# EUROPEAN PATENT SPECIFICATION

(11) **EP 2 857 509 B1**
(45) Date of publication and mention of the grant of the patent: **09.01.2019**
(21) Application number: 13796298.1
(22) Date of filing: 12.03.2013
(51) Int. Cl.: C12P 5/00, C08F 36/08, C12N 9/88

(54) **ISOPRENE SYNTHASE AND POLYNUCLEOTIDE ENCODING SAME, AND METHOD FOR PRODUCING ISOPRENE MONOMER**
ISOPRENSYNTHASE UND DAFÜR CODIERENDES POLYNUKLEOTID SOWIE VERFAHREN ZUR HERSTELLUNG VON ISOPRENMONOMER
ISOPRÈNE SYNTHASE ET POLYNUCLÉOTIDE CODANT POUR CELLE-CI, ET PROCÉDÉ DE PRODUCTION DU MONOMÈRE ISOPRÈNE

(30) Priority: 30.05.2012 JP 2012123724; 30.05.2012 JP 2012123728
(43) Date of publication of application: 08.04.2015
(73) Proprietor: Bridgestone Corporation, Tokyo 104-8340 (JP); Ajinomoto Co., Inc., Tokyo 104-8315 (JP)
(72) Inventor: HAYASHI, Yasuyuki, Kodaira-shi Tokyo 187-8531 (JP); HARADA, Minako, Kodaira-shi Tokyo 187-8531 (JP); TAKAOKA, Saaya, Kodaira-shi Tokyo 187-8531 (JP); FUKUSHIMA, Yasuo, Kodaira-shi Tokyo 187-8531 (JP); YOKOYAMA, Keiichi, Kawasaki-shi Kanagawa 210-8681 (JP); NISHIO, Yosuke, Kawasaki-shi Kanagawa 210-8681 (JP); TAJIMA, Yoshinori, Kawasaki-shi Kanagawa 210-8681 (JP); MIHARA, Yoko, Kawasaki-shi Kanagawa 210-8681 (JP); NAKATA, Kunio, Kawasaki-shi Kanagawa 210-8681 (JP)
(74) Representative: Grünecker Patent- und Rechtsanwälte PartG mbB
(86) International application number: PCT/JP2013/056866
(87) International publication number: WO 2013/179722

(56) References cited:
- WO-A1-2013/166320
- WO-A2-2009/100231
- JP-A- 2011 505 841
- JP-A- 2011 518 564
- RUSSELL K MONSON ET AL: "Relationships among Isoprene Emission Rate, Photosynthesis, and Isoprene Synthase Activity as Influenced by Temperature", PLANT PHYSIOL, vol. 98, no. 3, 1 January 1992 (1992-01-01), pages 1175-1180, XP055180049,
- JENNIFER KUZMA ET AL: "Leaf lsoprene Emission Rate 1s Dependent on Leaf Development and the Leve1 of lsoprene Synthase'", PLANT PHYSIOL, vol. 101, no. 2, 1 January 1993 (1993-01-01), pages 435-440, XP055180041,
- KUZMA J ET AL.: 'Leaf Isoprene Emission Rate Is Dependent on Leaf Development and the Level of Isoprene Synthase' PLANT PHYSIOL. vol. 101, no. 2, 1993, pages 435 - 440, XP055180041
- MONSON RK ET AL.: 'Relationships among Isoprene Emission Rate, Photosynthesis, and Isoprene Synthase Activity as Influenced by Temperature' PLANT PHYSIOL. vol. 98, no. 3, 1992, pages 1175 - 1180, XP055180049
- ZHAO Y ET AL.: 'Biosynthesis of isoprene in Escherichia coli via methylerythritol phosphate (MEP) pathway' APPL. MICROBIOL. BIOTECHNOL. vol. 90, no. 6, June 2011, pages 1915 - 1922, XP019908970

### SEQUENCE LISTING

<110> BRIDGESTONE CORPORATION
   Ajinomoto Co., Inc.
<120> Isoprene synthase and gene encoding the same, and method for producing isoprene monomer
<130> EP97367JHSZ298pau
<140> EP13796298.1
   <141> 2013-03-12
<150> PCT/JP2013/056866
   <151> 2013-03-12
<150> JP 2012-123728
   <151> 2012-05-30
<150> JP 2012-123724
   <151> 2012-05-30
<160> 98
<170> PatentIn version 3.5
<210> 1
   <211> 1785
   <212> DNA
   <213> Mucuna pruriens
<220>
   <221> CDS
   <222> (1)..(1785)
<400> 1
   atg gca acc aac cct tca tgc tta tct act cca ttt ttg tcc tcc aca       48
   Met Ala Thr Asn Pro Ser Cys Leu Ser Thr Pro Phe Leu Ser Ser Thr
   1               5                   10                  15
   cca gca cta agt act aga ttt cca tta agt gag aac ttc aca caa aaa       96
   Pro Ala Leu Ser Thr Arg Phe Pro Leu Ser Glu Asn Phe Thr Gln Lys
               20                  25                  30
   aca tct ctt gtc aat ccc aaa cct tgg cca ctt att tct gca gtc agc      144
   Thr Ser Leu Val Asn Pro Lys Pro Trp Pro Leu Ile Ser Ala Val Ser
           35                  40                  45
   tct caa ttt agc caa ata gca gaa gat aat agt cgt cgt tca gct aat      192
   Ser Gln Phe Ser Gln Ile Ala Glu Asp Asn Ser Arg Arg Ser Ala Asn
       50                  55                  60
   tac cac cca aac ctc tgg gat ttt gaa ttt ctg cag tct ctc gaa aat      240
   Tyr His Pro Asn Leu Trp Asp Phe Glu Phe Leu Gln Ser Leu Glu Asn
   65                  70                  75                  80
   gac tct aag atg gaa aag ctg gaa gag aaa gca aca aag ttg gag gag      288
   Asp Ser Lys Met Glu Lys Leu Glu Glu Lys Ala Thr Lys Leu Glu Glu
                   85                  90                  95
   gaa gtg cga aac atg atg aac gaa gca aag aca gaa gca cta agc tta      336
   Glu Val Arg Asn Met Met Asn Glu Ala Lys Thr Glu Ala Leu Ser Leu
               100                 105                 110
   ttg gaa ttg ata gac gac gtc cag cgt ctg gga ttg acc tac aag ttt        384
   Leu Glu Leu Ile Asp Asp Val Gln Arg Leu Gly Leu Thr Tyr Lys Phe
           115                 120                 125
   gag aag gac ata atc aaa gcc ctt gag aag att gtt cca ttg gat gag        432
   Glu Lys Asp Ile Ile Lys Ala Leu Glu Lys Ile Val Pro Leu Asp Glu
       130                 135                 140
   agt ggg ctg cat gtt act tct ctc agc ttc cgt ata ctt aga caa cat        480
   Ser Gly Leu His Val Thr Ser Leu Ser Phe Arg Ile Leu Arg Gln His
   145                 150                 155                 160
   ggc ttt gag gtt tcc caa gat gtg ttt aag aga ttt aag gac aag gag        528
   Gly Phe Glu Val Ser Gln Asp Val Phe Lys Arg Phe Lys Asp Lys Glu
                   165                 170                 175
   gga ggt ttt tgt gct gaa ctt aaa gac gat gtt caa ggg ttg cta agt        576
   Gly Gly Phe Cys Ala Glu Leu Lys Asp Asp Val Gln Gly Leu Leu Ser
               180                 185                 190
   cta tat gaa gca tcc tat ctt ggt ttt gag gga gaa agt ctc tta gac        624
   Leu Tyr Glu Ala Ser Tyr Leu Gly Phe Glu Gly Glu Ser Leu Leu Asp
           195                 200                 205
   gag gca agg gca ttt tca ata aca cat ctc aag aac aac cta aac aaa        672
   Glu Ala Arg Ala Phe Ser Ile Thr His Leu Lys Asn Asn Leu Asn Lys
       210                 215                 220
   gga ata aac acc aaa gta gcc caa caa gtt agc cat gca ctg gaa ctt        720
   Gly Ile Asn Thr Lys Val Ala Gln Gln Val Ser His Ala Leu Glu Leu
   225                 230                 235                 240
   cct tat cat cga aga ctg cat aga ctg gaa gca cga tgg ctc ctt gac        768
   Pro Tyr His Arg Arg Leu His Arg Leu Glu Ala Arg Trp Leu Leu Asp
                   245                 250                 255
   aaa tat gaa cca aag gaa ccc cac cat cat tta cta cac gag ctt gca        816
   Lys Tyr Glu Pro Lys Glu Pro His His His Leu Leu His Glu Leu Ala
               260                 265                 270
   aag ttg gat ttc aat ttg gtc caa tca ttg tac cag aaa gag ttg cga        864
   Lys Leu Asp Phe Asn Leu Val Gln Ser Leu Tyr Gln Lys Glu Leu Arg
           275                 280                 285
   gaa ttg tca ctg tgg tgg agg gag att ggg ctc aca agc aag ttg gac        912
   Glu Leu Ser Leu Trp Trp Arg Glu Ile Gly Leu Thr Ser Lys Leu Asp
       290                 295                 300
   ttt gtt cga gac aga tta atg gaa gtg tac ttt tgg gcg ctg gga atg        960
   Phe Val Arg Asp Arg Leu Met Glu Val Tyr Phe Trp Ala Leu Gly Met
   305                 310                 315                 320
   gca cct gat cct caa ttt agt gaa tgt cgt aaa gtc gtc act aaa atg       1008
   Ala Pro Asp Pro Gln Phe Ser Glu Cys Arg Lys Val Val Thr Lys Met
                   325                 330                 335
   ttt ggg cta gtt act atc atc gat gat gta tat gac gtt tac ggt act       1056
   Phe Gly Leu Val Thr Ile Ile Asp Asp Val Tyr Asp Val Tyr Gly Thr
               340                 345                 350
   ttg gac gag cta caa ctc ttc acc gat gct gtt gag aga tgg gac gtg       1104
   Leu Asp Glu Leu Gln Leu Phe Thr Asp Ala Val Glu Arg Trp Asp Val
           355                 360                 365
   aat gcg ata aat aca ctt cca gac tat atg aaa ttg tgc tat tta gcc      1152
   Asn Ala Ile Asn Thr Leu Pro Asp Tyr Met Lys Leu Cys Tyr Leu Ala
       370                 375                 380
   ctt tat aac acc gtc aat gac aca gct tat agc atc ctt aaa gaa aag      1200
   Leu Tyr Asn Thr Val Asn Asp Thr Ala Tyr Ser Ile Leu Lys Glu Lys
   385                 390                 395                 400
   gga cat aac aac att tct tat ttg aca aaa tct tgg tgt gag ttg tgc      1248
   Gly His Asn Asn Ile Ser Tyr Leu Thr Lys Ser Trp Cys Glu Leu Cys
                   405                 410                 415
   aaa gca ttc ctc caa gaa gca aaa tgg tca aac aac aaa atc att cca      1296
   Lys Ala Phe Leu Gln Glu Ala Lys Trp Ser Asn Asn Lys Ile Ile Pro
               420                 425                 430
   gca ttc aac aag tac cta gac aat gca tcg gtg tcc tcc tct ggt gtg      1344
   Ala Phe Asn Lys Tyr Leu Asp Asn Ala Ser Val Ser Ser Ser Gly Val
           435                 440                 445
   gct ttg ctt gct cct tcc tac ttc tta gtg tgc caa gaa caa gac att      1392
   Ala Leu Leu Ala Pro Ser Tyr Phe Leu Val Cys Gln Glu Gln Asp Ile
       450                 455                 460
   tca gac caa gct ctt cat tcc tta act aat ttc cat ggc ctt gtg cgt      1440
   Ser Asp Gln Ala Leu His Ser Leu Thr Asn Phe His Gly Leu Val Arg
   465                 470                 475                 480
   tca tca tgc acc att ttt agg ctt tgc aat gat ctg gct acc tca tcg      1488
   Ser Ser Cys Thr Ile Phe Arg Leu Cys Asn Asp Leu Ala Thr Ser Ser
                   485                 490                 495
   gct gag cta gag aga ggt gaa aca aca aat tca atc aca tcg tac atg      1536
   Ala Glu Leu Glu Arg Gly Glu Thr Thr Asn Ser Ile Thr Ser Tyr Met
               500                 505                 510
   cat gag aat gag act tct gag gag caa gca tgt aag gag ttg aga aat      1584
   His Glu Asn Glu Thr Ser Glu Glu Gln Ala Cys Lys Glu Leu Arg Asn
           515                 520                 525
   ttg atc gat gca gag tgg aag aag atg aat gaa gag cga gtt tca aat      1632
   Leu Ile Asp Ala Glu Trp Lys Lys Met Asn Glu Glu Arg Val Ser Asn
       530                 535                 540
   tct aca ctc cca aaa gca ttt agg gaa ata gct att aac atg gct cgg      1680
   Ser Thr Leu Pro Lys Ala Phe Arg Glu Ile Ala Ile Asn Met Ala Arg
   545                 550                 555                 560
   att tcc cat tgc aca tac caa tat gga gac gga ctt gga agg ccc gac      1728
   Ile Ser His Cys Thr Tyr Gln Tyr Gly Asp Gly Leu Gly Arg Pro Asp
                   565                 570                 575
   tac acc aca gag aac agg ata aag ttg cta cta ata gac cct ttt cca      1776
   Tyr Thr Thr Glu Asn Arg Ile Lys Leu Leu Leu Ile Asp Pro Phe Pro
               580                 585                 590
   att aat tag                                                          1785
   Ile Asn
<210> 2
   <211> 594
   <212> PRT
   <213> Mucuna pruriens
<400> 2
   Met Ala Thr Asn Pro Ser Cys Leu Ser Thr Pro Phe Leu Ser Ser Thr
   1               5                   10                  15
   Pro Ala Leu Ser Thr Arg Phe Pro Leu Ser Glu Asn Phe Thr Gln Lys
               20                  25                  30
   Thr Ser Leu Val Asn Pro Lys Pro Trp Pro Leu Ile Ser Ala Val Ser
           35                  40                  45
   Ser Gln Phe Ser Gln Ile Ala Glu Asp Asn Ser Arg Arg Ser Ala Asn
       50                  55                  60
   Tyr His Pro Asn Leu Trp Asp Phe Glu Phe Leu Gln Ser Leu Glu Asn
   65                  70                  75                  80
   Asp Ser Lys Met Glu Lys Leu Glu Glu Lys Ala Thr Lys Leu Glu Glu
                   85                  90                  95
   Glu Val Arg Asn Met Met Asn Glu Ala Lys Thr Glu Ala Leu Ser Leu
               100                 105                 110
   Leu Glu Leu Ile Asp Asp Val Gln Arg Leu Gly Leu Thr Tyr Lys Phe
           115                 120                 125
   Glu Lys Asp Ile Ile Lys Ala Leu Glu Lys Ile Val Pro Leu Asp Glu
       130                 135                 140
   Ser Gly Leu His Val Thr Ser Leu Ser Phe Arg Ile Leu Arg Gln His
   145                 150                 155                 160
   Gly Phe Glu Val Ser Gln Asp Val Phe Lys Arg Phe Lys Asp Lys Glu
                   165                 170                 175
   Gly Gly Phe Cys Ala Glu Leu Lys Asp Asp Val Gln Gly Leu Leu Ser
               180                 185                 190
   Leu Tyr Glu Ala Ser Tyr Leu Gly Phe Glu Gly Glu Ser Leu Leu Asp
           195                 200                 205
   Glu Ala Arg Ala Phe Ser Ile Thr His Leu Lys Asn Asn Leu Asn Lys
       210                 215                 220
   Gly Ile Asn Thr Lys Val Ala Gln Gln Val Ser His Ala Leu Glu Leu
   225                 230                 235                 240
   Pro Tyr His Arg Arg Leu His Arg Leu Glu Ala Arg Trp Leu Leu Asp
                   245                 250                 255
   Lys Tyr Glu Pro Lys Glu Pro His His His Leu Leu His Glu Leu Ala
               260                 265                 270
   Lys Leu Asp Phe Asn Leu Val Gln Ser Leu Tyr Gln Lys Glu Leu Arg
           275                 280                 285
   Glu Leu Ser Leu Trp Trp Arg Glu Ile Gly Leu Thr Ser Lys Leu Asp
       290                 295                 300
   Phe Val Arg Asp Arg Leu Met Glu Val Tyr Phe Trp Ala Leu Gly Met
   305                 310                 315                 320
   Ala Pro Asp Pro Gln Phe Ser Glu Cys Arg Lys Val Val Thr Lys Met
                   325                 330                 335
   Phe Gly Leu Val Thr Ile Ile Asp Asp Val Tyr Asp Val Tyr Gly Thr
               340                 345                 350
   Leu Asp Glu Leu Gln Leu Phe Thr Asp Ala Val Glu Arg Trp Asp Val
           355                 360                 365
   Asn Ala Ile Asn Thr Leu Pro Asp Tyr Met Lys Leu Cys Tyr Leu Ala
       370                 375                 380
   Leu Tyr Asn Thr Val Asn Asp Thr Ala Tyr Ser Ile Leu Lys Glu Lys
   385                 390                 395                 400
   Gly His Asn Asn Ile Ser Tyr Leu Thr Lys Ser Trp Cys Glu Leu Cys
                   405                 410                 415
   Lys Ala Phe Leu Gln Glu Ala Lys Trp Ser Asn Asn Lys Ile Ile Pro
               420                 425                 430
   Ala Phe Asn Lys Tyr Leu Asp Asn Ala Ser Val Ser Ser Ser Gly Val
           435                 440                 445
   Ala Leu Leu Ala Pro Ser Tyr Phe Leu Val Cys Gln Glu Gln Asp Ile
       450                 455                 460
   Ser Asp Gln Ala Leu His Ser Leu Thr Asn Phe His Gly Leu Val Arg
   465                 470                 475                 480
   Ser Ser Cys Thr Ile Phe Arg Leu Cys Asn Asp Leu Ala Thr Ser Ser
                   485                 490                 495
   Ala Glu Leu Glu Arg Gly Glu Thr Thr Asn Ser Ile Thr Ser Tyr Met
               500                 505                 510
   His Glu Asn Glu Thr Ser Glu Glu Gln Ala Cys Lys Glu Leu Arg Asn
           515                 520                 525
   Leu Ile Asp Ala Glu Trp Lys Lys Met Asn Glu Glu Arg Val Ser Asn
       530                 535                 540
   Ser Thr Leu Pro Lys Ala Phe Arg Glu Ile Ala Ile Asn Met Ala Arg
   545                 550                 555                 560
   Ile Ser His Cys Thr Tyr Gln Tyr Gly Asp Gly Leu Gly Arg Pro Asp
                   565                 570                 575
   Tyr Thr Thr Glu Asn Arg Ile Lys Leu Leu Leu Ile Asp Pro Phe Pro
               580                 585                 590
   Ile Asn
<210> 3
   <211> 608
   <212> PRT
   <213> Pueraria montana
<400> 3
   Met Ala Thr Asn Leu Leu Cys Leu Ser Asn Lys Leu Ser Ser Pro Thr
   1               5                   10                  15
   Pro Thr Pro Ser Thr Arg Phe Pro Gln Ser Lys Asn Phe Ile Thr Gln
               20                  25                  30
   Lys Thr Ser Leu Ala Asn Pro Lys Pro Trp Arg Val Ile Cys Ala Thr
           35                  40                  45
   Ser Ser Gln Phe Thr Gln Ile Thr Glu His Asn Ser Arg Arg Ser Ala
       50                  55                  60
   Asn Tyr Gln Pro Asn Leu Trp Asn Phe Glu Phe Leu Gln Ser Leu Glu
   65                  70                  75                  80
   Asn Asp Leu Lys Val Glu Lys Leu Glu Glu Lys Ala Thr Lys Leu Glu
                   85                  90                  95
   Glu Glu Val Arg Cys Met Ile Asn Arg Val Asp Thr Gln Pro Leu Ser
   100 105 110
   Leu Leu Glu Leu Ile Asp Asp Val Gln Arg Leu Gly Leu Thr Tyr Lys
   115 120 125
   Phe Glu Lys Asp Ile Ile Lys Ala Leu Glu Asn Ile Val Leu Leu Asp
   130 135 140
   Glu Asn Lys Lys Asn Lys Ser Asp Leu His Ala Thr Ala Leu Ser Phe
   145 150 155 160
   Arg Leu Leu Arg Gln His Gly Phe Glu Val Ser Gln Asp Val Phe Glu
   165 170 175
   Arg Phe Lys Asp Lys Glu Gly Gly Phe Ser Gly Glu Leu Lys Gly Asp
   180 185 190
   Val Gln Gly Leu Leu Ser Leu Tyr Glu Ala Ser Tyr Leu Gly Phe Glu
   195 200 205
   Gly Glu Asn Leu Leu Glu Glu Ala Arg Thr Phe Ser Ile Thr His Leu
   210 215 220
   Lys Asn Asn Leu Lys Glu Gly Ile Asn Thr Lys Val Ala Glu Gln Val
   225 230 235 240
   Ser His Ala Leu Glu Leu Pro Tyr His Gln Arg Leu His Arg Leu Glu
   245 250 255
   Ala Arg Trp Phe Leu Asp Lys Tyr Glu Pro Lys Glu Pro His His Gln
   260 265 270
   Leu Leu Leu Glu Leu Ala Lys Leu Asp Phe Asn Met Val Gln Thr Leu
   275 280 285
   His Gln Lys Glu Leu Gln Asp Leu Ser Arg Trp Trp Thr Glu Met Gly
   290 295 300
   Leu Ala Ser Lys Leu Asp Phe Val Arg Asp Arg Leu Met Glu Val Tyr
   305 310 315 320
   Phe Trp Ala Leu Gly Met Ala Pro Asp Pro Gln Phe Gly Glu Cys Arg
   325 330 335
   Lys Ala Val Thr Lys Met Phe Gly Leu Val Thr Ile Ile Asp Asp Val
   340 345 350
   Tyr Asp Val Tyr Gly Thr Leu Asp Glu Leu Gln Leu Phe Thr Asp Ala
   355 360 365
   Val Glu Arg Trp Asp Val Asn Ala Ile Asn Thr Leu Pro Asp Tyr Met
   370 375 380
   Lys Leu Cys Phe Leu Ala Leu Tyr Asn Thr Val Asn Asp Thr Ser Tyr
   385 390 395 400
   Ser Ile Leu Lys Glu Lys Gly His Asn Asn Leu Ser Tyr Leu Thr Lys
   405 410 415
   Ser Trp Arg Glu Leu Cys Lys Ala Phe Leu Gln Glu Ala Lys Trp Ser
   420 425 430
   Asn Asn Lys Ile Ile Pro Ala Phe Ser Lys Tyr Leu Glu Asn Ala Ser
   435 440 445
   Val Ser Ser Ser Gly Val Ala Leu Leu Ala Pro Ser Tyr Phe Ser Val
   450 455 460
   Cys Gln Gln Gln Glu Asp Ile Ser Asp His Ala Leu Arg Ser Leu Thr
   465 470 475 480
   Asp Phe His Gly Leu Val Arg Ser Ser Cys Val Ile Phe Arg Leu Cys
   485 490 495
   Asn Asp Leu Ala Thr Ser Ala Ala Glu Leu Glu Arg Gly Glu Thr Thr
   500 505 510
   Asn Ser Ile Ile Ser Tyr Met His Glu Asn Asp Gly Thr Ser Glu Glu
   515 520 525
   Gln Ala Arg Glu Glu Leu Arg Lys Leu Ile Asp Ala Glu Trp Lys Lys
   530 535 540
   Met Asn Arg Glu Arg Val Ser Asp Ser Thr Leu Leu Pro Lys Ala Phe
   545 550 555 560
   Met Glu Ile Ala Val Asn Met Ala Arg Val Ser His Cys Thr Tyr Gln
   565 570 575
   Tyr Gly Asp Gly Leu Gly Arg Pro Asp Tyr Ala Thr Glu Asn Arg Ile
   580 585 590
   Lys Leu Leu Leu Ile Asp Pro Phe Pro Ile Asn Gln Leu Met Tyr Val
   595 600 605
<210> 4
   <211> 1827
   <212> DNA
   <213> Pueraria montana
<400> 4
   atggcaacca accttttatg cttgtctaat aaattatcgt cccccacacc aacaccaagt 60
   actagatttc cacaaagtaa gaacttcatc acacaaaaaa catctcttgc caatcccaaa 120
   ccttggcgag ttatttgtgc tacgagctct caatttaccc aaataacaga acataatagt 180
   cggcgttcag ctaattacca gccaaacctc tggaattttg aatttctgca gtctctggaa 240
   aatgacctta aggtggaaaa actagaagag aaggcaacaa agctagagga ggaggtacga 300
   tgcatgatca acagagtaga cacacaacca ttaagcttac tagaattgat cgacgatgtc 360
   cagcgtctag gattgaccta caagtttgag aaggacataa tcaaagccct tgagaatatt 420
   gttttgctgg atgagaataa gaaaaataaa agtgacctcc atgctactgc tctcagcttc 480
   cgtttactta gacaacatgg ctttgaggtt tcccaagatg tgtttgagag atttaaggac 540
   aaggagggag gtttcagtgg tgaacttaaa ggtgatgtgc aagggttgct gagtctatat 600
   gaagcatcct atcttggctt tgagggagaa aatctcttgg aggaggcaag gacattttca 660
   ataacacatc tcaagaacaa cctaaaagaa ggaataaaca ccaaagtggc agaacaagtt 720
   agtcatgcac tggaacttcc ctatcatcaa agattgcata gactagaagc acgatggttc 780
   cttgacaaat atgaaccaaa ggaaccccac catcagttac tactcgagct tgcaaagcta 840
   gatttcaata tggtgcaaac attgcaccag aaagaactgc aagacctgtc aaggtggtgg 900
   acggagatgg ggctagcaag caagctagac tttgtccgag acagattaat ggaagtgtat 960
   ttttgggcgt tgggaatggc acctgatcct caattcggtg aatgtcgtaa agctgtcact 1020
   aaaatgtttg gattggtcac catcatcgat gatgtatatg acgtttatgg tactttggat 1080
   gagctacaac tcttcactga tgctgttgag agatgggacg tgaatgccat aaacacactt 1140
   ccagactaca tgaagttgtg cttcctagca ctttataaca ccgtcaatga cacgtcttat 1200
   agcatcctta aagaaaaagg acacaacaac ctttcctatt tgacaaaatc ttggcgtgag 1260
   ttatgcaaag cattccttca agaagcaaaa tggtcgaaca acaaaatcat tccagcattt 1320
   agcaagtacc tggaaaatgc atcggtgtcc tcctccggtg tggctttgct tgctccttcc 1380
   tacttctcag tgtgccaaca acaagaagat atctcagacc atgctcttcg ttctttaact 1440
   gatttccatg gccttgtgcg ctcctcatgc gtcattttca gactctgcaa tgatttggct 1500
   acctcagcgg ctgagctaga gaggggtgag acgacaaatt caataatatc ttatatgcat 1560
   gagaatgacg gcacttctga agagcaagca cgtgaggagt tgagaaaatt gatcgatgca 1620
   gagtggaaga agatgaaccg agagcgagtt tcagattcta cactactccc aaaagctttt 1680
   atggaaatag ctgttaacat ggctcgagtt tcgcattgca cataccaata tggagacgga 1740
   cttggaaggc cagactacgc cacagagaat agaatcaagt tgctacttat agaccccttt 1800
   ccaatcaatc aactaatgta cgtgtaa 1827
<210> 5
   <211> 1695
   <212> DNA
   <213> Artificial Sequence
<220>
   <223> Polynucleotide having modified codons, which encodes isoprene synthase derived from Pueraria montana (IspSK gene)
<400> 5
   atgtgtgcga cctcttctca atttactcag attaccgagc ataattcccg tcgttccgca 60
   aactatcagc caaacctgtg gaatttcgaa ttcctgcaat ccctggagaa cgacctgaaa 120
   gtggaaaagc tggaggagaa agcgaccaaa ctggaggaag aagttcgctg catgatcaac 180
   cgtgtagaca cccagccgct gtccctgctg gagctgatcg acgatgtgca gcgcctgggt 240
   ctgacctaca aatttgaaaa agacatcatt aaagccctgg aaaacatcgt actgctggac 300
   gaaaacaaaa agaacaaatc tgacctgcac gcaaccgctc tgtctttccg tctgctgcgt 360
   cagcacggtt tcgaggtttc tcaggatgtt tttgagcgtt tcaaggataa agaaggtggt 420
   ttcagcggtg aactgaaagg tgacgtccaa ggcctgctga gcctgtatga agcgtcttac 480
   ctgggtttcg agggtgagaa cctgctggag gaggcgcgta ccttttccat cacccacctg 540
   aagaacaacc tgaaagaagg cattaatacc aaggttgcag aacaagtgag ccacgccctg 600
   gaactgccat atcaccagcg tctgcaccgt ctggaggcac gttggttcct ggataaatac 660
   gaaccgaaag aaccgcatca ccagctgctg ctggagctgg cgaagctgga ttttaacatg 720
   gtacagaccc tgcaccagaa agagctgcaa gatctgtccc gctggtggac cgagatgggc 780
   ctggctagca aactggattt tgtacgcgac cgcctgatgg aagtttattt ctgggcactg 840
   ggtatggcgc cagacccgca gtttggtgaa tgtcgcaaag ctgttactaa aatgtttggt 900
   ctggtgacga tcatcgatga cgtgtatgac gtttatggca ctctggacga actgcaactg 960
   ttcaccgatg ctgtagagcg ctgggacgtt aacgctatta acaccctgcc ggactatatg 1020
   aaactgtgtt tcctggcact gtacaacacc gttaacgaca cgtcctattc tattctgaaa 1080
   gagaaaggtc ataacaacct gtcctatctg acgaaaagct ggcgtgaact gtgcaaagcc 1140
   tttctgcaag aggcgaaatg gtccaacaac aaaattatcc cggctttctc caagtacctg 1200
   gaaaacgcca gcgtttcctc ctccggtgta gcgctgctgg cgccgtctta cttttccgta 1260
   tgccagcagc aggaagacat ctccgaccac gcgctgcgtt ccctgaccga cttccatggt 1320
   ctggtgcgtt ctagctgcgt tatcttccgc ctgtgcaacg atctggccac ctctgcggcg   1380
   gagctggaac gtggcgagac taccaattct atcattagct acatgcacga aaacgatggt   1440
   accagcgagg aacaggcccg cgaagaactg cgtaaactga tcgacgccga atggaaaaag   1500
   atgaatcgtg aacgcgttag cgactccacc ctgctgccta aagcgttcat ggaaatcgca   1560
   gttaacatgg cacgtgtttc ccactgcacc taccagtatg gcgatggtct gggtcgccca   1620
   gactacgcga ctgaaaaccg catcaaactg ctgctgattg accctttccc gattaaccag   1680
   ctgatgtatg tctaa                                                      1695
<210> 6
   <211> 595
   <212> PRT
   <213> Populus alba x Populus tremula
<400> 6
   Met Ala Thr Glu Leu Leu Cys Leu His Arg Pro Ile Ser Leu Thr His
   1               5                   10                  15
   Lys Leu Phe Arg Asn Pro Leu Pro Lys Val Ile Gln Ala Thr Pro Leu
               20                  25                  30
   Thr Leu Lys Leu Arg Cys Ser Val Ser Thr Glu Asn Val Ser Phe Thr
           35                  40                  45
   Glu Thr Glu Thr Glu Ala Arg Arg Ser Ala Asn Tyr Glu Pro Asn Ser
       50                  55                  60
   Trp Asp Tyr Asp Phe Leu Leu Ser Ser Asp Thr Asp Glu Ser Ile Glu
   65                  70                  75                  80
   Val Tyr Lys Asp Lys Ala Lys Lys Leu Glu Ala Glu Val Arg Arg Glu
                   85                  90                  95
   Ile Asn Asn Glu Lys Ala Glu Phe Leu Thr Leu Leu Glu Leu Ile Asp
               100                 105                 110
   Asn Val Gln Arg Leu Gly Leu Gly Tyr Arg Phe Glu Ser Asp Ile Arg
           115                 120                 125
   Arg Ala Leu Asp Arg Phe Val Ser Ser Gly Gly Phe Asp Gly Val Thr
       130                 135                 140
   Lys Thr Ser Leu His Ala Thr Ala Leu Ser Phe Arg Leu Leu Arg Gln
   145                 150                 155                 160
   His Gly Phe Glu Val Ser Gln Glu Ala Phe Ser Gly Phe Lys Asp Gln
                   165                 170                 175
   Asn Gly Asn Phe Leu Glu Asn Leu Lys Glu Asp Thr Lys Ala Ile Leu
               180                 185                 190
   Ser Leu Tyr Glu Ala Ser Phe Leu Ala Leu Glu Gly Glu Asn Ile Leu
           195                 200                 205
   Asp Glu Ala Arg Val Phe Ala Ile Ser His Leu Lys Glu Leu Ser Glu
       210                 215                 220
   Glu Lys Ile Gly Lys Glu Leu Ala Glu Gln Val Asn His Ala Leu Glu
   225                 230                 235                 240
   Leu Pro Leu His Arg Arg Thr Gln Arg Leu Glu Ala Val Trp Ser Ile
                   245                 250                 255
   Glu Ala Tyr Arg Lys Lys Glu Asp Ala Asn Gln Val Leu Leu Glu Leu
               260                 265                 270
   Ala Ile Leu Asp Tyr Asn Met Ile Gln Ser Val Tyr Gln Arg Asp Leu
           275                 280                 285
   Arg Glu Thr Ser Arg Trp Trp Arg Arg Val Gly Leu Ala Thr Lys Leu
       290                 295                 300
   His Phe Ala Lys Asp Arg Leu Ile Glu Ser Phe Tyr Trp Ala Val Gly
   305                 310                 315                 320
   Val Ala Phe Glu Pro Gln Tyr Ser Asp Cys Arg Asn Ser Val Ala Lys
                   325                 330                 335
   Met Phe Ser Phe Val Thr Ile Ile Asp Asp Ile Tyr Asp Val Tyr Gly
               340                 345                 350
   Thr Leu Asp Glu Leu Glu Leu Phe Thr Asp Ala Val Glu Arg Trp Asp
           355                 360                 365
   Val Asn Ala Ile Asn Asp Leu Pro Asp Tyr Met Lys Leu Cys Phe Leu
       370                 375                 380
   Ala Leu Tyr Asn Thr Ile Asn Glu Ile Ala Tyr Asp Asn Leu Lys Asp
   385                 390                 395                 400
   Lys Gly Glu Asn Ile Leu Pro Tyr Leu Thr Lys Ala Trp Ala Asp Leu
                   405                 410                 415
   Cys Asn Ala Phe Leu Gln Glu Ala Lys Trp Leu Tyr Asn Lys Ser Thr
   420 425 430
   Pro Thr Phe Asp Asp Tyr Phe Gly Asn Ala Trp Lys Ser Ser Ser Gly
   435 440 445
   Pro Leu Gln Leu Ile Phe Ala Tyr Phe Ala Val Val Gln Asn Ile Lys
   450 455 460
   Lys Glu Glu Ile Glu Asn Leu Gln Lys Tyr His Asp Ile Ile Ser Arg
   465 470 475 480
   Pro Ser His Ile Phe Arg Leu Cys Asn Asp Leu Ala Ser Ala Ser Ala
   485 490 495
   Glu Ile Ala Arg Gly Glu Thr Ala Asn Ser Val Ser Cys Tyr Met Arg
   500 505 510
   Thr Lys Gly Ile Ser Glu Glu Leu Ala Thr Glu Ser Val Met Asn Leu
   515 520 525
   Ile Asp Glu Thr Cys Lys Lys Met Asn Lys Glu Lys Leu Gly Gly Ser
   530 535 540
   Leu Phe Ala Lys Pro Phe Val Glu Thr Ala Ile Asn Leu Ala Arg Gln
   545 550 555 560
   Ser His Cys Thr Tyr His Asn Gly Asp Ala His Thr Ser Pro Asp Glu
   565 570 575
   Leu Thr Arg Lys Arg Val Leu Ser Val Ile Thr Glu Pro Ile Leu Pro
   580 585 590
   Phe Glu Arg
   595
<210> 7
   <211> 1788
   <212> DNA
   <213> Populus alba x Populus tremula
<400> 7
   atggcaactg aattattgtg cttgcaccgt ccaatctcac tgacacacaa actgttcaga 60
   aatcccttac ctaaagtcat ccaggccact cccttaactt tgaaactcag atgttctgta 120
   agcacagaaa acgtcagctt cacagaaaca gaaacagaag ccagacggtc tgccaattat 180
   gaaccaaata gctgggatta tgattttttg ctgtcttcag acactgacga atcgattgaa 240
   gtatacaaag acaaggccaa aaagctggag gctgaggtga gaagagagat taacaatgaa    300
   aaggcagagt ttttgactct gcttgaactg atagataatg tccaaaggtt aggattgggt    360
   taccggttcg agagtgacat aaggagagcc ctcgacagat ttgtttcttc aggaggattt    420
   gatggtgtta caaaaactag ccttcatgct actgctctta gcttcaggct tctcagacag    480
   catggctttg aggtctctca agaagcgttc agtggattca aggatcaaaa tggcaatttc    540
   ttggaaaacc ttaaggagga caccaaggca atactaagcc tatatgaagc ttcatttctt    600
   gcattagaag gagaaaatat cttggatgag gccagggtgt ttgcaatatc acatctaaaa    660
   gagctcagcg aagaaaagat tggaaaagag ctggccgaac aggtgaatca tgcattggag    720
   cttccattgc atcgcaggac gcaaagacta gaagctgttt ggagtattga agcataccgt    780
   aaaaaggaag atgcaaatca agtactgcta gaacttgcta tattggacta caacatgatt    840
   caatcagtat accaaagaga tcttcgcgag acatcaaggt ggtggaggcg agtgggtctt    900
   gcaacaaagt tgcattttgc taaagacagg ttaattgaaa gcttttactg ggcagttgga    960
   gttgcgttcg aacctcaata cagtgattgc cgtaattcag tagcaaaaat gttttcattt   1020
   gtaacaatca ttgatgatat ctatgatgtt tatggtactc tggatgagct ggagctattt   1080
   acagatgctg ttgagagatg ggatgttaac gccatcaatg atcttccgga ttatatgaag   1140
   ctctgcttcc tagctctcta caacactatc aatgagatag cttatgacaa tctgaaggac   1200
   aagggggaaa acattcttcc atacctaaca aaagcgtggg cagatttatg caatgcattc   1260
   ctacaagaag caaaatggct gtacaataag tccacaccaa catttgatga ctatttcgga   1320
   aatgcatgga aatcatcctc agggcctctt caactaattt ttgcctactt tgccgtggtt   1380
   caaaacatca agaaagagga aattgaaaac ttacaaaagt atcatgatat catcagtagg   1440
   ccttcccaca tctttcgtct ttgcaacgac ctggcttcag catcggctga gatagcgaga   1500
   ggtgaaactg cgaattccgt atcctgctac atgcgtacaa aaggcatttc tgaggaactt   1560
   gctactgaat ccgtaatgaa tttgatcgac gaaacctgta aaaagatgaa caaagaaaag   1620
   cttggtggct ctttgtttgc aaaacctttt gtcgaaacag ctattaacct tgcacggcaa   1680
   tcccattgca cttatcataa cggagatgcg catacttcac cagacgagct aactaggaaa   1740
   cgtgtcctgt cagtaatcac agagcctatt ctaccctttg agagataa                1788
<210> 8
   <211> 1680
   <212> DNA
   <213> Artificial Sequence
<220>
   <223> Polynucleotide having modified codons, which encodes isoprene synthase derived from Populus alba x Populus tremula (IspSP gene)
<400> 8
   atgtgctctg tttctaccga gaacgtttcc ttcactgaga cggaaaccga ggcacgtcgt      60
   agcgcgaact acgagccgaa tagctgggac tacgatttcc tgctgtcttc cgatactgac     120
   gaatctattg aggtgtacaa agacaaagca aagaaactgg aggctgaagt gcgccgcgaa     180
   attaacaacg agaaagctga attcctgact ctgctggagc tgatcgataa cgtacagcgc     240
   ctgggtctgg gttaccgctt cgaatctgat atccgtcgcg cactggatcg tttcgtaagc     300
   agcggcggtt tcgatggcgt gaccaaaacg agcctgcacg ctaccgcgct gtccttccgt     360
   ctgctgcgtc agcacggctt cgaagtttct caggaagcat tctccggttt caaagatcaa     420
   aacggtaact tcctggaaaa cctgaaagaa gacactaagg cgatcctgag cctgtatgag     480
   gcaagctttc tggccctgga gggtgagaac atcctggatg aggcgcgcgt attcgccatc     540
   tcccatctga aagagctgtc tgaagagaaa atcggtaagg aactggcaga gcaggttaat     600
   cacgcactgg aactgccgct gcatcgtcgt acccagcgtc tggaggcggt ttggtccatc     660
   gaagcgtacc gcaaaaagga ggatgctaac caggttctgc tggaactggc catcctggac     720
   tacaacatga tccagtccgt ttaccagcgt gatctgcgtg aaacctcccg ttggtggcgc     780
   cgtgtgggcc tggcgaccaa actgcacttc gctaaggacc gcctgattga gtctttttac     840
   tgggcagtcg gcgttgcgtt cgaacctcag tattctgact gccgtaacag cgttgcgaaa     900
   atgttcagct tcgttactat tatcgacgac atctacgacg tttacggtac tctggacgag     960
   ctggaactgt ttaccgacgc tgtcgaacgt tgggatgtta acgccatcaa cgatctgcct    1020
   gactacatga aactgtgctt cctggcactg tataacacga tcaacgaaat tgcatacgac    1080
   aacctgaaag acaaaggtga aaacatcctg ccgtacctga ctaaagcgtg ggcggatctg    1140
   tgtaacgctt ttctgcaaga agcgaaatgg ctgtataaca aatccactcc gacctttgac    1200
   gattatttcg gcaatgcctg gaaatccagc tctggcccgc tgcaactgat cttcgcttat    1260
   tttgcggttg tccaaaacat caaaaaggag gaaattgaaa acctgcaaaa ataccacgat    1320
   atcattagcc gtccttctca tatctttcgc ctgtgcaacg acctggcaag cgcgtccgca    1380
   gagatcgcac gtggcgaaac cgctaactct gtttcctgct acatgcgcac caagggcatt    1440
   tccgaagagc tggcaaccga gagcgtaatg aatctgatcg acgaaacctg taagaaaatg    1500
   aacaaagaaa aactgggtgg ctccctgttc gctaaaccgt tcgtagagac tgctattaac    1560
   ctggcacgtc agagccactg cacctaccac aatggtgacg cacatactag cccggatgaa    1620
   ctgactcgta aacgtgtact gtctgttatc accgaaccga ttctgccgtt cgaacgttaa    1680
<210> 9
   <211> 1785
   <212> DNA
   <213> Artificial Sequence
<220>
   <223> Polynucleotide which encodes isoprene synthase derived from Mucuna pururiens and which is fused with chloroplast-localization signal (IspSM(L) gene)
<400> 9
   atggctacca acccgtcctg tctgtcaacc ccgttcctgt cttcaacccc ggctctgtcc 60
   acccgcttcc cgctgtccga aaacttcacc cagaaaacga gcctggttaa cccgaaaccg 120
   tggccgctga tttctgcggt cagctctcag tttagtcaaa tcgcggaaga taattctcgt 180
   cgcagtgcca actatcatcc gaatctgtgg gattttgaat tcctgcagtc gctggaaaac 240
   gacagcaaaa tggaaaaact ggaagaaaaa gcgaccaaac tggaagaaga agtgcgtaac 300
   atgatgaatg aagcgaaaac ggaagccctg tctctgctgg aactgattga tgacgttcaa 360
   cgcctgggtc tgacctacaa attcgaaaaa gatatcatca aagccctgga aaaaattgtc 420
   ccgctggacg aatcaggtct gcacgtgacc tccctgtcat ttcgtatcct gcgccagcat 480
   ggcttcgaag tttcgcaaga tgtctttaaa cgtttcaaag acaaagaagg cggtttctgc 540
   gcagaactga aagatgacgt gcagggtctg ctgtctctgt atgaagctag ttacctgggt 600
   tttgaaggcg aatccctgct ggatgaagcg cgcgccttct caattaccca cctgaaaaac 660
   aatctgaaca aaggcatcaa tacgaaagtg gcacagcaag ttagtcatgc tctggaactg 720
   ccgtatcacc gtcgcctgca tcgtctggaa gcccgctggc tgctggataa atacgaaccg 780
   aaagaaccgc atcaccatct gctgcacgaa ctggcaaaac tggactttaa tctggttcag 840
   tcgctgtatc aaaaagaact gcgtgaactg agcctgtggt ggcgcgaaat tggtctgacc 900
   tctaaactgg attttgtgcg tgaccgcctg atggaagttt acttctgggc actgggcatg 960
   gctccggatc cgcagtttag cgaatgccgt aaagtggtta ccaaaatgtt cggtctggtg 1020
   acgattatcg atgacgtcta tgatgtgtac ggcaccctgg acgaactgca actgttcacg 1080
   gatgcagtcg aacgctggga cgtgaacgct atcaataccc tgccggatta tatgaaactg 1140
   tgttatctgg cactgtacaa caccgttaat gacacggctt acagcatcct gaaagaaaaa 1200
   ggtcataaca acatctccta cctgaccaaa tcatggtgcg aactgtgtaa agcgtttctg 1260
   caggaagcca aatggtctaa caataaaatt atcccggcgt tcaacaaata tctggataat 1320
   gccagtgtta gttcctcagg cgtcgcactg ctggctccgt cctactttct ggtctgtcag 1380
   gaacaagata tttcggacca ggcactgcac agcctgacca actttcatgg tctggttcgt 1440
   tcgagctgca ccatcttccg cctgtgtaat gatctggcga cgtctagtgc cgaactggaa 1500
   cgtggcgaaa ccacgaactc cattacctca tatatgcacg aaaatgaaac gagtgaagaa 1560
   caggcgtgca aagaactgcg taacctgatc gatgccgaat ggaagaaaat gaacgaagaa 1620
   cgcgtgtcga atagcaccct gccgaaagcc tttcgtgaaa ttgcaatcaa tatggctcgc 1680
   atttcccatt gtacgtatca gtacggcgat ggtctgggcc gcccggacta cacgaccgaa 1740
   aaccgcatta aactgctgct gattgacccg ttcccgatta actga 1785
<210> 10
   <211> 1656
   <212> DNA
   <213> Artificial Sequence
<220>
   <223> Polynucleotide without chloroplast-localization signal, which encodes isoprene synthase derived from Mucuna pururiens (IspSM gene)
<400> 10
   atgtccgccg tttcaagcca gttctctcaa atcgccgaag acaatagccg tcgctcagca     60
   aattatcatc cgaatctgtg ggactttgaa tttctgcagt ctctggaaaa cgatagtaaa    120
   atggaaaaac tggaagaaaa agccaccaaa ctggaagaag aagtgcgtaa catgatgaat    180
   gaagcgaaaa cggaagccct gagcctgctg gaactgattg atgacgtcca acgcctgggt    240
   ctgacctaca aattcgaaaa agatatcatc aaagcactgg aaaaaattgt cccgctggac    300
   gaatcaggtc tgcacgtgac gtctctgagt tttcgtatcc tgcgccagca tggcttcgaa    360
   gtttcgcaag atgtctttaa acgtttcaaa gacaaagaag gcggtttctg cgcggaactg    420
   aaagatgacg tgcagggtct gctgtccctg tatgaagcct catacctggg ttttgaaggc    480
   gaatccctgc tggatgaagc gcgcgccttc tcaattaccc acctgaaaaa caatctgaac    540
   aaaggcatca atacgaaagt ggcacagcaa gttagccatg ctctggaact gccgtatcac    600
   cgtcgcctgc atcgtctgga agcacgctgg ctgctggata aatacgaacc gaaagaaccg    660
   catcaccatc tgctgcacga actggcgaaa ctggacttta atctggttca gtcgctgtat    720
   caaaaagaac tgcgtgaact gagcctgtgg tggcgcgaaa ttggtctgac ctctaaactg    780
   gattttgtgc gtgaccgcct gatggaagtt tacttctggg cactgggcat ggctccggat    840
   ccgcagttta gcgaatgccg taaagtggtt accaaaatgt tcggtctggt cacgattatc    900
   gatgacgtct atgatgtgta cggcaccctg gacgaactgc aactgttcac ggatgcggtc    960
   gaacgctggg acgtgaacgc catcaatacc ctgccggatt atatgaaact gtgttatctg   1020
   gcgctgtaca acaccgttaa tgacacggcc tatagcatcc tgaaagaaaa aggtcataac   1080
   aacatctcgt acctgaccaa aagctggtgc gaactgtgta aagcgtttct gcaggaagcc   1140
   aaatggtcta acaacaaaat catcccggca ttcaacaaat acctggataa tgctagtgtt   1200
   agctctagtg gcgtcgcact gctggctccg tcctactttc tggtgtgtca ggaacaagat   1260
   atttctgacc aggcgctgca cagtctgacc aactttcatg gtctggttcg ttcctcatgc   1320
   accatcttcc gcctgtgtaa tgatctggcg acgtcgagcg ccgaactgga acgtggcgaa   1380
   accacgaact cgattaccag ctatatgcac gaaaatgaaa cgagtgaaga acaggcatgc   1440
   aaagaactgc gtaacctgat cgatgctgaa tggaagaaaa tgaacgaaga acgcgtgtcc   1500
   aattcaaccc tgccgaaagc ctttcgtgaa attgcaatca atatggctcg catttcccat 1560
   tgtacgtatc agtacggcga tggtctgggc cgcccggact acacgaccga aaaccgtatt 1620
   aaactgctgc tgattgaccc gttcccgatt aactaa 1656
<210> 11
   <211> 399
   <212> DNA
   <213> Artificial Sequence
<220>
   <223> Ptac-Ttrp
<400> 11
   ggtaccagat ctccctgttg acaattaatc atcggctcta taatgtgtgg aatcgtgagc 60
   ggataacaat ttcacacaag gagactcccg ggagccgcca gttccgctgg cggcatttta 120
   actttcttta atgaagccgg aaaaatccta aattcattta atatttatct ttttaccgtt 180
   tcgcttaccc cggtcgaacg tcaacttacg tcatttttcc gcccaacagt aatataatca 240
   aacaaattaa tcccgcaaca taacaccagt aaaatcaata attttctcta agtcacttat 300
   tcctcaggta attgttaata tatccagaat gttcctcaaa atatattttc cctctatctt 360
   ctcgttgcgc ttaatttgac taattctcat tagggatcc 399
<210> 12
   <211> 3383
   <212> DNA
   <213> Artificial Sequence
<220>
   <223> Expression plasmid pSTV28-Ptac-Ttrp
<400> 12
   cgtatggcaa tgaaagacgg tgagctggtg atatgggata gtgttcaccc ttgttacacc     60
   gttttccatg agcaaactga aacgttttca tcgctctgga gtgaatacca cgacgatttc    120
   cggcagtttc tacacatata ttcgcaagat gtggcgtgtt acggtgaaaa cctggcctat    180
   ttccctaaag ggtttattga gaatatgttt ttcgtctcag ccaatccctg ggtgagtttc    240
   accagttttg atttaaacgt ggccaatatg gacaacttct tcgcccccgt tttcaccatg    300
   ggcaaatatt atacgcaagg cgacaaggtg ctgatgccgc tggcgattca ggttcatcat    360
   gccgtttgtg atggcttcca tgtcggcaga atgcttaatg aattacaaca gtactgcgat    420
   gagtggcagg gcggggcgta atttttttaa ggcagttatt ggtgccctta aacgcctggt    480
   gctacgcctg aataagtgat aataagcgga tgaatggcag aaattcgaaa gcaaattcga    540
   cccggtcgtc ggttcagggc agggtcgtta aatagccgct tatgtctatt gctggtttac    600
   cggtttattg actaccggaa gcagtgtgac cgtgtgcttc tcaaatgcct gaggccagtt    660
   tgctcaggct ctccccgtgg aggtaataat tgacgatatg atcatttatt ctgcctccca 720
   gagcctgata aaaacggtta gcgcttcgtt aatacagatg taggtgttcc acagggtagc 780
   cagcagcatc ctgcgatgca gatccggaac ataatggtgc agggcgcttg tttcggcgtg 840
   ggtatggtgg caggccccgt ggccggggga ctgttgggcg ctgccggcac ctgtcctacg 900
   agttgcatga taaagaagac agtcataagt gcggcgacga tagtcatgcc ccgcgcccac 960
   cggaaggagc taccggacag cggtgcggac tgttgtaact cagaataaga aatgaggccg 1020
   ctcatggcgt tccaatacgc aaaccgcctc tccccgcgcg ttggccgatt cattaatgca 1080
   gctggcacga caggtttccc gactggaaag cgggcagtga gcgcaacgca attaatgtga 1140
   gttagctcac tcattaggca ccccaggctt tacactttat gcttccggct cgtatgttgt 1200
   gtggaattgt gagcggataa caatttcaca caggaaacag ctatgaccat gattacgaat 1260
   tcgagctcgg taccagatct ccctgttgac aattaatcat cggctctata atgtgtggaa 1320
   tcgtgagcgg ataacaattt cacacaagga gactcccggg agccgccagt tccgctggcg 1380
   gcattttaac tttctttaat gaagccggaa aaatcctaaa ttcatttaat atttatcttt 1440
   ttaccgtttc gcttaccccg gtcgaacgtc aacttacgtc atttttccgc ccaacagtaa 1500
   tataatcaaa caaattaatc ccgcaacata acaccagtaa aatcaataat tttctctaag 1560
   tcacttattc ctcaggtaat tgttaatata tccagaatgt tcctcaaaat atattttccc 1620
   tctatcttct cgttgcgctt aatttgacta attctcatta gggatcctct agagtcgacc 1680
   tgcaggcatg caagcttggc actggccgtc gttttacaac gtcgtgactg ggaaaaccct 1740
   ggcgttaccc aacttaatcg ccttgcagca catccccctt tcgccagctg gcgtaatagc 1800
   gaagaggccc gcaccgatcg cccttcccaa cagttgcgca gcctgaatgg cgaatgagct 1860
   tatcgatgat aagctgtcaa acatgagaat tacaacttat atcgtatggg gctgacttca 1920
   ggtgctacat ttgaagagat aaattgcact gaaatctaga aatattttat ctgattaata 1980
   agatgatctt cttgagatcg ttttggtctg cgcgtaatct cttgctctga aaacgaaaaa 2040
   accgccttgc agggcggttt ttcgaaggtt ctctgagcta ccaactcttt gaaccgaggt 2100
   aactggcttg gaggagcgca gtcaccaaaa cttgtccttt cagtttagcc ttaaccggcg 2160
   catgacttca agactaactc ctctaaatca attaccagtg gctgctgcca gtggtgcttt 2220
   tgcatgtctt tccgggttgg actcaagacg atagttaccg gataaggcgc agcggtcgga 2280
   ctgaacgggg ggttcgtgca tacagtccag cttggagcga actgcctacc cggaactgag 2340
   tgtcaggcgt ggaatgagac aaacgcggcc ataacagcgg aatgacaccg gtaaaccgaa 2400
   aggcaggaac aggagagcgc acgagggagc cgccagggga aacgcctggt atctttatag 2460
   tcctgtcggg tttcgccacc actgatttga gcgtcagatt tcgtgatgct tgtcaggggg 2520
   gcggagccta tggaaaaacg gctttgccgc ggccctctca cttccctgtt aagtatcttc 2580
   ctggcatctt ccaggaaatc tccgccccgt tcgtaagcca tttccgctcg ccgcagtcga   2640
   acgaccgagc gtagcgagtc agtgagcgag gaagcggaat atatcctgta tcacatattc   2700
   tgctgacgca ccggtgcagc cttttttctc ctgccacatg aagcacttca ctgacaccct   2760
   catcagtgcc aacatagtaa gccagtatac actccgctag cgctgatgtc cggcggtgct   2820
   tttgccgtta cgcaccaccc cgtcagtagc tgaacaggag ggacagctga tagaaacaga   2880
   agccactgga gcacctcaaa aacaccatca tacactaaat cagtaagttg gcagcatcac   2940
   ccgacgcact ttgcgccgaa taaatacctg tgacggaaga tcacttcgca gaataaataa   3000
   atcctggtgt ccctgttgat accgggaagc cctgggccaa cttttggcga aaatgagacg   3060
   ttgatcggca cgtaagaggt tccaactttc accataatga aataagatca ctaccgggcg   3120
   tattttttga gttatcgaga ttttcaggag ctaaggaagc taaaatggag aaaaaaatca   3180
   ctggatatac caccgttgat atatcccaat ggcatcgtaa agaacatttt gaggcatttc   3240
   agtcagttgc tcaatgtacc tataaccaga ccgttcagct ggatattacg gcctttttaa   3300
   agaccgtaaa gaaaaataag cacaagtttt atccggcctt tattcacatt cttgcccgcc   3360
   tgatgaatgc tcatccggaa ttt                                           3383
<210> 13
   <211> 79
   <212> DNA
   <213> Artificial Sequence
<220>
   <223> Primer for amplifying IspSK gene (Ptac-IspS(K)F)
<400> 13
   gataacaatt tcacacaata attttgttta actttaagaa ggagatataa tgtgtgcgac     60
   ctcttctcaa tttactcag                                                  79
<210> 14
   <211> 42
   <212> DNA
   <213> Artificial Sequence
<220>
   <223> Primer for amplifying IspSK gene (IspS(K)R-MCSR)
<400> 14
   acggccagtg aattcttaga catacatcag ctggttaatc gg 42
<210> 15
   <211> 79
   <212> DNA
   <213> Artificial Sequence
<220>
   <223> Primer for amplifying IspSP gene (Ptac-IspS(P)F)
<400> 15
   gataacaatt tcacacaata attttgttta actttaagaa ggagatataa tgtgctctgt 60
   ttctaccgag aacgtttcc 79
<210> 16
   <211> 43
   <212> DNA
   <213> Artificial Sequence
<220>
   <223> Primer for amplifying IspSP gene (IspS(P)R-MCSR)
<400> 16
   acggccagtg aattcttaac gttcgaacgg cagaatcggt tcg 43
<210> 17
   <211> 69
   <212> DNA
   <213> Artificial Sequence
<220>
   <223> Primer for amplifying IspSM gene (Ptac-IspS(K)F)
<400> 17
   gataacaatt tcacacaata attttgttta actttaagaa ggagatataa tgtccgccgt 60
   ttcaagcca 69
<210> 18
   <211> 35
   <212> DNA
   <213> Artificial Sequence
<220>
   <223> Primer for amplifying IspSM gene
<400> 18
   acggccagtg aattcttagt taatcgggaa cgggt 35
<210> 19
   <211> 79
   <212> DNA
   <213> Artificial Sequence
<220>
   <223> Primer for amplifying IspSM(L) gene
<400> 19
   gataacaatt tcacacaata attttgttta actttaagaa ggagatataa tggctaccaa 60
   cccgtcctgt ctgtcaacc 79
<210> 20
   <211> 35
   <212> DNA
   <213> Artificial Sequence
<220>
   <223> Primer for amplifying IspSM(L) gene
<400> 20
   acggccagtg aattctcagt taatcgggaa cgggt 35
<210> 21
   <211> 29
   <212> DNA
   <213> Artificial Sequence
<220>
   <223> Primer for amplifying IpSTV28-Ptac-Ttrp construct (pSTV28-F)
<400> 21
   gtgtgaaatt gttatccgct cacaattcc 29
<210> 22
   <211> 27
   <212> DNA
   <213> Artificial Sequence
<220>
   <223> Primer for amplifying pSTV28-Ptac-Ttrp construct (pSTV28-R)
<400> 22
   gaattcactg gccgtcgttt tacaacg 27
<210> 23
   <211> 41
   <212> DNA
   <213> Artificial Sequence
<220>
   <223> Primer for amplifying dxs gene (dxs-F)
<400> 23
   caggaaacag ctatgagttt tgatattgcc aaatacccga c 41
<210> 24
   <211> 28
   <212> DNA
   <213> Artificial Sequence
<220>
   <223> Primer for amplifying dxs gene (dxs-R)
<400> 24
   gctgccactc ctgctatact cgtcatac 28
<210> 25
   <211> 30
   <212> DNA
   <213> Artificial Sequence
<220>
   <223> Primer for amplifying dxs gene (pMW219-F)
<400> 25
   catagctgtt tcctgtgtga aattgttatc 30
<210> 26
   <211> 40
   <212> DNA
   <213> Artificial Sequence
<220>
   <223> Primer for amplifying dxs gene (pMW219-R)
<400> 26
   agcaggagtg gcagcgaatt cgagctcggt acccggggat 40
<210> 27
   <211> 40
   <212> DNA
   <213> Artificial Sequence
<220>
   <223> Primer for amplifying ERG12 gene (MVK-IFS_5742-33-1)
<400> 27
   acacaaggag actcccatgt cattaccgtt cttaacttct 40
<210> 28
   <211> 46
   <212> DNA
   <213> Artificial Sequence
<220>
   <223> Primer for amplifying ERG12 gene (MVK-IFA_5742-33-2)
<400> 28
   ggaactggcg gctcccgggt tattatgaag tccatggtaa attcgt 46
<210> 29
   <211> 38
   <212> DNA
   <213> Artificial Sequence
<220>
   <223> Primer for amplifying ERG8 gene (PMK-IFS_5742-33-3)
<400> 29
   acacaaggag actcccatgt cagagttgag agccttca 38
<210> 30
   <211> 46
   <212> DNA
   <213> Artificial Sequence
<220>
   <223> Primer for amplifying ERG8 gene (PMK-IFA_5742-33-4)
<400> 30
   ggaactggcg gctcccgggt tattatttat caagataagt ttccgg 46
<210> 31
   <211> 37
   <212> DNA
   <213> Artificial Sequence
<220>
   <223> Primer for amplifying ERG19 gene (MVD-IFS_5742-33-5)
<400> 31
   acacaaggag actcccatga ccgtttacac agcatcc 37
<210> 32
   <211> 46
   <212> DNA
   <213> Artificial Sequence
<220>
   <223> Primer for amplifying ERG19 gene (MVD-IFA_5742-33-6)
<400> 32
   ggaactggcg gctcccgggt tattattcct ttggtagacc agtctt 46
<210> 33
   <211> 36
   <212> DNA
   <213> Artificial Sequence
<220>
   <223> Primer for amplifying IDI1 gene (yIDI-IFS_5742-33-7)
<400> 33
   acacaaggag actcccatgc cccatggtgc agtatc 36
<210> 34
   <211> 49
   <212> DNA
   <213> Artificial Sequence
<220>
   <223> Primer for amplifying IDI1 gene (yIDI-IFA_5742-33-8)
<400> 34
   ggaactggcg gctcccgggt tattatagca ttctatgaat ttgcctgtc 49
<210> 35
   <211> 5892
   <212> DNA
   <213> Artificial Sequence
<220>
   <223> Nucleotide sequence of prepared plasmid pUC-mvk-pmk
<400> 35
   agcgcccaat acgcaaaccg cctctccccg cgcgttggcc gattcattaa tgcagctggc     60
   acgacaggtt tcccgactgg aaagcgggca gtgagcgcaa cgcaattaat gtgagttagc    120
   tcactcatta ggcaccccag gctttacact ttatgcttcc ggctcgtatg ttgtgtggaa    180
   ttgtgagcgg ataacaattt cacacaggaa acagctatga ccatgattac gaattcgagc    240
   tcggtaccca tgtcattacc gttcttaact tctgcaccgg gaaaggttat tatttttggt    300
   gaacactctg ctgtgtacaa caagcctgcc gtcgctgcta gtgtgtctgc gttgagaacc    360
   tacctgctaa taagcgagtc atctgcacca gatactattg aattggactt cccggacatt    420
   agctttaatc ataagtggtc catcaatgat ttcaatgcca tcaccgagga tcaagtaaac    480
   tcccaaaaat tggccaaggc tcaacaagcc accgatggct tgtctcagga actcgttagt    540
   cttttggatc cgttgttagc tcaactatcc gaatccttcc actaccatgc agcgttttgt    600
   ttcctgtata tgtttgtttg cctatgcccc catgccaaga atattaagtt ttctttaaag    660
   tctactttac ccatcggtgc tgggttgggc tcaagcgcct ctatttctgt atcactggcc    720
   ttagctatgg cctacttggg ggggttaata ggatctaatg acttggaaaa gctgtcagaa    780
   aacgataagc atatagtgaa tcaatgggcc ttcataggtg aaaagtgtat tcacggtacc    840
   ccttcaggaa tagataacgc tgtggccact tatggtaatg ccctgctatt tgaaaaagac    900
   tcacataatg gaacaataaa cacaaacaat tttaagttct tagatgattt cccagccatt    960
   ccaatgatcc taacctatac tagaattcca aggtctacaa aagatcttgt tgctcgcgtt   1020
   cgtgtgttgg tcaccgagaa atttcctgaa gttatgaagc caattctaga tgccatgggt   1080
   gaatgtgccc tacaaggctt agagatcatg actaagttaa gtaaatgtaa aggcaccgat   1140
   gacgaggctg tagaaactaa taatgaactg tatgaacaac tattggaatt gataagaata   1200
   aatcatggac tgcttgtctc aatcggtgtt tctcatcctg gattagaact tattaaaaat   1260
   ctgagcgatg atttgagaat tggctccaca aaacttaccg gtgctggtgg cggcggttgc   1320
   tctttgactt tgttacgaag agacattact caagagcaaa ttgacagctt caaaaagaaa   1380
   ttgcaagatg attttagtta cgagacattt gaaacagact tgggtgggac tggctgctgt   1440
   ttgttaagcg caaaaaattt gaataaagat cttaaaatca aatccctagt attccaatta   1500
   tttgaaaata aaactaccac aaagcaacaa attgacgatc tattattgcc aggaaacacg   1560
   aatttaccat ggacttcata agctaatttg cgataggcct gcacccttaa ggaggaaaaa   1620
   aacatgtcag agttgagagc cttcagtgcc ccagggaaag cgttactagc tggtggatat   1680
   ttagttttag atacaaaata tgaagcattt gtagtcggat tatcggcaag aatgcatgct   1740
   gtagcccatc cttacggttc attgcaaggg tctgataagt ttgaagtgcg tgtgaaaagt   1800
   aaacaattta aagatgggga gtggctgtac catataagtc ctaaaagtgg cttcattcct   1860
   gtttcgatag gcggatctaa gaaccctttc attgaaaaag ttatcgctaa cgtatttagc   1920
   tactttaaac ctaacatgga cgactactgc aatagaaact tgttcgttat tgatattttc   1980
   tctgatgatg cctaccattc tcaggaggat agcgttaccg aacatcgtgg caacagaaga   2040
   ttgagttttc attcgcacag aattgaagaa gttcccaaaa cagggctggg ctcctcggca 2100
   ggtttagtca cagttttaac tacagctttg gcctcctttt ttgtatcgga cctggaaaat 2160
   aatgtagaca aatatagaga agttattcat aatttagcac aagttgctca ttgtcaagct 2220
   cagggtaaaa ttggaagcgg gtttgatgta gcggcggcag catatggatc tatcagatat 2280
   agaagattcc cacccgcatt aatctctaat ttgccagata ttggaagtgc tacttacggc 2340
   agtaaactgg cgcatttggt tgatgaagaa gactggaata ttacgattaa aagtaaccat 2400
   ttaccttcgg gattaacttt atggatgggc gatattaaga atggttcaga aacagtaaaa 2460
   ctggtccaga aggtaaaaaa ttggtatgat tcgcatatgc cagaaagctt gaaaatatat 2520
   acagaactcg atcatgcaaa ttctagattt atggatggac tatctaaact agatcgctta 2580
   cacgagactc atgacgatta cagcgatcag atatttgagt ctcttgagag gaatgactgt 2640
   acctgtcaaa agtatcctga aatcacagaa gttagagatg cagttgccac aattagacgt 2700
   tcctttagaa aaataactaa agaatctggt gccgatatcg aacctcccgt acaaactagc 2760
   ttattggatg attgccagac cttaaaagga gttcttactt gcttaatacc tggtgctggt 2820
   ggttatgacg ccattgcagt gattactaag caagatgttg atcttagggc tcaaaccgct 2880
   aatgacaaaa gattttctaa ggttcaatgg ctggatgtaa ctcaggctga ctggggtgtt 2940
   aggaaagaaa aagatccgga aacttatctt gataaataag gggatcctct agagtcgacc 3000
   tgcaggcatg caagcttggc actggccgtc gttttacaac gtcgtgactg ggaaaaccct 3060
   ggcgttaccc aacttaatcg ccttgcagca catccccctt tcgccagctg gcgtaatagc 3120
   gaagaggccc gcaccgatcg cccttcccaa cagttgcgca gcctgaatgg cgaatggcgc 3180
   ctgatgcggt attttctcct tacgcatctg tgcggtattt cacaccgcat acgtcaaagc 3240
   aaccatagta cgcgccctgt agcggcgcat taagcgcggc gggtgtggtg gttacgcgca 3300
   gcgtgaccgc tacacttgcc agcgccctag cgcccgctcc tttcgctttc ttcccttcct 3360
   ttctcgccac gttcgccggc tttccccgtc aagctctaaa tcgggggctc cctttagggt 3420
   tccgatttag tgctttacgg cacctcgacc ccaaaaaact tgatttgggt gatggttcac 3480
   gtagtgggcc atcgccctga tagacggttt ttcgcccttt gacgttggag tccacgttct 3540
   ttaatagtgg actcttgttc caaactggaa caacactcaa ccctatctcg ggctattctt 3600
   ttgatttata agggattttg ccgatttcgg cctattggtt aaaaaatgag ctgatttaac 3660
   aaaaatttaa cgcgaatttt aacaaaatat taacgtttac aattttatgg tgcactctca 3720
   gtacaatctg ctctgatgcc gcatagttaa gccagccccg acacccgcca acacccgctg 3780
   acgcgccctg acgggcttgt ctgctcccgg catccgctta cagacaagct gtgaccgtct 3840
   ccgggagctg catgtgtcag aggttttcac cgtcatcacc gaaacgcgcg agacgaaagg 3900
   gcctcgtgat acgcctattt ttataggtta atgtcatgat aataatggtt tcttagacgt 3960
   caggtggcac ttttcgggga aatgtgcgcg gaacccctat ttgtttattt ttctaaatac 4020
   attcaaatat gtatccgctc atgagacaat aaccctgata aatgcttcaa taatattgaa 4080
   aaaggaagag tatgagtatt caacatttcc gtgtcgccct tattcccttt tttgcggcat 4140
   tttgccttcc tgtttttgct cacccagaaa cgctggtgaa agtaaaagat gctgaagatc 4200
   agttgggtgc acgagtgggt tacatcgaac tggatctcaa cagcggtaag atccttgaga 4260
   gttttcgccc cgaagaacgt tttccaatga tgagcacttt taaagttctg ctatgtggcg 4320
   cggtattatc ccgtattgac gccgggcaag agcaactcgg tcgccgcata cactattctc 4380
   agaatgactt ggttgagtac tcaccagtca cagaaaagca tcttacggat ggcatgacag 4440
   taagagaatt atgcagtgct gccataacca tgagtgataa cactgcggcc aacttacttc 4500
   tgacaacgat cggaggaccg aaggagctaa ccgctttttt gcacaacatg ggggatcatg 4560
   taactcgcct tgatcgttgg gaaccggagc tgaatgaagc cataccaaac gacgagcgtg 4620
   acaccacgat gcctgtagca atggcaacaa cgttgcgcaa actattaact ggcgaactac 4680
   ttactctagc ttcccggcaa caattaatag actggatgga ggcggataaa gttgcaggac 4740
   cacttctgcg ctcggccctt ccggctggct ggtttattgc tgataaatct ggagccggtg 4800
   agcgtgggtc tcgcggtatc attgcagcac tggggccaga tggtaagccc tcccgtatcg 4860
   tagttatcta cacgacgggg agtcaggcaa ctatggatga acgaaataga cagatcgctg 4920
   agataggtgc ctcactgatt aagcattggt aactgtcaga ccaagtttac tcatatatac 4980
   tttagattga tttaaaactt catttttaat ttaaaaggat ctaggtgaag atcctttttg 5040
   ataatctcat gaccaaaatc ccttaacgtg agttttcgtt ccactgagcg tcagaccccg 5100
   tagaaaagat caaaggatct tcttgagatc ctttttttct gcgcgtaatc tgctgcttgc 5160
   aaacaaaaaa accaccgcta ccagcggtgg tttgtttgcc ggatcaagag ctaccaactc 5220
   tttttccgaa ggtaactggc ttcagcagag cgcagatacc aaatactgtc cttctagtgt 5280
   agccgtagtt aggccaccac ttcaagaact ctgtagcacc gcctacatac ctcgctctgc 5340
   taatcctgtt accagtggct gctgccagtg gcgataagtc gtgtcttacc gggttggact 5400
   caagacgata gttaccggat aaggcgcagc ggtcgggctg aacggggggt tcgtgcacac 5460
   agcccagctt ggagcgaacg acctacaccg aactgagata cctacagcgt gagctatgag 5520
   aaagcgccac gcttcccgaa gggagaaagg cggacaggta tccggtaagc ggcagggtcg 5580
   gaacaggaga gcgcacgagg gagcttccag ggggaaacgc ctggtatctt tatagtcctg 5640
   tcgggtttcg ccacctctga cttgagcgtc gatttttgtg atgctcgtca ggggggcgga 5700
   gcctatggaa aaacgccagc aacgcggcct ttttacggtt cctggccttt tgctggcctt 5760
   ttgctcacat gttctttcct gcgttatccc ctgattctgt ggataaccgt attaccgcct 5820
   ttgagtgagc tgataccgct cgccgcagcc gaacgaccga gcgcagcgag tcagtgagcg 5880
   aggaagcgga ag 5892
<210> 36
   <211> 39
   <212> DNA
   <213> Artificial Sequence
<220>
   <223> Primer for amplifying ERG12 gene (KKS1-6038-2-1)
<400> 36
   tcgagctcgg tacccatgtc attaccgttc ttaacttct 39
<210> 37
   <211> 53
   <212> DNA
   <213> Artificial Sequence
<220>
   <223> Primer for amplifying ERG12 gene (KKA1-6038-2-2)
<400> 37
   ttaagggtgc aggcctatcg caaattagct tatgaagtcc atggtaaatt cgt 53
<210> 38
   <211> 50
   <212> DNA
   <213> Artificial Sequence
<220>
   <223> Primer for amplifying ERG8 gene (KKS2-6083-2-3)
<400> 38
   ggcctgcacc cttaaggagg aaaaaaacat gtcagagttg agagccttca 50
<210> 39
   <211> 39
   <212> DNA
   <213> Artificial Sequence
<220>
   <223> Primer for amplifying ERG8 gene (KKA2-6083-2-4)
<400> 39
   ctctagagga tccccttatt tatcaagata agtttccgg 39
<210> 40
   <211> 6135
   <212> DNA
   <213> Artificial Sequence
<220>
   <223> Nucleotide sequence of prepared plasmid pTWV-dmd-yidi
<400> 40
   gacgaaaggg cctcgtgata cgcctatttt tataggttaa tgtcatgata ataatggttt     60
   cttagacgtc aggtggcact tttcggggaa atgtgcgcgg aacccctatt tgtttatttt    120
   tctaaataca ttcaaatatg tatccgctca tgagacaata accctgataa atgcttcaat    180
   aatattgaaa aaggaagagt atgagtattc aacatttccg tgtcgccctt attccctttt    240
   ttgcggcatt ttgccttcct gtttttgctc acccagaaac gctggtgaaa gtaaaagatg    300
   ctgaagatca gttgggtgca cgagtgggtt acatcgaact ggatctcaac agcggtaaga    360
   tccttgagag ttttcgcccc gaagaacgtt ttccaatgat gagcactttt aaagttctgc    420
   tatgtggcgc ggtattatcc cgtattgacg ccgggcaaga gcaactcggt cgccgcatac    480
   actattctca gaatgacttg gttgagtact caccagtcac agaaaagcat cttacggatg    540
   gcatgacagt aagagaatta tgcagtgctg ccataaccat gagtgataac actgcggcca    600
   acttacttct gacaacgatc ggaggaccga aggagctaac cgcttttttg cacaacatgg    660
   gggatcatgt aactcgcctt gatcgttggg aaccggagct gaatgaagcc ataccaaacg    720
   acgagcgtga caccacgatg cctgtagcaa tggcaacaac gttgcgcaaa ctattaactg    780
   gcgaactact tactctagct tcccggcaac aattaataga ctggatggag gcggataaag    840
   ttgcaggacc acttctgcgc tcggcccttc cggctggctg gtttattgct gataaatctg    900
   gagccggtga gcgtgggtct cgcggtatca ttgcagcact ggggccagat ggtaagccct    960
   cccgtatcgt agttatctac acgacgggga gtcaggcaac tatggatgaa cgaaatagac   1020
   agatcgctga gataggtgcc tcactgatta agcattggta actgtcagac caagtttact   1080
   catatatact ttagattgat ttaaaacttc atttttaatt taaaaggatc taggtgaaga   1140
   tcctttttga taatctcatg accaaaatcc cttaacgtga gttttcgttc cactgagcgt   1200
   cagaccccgt agaaaagatc aaaggatctt cttgagatcc tttttttctg cgcgtaatct   1260
   gctgcttgca aacaaaaaaa ccaccgctac cagcggtggt ttgtttgccg gatcaagagc   1320
   taccaactct ttttccgaag gtaactggct tcagcagagc gcagatacca aatactgtcc   1380
   ttctagtgta gccgtagtta ggccaccact tcaagaactc tgtagcaccg cctacatacc   1440
   tcgctctgct aatcctgtta ccagtggctg ctgccagtgg cgataagtcg tgtcttaccg   1500
   ggttggactc aagacgatag ttaccggata aggcgcagcg gtcgggctga acggggggtt   1560
   cgtgcacaca gcccagcttg gagcgaacga cctacaccga actgagatac ctacagcgtg   1620
   agctatgaga aagcgccacg cttcccgaag ggagaaaggc ggacaggtat ccggtaagcg   1680
   gcagggtcgg aacaggagag cgcacgaggg agcttccagg gggaaacgcc tggtatcttt   1740
   atagtcctgt cgggtttcgc cacctctgac ttgagcgtcg atttttgtga tgctcgtcag   1800
   gggggcggag cctatggaaa aacgccagca acgcggcctt tttacggttc ctggcctttt   1860
   gctggccttt tgctcacatg ttctttcctg cgttatcccc tgattctgtg gataaccgta   1920
   ttaccgcctt tgagtgagct gataccgctc gccgcagccg aacgaccgag cgcagcgagt 1980
   cagtgagcga ggaagcggaa gagcgcctga tgcggtattt tctccttacg catctgtgcg 2040
   gtatttcaca ccgcatatgg tgcactctca gtacaatctg ctctgatgcc gcatagttaa 2100
   gccagtatac actccgctat cgctacgtga ctgggtcatg gctgcgcccc gacacccgcc 2160
   aacacccgct gacgcgccct gacgggcttg tctgctcccg gcatccgctt acagacaagc 2220
   tgtgaccgtc tccgggagct gcatgtgtca gaggttttca ccgtcatcac cgaaacgcgc 2280
   gaggcagctg cggtaaagct catcagcgtg gtcgtgaagc gattcacaga tgtctgcctg 2340
   ttcatccgcg tccagctcgt tgagtttctc cagaagcgtt aatgtctggc ttctgataaa 2400
   gcgggccatg ttaagggcgg ttttttcctg tttggtcact gatgcctccg tgtaaggggg 2460
   atttctgttc atgggggtaa tgataccgat gaaacgagag aggatgctca cgatacgggt 2520
   tactgatgat gaacatgccc ggttactgga acgttgtgag ggtaaacaac tggcggtatg 2580
   gatgcggcgg gaccagagaa aaatcactca gggtcaatgc cagcgcttcg ttaatacaga 2640
   tgtaggtgtt ccacagggta gccagcagca tcctgcgatg cagatccgga acataatggt 2700
   gcagggcgct gacttccgcg tttccagact ttacgaaaca cggaaaccga agaccattca 2760
   tgttgttgct caggtcgcag acgttttgca gcagcagtcg cttcacgttc gctcgcgtat 2820
   cggtgattca ttctgctaac cagtaaggca accccgccag cctagccggg tcctcaacga 2880
   caggagcacg atcatgcgca cccgtggcca ggacccaacg ctgcccgaga tgcgccgcgt 2940
   gcggctgctg gagatggcgg acgcgatgga tatgttctgc caagtaatgt gagttagctc 3000
   actcattagg caccccaggc tttacacttt atgcttccgg ctcgtatgtt gtgtggaatt 3060
   gtgagcggat aacaatttca cacaggaaac agctatgacc atgattacga attcgagctc 3120
   ggtacccatg accgtttaca cagcatccgt taccgcaccc gtcaacatcg caacccttaa 3180
   gtattggggg aaaagggaca cgaagttgaa tctgcccacc aattcgtcca tatcagtgac 3240
   tttatcgcaa gatgacctca gaacgttgac ctctgcggct actgcacctg agtttgaacg 3300
   cgacactttg tggttaaatg gagaaccaca cagcatcgac aatgaaagaa ctcaaaattg 3360
   tctgcgcgac ctacgccaat taagaaagga aatggaatcg aaggacgcct cattgcccac 3420
   attatctcaa tggaaactcc acattgtctc cgaaaataac tttcctacag cagctggttt 3480
   agcttcctcc gctgctggct ttgctgcatt ggtctctgca attgctaagt tataccaatt 3540
   accacagtca acttcagaaa tatctagaat agcaagaaag gggtctggtt cagcttgtag 3600
   atcgttgttt ggcggatacg tggcctggga aatgggaaaa gctgaagatg gtcatgattc 3660
   catggcagta caaatcgcag acagctctga ctggcctcag atgaaagctt gtgtcctagt 3720
   tgtcagcgat attaaaaagg atgtgagttc cactcagggt atgcaattga ccgtggcaac 3780
   ctccgaacta tttaaagaaa gaattgaaca tgtcgtacca aagagatttg aagtcatgcg 3840
   taaagccatt gttgaaaaag atttcgccac ctttgcaaag gaaacaatga tggattccaa 3900
   ctctttccat gccacatgtt tggactcttt ccctccaata ttctacatga atgacacttc 3960
   caagcgtatc atcagttggt gccacaccat taatcagttt tacggagaaa caatcgttgc 4020
   atacacgttt gatgcaggtc caaatgctgt gttgtactac ttagctgaaa atgagtcgaa 4080
   actctttgca tttatctata aattgtttgg ctctgttcct ggatgggaca agaaatttac 4140
   tactgagcag cttgaggctt tcaaccatca atttgaatca tctaacttta ctgcacgtga 4200
   attggatctt gagttgcaaa aggatgttgc cagagtgatt ttaactcaag tcggttcagg 4260
   cccacaagaa acaaacgaat ctttgattga cgcaaagact ggtctaccaa aggaataaga 4320
   tcaattcgct gcatcgccct taggaggtaa aaaaaaatga ctgccgacaa caatagtatg 4380
   ccccatggtg cagtatctag ttacgccaaa ttagtgcaaa accaaacacc tgaagacatt 4440
   ttggaagagt ttcctgaaat tattccatta caacaaagac ctaatacccg atctagtgag 4500
   acgtcaaatg acgaaagcgg agaaacatgt ttttctggtc atgatgagga gcaaattaag 4560
   ttaatgaatg aaaattgtat tgttttggat tgggacgata atgctattgg tgccggtacc 4620
   aagaaagttt gtcatttaat ggaaaatatt gaaaagggtt tactacatcg tgcattctcc 4680
   gtctttattt tcaatgaaca aggtgaatta cttttacaac aaagagccac tgaaaaaata 4740
   actttccctg atctttggac taacacatgc tgctctcatc cactatgtat tgatgacgaa 4800
   ttaggtttga agggtaagct agacgataag attaagggcg ctattactgc ggcggtgaga 4860
   aaactagatc atgaattagg tattccagaa gatgaaacta agacaagggg taagtttcac 4920
   tttttaaaca gaatccatta catggcacca agcaatgaac catggggtga acatgaaatt 4980
   gattacatcc tattttataa gatcaacgct aaagaaaact tgactgtcaa cccaaacgtc 5040
   aatgaagtta gagacttcaa atgggtttca ccaaatgatt tgaaaactat gtttgctgac 5100
   ccaagttaca agtttacgcc ttggtttaag attatttgcg agaattactt attcaactgg 5160
   tgggagcaat tagatgacct ttctgaagtg gaaaatgaca ggcaaattca tagaatgcta 5220
   taaggggatc ctctagagtc gacctgcagg catgcaagct tggcactggc cgtcgtttta 5280
   caacgtcgtg actgggaaaa ccctggcgtt acccaactta atcgccttgc agcacatccc 5340
   cctttcgcca gctggcgtaa tagcgaagag gcccgcaccg atcgcccttc ccaacagttg 5400
   cgcagcctga atggcgaatg gcgcctgatg cggtattttc tccttacgca tctgtgcggt 5460
   atttcacacc gcatacgtca aagcaaccat agtacgcgcc ctgtagcggc gcattaagcg 5520
   cggcgggtgt ggtggttacg cgcagcgtga ccgctacact tgccagcgcc ctagcgcccg 5580
   ctcctttcgc tttcttccct tcctttctcg ccacgttcgc cggctttccc cgtcaagctc 5640
   taaatcgggg gctcccttta gggttccgat ttagtgcttt acggcacctc gaccccaaaa 5700
   aacttgattt gggtgatggt tcacgtagtg ggccatcgcc ctgatagacg gtttttcgcc   5760
   ctttgacgtt ggagtccacg ttctttaata gtggactctt gttccaaact ggaacaacac   5820
   tcaaccctat ctcgggctat tcttttgatt tataagggat tttgccgatt tcggcctatt   5880
   ggttaaaaaa tgagctgatt taacaaaaat ttaacgcgaa ttttaacaaa atattaacgt   5940
   ttacaatttt atggtgcact ctcagtacaa tctgctctga tgccgcatag ttaagccagc   6000
   cccgacaccc gccaacaccc gctgacgcgc cctgacgggc ttgtctgctc ccggcatccg   6060
   cttacagaca agctgtgacc gtctccggga gctgcatgtg tcagaggttt tcaccgtcat   6120
   caccgaaacg cgcga                                                    6135
<210> 41
   <211> 36
   <212> DNA
   <213> Artificial Sequence
<220>
   <223> Primer for amplifying ERG19 gene (DyIS1-6083-2-5)
<400> 41
   tcgagctcgg tacccatgac cgtttacaca gcatcc 36
<210> 42
   <211> 61
   <212> DNA
   <213> Artificial Sequence
<220>
   <223> Primer for amplifying ERG19 gene (DyIA1-6083-2-6)
<400> 42
   tttttttacc tcctaagggc gatgcagcga attgatctta ttcctttggt agaccagtct     60
   t                                                                    61
<210> 43
   <211> 57
   <212> DNA
   <213> Artificial Sequence
<220>
   <223> Primer for amplifying IDI1 gene (DyIS2-6083-2-7)
<400> 43
   taggaggtaa aaaaaaatga ctgccgacaa caatagtatg ccccatggtg cagtatc 57
<210> 44
   <211> 42
   <212> DNA
   <213> Artificial Sequence
<220>
   <223> Primer for amplifying IDI1 gene (DyIA2-6083-2-8)
<400> 44
   ctctagagga tccccttata gcattctatg aatttgcctg tc 42
<210> 45
   <211> 9257
   <212> DNA
   <213> Artificial Sequence
<220>
   <223> Nucleotide sequence of prepared plasmid pTrc-KKDyI(beta))
<400> 45
   gtttgacagc ttatcatcga ctgcacggtg caccaatgct tctggcgtca ggcagccatc      60
   ggaagctgtg gtatggctgt gcaggtcgta aatcactgca taattcgtgt cgctcaaggc     120
   gcactcccgt tctggataat gttttttgcg ccgacatcat aacggttctg gcaaatattc     180
   tgaaatgagc tgttgacaat taatcatccg gctcgtataa tgtgtggaat tgtgagcgga     240
   taacaatttc acacaggaaa cagcgccgct gagaaaaagc gaagcggcac tgctctttaa     300
   caatttatca gacaatctgt gtgggcactc gaccggaatt atcgattaac tttattatta     360
   aaaattaaag aggtatatat taatgtatcg attaaataag gaggaataaa ccatgtcatt     420
   accgttctta acttctgcac cgggaaaggt tattattttt ggtgaacact ctgctgtgta     480
   caacaagcct gccgtcgctg ctagtgtgtc tgcgttgaga acctacctgc taataagcga     540
   gtcatctgca ccagatacta ttgaattgga cttcccggac attagcttta atcataagtg     600
   gtccatcaat gatttcaatg ccatcaccga ggatcaagta aactcccaaa aattggccaa     660
   ggctcaacaa gccaccgatg gcttgtctca ggaactcgtt agtcttttgg atccgttgtt     720
   agctcaacta tccgaatcct tccactacca tgcagcgttt tgtttcctgt atatgtttgt     780
   ttgcctatgc ccccatgcca agaatattaa gttttcttta aagtctactt tacccatcgg     840
   tgctgggttg ggctcaagcg cctctatttc tgtatcactg gccttagcta tggcctactt     900
   gggggggtta ataggatcta atgacttgga aaagctgtca gaaaacgata agcatatagt     960
   gaatcaatgg gccttcatag gtgaaaagtg tattcacggt accccttcag gaatagataa    1020
   cgctgtggcc acttatggta atgccctgct atttgaaaaa gactcacata atggaacaat    1080
   aaacacaaac aattttaagt tcttagatga tttcccagcc attccaatga tcctaaccta    1140
   tactagaatt ccaaggtcta caaaagatct tgttgctcgc gttcgtgtgt tggtcaccga    1200
   gaaatttcct gaagttatga agccaattct agatgccatg ggtgaatgtg ccctacaagg    1260
   cttagagatc atgactaagt taagtaaatg taaaggcacc gatgacgagg ctgtagaaac    1320
   taataatgaa ctgtatgaac aactattgga attgataaga ataaatcatg gactgcttgt    1380
   ctcaatcggt gtttctcatc ctggattaga acttattaaa aatctgagcg atgatttgag    1440
   aattggctcc acaaaactta ccggtgctgg tggcggcggt tgctctttga ctttgttacg    1500
   aagagacatt actcaagagc aaattgacag cttcaaaaag aaattgcaag atgattttag 1560
   ttacgagaca tttgaaacag acttgggtgg gactggctgc tgtttgttaa gcgcaaaaaa 1620
   tttgaataaa gatcttaaaa tcaaatccct agtattccaa ttatttgaaa ataaaactac 1680
   cacaaagcaa caaattgacg atctattatt gccaggaaac acgaatttac catggacttc 1740
   ataagctaat ttgcgatagg cctgcaccct taaggaggaa aaaaacatgt cagagttgag 1800
   agccttcagt gccccaggga aagcgttact agctggtgga tatttagttt tagatacaaa 1860
   atatgaagca tttgtagtcg gattatcggc aagaatgcat gctgtagccc atccttacgg 1920
   ttcattgcaa gggtctgata agtttgaagt gcgtgtgaaa agtaaacaat ttaaagatgg 1980
   ggagtggctg taccatataa gtcctaaaag tggcttcatt cctgtttcga taggcggatc 2040
   taagaaccct ttcattgaaa aagttatcgc taacgtattt agctacttta aacctaacat 2100
   ggacgactac tgcaatagaa acttgttcgt tattgatatt ttctctgatg atgcctacca 2160
   ttctcaggag gatagcgtta ccgaacatcg tggcaacaga agattgagtt ttcattcgca 2220
   cagaattgaa gaagttccca aaacagggct gggctcctcg gcaggtttag tcacagtttt 2280
   aactacagct ttggcctcct tttttgtatc ggacctggaa aataatgtag acaaatatag 2340
   agaagttatt cataatttag cacaagttgc tcattgtcaa gctcagggta aaattggaag 2400
   cgggtttgat gtagcggcgg cagcatatgg atctatcaga tatagaagat tcccacccgc 2460
   attaatctct aatttgccag atattggaag tgctacttac ggcagtaaac tggcgcattt 2520
   ggttgatgaa gaagactgga atattacgat taaaagtaac catttacctt cgggattaac 2580
   tttatggatg ggcgatatta agaatggttc agaaacagta aaactggtcc agaaggtaaa 2640
   aaattggtat gattcgcata tgccagaaag cttgaaaata tatacagaac tcgatcatgc 2700
   aaattctaga tttatggatg gactatctaa actagatcgc ttacacgaga ctcatgacga 2760
   ttacagcgat cagatatttg agtctcttga gaggaatgac tgtacctgtc aaaagtatcc 2820
   tgaaatcaca gaagttagag atgcagttgc cacaattaga cgttccttta gaaaaataac 2880
   taaagaatct ggtgccgata tcgaacctcc cgtacaaact agcttattgg atgattgcca 2940
   gaccttaaaa ggagttctta cttgcttaat acctggtgct ggtggttatg acgccattgc 3000
   agtgattact aagcaagatg ttgatcttag ggctcaaacc gctaatgaca aaagattttc 3060
   taaggttcaa tggctggatg taactcaggc tgactggggt gttaggaaag aaaaagatcc 3120
   ggaaacttat cttgataaat aacttaaggt agctgcatgc agaattcgcc cttaaggagg 3180
   aaaaaaaaat gaccgtttac acagcatccg ttaccgcacc cgtcaacatc gcaaccctta 3240
   agtattgggg gaaaagggac acgaagttga atctgcccac caattcgtcc atatcagtga 3300
   ctttatcgca agatgacctc agaacgttga cctctgcggc tactgcacct gagtttgaac 3360
   gcgacacttt gtggttaaat ggagaaccac acagcatcga caatgaaaga actcaaaatt 3420
   gtctgcgcga cctacgccaa ttaagaaagg aaatggaatc gaaggacgcc tcattgccca 3480
   cattatctca atggaaactc cacattgtct ccgaaaataa ctttcctaca gcagctggtt 3540
   tagcttcctc cgctgctggc tttgctgcat tggtctctgc aattgctaag ttataccaat 3600
   taccacagtc aacttcagaa atatctagaa tagcaagaaa ggggtctggt tcagcttgta 3660
   gatcgttgtt tggcggatac gtggcctggg aaatgggaaa agctgaagat ggtcatgatt 3720
   ccatggcagt acaaatcgca gacagctctg actggcctca gatgaaagct tgtgtcctag 3780
   ttgtcagcga tattaaaaag gatgtgagtt ccactcaggg tatgcaattg accgtggcaa 3840
   cctccgaact atttaaagaa agaattgaac atgtcgtacc aaagagattt gaagtcatgc 3900
   gtaaagccat tgttgaaaaa gatttcgcca cctttgcaaa ggaaacaatg atggattcca 3960
   actctttcca tgccacatgt ttggactctt tccctccaat attctacatg aatgacactt 4020
   ccaagcgtat catcagttgg tgccacacca ttaatcagtt ttacggagaa acaatcgttg 4080
   catacacgtt tgatgcaggt ccaaatgctg tgttgtacta cttagctgaa aatgagtcga 4140
   aactctttgc atttatctat aaattgtttg gctctgttcc tggatgggac aagaaattta 4200
   ctactgagca gcttgaggct ttcaaccatc aatttgaatc atctaacttt actgcacgtg 4260
   aattggatct tgagttgcaa aaggatgttg ccagagtgat tttaactcaa gtcggttcag 4320
   gcccacaaga aacaaacgaa tctttgattg acgcaaagac tggtctacca aaggaataag 4380
   atcaattcgc tgcatcgccc ttaggaggta aaaaaaaatg actgccgaca acaatagtat 4440
   gccccatggt gcagtatcta gttacgccaa attagtgcaa aaccaaacac ctgaagacat 4500
   tttggaagag tttcctgaaa ttattccatt acaacaaaga cctaataccc gatctagtga 4560
   gacgtcaaat gacgaaagcg gagaaacatg tttttctggt catgatgagg agcaaattaa 4620
   gttaatgaat gaaaattgta ttgttttgga ttgggacgat aatgctattg gtgccggtac 4680
   caagaaagtt tgtcatttaa tggaaaatat tgaaaagggt ttactacatc gtgcattctc 4740
   cgtctttatt ttcaatgaac aaggtgaatt acttttacaa caaagagcca ctgaaaaaat 4800
   aactttccct gatctttgga ctaacacatg ctgctctcat ccactatgta ttgatgacga 4860
   attaggtttg aagggtaagc tagacgataa gattaagggc gctattactg cggcggtgag 4920
   aaaactagat catgaattag gtattccaga agatgaaact aagacaaggg gtaagtttca 4980
   ctttttaaac agaatccatt acatggcacc aagcaatgaa ccatggggtg aacatgaaat 5040
   tgattacatc ctattttata agatcaacgc taaagaaaac ttgactgtca acccaaacgt 5100
   caatgaagtt agagacttca aatgggtttc accaaatgat ttgaaaacta tgtttgctga 5160
   cccaagttac aagtttacgc cttggtttaa gattatttgc gagaattact tattcaactg 5220
   gtgggagcaa ttagatgacc tttctgaagt ggaaaatgac aggcaaattc atagaatgct 5280
   ataactgcag ctggtaccat atgggaattc gaagctttct agaacaaaaa ctcatctcag   5340
   aagaggatct gaatagcgcc gtcgaccatc atcatcatca tcattgagtt taaacggtct   5400
   ccagcttggc tgttttggcg gatgagagaa gattttcagc ctgatacaga ttaaatcaga   5460
   acgcagaagc ggtctgataa aacagaattt gcctggcggc agtagcgcgg tggtcccacc   5520
   tgaccccatg ccgaactcag aagtgaaacg ccgtagcgcc gatggtagtg tggggtctcc   5580
   ccatgcgaga gtagggaact gccaggcatc aaataaaacg aaaggctcag tcgaaagact   5640
   gggcctttcg ttttatctgt tgtttgtcgg tgaacgctct cctgagtagg acaaatccgc   5700
   cgggagcgga tttgaacgtt gcgaagcaac ggcccggagg gtggcgggca ggacgcccgc   5760
   cataaactgc caggcatcaa attaagcaga aggccatcct gacggatggc ctttttgcgt   5820
   ttctacaaac tctttttgtt tatttttcta aatacattca aatatgtatc cgctcatgag   5880
   acaataaccc tgataaatgc ttcaataata ttgaaaaagg aagagtatga gtattcaaca   5940
   tttccgtgtc gcccttattc ccttttttgc ggcattttgc cttcctgttt ttgctcaccc   6000
   agaaacgctg gtgaaagtaa aagatgctga agatcagttg ggtgcacgag tgggttacat   6060
   cgaactggat ctcaacagcg gtaagatcct tgagagtttt cgccccgaag aacgttttcc   6120
   aatgatgagc acttttaaag ttctgctatg tggcgcggta ttatcccgtg ttgacgccgg   6180
   gcaagagcaa ctcggtcgcc gcatacacta ttctcagaat gacttggttg agtactcacc   6240
   agtcacagaa aagcatctta cggatggcat gacagtaaga gaattatgca gtgctgccat   6300
   aaccatgagt gataacactg cggccaactt acttctgaca acgatcggag gaccgaagga   6360
   gctaaccgct tttttgcaca acatggggga tcatgtaact cgccttgatc gttgggaacc   6420
   ggagctgaat gaagccatac caaacgacga gcgtgacacc acgatgcctg tagcaatggc   6480
   aacaacgttg cgcaaactat taactggcga actacttact ctagcttccc ggcaacaatt   6540
   aatagactgg atggaggcgg ataaagttgc aggaccactt ctgcgctcgg cccttccggc   6600
   tggctggttt attgctgata aatctggagc cggtgagcgt gggtctcgcg gtatcattgc   6660
   agcactgggg ccagatggta agccctcccg tatcgtagtt atctacacga cggggagtca   6720
   ggcaactatg gatgaacgaa atagacagat cgctgagata ggtgcctcac tgattaagca   6780
   ttggtaactg tcagaccaag tttactcata tatactttag attgatttaa aacttcattt   6840
   ttaatttaaa aggatctagg tgaagatcct ttttgataat ctcatgacca aaatccctta   6900
   acgtgagttt tcgttccact gagcgtcaga ccccgtagaa aagatcaaag gatcttcttg   6960
   agatcctttt tttctgcgcg taatctgctg cttgcaaaca aaaaaaccac cgctaccagc   7020
   ggtggtttgt ttgccggatc aagagctacc aactcttttt ccgaaggtaa ctggcttcag   7080
   cagagcgcag ataccaaata ctgtccttct agtgtagccg tagttaggcc accacttcaa   7140
   gaactctgta gcaccgccta catacctcgc tctgctaatc ctgttaccag tggctgctgc 7200
   cagtggcgat aagtcgtgtc ttaccgggtt ggactcaaga cgatagttac cggataaggc 7260
   gcagcggtcg ggctgaacgg ggggttcgtg cacacagccc agcttggagc gaacgaccta 7320
   caccgaactg agatacctac agcgtgagct atgagaaagc gccacgcttc ccgaagggag 7380
   aaaggcggac aggtatccgg taagcggcag ggtcggaaca ggagagcgca cgagggagct 7440
   tccaggggga aacgcctggt atctttatag tcctgtcggg tttcgccacc tctgacttga 7500
   gcgtcgattt ttgtgatgct cgtcaggggg gcggagccta tggaaaaacg ccagcaacgc 7560
   ggccttttta cggttcctgg ccttttgctg gccttttgct cacatgttct ttcctgcgtt 7620
   atcccctgat tctgtggata accgtattac cgcctttgag tgagctgata ccgctcgccg 7680
   cagccgaacg accgagcgca gcgagtcagt gagcgaggaa gcggaagagc gcctgatgcg 7740
   gtattttctc cttacgcatc tgtgcggtat ttcacaccgc atatggtgca ctctcagtac 7800
   aatctgctct gatgccgcat agttaagcca gtatacactc cgctatcgct acgtgactgg 7860
   gtcatggctg cgccccgaca cccgccaaca cccgctgacg cgccctgacg ggcttgtctg 7920
   ctcccggcat ccgcttacag acaagctgtg accgtctccg ggagctgcat gtgtcagagg 7980
   ttttcaccgt catcaccgaa acgcgcgagg cagcagatca attcgcgcgc gaaggcgaag 8040
   cggcatgcat ttacgttgac accatcgaat ggtgcaaaac ctttcgcggt atggcatgat 8100
   agcgcccgga agagagtcaa ttcagggtgg tgaatgtgaa accagtaacg ttatacgatg 8160
   tcgcagagta tgccggtgtc tcttatcaga ccgtttcccg cgtggtgaac caggccagcc 8220
   acgtttctgc gaaaacgcgg gaaaaagtgg aagcggcgat ggcggagctg aattacattc 8280
   ccaaccgcgt ggcacaacaa ctggcgggca aacagtcgtt gctgattggc gttgccacct 8340
   ccagtctggc cctgcacgcg ccgtcgcaaa ttgtcgcggc gattaaatct cgcgccgatc 8400
   aactgggtgc cagcgtggtg gtgtcgatgg tagaacgaag cggcgtcgaa gcctgtaaag 8460
   cggcggtgca caatcttctc gcgcaacgcg tcagtgggct gatcattaac tatccgctgg 8520
   atgaccagga tgccattgct gtggaagctg cctgcactaa tgttccggcg ttatttcttg 8580
   atgtctctga ccagacaccc atcaacagta ttattttctc ccatgaagac ggtacgcgac 8640
   tgggcgtgga gcatctggtc gcattgggtc accagcaaat cgcgctgtta gcgggcccat 8700
   taagttctgt ctcggcgcgt ctgcgtctgg ctggctggca taaatatctc actcgcaatc 8760
   aaattcagcc gatagcggaa cgggaaggcg actggagtgc catgtccggt tttcaacaaa 8820
   ccatgcaaat gctgaatgag ggcatcgttc ccactgcgat gctggttgcc aacgatcaga 8880
   tggcgctggg cgcaatgcgc gccattaccg agtccgggct gcgcgttggt gcggatatct 8940
   cggtagtggg atacgacgat accgaagaca gctcatgtta tatcccgccg tcaaccacca 9000
   tcaaacagga ttttcgcctg ctggggcaaa ccagcgtgga ccgcttgctg caactctctc 9060
   agggccaggc ggtgaagggc aatcagctgt tgcccgtctc actggtgaaa agaaaaacca 9120
   ccctggcgcc caatacgcaa accgcctctc cccgcgcgtt ggccgattca ttaatgcagc 9180
   tggcacgaca ggtttcccga ctggaaagcg ggcagtgagc gcaacgcaat taatgtgagt 9240
   tagcgcgaat tgatctg 9257
<210> 46
   <211> 36
   <212> DNA
   <213> Artificial Sequence
<220>
   <223> Primer (KKDS2_6038-3-2)
<400> 46
   gaggaataaa ccatgtcatt accgttctta acttct 36
<210> 47
   <211> 55
   <212> DNA
   <213> Artificial Sequence
<220>
   <223> Primer (KKMyIA_6038-2-9)
<400> 47
   aagggcgaat tctgcatgca gctaccttaa gttatttatc aagataagtt tccgg 55
<210> 48
   <211> 51
   <212> DNA
   <213> Artificial Sequence
<220>
   <223> Primer (KMS_6038-6-1)
<400> 48
   gcagaattcg cccttaagga ggaaaaaaaa atgaccgttt acacagcatc c 51
<210> 49
   <211> 47
   <212> DNA
   <213> Artificial Sequence
<220>
   <223> Primer (KDyIA_6038-3-3)
<400> 49
   ccatatggta ccagctgcag ttatagcatt ctatgaattt gcctgtc 47
<210> 50
   <211> 11386
   <212> DNA
   <213> Artificial Sequence
<220>
   <223> Nucleotide sequence of prepared plasmid pMW219-KKDyI-TaspA
<400> 50
   gacagtaaga cgggtaagcc tgttgatgat accgctgcct tactgggtgc attagccagt 60
   ctgaatgacc tgtcacggga taatccgaag tggtcagact ggaaaatcag agggcaggaa 120
   ctgcagaaca gcaaaaagtc agatagcacc acatagcaga cccgccataa aacgccctga 180
   gaagcccgtg acgggctttt cttgtattat gggtagtttc cttgcatgaa tccataaaag 240
   gcgcctgtag tgccatttac ccccattcac tgccagagcc gtgagcgcag cgaactgaat 300
   gtcacgaaaa agacagcgac tcaggtgcct gatggtcgga gacaaaagga atattcagcg 360
   atttgcccga gcttgcgagg gtgctactta agcctttagg gttttaaggt ctgttttgta 420
   gaggagcaaa cagcgtttgc gacatccttt tgtaatactg cggaactgac taaagtagtg 480
   agttatacac agggctggga tctattcttt ttatcttttt ttattctttc tttattctat 540
   aaattataac cacttgaata taaacaaaaa aaacacacaa aggtctagcg gaatttacag 600
   agggtctagc agaatttaca agttttccag caaaggtcta gcagaattta cagataccca 660
   caactcaaag gaaaaggact agtaattatc attgactagc ccatctcaat tggtatagtg 720
   attaaaatca cctagaccaa ttgagatgta tgtctgaatt agttgttttc aaagcaaatg 780
   aactagcgat tagtcgctat gacttaacgg agcatgaaac caagctaatt ttatgctgtg 840
   tggcactact caaccccacg attgaaaacc ctacaaggaa agaacggacg gtatcgttca 900
   cttataacca atacgctcag atgatgaaca tcagtaggga aaatgcttat ggtgtattag 960
   ctaaagcaac cagagagctg atgacgagaa ctgtggaaat caggaatcct ttggttaaag 1020
   gctttgagat tttccagtgg acaaactatg ccaagttctc aagcgaaaaa ttagaattag 1080
   tttttagtga agagatattg ccttatcttt tccagttaaa aaaattcata aaatataatc 1140
   tggaacatgt taagtctttt gaaaacaaat actctatgag gatttatgag tggttattaa 1200
   aagaactaac acaaaagaaa actcacaagg caaatataga gattagcctt gatgaattta 1260
   agttcatgtt aatgcttgaa aataactacc atgagtttaa aaggcttaac caatgggttt 1320
   tgaaaccaat aagtaaagat ttaaacactt acagcaatat gaaattggtg gttgataagc 1380
   gaggccgccc gactgatacg ttgattttcc aagttgaact agatagacaa atggatctcg 1440
   taaccgaact tgagaacaac cagataaaaa tgaatggtga caaaatacca acaaccatta 1500
   catcagattc ctacctacat aacggactaa gaaaaacact acacgatgct ttaactgcaa 1560
   aaattcagct caccagtttt gaggcaaaat ttttgagtga catgcaaagt aagcatgatc 1620
   tcaatggttc gttctcatgg ctcacgcaaa aacaacgaac cacactagag aacatactgg 1680
   ctaaatacgg aaggatctga ggttcttatg gctcttgtat ctatcagtga agcatcaaga 1740
   ctaacaaaca aaagtagaac aactgttcac cgttacatat caaagggaaa actgtccata 1800
   tgcacagatg aaaacggtgt aaaaaagata gatacatcag agcttttacg agtttttggt 1860
   gcattcaaag ctgttcacca tgaacagatc gacaatgtaa cagatgaaca gcatgtaaca 1920
   cctaatagaa caggtgaaac cagtaaaaca aagcaactag aacatgaaat tgaacacctg 1980
   agacaacttg ttacagctca acagtcacac atagacagcc tgaaacaggc gatgctgctt 2040
   atcgaatcaa agctgccgac aacacgggag ccagtgacgc ctcccgtggg gaaaaaatca 2100
   tggcaattct ggaagaaata gcgccattcg ccattcaggc tgcgcaactg ttgggaaggg 2160
   cgatcggtgc gggcctcttc gctattacgc cagctggcga aagggggatg tgctgcaagg 2220
   cgattaagtt gggtaacgcc agggttttcc cagtcacgac gttgtaaaac gacggccagt 2280
   gaattcgagc tcggtaccct gtttttccac tcttcgttca ctttcgccag gtagctggtg 2340
   aagacgaagg aagtcccgga gccatctgcg cggcgtacta cagcaatgtt ttgtgaaggc 2400
   agtttcagac ccggattcag tttggcgatg gcttcatcat cccacttctt gattttgccc 2460
   aggtagatgt cgccgagggt tttaccatcc agcaccagtt cgccagactt cagccctgga 2520
   atgttaaccg ccagcaccac gccgccaatc acggtcggga actggaacag accttcctga 2580
   gccagttttt cgtcagacag cggcgcgtca gaggcaccaa aatcaacggt attagcgata 2640
   atctgtttta cgccaccgga agaaccgata ccctggtagt taactttatt accggtttct 2700
   ttctggtaag tgtcagccca tttggcatac accggcgcag ggaaggttgc acctgcacct 2760
   gtcaggcttg cttctgcaaa cacagagaaa gcactcatcg ataaggtcgc ggcgacaaca 2820
   gttgcgacgg tggtacgcat aactttcata atgtctcctg ggaggattca taaagcattg 2880
   tttgttggct acgagaagca aaataggaca aacaggtgac agttatatgt aaggaatatg 2940
   acagttttat gacagagaga taaagtcttc agtctgattt aaataagcgt tgatattcag 3000
   tcaattacaa acattaataa cgaagagatg acagaaaaat tttcattctg tgacagagaa 3060
   aaagtagccg aagatgacgg tttgtcacat ggagttggca ggatgtttga ttaaaatgaa 3120
   gcctgctttt ttatactaag ttggcattat aaaaaagcat tgcttatcaa tttgttgcaa 3180
   cgaacaggtc actatcagtc aaaataaaat cattatttga tttcgaattc tcatgtttga 3240
   cagcttatca tcgataagct ttaatgcggt agtttatcac agttaaattg ctaacgcagt 3300
   caggcaccgt gtatgaaatc taacaatgcg ctcatcgtca tcctcggcac cgtcaccctg 3360
   gatgctgtag gcataggctt ggttatgccg gtactgccgg gcctcttgcg ggatatcgtc 3420
   cattccgaca gcatcgccag tcactatggc gtgctgctag cgctatatgc gttgatgcaa 3480
   tttctatgcg cacccgttct cggagcactg tccgaccgct ttggccgccg cccagtcctg 3540
   ctcgcttcgc tacttggagc cactatcgac tacgcgatca tggcgaccac acccgtcctg 3600
   tggatctccg gataagtaga cagcctgata agtcgcacga aaaacaggta ttgacaacat 3660
   gaagtaacat gcagtaagat acaaatcgct aggtaacact agcagcgtca accgggcgct 3720
   ctagctagag ccaagctagc ttggccggat ccgagatttt caggagctaa ggaagctaaa 3780
   atggagaaaa aaatcactgg atataccacc gttgatatat cccaatggca tcgtaaagaa 3840
   cattttgagg catttcagtc agttgctcaa tgtacctata accagaccgt tcagctggat 3900
   attacggcct ttttaaagac cgtaaagaaa aataagcaca agttttatcc ggcctttatt 3960
   cacattcttg cccgcctgat gaatgctcat ccggaattcc gtatggcaat gaaagacggt 4020
   gagctggtga tatgggatag tgttcaccct tgttacaccg ttttccatga gcaaactgaa 4080
   acgttttcat cgctctggag tgaataccac gacgatttcc ggcagtttct acacatatat 4140
   tcgcaagatg tggcgtgtta cggtgaaaac ctggcctatt tccctaaagg gtttattgag 4200
   aatatgtttt tcgtctcagc caatccctgg gtgagtttca ccagttttga tttaaacgtg 4260
   gccaatatgg acaacttctt cgcccccgtt ttcaccatgg gcaaatatta tacgcaaggc 4320
   gacaaggtgc tgatgccgct ggcgattcag gttcatcatg ccgtctgtga tggcttccat 4380
   gtcggcagaa tgcttaatga attacaacag tactgcgatg agtggcaggg cggggcgtaa 4440
   tttttttaag gcagttattg gtgcccttaa acgcctggtg ctacgcctga ataagtgata 4500
   ataagcggat gaatggcaga aattcgtcga agcttaacac agaaaaaagc ccgcacctga 4560
   cagtgcgggc tttttttttc gaccactgca gtctgttaca ggtcactaat accatctaag 4620
   tagttgattc atagtgactg catatgttgt gttttacagt attatgtagt ctgtttttta 4680
   tgcaaaatct aatttaatat attgatattt atatcatttt acgtttctcg ttcagctttt 4740
   ttatactaac ttgagcggcc cttgacgatg ccacatcctg agcaaataat tcaaccacta 4800
   attgtgagcg gataacacaa ggaggaaaca gctatgtcat taccgttctt aacttctgca 4860
   ccgggaaagg ttattatttt tggtgaacac tctgctgtgt acaacaagcc tgccgtcgct 4920
   gctagtgtgt ctgcgttgag aacctacctg ctaataagcg agtcatctgc accagatact 4980
   attgaattgg acttcccgga cattagcttt aatcataagt ggtccatcaa tgatttcaat 5040
   gccatcaccg aggatcaagt aaactcccaa aaattggcca aggctcaaca agccaccgat 5100
   ggcttgtctc aggaactcgt tagtcttttg gatccgttgt tagctcaact atccgaatcc 5160
   ttccactacc atgcagcgtt ttgtttcctg tatatgtttg tttgcctatg cccccatgcc 5220
   aagaatatta agttttcctt aaagtctact ttacccatcg gtgctgggtt gggctcaagc 5280
   gcctctattt ctgtatcact ggccttagct atggcctact tgggggggtt aataggatct 5340
   aatgacttgg aaaagctgtc agaaaacgat aagcatatag tgaatcaatg ggccttcata 5400
   ggtgaaaagt gtattcacgg taccccttca ggaatagata acgctgtggc cacttatggt 5460
   aatgccctgc tatttgaaaa agactcacat aatggaacaa taaacacaaa caattttaag 5520
   ttcttagatg atttcccagc cattccaatg atcctaacct atactagaat tccaaggtct 5580
   acaaaagatc ttgttgctcg cgttcgtgtg ttggtcaccg agaaatttcc tgaagttatg 5640
   aagccaattc tagatgccat gggtgaatgt gccctacaag gcttagagat catgactaag 5700
   ttaagtaaat gtaaaggcac cgatgacgag gctgtagaaa ctaataatga actgtatgaa 5760
   caactattgg aattgataag aataaatcat ggactgcttg tctcaatcgg tgtttctcat 5820
   cctggattag aacttattaa aaatctgagc gatgatttga gaattggctc cacaaaactt 5880
   accggtgctg gtggcggcgg ttgctctttg actttgttac gaagagacat tactcaagag 5940
   caaattgaca gcttcaaaaa gaaattgcaa gatgatttta gttacgagac atttgaaaca 6000
   gacttgggtg ggactggctg ctgtttgtta agcgcaaaaa atttgaataa agatcttaaa 6060
   atcaaatccc tagtattcca attatttgaa aataaaacta ccacaaagca acaaattgac 6120
   gatctattat tgccaggaaa cacgaattta ccatggactt cataagctaa tttgcgatag 6180
   gcctgcaccc ttaaggagga aaaaaacatg tcagagttga gagccttcag tgccccaggg 6240
   aaagcgttac tagctggtgg atatttagtt ttagatacaa aatatgaagc atttgtagtc 6300
   ggattatcgg caagaatgca tgctgtagcc catccttacg gttcattgca agggtctgat 6360
   aagtttgaag tgcgtgtgaa aagtaaacaa tttaaagatg gggagtggct gtaccatata 6420
   agtcctaaaa gtggcttcat tcctgtttcg ataggcggat ctaagaaccc tttcattgaa 6480
   aaagttatcg ctaacgtatt tagctacttt aaacctaaca tggacgacta ctgcaataga 6540
   aacttgttcg ttattgatat tttctctgat gatgcctacc attctcagga ggatagcgtt 6600
   accgaacatc gtggcaacag aagattgagt tttcattcgc acagaattga agaagttccc 6660
   aaaacagggc tgggctcctc ggcaggttta gtcacagttt taactacagc tttggcctcc 6720
   ttttttgtat cggacctgga aaataatgta gacaaatata gagaagttat tcataattta 6780
   gcacaagttg ctcattgtca agctcagggt aaaattggaa gcgggtttga tgtagcggcg 6840
   gcagcatatg gatctatcag atatagaaga ttcccacccg cattaatctc taatttgcca 6900
   gatattggaa gtgctactta cggcagtaaa ctggcgcatt tggttgatga agaagactgg 6960
   aatattacga ttaaaagtaa ccatttacct tcgggattaa ctttatggat gggcgatatt 7020
   aagaatggtt cagaaacagt aaaactggtc cagaaggtaa aaaattggta tgattcgcat 7080
   atgccagaaa gcttgaaaat atatacagaa ctcgatcatg caaattctag atttatggat 7140
   ggactatcta aactagatcg cttacacgag actcatgacg attacagcga tcagatattt 7200
   gagtctcttg agaggaatga ctgtacctgt caaaagtatc ctgaaatcac agaagttaga 7260
   gatgcagttg ccacaattag acgttccttt agaaaaataa ctaaagaatc tggtgccgat 7320
   atcgaacctc ccgtacaaac tagcttattg gatgattgcc agaccttaaa aggagttctt 7380
   acttgcttaa tacctggtgc tggtggttat gacgccattg cagtgattac taagcaagat 7440
   gttgatctta gggctcaaac cgctaatgac aaaagatttt ctaaggttca atggctggat 7500
   gtaactcagg ctgactgggg tgttaggaaa gaaaaagatc cggaaactta tcttgataaa 7560
   taacttaagg tagctgcatg cagaattcgc ccttaaggag gaaaaaaaaa tgaccgttta 7620
   cacagcatcc gttaccgcac ccgtcaacat cgcaaccctt aagtattggg ggaaaaggga 7680
   cacgaagttg aatctgccca ccaattcgtc catatcagtg actttatcgc aagatgacct 7740
   cagaacgttg acctctgcgg ctactgcacc tgagtttgaa cgcgacactt tgtggttaaa 7800
   tggagaacca cacagcatcg acaatgaaag aactcaaaat tgtctgcgcg acctacgcca 7860
   attaagaaag gaaatggaat cgaaggacgc ctcattgccc acattatctc aatggaaact 7920
   ccacattgtc tccgaaaata actttcctac agcagctggt ttagcttcct ccgctgctgg 7980
   ctttgctgca ttggtctctg caattgctaa gttataccaa ttaccacagt caacttcaga 8040
   aatatctaga atagcaagaa aggggtctgg ttcagcttgt agatcgttgt ttggcggata 8100
   cgtggcctgg gaaatgggaa aagctgaaga tggtcatgat tccatggcag tacaaatcgc 8160
   agacagctct gactggcctc agatgaaagc ttgtgtccta gttgtcagcg atattaaaaa 8220
   ggatgtgagt tccactcagg gtatgcaatt gaccgtggca acctccgaac tatttaaaga 8280
   aagaattgaa catgtcgtac caaagagatt tgaagtcatg cgtaaagcca ttgttgaaaa 8340
   agatttcgcc acctttgcaa aggaaacaat gatggattcc aactctttcc atgccacatg 8400
   tttggactct ttccctccaa tattctacat gaatgacact tccaagcgta tcatcagttg 8460
   gtgccacacc attaatcagt tttacggaga aacaatcgtt gcatacacgt ttgatgcagg 8520
   tccaaatgct gtgttgtact acttagctga aaatgagtcg aaactctttg catttatcta 8580
   taaattgttt ggctctgttc ctggatggga caagaaattt actactgagc agcttgaggc 8640
   tttcaaccat caatttgaat catctaactt tactgcacgt gaattggatc ttgagttgca 8700
   aaaggatgtt gccagagtga ttttaactca agtcggttca ggcccacaag aaacaaacga 8760
   atctttgatt gacgcaaaga ctggtctacc aaaggaataa gatcaattcg ctgcatcgcc 8820
   cttaggaggt aaaaaaaaat gactgccgac aacaatagta tgccccatgg tgcagtatct 8880
   agttacgcca aattagtgca aaaccaaaca cctgaagaca ttttggaaga gtttcctgaa 8940
   attattccat tacaacaaag acctaatacc cgatctagtg agacgtcaaa tgacgaaagc 9000
   ggagaaacat gtttttctgg tcatgatgag gagcaaatta agttaatgaa tgaaaattgt 9060
   attgttttgg attgggacga taatgctatt ggtgccggta ccaagaaagt ttgtcattta 9120
   atggaaaata ttgaaaaggg tttactacat cgtgcattct ccgtctttat tttcaatgaa 9180
   caaggtgaat tacttttaca acaaagagcc actgaaaaaa taactttccc tgatctttgg 9240
   actaacacat gctgctctca tccactatgt attgatgacg aattaggttt gaagggtaag 9300
   ctagacgata agattaaggg cgctattact gcggcggtga gaaaactaga tcatgaatta 9360
   ggtattccag aagatgaaac taagacaagg ggtaagtttc actttttaaa cagaatccat 9420
   tacatggcac caagcaatga accatggggt gaacatgaaa ttgattacat cctattttat 9480
   aagatcaacg ctaaagaaaa cttgactgtc aacccaaacg tcaatgaagt tagagacttc 9540
   aaatgggttt caccaaatga tttgaaaact atgtttgctg acccaagtta caagtttacg 9600
   ccttggttta agattatttg cgagaattac ttattcaact ggtgggagca attagatgac 9660
   ctttctgaag tggaaaatga caggcaaatt catagaatgc tataacaacg cgtctacaaa 9720
   taaaaaaggc acgtcagatg acgtgccttt tttcttgggg ccggggatcc tctagagtcg 9780
   acctgcaggc atgcaagctt ggcgtaatca tggtcatagc tgtttcctgt gtgaaattgt 9840
   tatccgctca caattccaca caacatacga gccggaagca taaagtgtaa agcctggggt 9900
   gcctaatgag tgagctaact cacattaatt gcgttgcgct cactgcccgc tttccagtcg 9960
   ggaaacctgt cgtgccagct gcattaatga atcggccaac gcgcggggag aggcggtttg 10020
   cgtattgggc gctttctcat agctcacgct gtaggtatct cagttcggtg taggtcgttc 10080
   gctccaagct gggctgtgtg ccgaacccca gagtcccgct cagaagaact cgtcaagaag 10140
   gcgatagaag gcgatgcgct gcgaatcggg agcggcgata ccgtaaagca cgaggaagcg 10200
   gtcagcccat tcgccgccaa gctcttcagc aatatcacgg gtagccaacg ctatgtcctg 10260
   atagcggtcc gccacaccca gccggccaca gtcgatgaat ccagaaaagc ggccattttc 10320
   caccatgata ttcggcaagc aggcatcgcc atgggtcacg acgagatcct cgccgtcggg 10380
   catgcgcgcc ttgagcctgg cgaacagttc ggctggcgcg agcccctgat gctcttcgtc 10440
   cagatcatcc tgatcgacaa gaccggcttc catccgagta cgtgctcgct cgatgcgatg 10500
   tttcgcttgg tggtcgaatg ggcaggtagc cggatcaagc gtatgcagcc gccgcattgc 10560
   atcagccatg atggatactt tctcggcagg agcaaggtga gatgacagga gatcctgccc 10620
   cggcacttcg cccaatagca gccagtccct tcccgcttca gtgccaacgt cgagcacagc 10680
   tgcgcaagga acgcccgtcg tggccagcca cgatagccgc gctgcctcgt cctgcagttc 10740
   attcagggca ccggacaggt cggtcttgac aaaaagaacc gggcgcccct gcgctgacag 10800
   ccggaacacg gcggcatcag agcagccgat tgtctgttgt gcccagtcat agccgaatag 10860
   cctctccacc caagcggccg gagaacctgc gtgcaatcca tcttgttcaa tcatgcgaaa 10920
   cgatcctcat cctgtctctt gatcactacc gcattaaagc atatcgatga taagctgtca 10980
   aacatgagcg ttcttcgggg cgaaaactct caaggatctt accgctgttg agatccagtt 11040
   cgatgtaacc cactcgtgca cccaactgat cttcagcatc ttttactttc accagcgttt 11100
   ctgggtgagc aaaaacagga aggcaaaatg ccgcaaaaaa gggaataagg gcgacacgga 11160
   aatgttgaat actcatactc ttcctttttc aatattattg aagcatttat cagggttatt 11220
   gtctcatgag cggatacata tttgaatgta tttagaaaaa taaacaaata ggggttccgc  11280
   gcacatttcc ccgaaaagtg ccacctgacg tctaagaaac cattattatc atgacattaa  11340
   cctataaaaa taggcgtatc acgaggccct ttcgtcttca agaatt                 11386
<210> 51
   <211> 12232
   <212> DNA
   <213> Artificial Sequence
<220>
   <223> Nucleotide sequence of prepared plasmid pMW-Tn7-Pgi-KKDyI-TaspA-Tn7
<400> 51
   gacagtaaga cgggtaagcc tgttgatgat accgctgcct tactgggtgc attagccagt 60
   ctgaatgacc tgtcacggga taatccgaag tggtcagact ggaaaatcag agggcaggaa 120
   ctgcagaaca gcaaaaagtc agatagcacc acatagcaga cccgccataa aacgccctga 180
   gaagcccgtg acgggctttt cttgtattat gggtagtttc cttgcatgaa tccataaaag 240
   gcgcctgtag tgccatttac ccccattcac tgccagagcc gtgagcgcag cgaactgaat 300
   gtcacgaaaa agacagcgac tcaggtgcct gatggtcgga gacaaaagga atattcagcg 360
   atttgcccga gcttgcgagg gtgctactta agcctttagg gttttaaggt ctgttttgta 420
   gaggagcaaa cagcgtttgc gacatccttt tgtaatactg cggaactgac taaagtagtg 480
   agttatacac agggctggga tctattcttt ttatcttttt ttattctttc tttattctat 540
   aaattataac cacttgaata taaacaaaaa aaacacacaa aggtctagcg gaatttacag 600
   agggtctagc agaatttaca agttttccag caaaggtcta gcagaattta cagataccca 660
   caactcaaag gaaaaggact agtaattatc attgactagc ccatctcaat tggtatagtg 720
   attaaaatca cctagaccaa ttgagatgta tgtctgaatt agttgttttc aaagcaaatg 780
   aactagcgat tagtcgctat gacttaacgg agcatgaaac caagctaatt ttatgctgtg 840
   tggcactact caaccccacg attgaaaacc ctacaaggaa agaacggacg gtatcgttca 900
   cttataacca atacgctcag atgatgaaca tcagtaggga aaatgcttat ggtgtattag 960
   ctaaagcaac cagagagctg atgacgagaa ctgtggaaat caggaatcct ttggttaaag 1020
   gctttgagat tttccagtgg acaaactatg ccaagttctc aagcgaaaaa ttagaattag 1080
   tttttagtga agagatattg ccttatcttt tccagttaaa aaaattcata aaatataatc 1140
   tggaacatgt taagtctttt gaaaacaaat actctatgag gatttatgag tggttattaa 1200
   aagaactaac acaaaagaaa actcacaagg caaatataga gattagcctt gatgaattta 1260
   agttcatgtt aatgcttgaa aataactacc atgagtttaa aaggcttaac caatgggttt 1320
   tgaaaccaat aagtaaagat ttaaacactt acagcaatat gaaattggtg gttgataagc 1380
   gaggccgccc gactgatacg ttgattttcc aagttgaact agatagacaa atggatctcg 1440
   taaccgaact tgagaacaac cagataaaaa tgaatggtga caaaatacca acaaccatta 1500
   catcagattc ctacctacat aacggactaa gaaaaacact acacgatgct ttaactgcaa 1560
   aaattcagct caccagtttt gaggcaaaat tttgagtgca catgcaaagt aagcatgatc 1620
   tcaatggttc gttctcatgg ctcacgcaaa aacaacgaac cacactagag aacatactgg 1680
   ctaaatacgg aaggatctga ggttcttatg gctcttgtat ctatcagtga agcatcaaga 1740
   ctaacaaaca aaagtagaac aactgttcac cgttacatat caaagggaaa actgtccata 1800
   tgcacagatg aaaacggtgt aaaaaagata gatacatcag agcttttacg agtttttggt 1860
   gcattcaaag ctgttcacca tgaacagatc gacaatgtaa cagatgaaca gcatgtaaca 1920
   cctaatagaa caggtgaaac cagtaaaaca aagcaactag aacatgaaat tgaacacctg 1980
   agacaacttg ttacagctca acagtcacac atagacagcc tgaaacaggc gatgctgctt 2040
   atcgaatcaa agctgccgac aacacgggag ccagtgacgc ctcccgtggg gaaaaaatca 2100
   tggcaattct ggaagaaata gcgccattcg ccattcaggc tgcgcaactg ttgggaaggg 2160
   cgatcggtgc gggcctcttc gctattacgc cagctggcga aagggggatg tgctgcaagg 2220
   cgattaagtt gggtaacgcc agggttttcc cagtcacgac gttgtaaaac gacggccagt 2280
   gaattcgagc tcggtaccct gtttttccac tcttcgttca ctttcgccag gtagctggtg 2340
   aagacgaagg aagtcccgga gccatctgcg cggcgtacta cagcaatgtt ttgtgaaggc 2400
   agtttcagac ccggattcag tttggcgatg gcttcatcat cccacttctt gattttgccc 2460
   aggtagatgt cgccgagggt tttaccatcc agcaccagtt cgccagactt cagccctgga 2520
   atgttaaccg ccagcaccac gccgccaatc acggtcggga actggaacag accttcctga 2580
   gccagttttt cgtcagacag cggcgcgtca gaggcaccaa aatcaacggt attagcgata 2640
   atctgtttta cgccaccgga agaaccgata ccctggtagt taactttatt accggtttct 2700
   ttctggtaag tgtcagccca tttggcatac accggcgcag ggaaggttgc acctgcacct 2760
   gtcaggcttg cttctgcaaa cacagagaaa gcactcatcg ataaggtcgc ggcgacaaca 2820
   gttgcgacgg tggtacgcat aactttcata atgtctcctg ggaggattca taaagcattg 2880
   tttgttggct acgagaagca aaataggaca aacaggtgac agttatatgt aaggaatatg 2940
   acagttttat gacagagaga taaagtcttc agtctgattt aaataagcgt tgatattcag 3000
   tcaattacaa acattaataa cgaagagatg acagaaaaat tttcattctg tgacagagaa 3060
   aaagtagccg aagatgacgg tttgtcacat ggagttggca ggatgtttga ttaaaatgaa 3120
   gcctgctttt ttatactaag ttggcattat aaaaaagcat tgcttatcaa tttgttgcaa 3180
   cgaacaggtc actatcagtc aaaataaaat cattatttga tttcgaattc tcatgtttga 3240
   cagcttatca tcgataagct ttaatgcggt agtttatcac agttaaattg ctaacgcagt 3300
   caggcaccgt gtatgaaatc taacaatgcg ctcatcgtca tcctcggcac cgtcaccctg 3360
   gatgctgtag gcataggctt ggttatgccg gtactgccgg gcctcttgcg ggatatcgtc 3420
   cattccgaca gcatcgccag tcactatggc gtgctgctag cgctatatgc gttgatgcaa 3480
   tttctatgcg cacccgttct cggagcactg tccgaccgct ttggccgccg cccagtcctg 3540
   ctcgcttcgc tacttggagc cactatcgac tacgcgatca tggcgaccac acccgtcctg 3600
   tggatctccg gataagtaga cagcctgata agtcgcacga aaaacaggta ttgacaacat 3660
   gaagtaacat gcagtaagat acaaatcgct aggtaacact agcagcgtca accgggcgct 3720
   ctagctagag ccaagctagc ttggccggat ccgagatttt caggagctaa ggaagctaaa 3780
   atggagaaaa aaatcactgg atataccacc gttgatatat cccaatggca tcgtaaagaa 3840
   cattttgagg catttcagtc agttgctcaa tgtacctata accagaccgt tcagctggat 3900
   attacggcct ttttaaagac cgtaaagaaa aataagcaca agttttatcc ggcctttatt 3960
   cacattcttg cccgcctgat gaatgctcat ccggaattcc gtatggcaat gaaagacggt 4020
   gagctggtga tatgggatag tgttcaccct tgttacaccg ttttccatga gcaaactgaa 4080
   acgttttcat cgctctggag tgaataccac gacgatttcc ggcagtttct acacatatat 4140
   tcgcaagatg tggcgtgtta cggtgaaaac ctggcctatt tccctaaagg gtttattgag 4200
   aatatgtttt tcgtctcagc caatccctgg gtgagtttca ccagttttga tttaaacgtg 4260
   gccaatatgg acaacttctt cgcccccgtt ttcaccatgg gcaaatatta tacgcaaggc 4320
   gacaaggtgc tgatgccgct ggcgattcag gttcatcatg ccgtctgtga tggcttccat 4380
   gtcggcagaa tgcttaatga attacaacag tactgcgatg agtggcaggg cggggcgtaa 4440
   tttttttaag gcagttattg gtgcccttaa acgcctggtg ctacgcctga ataagtgata 4500
   ataagcggat gaatggcaga aattcgtcga agcttaacac agaaaaaagc ccgcacctga 4560
   cagtgcgggc tttttttttc gaccactgca gtctgttaca ggtcactaat accatctaag 4620
   tagttgattc atagtgactg catatgttgt gttttacagt attatgtagt ctgtttttta 4680
   tgcaaaatct aatttaatat attgatattt atatcatttt acgtttctcg ttcagctttt 4740
   ttatactaac ttgagcggcc cttgacgatg ccacatcctg agcaaataat tcaaccacta 4800
   attgtgagcg gataacacaa ggaggaaaca gctatgtcat taccgttctt aacttctgca 4860
   ccgggaaagg ttattatttt tggtgaacac tctgctgtgt acaacaagcc tgccgtcgct 4920
   gctagtgtgt ctgcgttgag aacctacctg ctaataagcg agtcatctgc accagatact 4980
   attgaattgg acttcccgga cattagcttt aatcataagt ggtccatcaa tgatttcaat 5040
   gccatcaccg aggatcaagt aaactcccaa aaattggcca aggctcaaca agccaccgat 5100
   ggcttgtctc aggaactcgt tagtcttttg gatccgttgt tagctcaact atccgaatcc 5160
   ttccactacc atgcagcgtt ttgtttcctg tatatgtttg tttgcctatg cccccatgcc 5220
   aagaatatta agttttcctt aaagtctact ttacccatcg gtgctgggtt gggctcaagc 5280
   gcctctattt ctgtatcact ggccttagct atggcctact tgggggggtt aataggatct 5340
   aatgacttgg aaaagctgtc agaaaacgat aagcatatag tgaatcaatg ggccttcata 5400
   ggtgaaaagt gtattcacgg taccccttca ggaatagata acgctgtggc cacttatggt 5460
   aatgccctgc tatttgaaaa agactcacat aatggaacaa taaacacaaa caattttaag 5520
   ttcttagatg atttcccagc cattccaatg atcctaacct atactagaat tccaaggtct 5580
   acaaaagatc ttgttgctcg cgttcgtgtg ttggtcaccg agaaatttcc tgaagttatg 5640
   aagccaattc tagatgccat gggtgaatgt gccctacaag gcttagagat catgactaag 5700
   ttaagtaaat gtaaaggcac cgatgacgag gctgtagaaa ctaataatga actgtatgaa 5760
   caactattgg aattgataag aataaatcat ggactgcttg tctcaatcgg tgtttctcat 5820
   cctggattag aacttattaa aaatctgagc gatgatttga gaattggctc cacaaaactt 5880
   accggtgctg gtggcggcgg ttgctctttg actttgttac gaagagacat tactcaagag 5940
   caaattgaca gcttcaaaaa gaaattgcaa gatgatttta gttacgagac atttgaaaca 6000
   gacttgggtg ggactggctg ctgtttgtta agcgcaaaaa atttgaataa agatcttaaa 6060
   atcaaatccc tagtattcca attatttgaa aataaaacta ccacaaagca acaaattgac 6120
   gatctattat tgccaggaaa cacgaattta ccatggactt cataagctaa tttgcgatag 6180
   gcctgcaccc ttaaggagga aaaaaacatg tcagagttga gagccttcag tgccccaggg 6240
   aaagcgttac tagctggtgg atatttagtt ttagatacaa aatatgaagc atttgtagtc 6300
   ggattatcgg caagaatgca tgctgtagcc catccttacg gttcattgca agggtctgat 6360
   aagtttgaag tgcgtgtgaa aagtaaacaa tttaaagatg gggagtggct gtaccatata 6420
   agtcctaaaa gtggcttcat tcctgtttcg ataggcggat ctaagaaccc tttcattgaa 6480
   aaagttatcg ctaacgtatt tagctacttt aaacctaaca tggacgacta ctgcaataga 6540
   aacttgttcg ttattgatat tttctctgat gatgcctacc attctcagga ggatagcgtt 6600
   accgaacatc gtggcaacag aagattgagt tttcattcgc acagaattga agaagttccc 6660
   aaaacagggc tgggctcctc ggcaggttta gtcacagttt taactacagc tttggcctcc 6720
   ttttttgtat cggacctgga aaataatgta gacaaatata gagaagttat tcataattta 6780
   gcacaagttg ctcattgtca agctcagggt aaaattggaa gcgggtttga tgtagcggcg 6840
   gcagcatatg gatctatcag atatagaaga ttcccacccg cattaatctc taatttgcca 6900
   gatattggaa gtgctactta cggcagtaaa ctggcgcatt tggttgatga agaagactgg 6960
   aatattacga ttaaaagtaa ccatttacct tcgggattaa ctttatggat gggcgatatt 7020
   aagaatggtt cagaaacagt aaaactggtc cagaaggtaa aaaattggta tgattcgcat 7080
   atgccagaaa gcttgaaaat atatacagaa ctcgatcatg caaattctag atttatggat 7140
   ggactatcta aactagatcg cttacacgag actcatgacg attacagcga tcagatattt 7200
   gagtctcttg agaggaatga ctgtacctgt caaaagtatc ctgaaatcac agaagttaga 7260
   gatgcagttg ccacaattag acgttccttt agaaaaataa ctaaagaatc tggtgccgat 7320
   atcgaacctc ccgtacaaac tagcttattg gatgattgcc agaccttaaa aggagttctt 7380
   acttgcttaa tacctggtgc tggtggttat gacgccattg cagtgattac taagcaagat 7440
   gttgatctta gggctcaaac cgctaatgac aaaagatttt ctaaggttca atggctggat 7500
   gtaactcagg ctgactgggg tgttaggaaa gaaaaagatc cggaaactta tcttgataaa 7560
   taacttaagg tagctgcatg cagaattcgc ccttaaggag gaaaaaaaaa tgaccgttta 7620
   cacagcatcc gttaccgcac ccgtcaacat cgcaaccctt aagtattggg ggaaaaggga 7680
   cacgaagttg aatctgccca ccaattcgtc catatcagtg actttatcgc aagatgacct 7740
   cagaacgttg acctctgcgg ctactgcacc tgagtttgaa cgcgacactt tgtggttaaa 7800
   tggagaacca cacagcatcg acaatgaaag aactcaaaat tgtctgcgcg acctacgcca 7860
   attaagaaag gaaatggaat cgaaggacgc ctcattgccc acattatctc aatggaaact 7920
   ccacattgtc tccgaaaata actttcctac agcagctggt ttagcttcct ccgctgctgg 7980
   ctttgctgca ttggtctctg caattgctaa gttataccaa ttaccacagt caacttcaga 8040
   aatatctaga atagcaagaa aggggtctgg ttcagcttgt agatcgttgt ttggcggata 8100
   cgtggcctgg gaaatgggaa aagctgaaga tggtcatgat tccatggcag tacaaatcgc 8160
   agacagctct gactggcctc agatgaaagc ttgtgtccta gttgtcagcg atattaaaaa 8220
   ggatgtgagt tccactcagg gtatgcaatt gaccgtggca acctccgaac tatttaaaga 8280
   aagaattgaa catgtcgtac caaagagatt tgaagtcatg cgtaaagcca ttgttgaaaa 8340
   agatttcgcc acctttgcaa aggaaacaat gatggattcc aactctttcc atgccacatg 8400
   tttggactct ttccctccaa tattctacat gaatgacact tccaagcgta tcatcagttg 8460
   gtgccacacc attaatcagt tttacggaga aacaatcgtt gcatacacgt ttgatgcagg 8520
   tccaaatgct gtgttgtact acttagctga aaatgagtcg aaactctttg catttatcta 8580
   taaattgttt ggctctgttc ctggatggga caagaaattt actactgagc agcttgaggc 8640
   tttcaaccat caatttgaat catctaactt tactgcacgt gaattggatc ttgagttgca 8700
   aaaggatgtt gccagagtga ttttaactca agtcggttca ggcccacaag aaacaaacga 8760
   atctttgatt gacgcaaaga ctggtctacc aaaggaataa gatcaattcg ctgcatcgcc 8820
   cttaggaggt aaaaaaaaat gactgccgac aacaatagta tgccccatgg tgcagtatct 8880
   agttacgcca aattagtgca aaaccaaaca cctgaagaca ttttggaaga gtttcctgaa 8940
   attattccat tacaacaaag acctaatacc cgatctagtg agacgtcaaa tgacgaaagc 9000
   ggagaaacat gtttttctgg tcatgatgag gagcaaatta agttaatgaa tgaaaattgt 9060
   attgttttgg attgggacga taatgctatt ggtgccggta ccaagaaagt ttgtcattta 9120
   atggaaaata ttgaaaaggg tttactacat cgtgcattct ccgtctttat tttcaatgaa 9180
   caaggtgaat tacttttaca acaaagagcc actgaaaaaa taactttccc tgatctttgg 9240
   actaacacat gctgctctca tccactatgt attgatgacg aattaggttt gaagggtaag 9300
   ctagacgata agattaaggg cgctattact gcggcggtga gaaaactaga tcatgaatta 9360
   ggtattccag aagatgaaac taagacaagg ggtaagtttc actttttaaa cagaatccat 9420
   tacatggcac caagcaatga accatggggt gaacatgaaa ttgattacat cctattttat 9480
   aagatcaacg ctaaagaaaa cttgactgtc aacccaaacg tcaatgaagt tagagacttc 9540
   aaatgggttt caccaaatga tttgaaaact atgtttgctg acccaagtta caagtttacg 9600
   ccttggttta agattatttg cgagaattac ttattcaact ggtgggagca attagatgac 9660
   ctttctgaag tggaaaatga caggcaaatt catagaatgc tataacaacg cgtctacaaa 9720
   taaaaaaggc acgtcagatg acgtgccttt tttcttgggg ccggggatcc tctagagtcg 9780
   aaagaaaaat gccccgctta cgcagggcat ccatttatta ctcaaccgta accgattttg 9840
   ccaggttacg cggctggtca acgtcggtgc ctttgatcag cgcgacatgg taagccagca 9900
   gctgcagcgg aacggtgtag aagatcggtg caatcacctc ttccacatgc ggcatctcga 9960
   tgatgtgcat gttatcgcta cttacaaaac ccgcatcctg atcggcgaag acatacaact 10020
   gaccgccacg cgcgcgaact tcttcaatgt tggatttcag tttttccagc aattcgttgt 10080
   tcggtgcaac aacaataacc ggcatatcgg catcaattag cgccagcgga ccgtgtttca 10140
   gttcgccagc agcgtaggct tcagcgtgaa tgtaagagat ctctttcaac ttcaatgcgc 10200
   cttccagcgc gattgggtac tgatcgccac ggcccaggaa cagcgcgtga tgtttgtcag 10260
   agaaatcttc tgccagcgct tcaatgcgtt tgtcctgaga cagcatctgc tcaatacggc 10320
   tcggcagcgc ctgcagacca tgcacgatgt catgttcaat ggaggcatcc agacctttca 10380
   ggcgagacag cttcgccacc agcatcaaca gcacagttaa ctgagtggtg aatgctttag 10440
   tggatgccac gccgatttct gtacccgcgt tggtcattag cgccagatcg gattcgcgca 10500
   ccagagaaga acccggaacg ttacagattg ccagtgaacc aaggtaaccc agctctttcg 10560
   acagacgcag gccagccagg gtatccgcgg tttcgccaga ctgtgacagg ggatcctcta 10620
   gagtcgacct gcaggcatgc aagcttggcg taatcatggt catagctgtt tcctgtgtga 10680
   aattgttatc cgctcacaat tccacacaac atacgagccg gaagcataaa gtgtaaagcc 10740
   tggggtgcct aatgagtgag ctaactcaca ttaattgcgt tgcgctcact gcccgctttc 10800
   cagtcgggaa acctgtcgtg ccagctgcat taatgaatcg gccaacgcgc ggggagaggc 10860
   ggtttgcgta ttgggcgctt tctcatagct cacgctgtag gtatctcagt tcggtgtagg 10920
   tcgttcgctc caagctgggc tgtgtgccga accccagagt cccgctcaga agaactcgtc 10980
   aagaaggcga tagaaggcga tgcgctgcga atcgggagcg gcgataccgt aaagcacgag 11040
   gaagcggtca gcccattcgc cgccaagctc ttcagcaata tcacgggtag ccaacgctat 11100
   gtcctgatag cggtccgcca cacccagccg gccacagtcg atgaatccag aaaagcggcc 11160
   attttccacc atgatattcg gcaagcaggc atcgccatgg gtcacgacga gatcctcgcc 11220
   gtcgggcatg cgcgccttga gcctggcgaa cagttcggct ggcgcgagcc cctgatgctc 11280
   ttcgtccaga tcatcctgat cgacaagacc ggcttccatc cgagtacgtg ctcgctcgat 11340
   gcgatgtttc gcttggtggt cgaatgggca ggtagccgga tcaagcgtat gcagccgccg 11400
   cattgcatca gccatgatgg atactttctc ggcaggagca aggtgagatg acaggagatc 11460
   ctgccccggc acttcgccca atagcagcca gtcccttccc gcttcagtgc caacgtcgag 11520
   cacagctgcg caaggaacgc ccgtcgtggc cagccacgat agccgcgctg cctcgtcctg 11580
   cagttcattc agggcaccgg acaggtcggt cttgacaaaa agaaccgggc gcccctgcgc 11640
   tgacagccgg aacacggcgg catcagagca gccgattgtc tgttgtgccc agtcatagcc 11700
   gaatagcctc tccacccaag cggccggaga acctgcgtgc aatccatctt gttcaatcat 11760
   gcgaaacgat cctcatcctg tctcttgatc actaccgcat taaagcatat cgatgataag 11820
   ctgtcaaaca tgagcgttct tcggggcgaa aactctcaag gatcttaccg ctgttgagat 11880
   ccagttcgat gtaacccact cgtgcaccca actgatcttc agcatctttt actttcacca 11940
   gcgtttctgg gtgagcaaaa acaggaaggc aaaatgccgc aaaaaaggga ataagggcga 12000
   cacggaaatg ttgaatactc atactcttcc tttttcaata ttattgaagc atttatcagg 12060
   gttattgtct catgagcgga tacatatttg aatgtattta gaaaaataaa caaatagggg 12120
   ttccgcgcac atttccccga aaagtgccac ctgacgtcta agaaaccatt attatcatga 12180
   cattaaccta taaaaatagg cgtatcacga ggccctttcg tcttcaagaa tt 12232
<210> 52
   <211> 9175
   <212> DNA
   <213> Artificial Sequence
<220>
   <223> Nucleotide sequence containing genomic sequence encoding downstream enzymes in the mevalonate pathway
<400> 52
   ttagtacagc ggcttaccgc tactgtcttt aatattggtc ttccacgcag cgcgaacctg 60
   ttcaactaca ctatccggca ggctggcgta atccaggtcg ttcgcctgtt tagccccggt 120
   tttgtacgcc cagtcgaaga atttcagcac ttctgtgcct tgttctggtt tcttctgatc 180
   tttgtggatc agaatgaacg tggtagaggt aataggccat gcatcttcgc ctttctggtt     240
   ggtcagatcc tgagcgaagg ttttgctcca gtctgcacct tttgctgcat tagcgaagtt     300
   ttcttcggtc ggactaaccg gtttaccatc agcggagatc agtttggtgt acgccaggtt     360
   gttctgcttc gcgtaagcat attcaacata accaattgca cccggcagac gctgaacgaa     420
   cgcggcgata ccgtcgttac ctttaccgcc cagaccgatc ggccatttta cggtagagcc     480
   agtaccaacg ttgtttttcc actcttcgtt cactttcgcc aggtagctgg tgaagacgaa     540
   ggaagtcccg gagccatctg cgcggcgtac tacagcaatg ttttgtgaag gcagtttcag     600
   acccggattc agtttggcga tggcttcatc atcccacttc ttgattttgc ccaggtagat     660
   gtcgccgagg gttttaccat ccagcaccag ttcgccagac ttcagccctg gaatgttaac     720
   cgccagcacc acgccgccaa tcacggtcgg gaactggaac agaccttcct gagccagttt     780
   ttcgtcagac agcggcgcgt cagaggcacc aaaatcaacg gtattagcga taatctgttt     840
   tacgccaccg gaagaaccga taccctggta gttaacttta ttaccggttt ctttctggta     900
   agtgtcagcc catttggcat acaccggcgc agggaaggtt gcacctgcac ctgtcaggct     960
   tgcttctgca aacacagaga aagcactcat cgataaggtc gcggcgacaa cagttgcgac    1020
   ggtggtacgc ataactttca taatgtctcc tgggaggatt cataaagcat tgtttgttgg    1080
   ctacgagaag caaaatagga caaacaggtg acagttatat gtaaggaata tgacagtttt    1140
   atgacagaga gataaagtct tcagtctgat ttaaataagc gttgatattc agtcaattac    1200
   aaacattaat aacgaagaga tgacagaaaa attttcattc tgtgacagag aaaaagtagc    1260
   cgaagatgac ggtttgtcac atggagttgg caggatgttt gattaaaatg aagcctgctt    1320
   ttttatacta agttggcatt ataaaaaagc attgcttatc aatttgttgc aacgaacagg    1380
   tcactatcag tcaaaataaa atcattattt gatttcgaat tctcatgttt gacagcttat    1440
   catcgataag ctttaatgcg gtagtttatc acagttaaat tgctaacgca gtcaggcacc    1500
   gtgtatgaaa tctaacaatg cgctcatcgt catcctcggc accgtcaccc tggatgctgt    1560
   aggcataggc ttggttatgc cggtactgcc gggcctcttg cgggatatcg tccattccga    1620
   cagcatcgcc agtcactatg gcgtgctgct agcgctatat gcgttgatgc aatttctatg    1680
   cgcacccgtt ctcggagcac tgtccgaccg ctttggccgc cgcccagtcc tgctcgcttc    1740
   gctacttgga gccactatcg actacgcgat catggcgacc acacccgtcc tgtggatctc    1800
   cggataagta gacagcctga taagtcgcac gaaaaacagg tattgacaac atgaagtaac    1860
   atgcagtaag atacaaatcg ctaggtaaca ctagcagcgt caaccgggcg ctctagctag    1920
   agccaagcta gcttggccgg atccgagatt ttcaggagct aaggaagcta aaatggagaa    1980
   aaaaatcact ggatatacca ccgttgatat atcccaatgg catcgtaaag aacattttga    2040
   ggcatttcag tcagttgctc aatgtaccta taaccagacc gttcagctgg atattacggc 2100
   ctttttaaag accgtaaaga aaaataagca caagttttat ccggccttta ttcacattct 2160
   tgcccgcctg atgaatgctc atccggaatt ccgtatggca atgaaagacg gtgagctggt 2220
   gatatgggat agtgttcacc cttgttacac cgttttccat gagcaaactg aaacgttttc 2280
   atcgctctgg agtgaatacc acgacgattt ccggcagttt ctacacatat attcgcaaga 2340
   tgtggcgtgt tacggtgaaa acctggccta tttccctaaa gggtttattg agaatatgtt 2400
   tttcgtctca gccaatccct gggtgagttt caccagtttt gatttaaacg tggccaatat 2460
   ggacaacttc ttcgcccccg ttttcaccat gggcaaatat tatacgcaag gcgacaaggt 2520
   gctgatgccg ctggcgattc aggttcatca tgccgtctgt gatggcttcc atgtcggcag 2580
   aatgcttaat gaattacaac agtactgcga tgagtggcag ggcggggcgt aattttttta 2640
   aggcagttat tggtgccctt aaacgcctgg tgctacgcct gaataagtga taataagcgg 2700
   atgaatggca gaaattcgtc gaagcttaac acagaaaaaa gcccgcacct gacagtgcgg 2760
   gctttttttt tcgaccactg cagtctgtta caggtcacta ataccatcta agtagttgat 2820
   tcatagtgac tgcatatgtt gtgttttaca gtattatgta gtctgttttt tatgcaaaat 2880
   ctaatttaat atattgatat ttatatcatt ttacgtttct cgttcagctt ttttatacta 2940
   acttgagcgg cccttgacga tgccacatcc tgagcaaata attcaaccac taattgtgag 3000
   cggataacac aaggaggaaa cagctatgtc attaccgttc ttaacttctg caccgggaaa 3060
   ggttattatt tttggtgaac actctgctgt gtacaacaag cctgccgtcg ctgctagtgt 3120
   gtctgcgttg agaacctacc tgctaataag cgagtcatct gcaccagata ctattgaatt 3180
   ggacttcccg gacattagct ttaatcataa gtggtccatc aatgatttca atgccatcac 3240
   cgaggatcaa gtaaactccc aaaaattggc caaggctcaa caagccaccg atggcttgtc 3300
   tcaggaactc gttagtcttt tggatccgtt gttagctcaa ctatccgaat ccttccacta 3360
   ccatgcagcg ttttgtttcc tgtatatgtt tgtttgccta tgcccccatg ccaagaatat 3420
   taagttttcc ttaaagtcta ctttacccat cggtgctggg ttgggctcaa gcgcctctat 3480
   ttctgtatca ctggccttag ctatggccta cttggggggg ttaataggat ctaatgactt 3540
   ggaaaagctg tcagaaaacg ataagcatat agtgaatcaa tgggccttca taggtgaaaa 3600
   gtgtattcac ggtacccctt caggaataga taacgctgtg gccacttatg gtaatgccct 3660
   gctatttgaa aaagactcac ataatggaac aataaacaca aacaatttta agttcttaga 3720
   tgatttccca gccattccaa tgatcctaac ctatactaga attccaaggt ctacaaaaga 3780
   tcttgttgct cgcgttcgtg tgttggtcac cgagaaattt cctgaagtta tgaagccaat 3840
   tctagatgcc atgggtgaat gtgccctaca aggcttagag atcatgacta agttaagtaa 3900
   atgtaaaggc accgatgacg aggctgtaga aactaataat gaactgtatg aacaactatt 3960
   ggaattgata agaataaatc atggactgct tgtctcaatc ggtgtttctc atcctggatt 4020
   agaacttatt aaaaatctga gcgatgattt gagaattggc tccacaaaac ttaccggtgc 4080
   tggtggcggc ggttgctctt tgactttgtt acgaagagac attactcaag agcaaattga 4140
   cagcttcaaa aagaaattgc aagatgattt tagttacgag acatttgaaa cagacttggg 4200
   tgggactggc tgctgtttgt taagcgcaaa aaatttgaat aaagatctta aaatcaaatc 4260
   cctagtattc caattatttg aaaataaaac taccacaaag caacaaattg acgatctatt 4320
   attgccagga aacacgaatt taccatggac ttcataagct aatttgcgat aggcctgcac 4380
   ccttaaggag gaaaaaaaca tgtcagagtt gagagccttc agtgccccag ggaaagcgtt 4440
   actagctggt ggatatttag ttttagatac aaaatatgaa gcatttgtag tcggattatc 4500
   ggcaagaatg catgctgtag cccatcctta cggttcattg caagggtctg ataagtttga 4560
   agtgcgtgtg aaaagtaaac aatttaaaga tggggagtgg ctgtaccata taagtcctaa 4620
   aagtggcttc attcctgttt cgataggcgg atctaagaac cctttcattg aaaaagttat 4680
   cgctaacgta tttagctact ttaaacctaa catggacgac tactgcaata gaaacttgtt 4740
   cgttattgat attttctctg atgatgccta ccattctcag gaggatagcg ttaccgaaca 4800
   tcgtggcaac agaagattga gttttcattc gcacagaatt gaagaagttc ccaaaacagg 4860
   gctgggctcc tcggcaggtt tagtcacagt tttaactaca gctttggcct ccttttttgt 4920
   atcggacctg gaaaataatg tagacaaata tagagaagtt attcataatt tagcacaagt 4980
   tgctcattgt caagctcagg gtaaaattgg aagcgggttt gatgtagcgg cggcagcata 5040
   tggatctatc agatatagaa gattcccacc cgcattaatc tctaatttgc cagatattgg 5100
   aagtgctact tacggcagta aactggcgca tttggttgat gaagaagact ggaatattac 5160
   gattaaaagt aaccatttac cttcgggatt aactttatgg atgggcgata ttaagaatgg 5220
   ttcagaaaca gtaaaactgg tccagaaggt aaaaaattgg tatgattcgc atatgccaga 5280
   aagcttgaaa atatatacag aactcgatca tgcaaattct agatttatgg atggactatc 5340
   taaactagat cgcttacacg agactcatga cgattacagc gatcagatat ttgagtctct 5400
   tgagaggaat gactgtacct gtcaaaagta tcctgaaatc acagaagtta gagatgcagt 5460
   tgccacaatt agacgttcct ttagaaaaat aactaaagaa tctggtgccg atatcgaacc 5520
   tcccgtacaa actagcttat tggatgattg ccagacctta aaaggagttc ttacttgctt 5580
   aatacctggt gctggtggtt atgacgccat tgcagtgatt actaagcaag atgttgatct 5640
   tagggctcaa accgctaatg acaaaagatt ttctaaggtt caatggctgg atgtaactca 5700
   ggctgactgg ggtgttagga aagaaaaaga tccggaaact tatcttgata aataacttaa 5760
   ggtagctgca tgcagaattc gcccttaagg aggaaaaaaa aatgaccgtt tacacagcat 5820
   ccgttaccgc acccgtcaac atcgcaaccc ttaagtattg ggggaaaagg gacacgaagt 5880
   tgaatctgcc caccaattcg tccatatcag tgactttatc gcaagatgac ctcagaacgt 5940
   tgacctctgc ggctactgca cctgagtttg aacgcgacac tttgtggtta aatggagaac 6000
   cacacagcat cgacaatgaa agaactcaaa attgtctgcg cgacctacgc caattaagaa 6060
   aggaaatgga atcgaaggac gcctcattgc ccacattatc tcaatggaaa ctccacattg 6120
   tctccgaaaa taactttcct acagcagctg gtttagcttc ctccgctgct ggctttgctg 6180
   cattggtctc tgcaattgct aagttatacc aattaccaca gtcaacttca gaaatatcta 6240
   gaatagcaag aaaggggtct ggttcagctt gtagatcgtt gtttggcgga tacgtggcct 6300
   gggaaatggg aaaagctgaa gatggtcatg attccatggc agtacaaatc gcagacagct 6360
   ctgactggcc tcagatgaaa gcttgtgtcc tagttgtcag cgatattaaa aaggatgtga 6420
   gttccactca gggtatgcaa ttgaccgtgg caacctccga actatttaaa gaaagaattg 6480
   aacatgtcgt accaaagaga tttgaagtca tgcgtaaagc cattgttgaa aaagatttcg 6540
   ccacctttgc aaaggaaaca atgatggatt ccaactcttt ccatgccaca tgtttggact 6600
   ctttccctcc aatattctac atgaatgaca cttccaagcg tatcatcagt tggtgccaca 6660
   ccattaatca gttttacgga gaaacaatcg ttgcatacac gtttgatgca ggtccaaatg 6720
   ctgtgttgta ctacttagct gaaaatgagt cgaaactctt tgcatttatc tataaattgt 6780
   ttggctctgt tcctggatgg gacaagaaat ttactactga gcagcttgag gctttcaacc 6840
   atcaatttga atcatctaac tttactgcac gtgaattgga tcttgagttg caaaaggatg 6900
   ttgccagagt gattttaact caagtcggtt caggcccaca agaaacaaac gaatctttga 6960
   ttgacgcaaa gactggtcta ccaaaggaat aagatcaatt cgctgcatcg cccttaggag 7020
   gtaaaaaaaa atgactgccg acaacaatag tatgccccat ggtgcagtat ctagttacgc 7080
   caaattagtg caaaaccaaa cacctgaaga cattttggaa gagtttcctg aaattattcc 7140
   attacaacaa agacctaata cccgatctag tgagacgtca aatgacgaaa gcggagaaac 7200
   atgtttttct ggtcatgatg aggagcaaat taagttaatg aatgaaaatt gtattgtttt 7260
   ggattgggac gataatgcta ttggtgccgg taccaagaaa gtttgtcatt taatggaaaa 7320
   tattgaaaag ggtttactac atcgtgcatt ctccgtcttt attttcaatg aacaaggtga 7380
   attactttta caacaaagag ccactgaaaa aataactttc cctgatcttt ggactaacac 7440
   atgctgctct catccactat gtattgatga cgaattaggt ttgaagggta agctagacga 7500
   taagattaag ggcgctatta ctgcggcggt gagaaaacta gatcatgaat taggtattcc 7560
   agaagatgaa actaagacaa ggggtaagtt tcacttttta aacagaatcc attacatggc 7620
   accaagcaat gaaccatggg gtgaacatga aattgattac atcctatttt ataagatcaa 7680
   cgctaaagaa aacttgactg tcaacccaaa cgtcaatgaa gttagagact tcaaatgggt 7740
   ttcaccaaat gatttgaaaa ctatgtttgc tgacccaagt tacaagttta cgccttggtt 7800
   taagattatt tgcgagaatt acttattcaa ctggtgggag caattagatg acctttctga 7860
   agtggaaaat gacaggcaaa ttcatagaat gctataacaa cgcgtctaca aataaaaaag 7920
   gcacgtcaga tgacgtgcct tttttcttgg ggccgaaaaa tgccccgctt acgcagggca 7980
   tccatttatt actcaaccgt aaccgatttt gccaggttac gcggctggtc aacgtcggtg 8040
   cctttgatca gcgcgacatg gtaagccagc agctgcagcg gaacggtgta gaagatcggt 8100
   gcaatcacct cttccacatg cggcatctcg atgatgtgca tgttatcgct acttacaaaa 8160
   cccgcatcct gatcggcgaa gacatacaac tgaccgccac gcgcgcgaac ttcttcaatg 8220
   ttggatttca gtttttccag caattcgttg ttcggtgcaa caacaataac cggcatatcg 8280
   gcatcaatta gcgccagcgg accgtgtttc agttcgccag cagcgtaggc ttcagcgtga 8340
   atgtaagaga tctctttcaa cttcaatgcg ccttccagcg cgattgggta ctgatcgcca 8400
   cggcccagga acagcgcgtg atgtttgtca gagaaatctt ctgccagcgc ttcaatgcgt 8460
   ttgtcctgag acagcatctg ctcaatacgg ctcggcagcg cctgcagacc atgcacgatg 8520
   tcatgttcaa tggaggcatc cagacctttc aggcgagaca gcttcgccac cagcatcaac 8580
   agcacagtta actgagtggt gaatgcttta gtggatgcca cgccgatttc tgtacccgcg 8640
   ttggtcatta gcgccagatc ggattcgcgc accagagaag aacccggaac gttacagatt 8700
   gccagtgaac caaggtaacc cagctctttc gacagacgca ggccagccag ggtatccgcg 8760
   gtttcgccag actgtgacaa ggtgatcatc aggctgttac gacgcacggc agatttgcga 8820
   tagcggaatt cagaggcgat ttcgacgtcg cacggaatac ctgctagcga ttcaaaccag 8880
   tagcgggaaa ccataccgga gttataagaa gtaccacagg cgaggatctg aatatgctca 8940
   accttcgaca gcagttcgtc ggcgttcggt cccagctcgc ttaaatcaac ctgaccgtgg 9000
   ctgatgcgtc cggtaagggt gtttttgatc gcgttcggct gttcgtagat ctctttctgc 9060
   atgtagtgac ggtaaatgcc tttatcgccc gcgtcatatt gcagattgga ttcgatatcc 9120
   tgacgtttta cttccgcgcc agttttatcg aagatgttta ccgaacggcg agtga 9175
<210> 53
   <211> 40
   <212> DNA
   <213> Artificial Sequence
<220>
   <223> Primer (Tn7dS_6038-7-1)
<400> 53
   tcgagctcgg taccctgttt ttccactctt cgttcacttt 40
<210> 54
   <211> 33
   <212> DNA
   <213> Artificial Sequence
<220>
   <223> Primer (Tn7dA_6038-7-2)
<400> 54
   aggcttcatt ttaatcaaac atcctgccaa ctc 33
<210> 55
   <211> 25
   <212> DNA
   <213> Artificial Sequence
<220>
   <223> Primer (Tn7dattLcmS_6038-7-4)
<400> 55
   attaaaatga agcctgcttt tttat 25
<210> 56
   <211> 32
   <212> DNA
   <213> Artificial Sequence
<220>
   <223> Primer (PgiattRcmA_6038-7-5)
<400> 56
   ggcatcgtca agggccgctc aagttagtat aa 32
<210> 57
   <211> 99
   <212> DNA
   <213> Artificial Sequence
<220>
   <223> Primer (gi1.2-MVK-S_6038-7-6)
<400> 57
   gcccttgacg atgccacatc ctgagcaaat aattcaacca ctaattgtga gcggataaca 60
   caaggaggaa acagctatgt cattaccgtt cttaacttc 99
<210> 58
   <211> 96
   <212> DNA
   <213> Artificial Sequence
<220>
   <223> Primer (pMW-TaspA-yIDIA_6038-7-7)
<400> 58
   ctctagagga tccccggccc caagaaaaaa ggcacgtcat ctgacgtgcc ttttttattt 60
   gtagacgcgt tgttatagca ttctatgaat ttgcct 96
<210> 59
   <211> 36
   <212> DNA
   <213> Artificial Sequence
<220>
   <223> Primer (Tn7upSv02_6038-24-1)
<400> 59
   atcctctaga gtcgaaagaa aaatgccccg cttacg 36
<210> 60
   <211> 37
   <212> DNA
   <213> Artificial Sequence
<220>
   <223> Primer (Tn7upAv02_6038-24-2)
<400> 60
   atgcctgcag gtcgactgtc acagtctggc gaaaccg 37
<210> 61
   <211> 20
   <212> DNA
   <213> Artificial Sequence
<220>
   <223> Primer (Tn7v02-F_6038-22-5)
<400> 61
   acgaactgct gtcgaaggtt 20
<210> 62
   <211> 20
   <212> DNA
   <213> Artificial Sequence
<220>
   <223> Primer (Tn7v02-R_6038-22-6)
<400> 62
   ggtgtacgcc aggttgttct 20
<210> 63
   <211> 2414
   <212> DNA
   <213> Artificial Sequence
<220>
   <223> Nucleotide sequence of genomic fragment containing attL-Tet-attR-Ptac
<400> 63
   tgaagcctgc ttttttatac taagttggca ttataaaaaa gcattgctta tcaatttgtt     60
   gcaacgaaca ggtcactatc agtcaaaata aaatcattat ttgatttcga attccccgga    120
   tccgtcgacc tgcagggaaa aaggttatgc tgcttttaag acccactttc acatttaagt    180
   tgtttttcta atccgcatat gatcaattca aggccgaata agaaggctgg ctctgcacct 240
   tggtgatcaa ataattcgat agcttgtcgt aataatggcg gcatactatc agtagtaggt 300
   gtttcccttt cttctttagc gacttgatgc tcttgatctt ccaatacgca acctaaagta 360
   aaatgcccca cagcgctgag tgcatataat gcattctcta gtgaaaaacc ttgttggcat 420
   aaaaaggcta attgattttc gagagtttca tactgttttt ctgtaggccg tgtacctaaa 480
   tgtacttttg ctccatcgcg atgacttagt aaagcacatc taaaactttt agcgttatta 540
   cgtaaaaaat cttgccagct ttccccttct aaagggcaaa agtgagtatg gtgcctatct 600
   aacatctcaa tggctaaggc gtcgagcaaa gcccgcttat tttttacatg ccaatacaat 660
   gtaggctgct ctacacctag cttctgggcg agtttacggg ttgttaaacc ttcgattccg 720
   acctcattaa gcagctctaa tgcgctgtta atcactttac ttttatctaa tctagacatc 780
   attaattcct aatttttgtt gacactctat cattgataga gttattttac cactccctat 840
   cagtgataga gaaaagtgaa atgaatagtt cgacaaagat cgcattggta attacgttac 900
   tcgatgccat ggggattggc cttatcatgc cagtcttgcc aacgttatta cgtgaattta 960
   ttgcttcgga agatatcgct aaccactttg gcgtattgct tgcactttat gcgttaatgc 1020
   aggttatctt tgctccttgg cttggaaaaa tgtctgaccg atttggtcgg cgcccagtgc 1080
   tgttgttgtc attaataggc gcatcgctgg attacttatt gctggctttt tcaagtgcgc 1140
   tttggatgct gtatttaggc cgtttgcttt cagggatcac aggagctact ggggctgtcg 1200
   cggcatcggt cattgccgat accacctcag cttctcaacg cgtgaagtgg ttcggttggt 1260
   taggggcaag ttttgggctt ggtttaatag cggggcctat tattggtggt tttgcaggag 1320
   agatttcacc gcatagtccc ttttttatcg ctgcgttgct aaatattgtc actttccttg 1380
   tggttatgtt ttggttccgt gaaaccaaaa atacacgtga taatacagat accgaagtag 1440
   gggttgagac gcaatcgaat tcggtataca tcactttatt taaaacgatg cccattttgt 1500
   tgattattta tttttcagcg caattgatag gccaaattcc cgcaacggtg tgggtgctat 1560
   ttaccgaaaa tcgttttgga tggaatagca tgatggttgg cttttcatta gcgggtcttg 1620
   gtcttttaca ctcagtattc caagcctttg tggcaggaag aatagccact aaatggggcg 1680
   aaaaaacggc agtactgctc gaatttattg cagatagtag tgcatttgcc tttttagcgt 1740
   ttatatctga aggttggtta gatttccctg ttttaatttt attggctggt ggtgggatcg 1800
   ctttacctgc attacaggga gtgatgtcta tccaaacaaa gagtcatgag caaggtgctt 1860
   tacagggatt attggtgagc cttaccaatg caaccggtgt tattggccca ttactgttta 1920
   ctgttattta taatcattca ctaccaattt gggatggctg gatttggatt attggtttag 1980
   cgttttactg tattattatc ctgctatcga tgaccttcat gttaacccct caagctcagg 2040
   ggagtaaaca ggagacaagt gcttagttat ttcgtcacca aatgatgtta ttccgcgaaa   2100
   tataatgacc ctcttgataa cccaagaggg cattttttac gataaagaag atttagcttc   2160
   tgcagtctgt tacaggtcac taataccatc taagtagttg attcatagtg actgcatatg   2220
   ttgtgtttta cagtattatg tagtctgttt tttatgcaaa atctaattta atatattgat   2280
   atttatatca ttttacgttt ctcgttcagc ttttttatac taacttgagc gagatctccc   2340
   tgttgacaat taatcatcgg ctctataatg tgtggaatcg tgagcggata acaatttcac   2400
   acaaggagac tgcc                                                     2414
<210> 64
   <211> 7464
   <212> DNA
   <213> Artificial Sequence
<220>
   <223> Nucleotide sequence containing genomic sequence encoding downstream enzymes in the mevalonate pathway under control of tac promoter
<400> 64
   ttagtacagc ggcttaccgc tactgtcttt aatattggtc ttccacgcag cgcgaacctg     60
   ttcaactaca ctatccggca ggctggcgta atccaggtcg ttcgcctgtt tagccccggt    120
   tttgtacgcc cagtcgaaga atttcagcac ttctgtgcct tgttctggtt tcttctgatc    180
   tttgtggatc agaatgaacg tggtagaggt aataggccat gcatcttcgc ctttctggtt    240
   ggtcagatcc tgagcgaagg ttttgctcca gtctgcacct tttgctgcat tagcgaagtt    300
   ttcttcggtc ggactaaccg gtttaccatc agcggagatc agtttggtgt acgccaggtt    360
   gttctgcttc gcgtaagcat attcaacata accaattgca cccggcagac gctgaacgaa    420
   cgcggcgata ccgtcgttac ctttaccgcc cagaccgatc ggccatttta cggtagagcc    480
   agtaccaacg ttgtttttcc actcttcgtt cactttcgcc aggtagctgg tgaagacgaa    540
   ggaagtcccg gagccatctg cgcggcgtac tacagcaatg ttttgtgaag gcagtttcag    600
   acccggattc agtttggcga tggcttcatc atcccacttc ttgattttgc ccaggtagat    660
   gtcgccgagg gttttaccat ccagcaccag ttcgccagac ttcagccctg gaatgttaac    720
   cgccagcacc acgccgccaa tcacggtcgg gaactggaac agaccttcct gagccagttt    780
   ttcgtcagac agcggcgcgt cagaggcacc aaaatcaacg gtattagcga taatctgttt    840
   tacgccaccg gaagaaccga taccctggta gttaacttta ttaccggttt ctttctggta    900
   agtgtcagcc catttggcat acaccggcgc agggaaggtt gcacctgcac ctgtcaggct    960
   tgcttctgca aacacagaga aagcactcat cgataaggtc gcggcgacaa cagttgcgac   1020
   ggtggtacgc ataactttca taatgtctcc tgggaggatt cataaagcat tgtttgttgg   1080
   ctacgagaag caaaatagga caaacaggtg acagttatat gtaaggaata tgacagtttt   1140
   atgacagaga gataaagtct tcagtctgat ttaaataagc gttgatattc agtcaattac 1200
   tgaagcctgc ttttttatac taacttgagc gagatctccc tgttgacaat taatcatcgg 1260
   ctctataatg tgtggaatcg tgagcggata acaatttcac acaaggagac tgccatgtca 1320
   ttaccgttct taacttctgc accgggaaag gttattattt ttggtgaaca ctctgctgtg 1380
   tacaacaagc ctgccgtcgc tgctagtgtg tctgcgttga gaacctacct gctaataagc 1440
   gagtcatctg caccagatac tattgaattg gacttcccgg acattagctt taatcataag 1500
   tggtccatca atgatttcaa tgccatcacc gaggatcaag taaactccca aaaattggcc 1560
   aaggctcaac aagccaccga tggcttgtct caggaactcg ttagtctttt ggatccgttg 1620
   ttagctcaac tatccgaatc cttccactac catgcagcgt tttgtttcct gtatatgttt 1680
   gtttgcctat gcccccatgc caagaatatt aagttttcct taaagtctac tttacccatc 1740
   ggtgctgggt tgggctcaag cgcctctatt tctgtatcac tggccttagc tatggcctac 1800
   ttgggggggt taataggatc taatgacttg gaaaagctgt cagaaaacga taagcatata 1860
   gtgaatcaat gggccttcat aggtgaaaag tgtattcacg gtaccccttc aggaatagat 1920
   aacgctgtgg ccacttatgg taatgccctg ctatttgaaa aagactcaca taatggaaca 1980
   ataaacacaa acaattttaa gttcttagat gatttcccag ccattccaat gatcctaacc 2040
   tatactagaa ttccaaggtc tacaaaagat cttgttgctc gcgttcgtgt gttggtcacc 2100
   gagaaatttc ctgaagttat gaagccaatt ctagatgcca tgggtgaatg tgccctacaa 2160
   ggcttagaga tcatgactaa gttaagtaaa tgtaaaggca ccgatgacga ggctgtagaa 2220
   actaataatg aactgtatga acaactattg gaattgataa gaataaatca tggactgctt 2280
   gtctcaatcg gtgtttctca tcctggatta gaacttatta aaaatctgag cgatgatttg 2340
   agaattggct ccacaaaact taccggtgct ggtggcggcg gttgctcttt gactttgtta 2400
   cgaagagaca ttactcaaga gcaaattgac agcttcaaaa agaaattgca agatgatttt 2460
   agttacgaga catttgaaac agacttgggt gggactggct gctgtttgtt aagcgcaaaa 2520
   aatttgaata aagatcttaa aatcaaatcc ctagtattcc aattatttga aaataaaact 2580
   accacaaagc aacaaattga cgatctatta ttgccaggaa acacgaattt accatggact 2640
   tcataagcta atttgcgata ggcctgcacc cttaaggagg aaaaaaacat gtcagagttg 2700
   agagccttca gtgccccagg gaaagcgtta ctagctggtg gatatttagt tttagataca 2760
   aaatatgaag catttgtagt cggattatcg gcaagaatgc atgctgtagc ccatccttac 2820
   ggttcattgc aagggtctga taagtttgaa gtgcgtgtga aaagtaaaca atttaaagat 2880
   ggggagtggc tgtaccatat aagtcctaaa agtggcttca ttcctgtttc gataggcgga 2940
   tctaagaacc ctttcattga aaaagttatc gctaacgtat ttagctactt taaacctaac 3000
   atggacgact actgcaatag aaacttgttc gttattgata ttttctctga tgatgcctac 3060
   cattctcagg aggatagcgt taccgaacat cgtggcaaca gaagattgag ttttcattcg 3120
   cacagaattg aagaagttcc caaaacaggg ctgggctcct cggcaggttt agtcacagtt 3180
   ttaactacag ctttggcctc cttttttgta tcggacctgg aaaataatgt agacaaatat 3240
   agagaagtta ttcataattt agcacaagtt gctcattgtc aagctcaggg taaaattgga 3300
   agcgggtttg atgtagcggc ggcagcatat ggatctatca gatatagaag attcccaccc 3360
   gcattaatct ctaatttgcc agatattgga agtgctactt acggcagtaa actggcgcat 3420
   ttggttgatg aagaagactg gaatattacg attaaaagta accatttacc ttcgggatta 3480
   actttatgga tgggcgatat taagaatggt tcagaaacag taaaactggt ccagaaggta 3540
   aaaaattggt atgattcgca tatgccagaa agcttgaaaa tatatacaga actcgatcat 3600
   gcaaattcta gatttatgga tggactatct aaactagatc gcttacacga gactcatgac 3660
   gattacagcg atcagatatt tgagtctctt gagaggaatg actgtacctg tcaaaagtat 3720
   cctgaaatca cagaagttag agatgcagtt gccacaatta gacgttcctt tagaaaaata 3780
   actaaagaat ctggtgccga tatcgaacct cccgtacaaa ctagcttatt ggatgattgc 3840
   cagaccttaa aaggagttct tacttgctta atacctggtg ctggtggtta tgacgccatt 3900
   gcagtgatta ctaagcaaga tgttgatctt agggctcaaa ccgctaatga caaaagattt 3960
   tctaaggttc aatggctgga tgtaactcag gctgactggg gtgttaggaa agaaaaagat 4020
   ccggaaactt atcttgataa ataacttaag gtagctgcat gcagaattcg cccttaagga 4080
   ggaaaaaaaa atgaccgttt acacagcatc cgttaccgca cccgtcaaca tcgcaaccct 4140
   taagtattgg gggaaaaggg acacgaagtt gaatctgccc accaattcgt ccatatcagt 4200
   gactttatcg caagatgacc tcagaacgtt gacctctgcg gctactgcac ctgagtttga 4260
   acgcgacact ttgtggttaa atggagaacc acacagcatc gacaatgaaa gaactcaaaa 4320
   ttgtctgcgc gacctacgcc aattaagaaa ggaaatggaa tcgaaggacg cctcattgcc 4380
   cacattatct caatggaaac tccacattgt ctccgaaaat aactttccta cagcagctgg 4440
   tttagcttcc tccgctgctg gctttgctgc attggtctct gcaattgcta agttatacca 4500
   attaccacag tcaacttcag aaatatctag aatagcaaga aaggggtctg gttcagcttg 4560
   tagatcgttg tttggcggat acgtggcctg ggaaatggga aaagctgaag atggtcatga 4620
   ttccatggca gtacaaatcg cagacagctc tgactggcct cagatgaaag cttgtgtcct 4680
   agttgtcagc gatattaaaa aggatgtgag ttccactcag ggtatgcaat tgaccgtggc 4740
   aacctccgaa ctatttaaag aaagaattga acatgtcgta ccaaagagat ttgaagtcat 4800
   gcgtaaagcc attgttgaaa aagatttcgc cacctttgca aaggaaacaa tgatggattc 4860
   caactctttc catgccacat gtttggactc tttccctcca atattctaca tgaatgacac 4920
   ttccaagcgt atcatcagtt ggtgccacac cattaatcag ttttacggag aaacaatcgt 4980
   tgcatacacg tttgatgcag gtccaaatgc tgtgttgtac tacttagctg aaaatgagtc 5040
   gaaactcttt gcatttatct ataaattgtt tggctctgtt cctggatggg acaagaaatt 5100
   tactactgag cagcttgagg ctttcaacca tcaatttgaa tcatctaact ttactgcacg 5160
   tgaattggat cttgagttgc aaaaggatgt tgccagagtg attttaactc aagtcggttc 5220
   aggcccacaa gaaacaaacg aatctttgat tgacgcaaag actggtctac caaaggaata 5280
   agatcaattc gctgcatcgc ccttaggagg taaaaaaaaa tgactgccga caacaatagt 5340
   atgccccatg gtgcagtatc tagttacgcc aaattagtgc aaaaccaaac acctgaagac 5400
   attttggaag agtttcctga aattattcca ttacaacaaa gacctaatac ccgatctagt 5460
   gagacgtcaa atgacgaaag cggagaaaca tgtttttctg gtcatgatga ggagcaaatt 5520
   aagttaatga atgaaaattg tattgttttg gattgggacg ataatgctat tggtgccggt 5580
   accaagaaag tttgtcattt aatggaaaat attgaaaagg gtttactaca tcgtgcattc 5640
   tccgtcttta ttttcaatga acaaggtgaa ttacttttac aacaaagagc cactgaaaaa 5700
   ataactttcc ctgatctttg gactaacaca tgctgctctc atccactatg tattgatgac 5760
   gaattaggtt tgaagggtaa gctagacgat aagattaagg gcgctattac tgcggcggtg 5820
   agaaaactag atcatgaatt aggtattcca gaagatgaaa ctaagacaag gggtaagttt 5880
   cactttttaa acagaatcca ttacatggca ccaagcaatg aaccatgggg tgaacatgaa 5940
   attgattaca tcctatttta taagatcaac gctaaagaaa acttgactgt caacccaaac 6000
   gtcaatgaag ttagagactt caaatgggtt tcaccaaatg atttgaaaac tatgtttgct 6060
   gacccaagtt acaagtttac gccttggttt aagattattt gcgagaatta cttattcaac 6120
   tggtgggagc aattagatga cctttctgaa gtggaaaatg acaggcaaat tcatagaatg 6180
   ctataacaac gcgtctacaa ataaaaaagg cacgtcagat gacgtgcctt ttttcttggg 6240
   gccgaaaaat gccccgctta cgcagggcat ccatttatta ctcaaccgta accgattttg 6300
   ccaggttacg cggctggtca acgtcggtgc ctttgatcag cgcgacatgg taagccagca 6360
   gctgcagcgg aacggtgtag aagatcggtg caatcacctc ttccacatgc ggcatctcga 6420
   tgatgtgcat gttatcgcta cttacaaaac ccgcatcctg atcggcgaag acatacaact 6480
   gaccgccacg cgcgcgaact tcttcaatgt tggatttcag tttttccagc aattcgttgt 6540
   tcggtgcaac aacaataacc ggcatatcgg catcaattag cgccagcgga ccgtgtttca 6600
   gttcgccagc agcgtaggct tcagcgtgaa tgtaagagat ctctttcaac ttcaatgcgc 6660
   cttccagcgc gattgggtac tgatcgccac ggcccaggaa cagcgcgtga tgtttgtcag 6720
   agaaatcttc tgccagcgct tcaatgcgtt tgtcctgaga cagcatctgc tcaatacggc 6780
   tcggcagcgc ctgcagacca tgcacgatgt catgttcaat ggaggcatcc agacctttca 6840
   ggcgagacag cttcgccacc agcatcaaca gcacagttaa ctgagtggtg aatgctttag 6900
   tggatgccac gccgatttct gtacccgcgt tggtcattag cgccagatcg gattcgcgca 6960
   ccagagaaga acccggaacg ttacagattg ccagtgaacc aaggtaaccc agctctttcg 7020
   acagacgcag gccagccagg gtatccgcgg tttcgccaga ctgtgacaag gtgatcatca 7080
   ggctgttacg acgcacggca gatttgcgat agcggaattc agaggcgatt tcgacgtcgc 7140
   acggaatacc tgctagcgat tcaaaccagt agcgggaaac cataccggag ttataagaag 7200
   taccacaggc gaggatctga atatgctcaa ccttcgacag cagttcgtcg gcgttcggtc 7260
   ccagctcgct taaatcaacc tgaccgtggc tgatgcgtcc ggtaagggtg tttttgatcg 7320
   cgttcggctg ttcgtagatc tctttctgca tgtagtgacg gtaaatgcct ttatcgcccg 7380
   cgtcatattg cagattggat tcgatatcct gacgttttac ttccgcgcca gttttatcga 7440
   agatgtttac cgaacggcga gtga 7464
<210> 65
   <211> 70
   <212> DNA
   <213> Artificial Sequence
<220>
   <223> Primer (APtacKKDyIv03_6038-36-5)
<400> 65
   gataaagtct tcagtctgat ttaaataagc gttgatattc agtcaattac tgaagcctgc 60
   ttttttatac 70
<210> 66
   <211> 71
   <212> DNA
   <213> Artificial Sequence
<220>
   <223> Primer (SPtacKKDyIv02_6038-36-3)
<400> 66
   tcaccaaaaa taataacctt tcccggtgca gaagttaaga acggtaatga catggcagtc 60
   tccttgtgtg a 71
<210> 67
   <211> 40
   <212> DNA
   <213> Artificial Sequence
<220>
   <223> Primer for amplifying IspSK gene
<400> 67
   gaagtccagg aggacataca atgtgtgcga cctcttctca 40
<210> 68
   <211> 40
   <212> DNA
   <213> Artificial Sequence
<220>
   <223> Primer for amplifying IspSK gene
<400> 68
   tgcctgcagg tcgactctag ttagacatac atcagctggt 40
<210> 69
   <211> 40
   <212> DNA
   <213> Artificial Sequence
<220>
   <223> Primer for amplifying IspSP gene
<400> 69
   gaagtccagg aggacataca atgtgctctg tttctaccga 40
<210> 70
   <211> 40
   <212> DNA
   <213> Artificial Sequence
<220>
   <223> Primer for amplifying IspSP gene
<400> 70
   tgcctgcagg tcgactctag ttaacgttcg aacggcagaa 40
<210> 71
   <211> 40
   <212> DNA
   <213> Artificial Sequence
<220>
   <223> Primer for amplifying IspSM gene
<400> 71
   gaagtccagg aggacataca atgtccgccg tttcaagcca 40
<210> 72
   <211> 40
   <212> DNA
   <213> Artificial Sequence
<220>
   <223> Primer for amplifying IspSM gene
<400> 72
   tgcctgcagg tcgactctag ttagttaatc gggaacgggt 40
<210> 73
   <211> 40
   <212> DNA
   <213> Artificial Sequence
<220>
   <223> Primer for amplifying nucleotide sequence of promoter region for elongation factor, Tu (P0480)
<400> 73
   ggtacccggg gatcctctag agatcgttta gatccgaagg 40
<210> 74
   <211> 40
   <212> DNA
   <213> Artificial Sequence
<220>
   <223> Primer for amplifying P0480 that is used in combination with ispSK
<400> 74
   tgagaagagg tcgcacacat tgtatgtcct cctggacttc 40
<210> 75
   <211> 40
   <212> DNA
   <213> Artificial Sequence
<220>
   <223> Primer for amplifying P0480 that is used in combination with ispSP
<400> 75
   tcggtagaaa cagagcacat tgtatgtcct cctggacttc 40
<210> 76
   <211> 40
   <212> DNA
   <213> Artificial Sequence
<220>
   <223> Primer for amplifying P0480 that is used in combination with ispSM
<400> 76
   tggcttgaaa cggcggacat tgtatgtcct cctggacttc 40
<210> 77
   <211> 362
   <212> DNA
   <213> Artificial Sequence
<220>
   <223> Nucleotide sequence of promoter region for elongation factor, Tu (P0480)
<400> 77
   agatcgttta gatccgaagg aaaacgtcga aaagcaattt gcttttcgac gccccacccc 60
   gcgcgtttta gcgtgtcagt agacgcgtag ggtaagtggg gtagcggctt gttagatatc    120
   ttgaaatcgg ctttcaacag cattgatttc gatgtattta gctggccgtt accctgcgaa    180
   tgtccacagg gtagctggta gtttgaaaat caacgccgtt gcccttagga ttcagtaact    240
   ggcacatttt gtaatgcgct agatctgtgt gcccagtctt ccaggctgct tatcacagtg    300
   aaagcaaaac caattcgtgg ctgcgaaagt cgtagccacc acgaagtcca ggaggacata    360
   ca                                                                   362
<210> 78
   <211> 5629
   <212> DNA
   <213> Artificial Sequence
<220>
   <223> Nucleotide sequence of shuttle vector for E.coli (pVK9)
<400> 78
   gaggtctgcc tcgtgaagaa ggtgttgctg actcatacca ggcctgaatc gccccatcat      60
   ccagccagaa agtgagggag ccacggttga tgagagcttt gttgtaggtg gacggggatc     120
   ttttctacgg ggtctgacgc tcagtggaac gaaaactcac gttaagggat tttggtcatg     180
   agattatcaa aaaggatcat gcgagcaacc tccataagat cagctaggcg atctttggga     240
   gcagtccttg tcgcgttacg aggtgagccg gtggggaacc gttagctgcc tatggtgtga     300
   gcccccctag agagcttcaa gagcaatcag cccgacctag aaaggaggcc aagagagaga     360
   cctacggggg gaaccgtttt ctgcctacga gatgggcaca ttactgggaa gctttacggc     420
   gtcctcgtgg aagttcaatg cccgcagact taagtgctct attcacggtc tgacgtgaca     480
   cgctaaattc agacatagct tcattgattg tcggccacga gccagtctct ccctcaacag     540
   tcataaacca acctgcaatg gtcaagcgat ttcctttagc tttcctagct tgtcgttgac     600
   tggacttagc tagtttttct cgctgtgctc gggcgtactc actgtttggg tctttccagc     660
   gttctgcggc ctttttaccg ccacgtcttc ccatagtggc cagagctttt cgccctcggc     720
   tgctctgcgt ctctgtctga cgagcaggga cgactggctg gcctttagcg acgtagccgc     780
   gcacacgtcg cgccatcgtc tggcggtcac gcatcggcgg cagatcaggc tcacggccgt     840
   ctgctccgac cgcctgagcg acggtgtagg cacgctcgta ggcgtcgatg atcttggtgt     900
   cttttaggcg ctcaccagcc gcttttaact ggtatcccac agtcaaagcg tggcgaaaag     960
   ccgtctcatc acgggcggca cgccctggag cagtccagag gacacggacg ccgtcgatca    1020
   gctctccaga cgcttcagcg gcgctcggca ggcttgcttc aagcgtggca agtgcttttg    1080
   cttccgcagt ggcttttctt gccgcttcga tacgtgcccg tccgctagaa aactcctgct    1140
   catagcgttt tttaggtttt tctgtgcctg agatcatgcg agcaacctcc ataagatcag    1200
   ctaggcgatc cacgcgattg tgctgggcat gccagcggta cgcggtggga tcgtcggaga    1260
   cgtgcagtgg ccaccggctc agcctatgtg aaaaagcctg gtcagcgccg aaaacgcggg 1320
   tcatttcctc ggtcgttgca gccagcaggc gcatattcgg gctgctcatg cctgctgcgg 1380
   catacaccgg atcaatgagc cagatgagct ggcatttccc gctcagtgga ttcacgccga 1440
   tccaagctgg cgctttttcc aggcgtgccc agcgctccaa aatcgcgtag acctcggggt 1500
   ttacgtgctc gattttcccg ccggcctggt ggctcggcac atcaatgtcc aggacaagca 1560
   cggctgcgtg ctgcgcgtgc gtcagagcaa catactggca ccgggcaagc gattttgaac 1620
   caactcggta taacttcggc tgtgtttctc ccgtgtccgg gtctttgatc caagcgctgg 1680
   cgaagtcgcg ggtcttgctg ccctggaaat tttctctgcc caggtgagcg aggaattcgc 1740
   ggcggtcttc gctcgtccag ccacgtgatc gcagcgcgag ctcgggatgg gtgtcgaaca 1800
   gatcagcgga aaatttccag gccggtgtgt caatgtctcg tgaatccgct agagtcattt 1860
   ttgagcgctt tctcccaggt ttggactggg ggttagccga cgccctgtga gttaccgctc 1920
   acggggcgtt caacattttt caggtattcg tgcagcttat cgcttcttgc cgcctgtgcg 1980
   ctttttcgac gcgcgacgct gctgccgatt cggtgcaggt ggtggcggcg ctgacacgtc 2040
   ctgggcggcc acggccacac gaaacgcggc atttacgatg tttgtcatgc ctgcgggcac 2100
   cgcgccacga tcgcggataa ttctcgctgc cgcttccagc tctgtgacga ccatggccaa 2160
   aatttcgctc gggggacgca cttccagcgc catttgcgac ctagccgcct ccagctcctc 2220
   ggcgtggcgt ttgttggcgc gctcgcggct ggctgcggca cgacacgcat ctgagcaata 2280
   ttttgcgcgc cgtcctcgcg ggtcaggccg gggaggaatc aggccaccgc agtaggcgca 2340
   actgattcga tcctccacta ctgtgcgtcc tcctggcgct gccgagcacg cagctcgtca 2400
   gccagctcct caagatccgc cacgagagtt tctaggtcgc tcgcggcact ggcccagtct 2460
   cgtgatgctg gcgcgtccgt cgtatcgaga gctcggaaaa atccgatcac cgtttttaaa 2520
   tcgacggcag catcgagcgc gtcggactcc agcgcgacat cagagagatc catagctgat 2580
   gattcgggcc aattttggta cttcgtcgtg aaggtcatga caccattata acgaacgttc 2640
   gttaaagttt ttggcggaaa atcacgcggc acgaaaattt tcacgaagcg ggactttgcg 2700
   cagctcaggg gtgctaaaaa ttttgtatcg cacttgattt ttccgaaaga cagattatct 2760
   gcaaacggtg tgtcgtattt ctggcttggt ttttaaaaaa tctggaatcg aaaatttgcg 2820
   gggcgaccga gaagtttttt acaaaaggca aaaacttttt cgggatcagc taggcgatcc 2880
   acgcgattgt gctgggcatg ccagcggtac gcggtgggat cgtcggagac gtgcagtggc 2940
   caccggctca gcctatgtga aaaagcctgg tcagcgccga aaacgcgggt catttcctcg 3000
   gtcgttgcag ccagcaggcg catattcggg ctgctcatgc ctgctgcggc atacaccgga 3060
   tcggaccagt tggtgatttt gaacttttgc tttgccacgg aacggtctgc gttgtcggga 3120
   agatgcgtga tctgatcctt caactcagca aaagttcgat ttattcaaca aagccacgtt 3180
   gtgtctcaaa atctctgatg ttacattgca caagataaaa atatatcatc atgaacaata 3240
   aaactgtctg cttacataaa cagtaataca aggggtgtta tgagccatat tcaacgggaa 3300
   acgtcttgct cgaagccgcg attaaattcc aacatggatg ctgatttata tgggtataaa 3360
   tgggctcgcg ataatgtcgg gcaatcaggt gcgacaatct atcgattgta tgggaagccc 3420
   gatgcgccag agttgtttct gaaacatggc aaaggtagcg ttgccaatga tgttacagat 3480
   gagatggtca gactaaactg gctgacggaa tttatgcctc ttccgaccat caagcatttt 3540
   atccgtactc ctgatgatgc atggttactc accactgcga tccccgggaa aacagcattc 3600
   caggtattag aagaatatcc tgattcaggt gaaaatattg ttgatgcgct ggcagtgttc 3660
   ctgcgccggt tgcattcgat tcctgtttgt aattgtcctt ttaacagcga tcgcgtattt 3720
   cgtctcgctc aggcgcaatc acgaatgaat aacggtttgg ttgatgcgag tgattttgat 3780
   gacgagcgta atggctggcc tgttgaacaa gtctggaaag aaatgcataa gcttttgcca 3840
   ttctcaccgg attcagtcgt cactcatggt gatttctcac ttgataacct tatttttgac 3900
   gaggggaaat taataggttg tattgatgtt ggacgagtcg gaatcgcaga ccgataccag 3960
   gatcttgcca tcctatggaa ctgcctcggt gagttttctc cttcattaca gaaacggctt 4020
   tttcaaaaat atggtattga taatcctgat atgaataaat tgcagtttca tttgatgctc 4080
   gatgagtttt tctaatcaga attggttaat tggttgtaac actggcagag cattacgctg 4140
   acttgacggg acggcggctt tgttgaataa atcgcattcg ccattcaggc tgcgcaactg 4200
   ttgggaaggg cgatcggtgc gggcctcttc gctattacgc cagctggcga aagggggatg 4260
   tgctgcaagg cgattaagtt gggtaacgcc agggttttcc cagtcacgac gttgtaaaac 4320
   gacggccagt gaattcgagc tcggtacccg gggatcctct agagtcgacc tgcaggcatg 4380
   caagcttggc gtaatcatgg tcatagctgt ttcctgtgtg aaattgttat ccgctcacaa 4440
   ttccacacaa catacgagcc ggaagcataa agtgtaaagc ctggggtgcc taatgagtga 4500
   gctaactcac attaattgcg ttgcgctcac tgcccgcttt ccagtcggga aacctgtcgt 4560
   gccagctgca ttaatgaatc ggccaacgcg cggggagagg cggtttgcgt attggcgaac 4620
   ttttgctgag ttgaaggatc agatcacgca tcttcccgac aacgcagacc gttccgtggc 4680
   aaagcaaaag ttcaaaatca gtaaccgtca gtgccgataa gttcaaagtt aaacctggtg 4740
   ttgataccaa cattgaaacg ctgatcgaaa acgcgctgaa aaacgctgct gaatgtgcga 4800
   gcttcttccg cttcctcgct cactgactcg ctgcgctcgg tcgttcggct gcggcgagcg 4860
   gtatcagctc actcaaaggc ggtaatacgg ttatccacag aatcagggga taacgcagga 4920
   aagaacatgt gagcaaaagg ccagcaaaag gccaggaacc gtaaaaaggc cgcgttgctg 4980
   gcgtttttcc ataggctccg cccccctgac gagcatcaca aaaatcgacg ctcaagtcag 5040
   aggtggcgaa acccgacagg actataaaga taccaggcgt ttccccctgg aagctccctc 5100
   gtgcgctctc ctgttccgac cctgccgctt accggatacc tgtccgcctt tctcccttcg 5160
   ggaagcgtgg cgctttctca atgctcacgc tgtaggtatc tcagttcggt gtaggtcgtt 5220
   cgctccaagc tgggctgtgt gcacgaaccc cccgttcagc ccgaccgctg cgccttatcc 5280
   ggtaactatc gtcttgagtc caacccggta agacacgact tatcgccact ggcagcagcc 5340
   actggtaaca ggattagcag agcgaggtat gtaggcggtg ctacagagtt cttgaagtgg 5400
   tggcctaact acggctacac tagaaggaca gtatttggta tctgcgctct gctgaagcca 5460
   gttaccttcg gaaaaagagt tggtagctct tgatccggca aacaaaccac cgctggtagc 5520
   ggtggttttt ttgtttgcaa gcagcagatt acgcgcagaa aaaaaggatc tcaagaagat 5580
   cctttgatct tttctacggg gtctgacgct cagtggaacg atccgtcga 5629
<210> 79
   <211> 7690
   <212> DNA
   <213> Artificial Sequence
<220>
   <223> Nucleotide sequence of plasmid for expressing ispSK gene (pVK9-P0480-ispSK)
<400> 79
   gaggtctgcc tcgtgaagaa ggtgttgctg actcatacca ggcctgaatc gccccatcat      60
   ccagccagaa agtgagggag ccacggttga tgagagcttt gttgtaggtg gacggggatc     120
   ttttctacgg ggtctgacgc tcagtggaac gaaaactcac gttaagggat tttggtcatg     180
   agattatcaa aaaggatcat gcgagcaacc tccataagat cagctaggcg atctttggga     240
   gcagtccttg tcgcgttacg aggtgagccg gtggggaacc gttagctgcc tatggtgtga     300
   gcccccctag agagcttcaa gagcaatcag cccgacctag aaaggaggcc aagagagaga     360
   cctacggggg gaaccgtttt ctgcctacga gatgggcaca ttactgggaa gctttacggc     420
   gtcctcgtgg aagttcaatg cccgcagact taagtgctct attcacggtc tgacgtgaca     480
   cgctaaattc agacatagct tcattgattg tcggccacga gccagtctct ccctcaacag     540
   tcataaacca acctgcaatg gtcaagcgat ttcctttagc tttcctagct tgtcgttgac     600
   tggacttagc tagtttttct cgctgtgctc gggcgtactc actgtttggg tctttccagc     660
   gttctgcggc ctttttaccg ccacgtcttc ccatagtggc cagagctttt cgccctcggc     720
   tgctctgcgt ctctgtctga cgagcaggga cgactggctg gcctttagcg acgtagccgc     780
   gcacacgtcg cgccatcgtc tggcggtcac gcatcggcgg cagatcaggc tcacggccgt     840
   ctgctccgac cgcctgagcg acggtgtagg cacgctcgta ggcgtcgatg atcttggtgt     900
   cttttaggcg ctcaccagcc gcttttaact ggtatcccac agtcaaagcg tggcgaaaag     960
   ccgtctcatc acgggcggca cgccctggag cagtccagag gacacggacg ccgtcgatca 1020
   gctctccaga cgcttcagcg gcgctcggca ggcttgcttc aagcgtggca agtgcttttg 1080
   cttccgcagt ggcttttctt gccgcttcga tacgtgcccg tccgctagaa aactcctgct 1140
   catagcgttt tttaggtttt tctgtgcctg agatcatgcg agcaacctcc ataagatcag 1200
   ctaggcgatc cacgcgattg tgctgggcat gccagcggta cgcggtggga tcgtcggaga 1260
   cgtgcagtgg ccaccggctc agcctatgtg aaaaagcctg gtcagcgccg aaaacgcggg 1320
   tcatttcctc ggtcgttgca gccagcaggc gcatattcgg gctgctcatg cctgctgcgg 1380
   catacaccgg atcaatgagc cagatgagct ggcatttccc gctcagtgga ttcacgccga 1440
   tccaagctgg cgctttttcc aggcgtgccc agcgctccaa aatcgcgtag acctcggggt 1500
   ttacgtgctc gattttcccg ccggcctggt ggctcggcac atcaatgtcc aggacaagca 1560
   cggctgcgtg ctgcgcgtgc gtcagagcaa catactggca ccgggcaagc gattttgaac 1620
   caactcggta taacttcggc tgtgtttctc ccgtgtccgg gtctttgatc caagcgctgg 1680
   cgaagtcgcg ggtcttgctg ccctggaaat tttctctgcc caggtgagcg aggaattcgc 1740
   ggcggtcttc gctcgtccag ccacgtgatc gcagcgcgag ctcgggatgg gtgtcgaaca 1800
   gatcagcgga aaatttccag gccggtgtgt caatgtctcg tgaatccgct agagtcattt 1860
   ttgagcgctt tctcccaggt ttggactggg ggttagccga cgccctgtga gttaccgctc 1920
   acggggcgtt caacattttt caggtattcg tgcagcttat cgcttcttgc cgcctgtgcg 1980
   ctttttcgac gcgcgacgct gctgccgatt cggtgcaggt ggtggcggcg ctgacacgtc 2040
   ctgggcggcc acggccacac gaaacgcggc atttacgatg tttgtcatgc ctgcgggcac 2100
   cgcgccacga tcgcggataa ttctcgctgc cgcttccagc tctgtgacga ccatggccaa 2160
   aatttcgctc gggggacgca cttccagcgc catttgcgac ctagccgcct ccagctcctc 2220
   ggcgtggcgt ttgttggcgc gctcgcggct ggctgcggca cgacacgcat ctgagcaata 2280
   ttttgcgcgc cgtcctcgcg ggtcaggccg gggaggaatc aggccaccgc agtaggcgca 2340
   actgattcga tcctccacta ctgtgcgtcc tcctggcgct gccgagcacg cagctcgtca 2400
   gccagctcct caagatccgc cacgagagtt tctaggtcgc tcgcggcact ggcccagtct 2460
   cgtgatgctg gcgcgtccgt cgtatcgaga gctcggaaaa atccgatcac cgtttttaaa 2520
   tcgacggcag catcgagcgc gtcggactcc agcgcgacat cagagagatc catagctgat 2580
   gattcgggcc aattttggta cttcgtcgtg aaggtcatga caccattata acgaacgttc 2640
   gttaaagttt ttggcggaaa atcacgcggc acgaaaattt tcacgaagcg ggactttgcg 2700
   cagctcaggg gtgctaaaaa ttttgtatcg cacttgattt ttccgaaaga cagattatct 2760
   gcaaacggtg tgtcgtattt ctggcttggt ttttaaaaaa tctggaatcg aaaatttgcg 2820
   gggcgaccga gaagtttttt acaaaaggca aaaacttttt cgggatcagc taggcgatcc 2880
   acgcgattgt gctgggcatg ccagcggtac gcggtgggat cgtcggagac gtgcagtggc 2940
   caccggctca gcctatgtga aaaagcctgg tcagcgccga aaacgcgggt catttcctcg 3000
   gtcgttgcag ccagcaggcg catattcggg ctgctcatgc ctgctgcggc atacaccgga 3060
   tcggaccagt tggtgatttt gaacttttgc tttgccacgg aacggtctgc gttgtcggga 3120
   agatgcgtga tctgatcctt caactcagca aaagttcgat ttattcaaca aagccacgtt 3180
   gtgtctcaaa atctctgatg ttacattgca caagataaaa atatatcatc atgaacaata 3240
   aaactgtctg cttacataaa cagtaataca aggggtgtta tgagccatat tcaacgggaa 3300
   acgtcttgct cgaagccgcg attaaattcc aacatggatg ctgatttata tgggtataaa 3360
   tgggctcgcg ataatgtcgg gcaatcaggt gcgacaatct atcgattgta tgggaagccc 3420
   gatgcgccag agttgtttct gaaacatggc aaaggtagcg ttgccaatga tgttacagat 3480
   gagatggtca gactaaactg gctgacggaa tttatgcctc ttccgaccat caagcatttt 3540
   atccgtactc ctgatgatgc atggttactc accactgcga tccccgggaa aacagcattc 3600
   caggtattag aagaatatcc tgattcaggt gaaaatattg ttgatgcgct ggcagtgttc 3660
   ctgcgccggt tgcattcgat tcctgtttgt aattgtcctt ttaacagcga tcgcgtattt 3720
   cgtctcgctc aggcgcaatc acgaatgaat aacggtttgg ttgatgcgag tgattttgat 3780
   gacgagcgta atggctggcc tgttgaacaa gtctggaaag aaatgcataa gcttttgcca 3840
   ttctcaccgg attcagtcgt cactcatggt gatttctcac ttgataacct tatttttgac 3900
   gaggggaaat taataggttg tattgatgtt ggacgagtcg gaatcgcaga ccgataccag 3960
   gatcttgcca tcctatggaa ctgcctcggt gagttttctc cttcattaca gaaacggctt 4020
   tttcaaaaat atggtattga taatcctgat atgaataaat tgcagtttca tttgatgctc 4080
   gatgagtttt tctaatcaga attggttaat tggttgtaac actggcagag cattacgctg 4140
   acttgacggg acggcggctt tgttgaataa atcgcattcg ccattcaggc tgcgcaactg 4200
   ttgggaaggg cgatcggtgc gggcctcttc gctattacgc cagctggcga aagggggatg 4260
   tgctgcaagg cgattaagtt gggtaacgcc agggttttcc cagtcacgac gttgtaaaac 4320
   gacggccagt gaattcgagc tcggtacccg gggatcctct agagatcgtt tagatccgaa 4380
   ggaaaacgtc gaaaagcaat ttgcttttcg acgccccacc ccgcgcgttt tagcgtgtca 4440
   gtagacgcgt agggtaagtg gggtagcggc ttgttagata tcttgaaatc ggctttcaac 4500
   agcattgatt tcgatgtatt tagctggccg ttaccctgcg aatgtccaca gggtagctgg 4560
   tagtttgaaa atcaacgccg ttgcccttag gattcagtaa ctggcacatt ttgtaatgcg 4620
   ctagatctgt gtgcccagtc ttccaggctg cttatcacag tgaaagcaaa accaattcgt 4680
   ggctgcgaaa gtcgtagcca ccacgaagtc caggaggaca tacaatgtgt gcgacctctt 4740
   ctcaatttac tcagattacc gagcataatt cccgtcgttc cgcaaactat cagccaaacc 4800
   tgtggaattt cgaattcctg caatccctgg agaacgacct gaaagtggaa aagctggagg 4860
   agaaagcgac caaactggag gaagaagttc gctgcatgat caaccgtgta gacacccagc 4920
   cgctgtccct gctggagctg atcgacgatg tgcagcgcct gggtctgacc tacaaatttg 4980
   aaaaagacat cattaaagcc ctggaaaaca tcgtactgct ggacgaaaac aaaaagaaca 5040
   aatctgacct gcacgcaacc gctctgtctt tccgtctgct gcgtcagcac ggtttcgagg 5100
   tttctcagga tgtttttgag cgtttcaagg ataaagaagg tggtttcagc ggtgaactga 5160
   aaggtgacgt ccaaggcctg ctgagcctgt atgaagcgtc ttacctgggt ttcgagggtg 5220
   agaacctgct ggaggaggcg cgtacctttt ccatcaccca cctgaagaac aacctgaaag 5280
   aaggcattaa taccaaggtt gcagaacaag tgagccacgc cctggaactg ccatatcacc 5340
   agcgtctgca ccgtctggag gcacgttggt tcctggataa atacgaaccg aaagaaccgc 5400
   atcaccagct gctgctggag ctggcgaagc tggattttaa catggtacag accctgcacc 5460
   agaaagagct gcaagatctg tcccgctggt ggaccgagat gggcctggct agcaaactgg 5520
   attttgtacg cgaccgcctg atggaagttt atttctgggc actgggtatg gcgccagacc 5580
   cgcagtttgg tgaatgtcgc aaagctgtta ctaaaatgtt tggtctggtg acgatcatcg 5640
   atgacgtgta tgacgtttat ggcactctgg acgaactgca actgttcacc gatgctgtag 5700
   agcgctggga cgttaacgct attaacaccc tgccggacta tatgaaactg tgtttcctgg 5760
   cactgtacaa caccgttaac gacacgtcct attctattct gaaagagaaa ggtcataaca 5820
   acctgtccta tctgacgaaa agctggcgtg aactgtgcaa agcctttctg caagaggcga 5880
   aatggtccaa caacaaaatt atcccggctt tctccaagta cctggaaaac gccagcgttt 5940
   cctcctccgg tgtagcgctg ctggcgccgt cttacttttc cgtatgccag cagcaggaag 6000
   acatctccga ccacgcgctg cgttccctga ccgacttcca tggtctggtg cgttctagct 6060
   gcgttatctt ccgcctgtgc aacgatctgg ccacctctgc ggcggagctg gaacgtggcg 6120
   agactaccaa ttctatcatt agctacatgc acgaaaacga tggtaccagc gaggaacagg 6180
   cccgcgaaga actgcgtaaa ctgatcgacg ccgaatggaa aaagatgaat cgtgaacgcg 6240
   ttagcgactc caccctgctg cctaaagcgt tcatggaaat cgcagttaac atggcacgtg 6300
   tttcccactg cacctaccag tatggcgatg gtctgggtcg cccagactac gcgactgaaa 6360
   accgcatcaa actgctgctg attgaccctt tcccgattaa ccagctgatg tatgtctaac 6420
   tagagtcgac ctgcaggcat gcaagcttgg cgtaatcatg gtcatagctg tttcctgtgt 6480
   gaaattgtta tccgctcaca attccacaca acatacgagc cggaagcata aagtgtaaag 6540
   cctggggtgc ctaatgagtg agctaactca cattaattgc gttgcgctca ctgcccgctt 6600
   tccagtcggg aaacctgtcg tgccagctgc attaatgaat cggccaacgc gcggggagag 6660
   gcggtttgcg tattggcgaa cttttgctga gttgaaggat cagatcacgc atcttcccga 6720
   caacgcagac cgttccgtgg caaagcaaaa gttcaaaatc agtaaccgtc agtgccgata 6780
   agttcaaagt taaacctggt gttgatacca acattgaaac gctgatcgaa aacgcgctga 6840
   aaaacgctgc tgaatgtgcg agcttcttcc gcttcctcgc tcactgactc gctgcgctcg 6900
   gtcgttcggc tgcggcgagc ggtatcagct cactcaaagg cggtaatacg gttatccaca 6960
   gaatcagggg ataacgcagg aaagaacatg tgagcaaaag gccagcaaaa ggccaggaac 7020
   cgtaaaaagg ccgcgttgct ggcgtttttc cataggctcc gcccccctga cgagcatcac 7080
   aaaaatcgac gctcaagtca gaggtggcga aacccgacag gactataaag ataccaggcg 7140
   tttccccctg gaagctccct cgtgcgctct cctgttccga ccctgccgct taccggatac 7200
   ctgtccgcct ttctcccttc gggaagcgtg gcgctttctc aatgctcacg ctgtaggtat 7260
   ctcagttcgg tgtaggtcgt tcgctccaag ctgggctgtg tgcacgaacc ccccgttcag 7320
   cccgaccgct gcgccttatc cggtaactat cgtcttgagt ccaacccggt aagacacgac 7380
   ttatcgccac tggcagcagc cactggtaac aggattagca gagcgaggta tgtaggcggt 7440
   gctacagagt tcttgaagtg gtggcctaac tacggctaca ctagaaggac agtatttggt 7500
   atctgcgctc tgctgaagcc agttaccttc ggaaaaagag ttggtagctc ttgatccggc 7560
   aaacaaacca ccgctggtag cggtggtttt tttgtttgca agcagcagat tacgcgcaga 7620
   aaaaaaggat ctcaagaaga tcctttgatc ttttctacgg ggtctgacgc tcagtggaac 7680
   gatccgtcga 7690
<210> 80
   <211> 7675
   <212> DNA
   <213> Artificial Sequence
<220>
   <223> Nucleotide sequence of plasmid for expressing ispSP gene (pVK9-P0480-ispSP)
<400> 80
<210> 81
   <211> 7647
   <212> DNA
   <213> Artificial Sequence
<220>
   <223> Nucleotide sequence of plasmid for expressing ispSK gene (pVK9-P0480-ispSM)
<210> 82
   <211> 41
   <212> DNA
   <213> Artificial Sequence
<220>
   <223> Primer for amplifying IspSK gene
<400> 82
   gggaatatta agcttggtac catgtgtgcg acctcttctc a 41
<210> 83
   <211> 41
   <212> DNA
   <213> Artificial Sequence
<220>
   <223> Primer for amplifying IspSK gene
<400> 83
   agtggatccg agctcggtac cttagacata catcagctgg t 41
<210> 84
   <211> 41
   <212> DNA
   <213> Artificial Sequence
<220>
   <223> Primer for amplifying IspSP gene
<400> 84
   gggaatatta agcttggtac catgtgctct gtttctaccg a 41
<210> 85
   <211> 41
   <212> DNA
   <213> Artificial Sequence
<220>
   <223> Primer for amplifying IspSP gene
<400> 85
   agtggatccg agctcggtac cttaacgttc gaacggcaga a 41
<210> 86
   <211> 41
   <212> DNA
   <213> Artificial Sequence
<220>
   <223> Primer for amplifying IspSM gene
<400> 86
   gggaatatta agcttggtac catgtccgcc gtttcaagcc a 41
<210> 87
   <211> 41
   <212> DNA
   <213> Artificial Sequence
<220>
   <223> Primer for amplifying IspSM gene
<400> 87
   agtggatccg agctcggtac cttagttaat cgggaacggg t 41
<210> 88
   <211> 24
   <212> DNA
   <213> Artificial Sequence
<220>
   <223> PCR primer 1 for pCold-TF
<400> 88
   cctaccttcg ataccaccac tacc 24
<210> 89
   <211> 30
   <212> DNA
   <213> Artificial Sequence
<220>
   <223> PCR primer 2 for pCold-TF
<400> 89
   taggtaatct ctgcttaaaa gcacagaatc 30
<210> 90
   <211> 44
   <212> DNA
   <213> Artificial Sequence
<220>
   <223> PCR primer 1 for IspSM gene
<400> 90
   ggtagtggtg gtatcgaagg taggatgtcc gccgtttcaa gcca 44
<210> 91
   <211> 53
   <212> DNA
   <213> Artificial Sequence
<220>
   <223> IspSM PCR primer 2
<400> 91
   gattctgtgc ttttaagcag agattaccta ttagttaatc gggaacgggt caa 53
<210> 92
   <211> 7365
   <212> DNA
   <213> Artificial Sequence
<220>
   <223> Nucleotide sequence of plasmid for expressing IspSM gene (pCold-TF-IspSM)
<400> 92
   aaggaatggt gtggccgatt aatcataaat atgaaaaata attgttgcat cacccgccaa      60
   tgcgtggctt aatgcacatc aaattgtgag cggataacaa tttgatgtgc tagcgcatat     120
   ccagtgtagt aaggcaagtc ccttcaagag ttatcgttga tacccctcgt agtgcacatt     180
   cctttaacgc ttcaaaatct gtaaagcacg ccatatcgcc gaaaggcaca cttaattatt     240
   aagaggtaat acaccatgaa tcacaaagtg catcatcatc atcatcacat gcaagtttca     300
   gttgaaacca ctcaaggcct tggccgccgt gtaacgatta ctatcgctgc tgacagcatc     360
   gagaccgctg ttaaaagcga gctggtcaac gttgcgaaaa aagtacgtat tgacggcttc     420
   cgcaagggca aagtgccaat gaatatcgtt gctcagcgtt atggcgcgtc tgtacgccag     480
   gacgttctgg gtgacctgat gagccgtaac ttcattgacg ccatcattaa agaaaaaatc     540
   aatccggctg gcgcaccgac ttatgttccg ggcgaataca agctgggtga agacttcact     600
   tactctgtag agtttgaagt ttatccggaa gttgaactgc aaggtctgga agcgatcgaa     660
   gttgaaaaac cgatcgttga agtgaccgac gctgacgttg acggcatgct ggatactctg     720
   cgtaaacagc aggcgacctg gaaagaaaaa gacggcgctg ttgaagcaga agaccgcgtg     780
   accatcgact tcaccggttc tgtagacggc gaagagttcg aaggcggtaa agcgtctgat     840
   ttcgtactgg cgatgggcca gggtcgtatg atcccgggct ttgaagacgg tatcaaaggc     900
   cacaaagctg gcgaagagtt caccatcgac gtgaccttcc cggaagaata ccacgcagaa     960
   aacctgaaag gtaaagcagc gaaattcgct atcaacctga agaaagttga agagcgtgaa    1020
   ctgccggaac tgaccgcaga gttcatcaaa cgtttcggcg ttgaagatgg ttccgtagaa    1080
   ggtctgcgcg ctgaagtgcg taaaaacatg gagcgcgagc tgaagagcgc catccgtaac    1140
   cgcgttaagt ctcaggcgat cgaaggtctg gtaaaagcta acgacatcga cgtaccggct    1200
   gcgctgatcg acagcgaaat cgacgttctg cgtcgccagg ctgcacagcg tttcggtggc    1260
   aacgaaaaac aagctctgga actgccgcgc gaactgttcg aagaacaggc taaacgccgc    1320
   gtagttgttg gcctgctgct gggcgaagtt atccgcacca acgagctgaa agctgacgaa    1380
   gagcgcgtga aaggcctgat cgaagagatg gcttctgcgt acgaagatcc gaaagaagtt    1440
   atcgagttct acagcaaaaa caaagaactg atggacaaca tgcgcaatgt tgctctggaa    1500
   gaacaggctg ttgaagctgt actggcgaaa gcgaaagtga ctgaaaaaga aaccactttc    1560
   aacgagctga tgaaccagca ggcgtccgcg ggtctggaag ttctgttcca ggggccctcc    1620
   gcgggtctgg tgccacgcgg tagtggtggt atcgaaggta ggatgtccgc cgtttcaagc 1680
   cagttctctc aaatcgccga agacaatagc cgtcgctcag caaattatca tccgaatctg 1740
   tgggactttg aatttctgca gtctctggaa aacgatagta aaatggaaaa actggaagaa 1800
   aaagccacca aactggaaga agaagtgcgt aacatgatga atgaagcgaa aacggaagcc 1860
   ctgagcctgc tggaactgat tgatgacgtc caacgcctgg gtctgaccta caaattcgaa 1920
   aaagatatca tcaaagcact ggaaaaaatt gtcccgctgg acgaatcagg tctgcacgtg 1980
   acgtctctga gttttcgtat cctgcgccag catggcttcg aagtttcgca agatgtcttt 2040
   aaacgtttca aagacaaaga aggcggtttc tgcgcggaac tgaaagatga cgtgcagggt 2100
   ctgctgtccc tgtatgaagc ctcatacctg ggttttgaag gcgaatccct gctggatgaa 2160
   gcgcgcgcct tctcaattac ccacctgaaa aacaatctga acaaaggcat caatacgaaa 2220
   gtggcacagc aagttagcca tgctctggaa ctgccgtatc accgtcgcct gcatcgtctg 2280
   gaagcacgct ggctgctgga taaatacgaa ccgaaagaac cgcatcacca tctgctgcac 2340
   gaactggcga aactggactt taatctggtt cagtcgctgt atcaaaaaga actgcgtgaa 2400
   ctgagcctgt ggtggcgcga aattggtctg acctctaaac tggattttgt gcgtgaccgc 2460
   ctgatggaag tttacttctg ggcactgggc atggctccgg atccgcagtt tagcgaatgc 2520
   cgtaaagtgg ttaccaaaat gttcggtctg gtcacgatta tcgatgacgt ctatgatgtg 2580
   tacggcaccc tggacgaact gcaactgttc acggatgcgg tcgaacgctg ggacgtgaac 2640
   gccatcaata ccctgccgga ttatatgaaa ctgtgttatc tggcgctgta caacaccgtt 2700
   aatgacacgg cctatagcat cctgaaagaa aaaggtcata acaacatctc gtacctgacc 2760
   aaaagctggt gcgaactgtg taaagcgttt ctgcaggaag ccaaatggtc taacaacaaa 2820
   atcatcccgg cattcaacaa atacctggat aatgctagtg ttagctctag tggcgtcgca 2880
   ctgctggctc cgtcctactt tctggtgtgt caggaacaag atatttctga ccaggcgctg 2940
   cacagtctga ccaactttca tggtctggtt cgttcctcat gcaccatctt ccgcctgtgt 3000
   aatgatctgg cgacgtcgag cgccgaactg gaacgtggcg aaaccacgaa ctcgattacc 3060
   agctatatgc acgaaaatga aacgagtgaa gaacaggcat gcaaagaact gcgtaacctg 3120
   atcgatgctg aatggaagaa aatgaacgaa gaacgcgtgt ccaattcaac cctgccgaaa 3180
   gcctttcgtg aaattgcaat caatatggct cgcatttccc attgtacgta tcagtacggc 3240
   gatggtctgg gccgcccgga ctacacgacc gaaaaccgta ttaaactgct gctgattgac 3300
   ccgttcccga ttaactaata ggtaatctct gcttaaaagc acagaatcta agatccctgc 3360
   catttggcgg ggattttttt atttgttttc aggaaataaa taatcgatcg cgtaataaaa 3420
   tctattatta tttttgtgaa gaataaattt gggtgcaatg agaatgcgca ggccctttcg 3480
   tctcgcgcgt ttcggtgatg acggtgaaaa cctctgacac atgcagctcc cggagacggt 3540
   cacagcttgt ctgtaagcgg atgccgggag cagacaagcc cgtcagggcg cgtcagcggg 3600
   tgttggcggg tgtcggggct ggcttaacta tgcggcatca gagcagattg tactgagagt 3660
   gcaccataaa attgtaaacg ttaatatttt gttaaaattc gcgttaaatt tttgttaaat 3720
   cagctcattt tttaaccaat aggccgaaat cggcaaaatc ccttataaat caaaagaata 3780
   gcccgagata gggttgagtg ttgttccagt ttggaacaag agtccactat taaagaacgt 3840
   ggactccaac gtcaaagggc gaaaaaccgt ctatcagggc gatggcccac tacgtgaacc 3900
   atcacccaaa tcaagttttt tggggtcgag gtgccgtaaa gcactaaatc ggaaccctaa 3960
   agggagcccc cgatttagag cttgacgggg aaagccggcg aacgtggcga gaaaggaagg 4020
   gaagaaagcg aaaggagcgg gcgctagggc gctggcaagt gtagcggtca cgctgcgcgt 4080
   aaccaccaca cccgccgcgc ttaatgcgcc gctacagggc gcgtactatg gttgctttga 4140
   cgtatgcggt gtgaaatacc gcacagatgc gtaaggagaa aataccgcat caggcgtcag 4200
   gtggcacttt tcggggaaat gtgcgcggaa cccctatttg tttatttttc taaatacatt 4260
   caaatatgta tccgctcatg agacaataac cctgataaat gcttcaataa tattgaaaaa 4320
   ggaagagtat gagtattcaa catttccgtg tcgcccttat tccctttttt gcggcatttt 4380
   gccttcctgt ttttgctcac ccagaaacgc tggtgaaagt aaaagatgct gaagatcagt 4440
   tgggtgcacg agtgggttac atcgaactgg atctcaacag cggtaagatc cttgagagtt 4500
   ttcgccccga agaacgtttt ccaatgatga gcacttttaa agttctgcta tgtggcgcgg 4560
   tattatcccg tattgacgcc gggcaagagc aactcggtcg ccgcatacac tattctcaga 4620
   atgacttggt tgagtactca ccagtcacag aaaagcatct tacggatggc atgacagtaa 4680
   gagaattatg cagtgctgcc ataaccatga gtgataacac tgcggccaac ttacttctga 4740
   caacgatcgg aggaccgaag gagctaaccg cttttttgca caacatgggg gatcatgtaa 4800
   ctcgccttga tcgttgggaa ccggagctga atgaagccat accaaacgac gagcgtgaca 4860
   ccacgatgcc tgtagcaatg gcaacaacgt tgcgcaaact attaactggc gaactactta 4920
   ctctagcttc ccggcaacaa ttaatagact ggatggaggc ggataaagtt gcaggaccac 4980
   ttctgcgctc ggcccttccg gctggctggt ttattgctga taaatctgga gccggtgagc 5040
   gtgggtctcg cggtatcatt gcagcactgg ggccagatgg taagccctcc cgtatcgtag 5100
   ttatctacac gacggggagt caggcaacta tggatgaacg aaatagacag atcgctgaga 5160
   taggtgcctc actgattaag cattggtaac tgtcagacca agtttactca tatatacttt 5220
   agattgattt aaaacttcat ttttaattta aaaggatcta ggtgaagatc ctttttgata 5280
   atctcatgac caaaatccct taacgtgagt tttcgttcca ctgagcgtca gaccccgtag 5340
   aaaagatcaa aggatcttct tgagatcctt tttttctgcg cgtaatctgc tgcttgcaaa 5400
   caaaaaaacc accgctacca gcggtggttt gtttgccgga tcaagagcta ccaactcttt 5460
   ttccgaaggt aactggcttc agcagagcgc agataccaaa tactgttctt ctagtgtagc 5520
   cgtagttagg ccaccacttc aagaactctg tagcaccgcc tacatacctc gctctgctaa 5580
   tcctgttacc agtggctgct gccagtggcg ataagtcgtg tcttaccggg ttggactcaa 5640
   gacgatagtt accggataag gcgcagcggt cgggctgaac ggggggttcg tgcacacagc 5700
   ccagcttgga gcgaacgacc tacaccgaac tgagatacct acagcgtgag ctatgagaaa 5760
   gcgccacgct tcccgaaggg agaaaggcgg acaggtatcc ggtaagcggc agggtcggaa 5820
   caggagagcg cacgagggag cttccagggg gaaacgcctg gtatctttat agtcctgtcg 5880
   ggtttcgcca cctctgactt gagcgtcgat ttttgtgatg ctcgtcaggg gggcggagcc 5940
   tatggaaaaa cgccagcaac gcggcctttt tacggttcct ggccttttgc tggccttttg 6000
   ctcacatagt catgccccgc gcccaccgga aggagctgac tgggttgaag gctctcaagg 6060
   gcatcggtcg agatcccggt gcctaatgag tgagctaact tacattaatt gcgttgcgct 6120
   cactgcccgc tttccagtcg ggaaacctgt cgtgccagct gcattaatga atcggccaac 6180
   gcgcggggag aggcggtttg cgtattgggc gccagggtgg tttttctttt caccagtgag 6240
   acgggcaaca gctgattgcc cttcaccgcc tggccctgag agagttgcag caagcggtcc 6300
   acgctggttt gccccagcag gcgaaaatcc tgtttgatgg tggttaacgg cgggatataa 6360
   catgagctgt cttcggtatc gtcgtatccc actaccgaga tatccgcacc aacgcgcagc 6420
   ccggactcgg taatggcgcg cattgcgccc agcgccatct gatcgttggc aaccagcatc 6480
   gcagtgggaa cgatgccctc attcagcatt tgcatggttt gttgaaaacc ggacatggca 6540
   ctccagtcgc cttcccgttc cgctatcggc tgaatttgat tgcgagtgag atatttatgc 6600
   cagccagcca gacgcagacg cgccgagaca gaacttaatg ggcccgctaa cagcgcgatt 6660
   tgctggtgac ccaatgcgac cagatgctcc acgcccagtc gcgtaccgtc ttcatgggag 6720
   aaaataatac tgttgatggg tgtctggtca gagacatcaa gaaataacgc cggaacatta 6780
   gtgcaggcag cttccacagc aatggcatcc tggtcatcca gcggatagtt aatgatcagc 6840
   ccactgacgc gttgcgcgag aagattgtgc accgccgctt tacaggcttc gacgccgctt 6900
   cgttctacca tcgacaccac cacgctggca cccagttgat cggcgcgaga tttaatcgcc 6960
   gcgacaattt gcgacggcgc gtgcagggcc agactggagg tggcaacgcc aatcagcaac 7020
   gactgtttgc ccgccagttg ttgtgccacg cggttgggaa tgtaattcag ctccgccatc 7080
   gccgcttcca ctttttcccg cgttttcgca gaaacgtggc tggcctggtt caccacgcgg 7140
   gaaacggtct gataagagac accggcatac tctgcgacat cgtataacgt tactggtttc 7200
   acattcacca ccctgaattg actctcttcc gggcgctatc atgccatacc gcgaaaggtt 7260
   ttgcgccatt cgatggtgtc cgggatctcg acgctctccc ttatgcgact cctgcattag 7320
   gaagcagccc agtagtaggt tgaggccgtt gagcaccgcc gccgc 7365
<210> 93
   <211> 48
   <212> DNA
   <213> Artificial Sequence
<220>
   <223> PCR primer 1 for amplifying IspSP gene
<400> 93
   ggtagtggtg gtatcgaagg taggatgtgc tctgtttcta ccgagaac 48
<210> 94
   <211> 52
   <212> DNA
   <213> Artificial Sequence
<220>
   <223> PCR primer 2 for amplifying IspSP gene
<400> 94
   gattctgtgc ttttaagcag agattaccta ttaacgttcg aacggcagaa tc 52
<210> 95
   <211> 7389
   <212> DNA
   <213> Artificial Sequence
<220>
   <223> Nucleotide sequence of plasmid for expressing IspSP gene (pCold-TF-IspSP)
<400> 95
   aaggaatggt gtggccgatt aatcataaat atgaaaaata attgttgcat cacccgccaa      60
   tgcgtggctt aatgcacatc aaattgtgag cggataacaa tttgatgtgc tagcgcatat     120
   ccagtgtagt aaggcaagtc ccttcaagag ttatcgttga tacccctcgt agtgcacatt    180
   cctttaacgc ttcaaaatct gtaaagcacg ccatatcgcc gaaaggcaca cttaattatt     240
   aagaggtaat acaccatgaa tcacaaagtg catcatcatc atcatcacat gcaagtttca     300
   gttgaaacca ctcaaggcct tggccgccgt gtaacgatta ctatcgctgc tgacagcatc     360
   gagaccgctg ttaaaagcga gctggtcaac gttgcgaaaa aagtacgtat tgacggcttc     420
   cgcaagggca aagtgccaat gaatatcgtt gctcagcgtt atggcgcgtc tgtacgccag     480
   gacgttctgg gtgacctgat gagccgtaac ttcattgacg ccatcattaa agaaaaaatc     540
   aatccggctg gcgcaccgac ttatgttccg ggcgaataca agctgggtga agacttcact     600
   tactctgtag agtttgaagt ttatccggaa gttgaactgc aaggtctgga agcgatcgaa     660
   gttgaaaaac cgatcgttga agtgaccgac gctgacgttg acggcatgct ggatactctg     720
   cgtaaacagc aggcgacctg gaaagaaaaa gacggcgctg ttgaagcaga agaccgcgtg 780
   accatcgact tcaccggttc tgtagacggc gaagagttcg aaggcggtaa agcgtctgat 840
   ttcgtactgg cgatgggcca gggtcgtatg atcccgggct ttgaagacgg tatcaaaggc 900
   cacaaagctg gcgaagagtt caccatcgac gtgaccttcc cggaagaata ccacgcagaa 960
   aacctgaaag gtaaagcagc gaaattcgct atcaacctga agaaagttga agagcgtgaa 1020
   ctgccggaac tgaccgcaga gttcatcaaa cgtttcggcg ttgaagatgg ttccgtagaa 1080
   ggtctgcgcg ctgaagtgcg taaaaacatg gagcgcgagc tgaagagcgc catccgtaac 1140
   cgcgttaagt ctcaggcgat cgaaggtctg gtaaaagcta acgacatcga cgtaccggct 1200
   gcgctgatcg acagcgaaat cgacgttctg cgtcgccagg ctgcacagcg tttcggtggc 1260
   aacgaaaaac aagctctgga actgccgcgc gaactgttcg aagaacaggc taaacgccgc 1320
   gtagttgttg gcctgctgct gggcgaagtt atccgcacca acgagctgaa agctgacgaa 1380
   gagcgcgtga aaggcctgat cgaagagatg gcttctgcgt acgaagatcc gaaagaagtt 1440
   atcgagttct acagcaaaaa caaagaactg atggacaaca tgcgcaatgt tgctctggaa 1500
   gaacaggctg ttgaagctgt actggcgaaa gcgaaagtga ctgaaaaaga aaccactttc 1560
   aacgagctga tgaaccagca ggcgtccgcg ggtctggaag ttctgttcca ggggccctcc 1620
   gcgggtctgg tgccacgcgg tagtggtggt atcgaaggta ggatgtgctc tgtttctacc 1680
   gagaacgttt ccttcactga gacggaaacc gaggcacgtc gtagcgcgaa ctacgagccg 1740
   aatagctggg actacgattt cctgctgtct tccgatactg acgaatctat tgaggtgtac 1800
   aaagacaaag caaagaaact ggaggctgaa gtgcgccgcg aaattaacaa cgagaaagct 1860
   gaattcctga ctctgctgga gctgatcgat aacgtacagc gcctgggtct gggttaccgc 1920
   ttcgaatctg atatccgtcg cgcactggat cgtttcgtaa gcagcggcgg tttcgatggc 1980
   gtgaccaaaa cgagcctgca cgctaccgcg ctgtccttcc gtctgctgcg tcagcacggc 2040
   ttcgaagttt ctcaggaagc attctccggt ttcaaagatc aaaacggtaa cttcctggaa 2100
   aacctgaaag aagacactaa ggcgatcctg agcctgtatg aggcaagctt tctggccctg 2160
   gagggtgaga acatcctgga tgaggcgcgc gtattcgcca tctcccatct gaaagagctg 2220
   tctgaagaga aaatcggtaa ggaactggca gagcaggtta atcacgcact ggaactgccg 2280
   ctgcatcgtc gtacccagcg tctggaggcg gtttggtcca tcgaagcgta ccgcaaaaag 2340
   gaggatgcta accaggttct gctggaactg gccatcctgg actacaacat gatccagtcc 2400
   gtttaccagc gtgatctgcg tgaaacctcc cgttggtggc gccgtgtggg cctggcgacc 2460
   aaactgcact tcgctaagga ccgcctgatt gagtcttttt actgggcagt cggcgttgcg 2520
   ttcgaacctc agtattctga ctgccgtaac agcgttgcga aaatgttcag cttcgttact 2580
   attatcgacg acatctacga cgtttacggt actctggacg agctggaact gtttaccgac 2640
   gctgtcgaac gttgggatgt taacgccatc aacgatctgc ctgactacat gaaactgtgc 2700
   ttcctggcac tgtataacac gatcaacgaa attgcatacg acaacctgaa agacaaaggt 2760
   gaaaacatcc tgccgtacct gactaaagcg tgggcggatc tgtgtaacgc ttttctgcaa 2820
   gaagcgaaat ggctgtataa caaatccact ccgacctttg acgattattt cggcaatgcc 2880
   tggaaatcca gctctggccc gctgcaactg atcttcgctt attttgcggt tgtccaaaac 2940
   atcaaaaagg aggaaattga aaacctgcaa aaataccacg atatcattag ccgtccttct 3000
   catatctttc gcctgtgcaa cgacctggca agcgcgtccg cagagatcgc acgtggcgaa 3060
   accgctaact ctgtttcctg ctacatgcgc accaagggca tttccgaaga gctggcaacc 3120
   gagagcgtaa tgaatctgat cgacgaaacc tgtaagaaaa tgaacaaaga aaaactgggt 3180
   ggctccctgt tcgctaaacc gttcgtagag actgctatta acctggcacg tcagagccac 3240
   tgcacctacc acaatggtga cgcacatact agcccggatg aactgactcg taaacgtgta 3300
   ctgtctgtta tcaccgaacc gattctgccg ttcgaacgtt aataggtaat ctctgcttaa 3360
   aagcacagaa tctaagatcc ctgccatttg gcggggattt ttttatttgt tttcaggaaa 3420
   taaataatcg atcgcgtaat aaaatctatt attatttttg tgaagaataa atttgggtgc 3480
   aatgagaatg cgcaggccct ttcgtctcgc gcgtttcggt gatgacggtg aaaacctctg 3540
   acacatgcag ctcccggaga cggtcacagc ttgtctgtaa gcggatgccg ggagcagaca 3600
   agcccgtcag ggcgcgtcag cgggtgttgg cgggtgtcgg ggctggctta actatgcggc 3660
   atcagagcag attgtactga gagtgcacca taaaattgta aacgttaata ttttgttaaa 3720
   attcgcgtta aatttttgtt aaatcagctc attttttaac caataggccg aaatcggcaa 3780
   aatcccttat aaatcaaaag aatagcccga gatagggttg agtgttgttc cagtttggaa 3840
   caagagtcca ctattaaaga acgtggactc caacgtcaaa gggcgaaaaa ccgtctatca 3900
   gggcgatggc ccactacgtg aaccatcacc caaatcaagt tttttggggt cgaggtgccg 3960
   taaagcacta aatcggaacc ctaaagggag cccccgattt agagcttgac ggggaaagcc 4020
   ggcgaacgtg gcgagaaagg aagggaagaa agcgaaagga gcgggcgcta gggcgctggc 4080
   aagtgtagcg gtcacgctgc gcgtaaccac cacacccgcc gcgcttaatg cgccgctaca 4140
   gggcgcgtac tatggttgct ttgacgtatg cggtgtgaaa taccgcacag atgcgtaagg 4200
   agaaaatacc gcatcaggcg tcaggtggca cttttcgggg aaatgtgcgc ggaaccccta 4260
   tttgtttatt tttctaaata cattcaaata tgtatccgct catgagacaa taaccctgat 4320
   aaatgcttca ataatattga aaaaggaaga gtatgagtat tcaacatttc cgtgtcgccc 4380
   ttattccctt ttttgcggca ttttgccttc ctgtttttgc tcacccagaa acgctggtga 4440
   aagtaaaaga tgctgaagat cagttgggtg cacgagtggg ttacatcgaa ctggatctca 4500
   acagcggtaa gatccttgag agttttcgcc ccgaagaacg ttttccaatg atgagcactt 4560
   ttaaagttct gctatgtggc gcggtattat cccgtattga cgccgggcaa gagcaactcg 4620
   gtcgccgcat acactattct cagaatgact tggttgagta ctcaccagtc acagaaaagc 4680
   atcttacgga tggcatgaca gtaagagaat tatgcagtgc tgccataacc atgagtgata 4740
   acactgcggc caacttactt ctgacaacga tcggaggacc gaaggagcta accgcttttt 4800
   tgcacaacat gggggatcat gtaactcgcc ttgatcgttg ggaaccggag ctgaatgaag 4860
   ccataccaaa cgacgagcgt gacaccacga tgcctgtagc aatggcaaca acgttgcgca 4920
   aactattaac tggcgaacta cttactctag cttcccggca acaattaata gactggatgg 4980
   aggcggataa agttgcagga ccacttctgc gctcggccct tccggctggc tggtttattg 5040
   ctgataaatc tggagccggt gagcgtgggt ctcgcggtat cattgcagca ctggggccag 5100
   atggtaagcc ctcccgtatc gtagttatct acacgacggg gagtcaggca actatggatg 5160
   aacgaaatag acagatcgct gagataggtg cctcactgat taagcattgg taactgtcag 5220
   accaagttta ctcatatata ctttagattg atttaaaact tcatttttaa tttaaaagga 5280
   tctaggtgaa gatccttttt gataatctca tgaccaaaat cccttaacgt gagttttcgt 5340
   tccactgagc gtcagacccc gtagaaaaga tcaaaggatc ttcttgagat cctttttttc 5400
   tgcgcgtaat ctgctgcttg caaacaaaaa aaccaccgct accagcggtg gtttgtttgc 5460
   cggatcaaga gctaccaact ctttttccga aggtaactgg cttcagcaga gcgcagatac 5520
   caaatactgt tcttctagtg tagccgtagt taggccacca cttcaagaac tctgtagcac 5580
   cgcctacata cctcgctctg ctaatcctgt taccagtggc tgctgccagt ggcgataagt 5640
   cgtgtcttac cgggttggac tcaagacgat agttaccgga taaggcgcag cggtcgggct 5700
   gaacgggggg ttcgtgcaca cagcccagct tggagcgaac gacctacacc gaactgagat 5760
   acctacagcg tgagctatga gaaagcgcca cgcttcccga agggagaaag gcggacaggt 5820
   atccggtaag cggcagggtc ggaacaggag agcgcacgag ggagcttcca gggggaaacg 5880
   cctggtatct ttatagtcct gtcgggtttc gccacctctg acttgagcgt cgatttttgt 5940
   gatgctcgtc aggggggcgg agcctatgga aaaacgccag caacgcggcc tttttacggt 6000
   tcctggcctt ttgctggcct tttgctcaca tagtcatgcc ccgcgcccac cggaaggagc 6060
   tgactgggtt gaaggctctc aagggcatcg gtcgagatcc cggtgcctaa tgagtgagct 6120
   aacttacatt aattgcgttg cgctcactgc ccgctttcca gtcgggaaac ctgtcgtgcc 6180
   agctgcatta atgaatcggc caacgcgcgg ggagaggcgg tttgcgtatt gggcgccagg 6240
   gtggtttttc ttttcaccag tgagacgggc aacagctgat tgcccttcac cgcctggccc 6300
   tgagagagtt gcagcaagcg gtccacgctg gtttgcccca gcaggcgaaa atcctgtttg 6360
   atggtggtta acggcgggat ataacatgag ctgtcttcgg tatcgtcgta tcccactacc 6420
   gagatatccg caccaacgcg cagcccggac tcggtaatgg cgcgcattgc gcccagcgcc   6480
   atctgatcgt tggcaaccag catcgcagtg ggaacgatgc cctcattcag catttgcatg   6540
   gtttgttgaa aaccggacat ggcactccag tcgccttccc gttccgctat cggctgaatt   6600
   tgattgcgag tgagatattt atgccagcca gccagacgca gacgcgccga gacagaactt   6660
   aatgggcccg ctaacagcgc gatttgctgg tgacccaatg cgaccagatg ctccacgccc   6720
   agtcgcgtac cgtcttcatg ggagaaaata atactgttga tgggtgtctg gtcagagaca   6780
   tcaagaaata acgccggaac attagtgcag gcagcttcca cagcaatggc atcctggtca   6840
   tccagcggat agttaatgat cagcccactg acgcgttgcg cgagaagatt gtgcaccgcc   6900
   gctttacagg cttcgacgcc gcttcgttct accatcgaca ccaccacgct ggcacccagt   6960
   tgatcggcgc gagatttaat cgccgcgaca atttgcgacg gcgcgtgcag ggccagactg   7020
   gaggtggcaa cgccaatcag caacgactgt ttgcccgcca gttgttgtgc cacgcggttg   7080
   ggaatgtaat tcagctccgc catcgccgct tccacttttt cccgcgtttt cgcagaaacg   7140
   tggctggcct ggttcaccac gcgggaaacg gtctgataag agacaccggc atactctgcg   7200
   acatcgtata acgttactgg tttcacattc accaccctga attgactctc ttccgggcgc   7260
   tatcatgcca taccgcgaaa ggttttgcgc cattcgatgg tgtccgggat ctcgacgctc   7320
   tcccttatgc gactcctgca ttaggaagca gcccagtagt aggttgaggc cgttgagcac   7380
   cgccgccgc                                                           7389
<210> 96
   <211> 50
   <212> DNA
   <213> Artificial Sequence
<220>
   <223> Primer 1 for amplifying IspSK gene
<400> 96
   ggtagtggtg gtatcgaagg taggatgtgt gcgacctctt ctcaatttac 50
<210> 97
   <211> 56
   <212> DNA
   <213> Artificial Sequence
<220>
   <223> Primer 2 for amplifying IspSK gene
<400> 97
   gattctgtgc ttttaagcag agattaccta ttagacatac atcagctggt taatcg 56
<210> 98
   <211> 7404
   <212> DNA
   <213> Artificial Sequence
<220>
   <223> Nucleotide sequence of plasmid for expressing IspSK gene (pCold-TF-IspSK)
<400> 98
   aaggaatggt gtggccgatt aatcataaat atgaaaaata attgttgcat cacccgccaa 60
   tgcgtggctt aatgcacatc aaattgtgag cggataacaa tttgatgtgc tagcgcatat     120
   ccagtgtagt aaggcaagtc ccttcaagag ttatcgttga tacccctcgt agtgcacatt     180
   cctttaacgc ttcaaaatct gtaaagcacg ccatatcgcc gaaaggcaca cttaattatt 240
   aagaggtaat acaccatgaa tcacaaagtg catcatcatc atcatcacat gcaagtttca 300
   gttgaaacca ctcaaggcct tggccgccgt gtaacgatta ctatcgctgc tgacagcatc 360
   gagaccgctg ttaaaagcga gctggtcaac gttgcgaaaa aagtacgtat tgacggcttc 420
   cgcaagggca aagtgccaat gaatatcgtt gctcagcgtt atggcgcgtc tgtacgccag 480
   gacgttctgg gtgacctgat gagccgtaac ttcattgacg ccatcattaa agaaaaaatc 540
   aatccggctg gcgcaccgac ttatgttccg ggcgaataca agctgggtga agacttcact 600
   tactctgtag agtttgaagt ttatccggaa gttgaactgc aaggtctgga agcgatcgaa 660
   gttgaaaaac cgatcgttga agtgaccgac gctgacgttg acggcatgct ggatactctg 720
   cgtaaacagc aggcgacctg gaaagaaaaa gacggcgctg ttgaagcaga agaccgcgtg 780
   accatcgact tcaccggttc tgtagacggc gaagagttcg aaggcggtaa agcgtctgat 840
   ttcgtactgg cgatgggcca gggtcgtatg atcccgggct ttgaagacgg tatcaaaggc 900
   cacaaagctg gcgaagagtt caccatcgac gtgaccttcc cggaagaata ccacgcagaa 960
   aacctgaaag gtaaagcagc gaaattcgct atcaacctga agaaagttga agagcgtgaa 1020
   ctgccggaac tgaccgcaga gttcatcaaa cgtttcggcg ttgaagatgg ttccgtagaa 1080
   ggtctgcgcg ctgaagtgcg taaaaacatg gagcgcgagc tgaagagcgc catccgtaac 1140
   cgcgttaagt ctcaggcgat cgaaggtctg gtaaaagcta acgacatcga cgtaccggct 1200
   gcgctgatcg acagcgaaat cgacgttctg cgtcgccagg ctgcacagcg tttcggtggc 1260
   aacgaaaaac aagctctgga actgccgcgc gaactgttcg aagaacaggc taaacgccgc 1320
   gtagttgttg gcctgctgct gggcgaagtt atccgcacca acgagctgaa agctgacgaa 1380
   gagcgcgtga aaggcctgat cgaagagatg gcttctgcgt acgaagatcc gaaagaagtt 1440
   atcgagttct acagcaaaaa caaagaactg atggacaaca tgcgcaatgt tgctctggaa 1500
   gaacaggctg ttgaagctgt actggcgaaa gcgaaagtga ctgaaaaaga aaccactttc 1560
   aacgagctga tgaaccagca ggcgtccgcg ggtctggaag ttctgttcca ggggccctcc 1620
   gcgggtctgg tgccacgcgg tagtggtggt atcgaaggta ggatgtgtgc gacctcttct 1680
   caatttactc agattaccga gcataattcc cgtcgttccg caaactatca gccaaacctg 1740
   tggaatttcg aattcctgca atccctggag aacgacctga aagtggaaaa gctggaggag 1800
   aaagcgacca aactggagga agaagttcgc tgcatgatca accgtgtaga cacccagccg 1860
   ctgtccctgc tggagctgat cgacgatgtg cagcgcctgg gtctgaccta caaatttgaa 1920
   aaagacatca ttaaagccct ggaaaacatc gtactgctgg acgaaaacaa aaagaacaaa 1980
   tctgacctgc acgcaaccgc tctgtctttc cgtctgctgc gtcagcacgg tttcgaggtt 2040
   tctcaggatg tttttgagcg tttcaaggat aaagaaggtg gtttcagcgg tgaactgaaa 2100
   ggtgacgtcc aaggcctgct gagcctgtat gaagcgtctt acctgggttt cgagggtgag 2160
   aacctgctgg aggaggcgcg taccttttcc atcacccacc tgaagaacaa cctgaaagaa 2220
   ggcattaata ccaaggttgc agaacaagtg agccacgccc tggaactgcc atatcaccag 2280
   cgtctgcacc gtctggaggc acgttggttc ctggataaat acgaaccgaa agaaccgcat 2340
   caccagctgc tgctggagct ggcgaagctg gattttaaca tggtacagac cctgcaccag 2400
   aaagagctgc aagatctgtc ccgctggtgg accgagatgg gcctggctag caaactggat 2460
   tttgtacgcg accgcctgat ggaagtttat ttctgggcac tgggtatggc gccagacccg 2520
   cagtttggtg aatgtcgcaa agctgttact aaaatgtttg gtctggtgac gatcatcgat 2580
   gacgtgtatg acgtttatgg cactctggac gaactgcaac tgttcaccga tgctgtagag 2640
   cgctgggacg ttaacgctat taacaccctg ccggactata tgaaactgtg tttcctggca 2700
   ctgtacaaca ccgttaacga cacgtcctat tctattctga aagagaaagg tcataacaac 2760
   ctgtcctatc tgacgaaaag ctggcgtgaa ctgtgcaaag cctttctgca agaggcgaaa 2820
   tggtccaaca acaaaattat cccggctttc tccaagtacc tggaaaacgc cagcgtttcc 2880
   tcctccggtg tagcgctgct ggcgccgtct tacttttccg tatgccagca gcaggaagac 2940
   atctccgacc acgcgctgcg ttccctgacc gacttccatg gtctggtgcg ttctagctgc 3000
   gttatcttcc gcctgtgcaa cgatctggcc acctctgcgg cggagctgga acgtggcgag 3060
   actaccaatt ctatcattag ctacatgcac gaaaacgatg gtaccagcga ggaacaggcc 3120
   cgcgaagaac tgcgtaaact gatcgacgcc gaatggaaaa agatgaatcg tgaacgcgtt 3180
   agcgactcca ccctgctgcc taaagcgttc atggaaatcg cagttaacat ggcacgtgtt 3240
   tcccactgca cctaccagta tggcgatggt ctgggtcgcc cagactacgc gactgaaaac 3300
   cgcatcaaac tgctgctgat tgaccctttc ccgattaacc agctgatgta tgtctaatag 3360
   gtaatctctg cttaaaagca cagaatctaa gatccctgcc atttggcggg gattttttta 3420
   tttgttttca ggaaataaat aatcgatcgc gtaataaaat ctattattat ttttgtgaag 3480
   aataaatttg ggtgcaatga gaatgcgcag gccctttcgt ctcgcgcgtt tcggtgatga 3540
   cggtgaaaac ctctgacaca tgcagctccc ggagacggtc acagcttgtc tgtaagcgga 3600
   tgccgggagc agacaagccc gtcagggcgc gtcagcgggt gttggcgggt gtcggggctg 3660
   gcttaactat gcggcatcag agcagattgt actgagagtg caccataaaa ttgtaaacgt 3720
   taatattttg ttaaaattcg cgttaaattt ttgttaaatc agctcatttt ttaaccaata 3780
   ggccgaaatc ggcaaaatcc cttataaatc aaaagaatag cccgagatag ggttgagtgt 3840
   tgttccagtt tggaacaaga gtccactatt aaagaacgtg gactccaacg tcaaagggcg 3900
   aaaaaccgtc tatcagggcg atggcccact acgtgaacca tcacccaaat caagtttttt 3960
   ggggtcgagg tgccgtaaag cactaaatcg gaaccctaaa gggagccccc gatttagagc 4020
   ttgacgggga aagccggcga acgtggcgag aaaggaaggg aagaaagcga aaggagcggg 4080
   cgctagggcg ctggcaagtg tagcggtcac gctgcgcgta accaccacac ccgccgcgct 4140
   taatgcgccg ctacagggcg cgtactatgg ttgctttgac gtatgcggtg tgaaataccg 4200
   cacagatgcg taaggagaaa ataccgcatc aggcgtcagg tggcactttt cggggaaatg 4260
   tgcgcggaac ccctatttgt ttatttttct aaatacattc aaatatgtat ccgctcatga 4320
   gacaataacc ctgataaatg cttcaataat attgaaaaag gaagagtatg agtattcaac 4380
   atttccgtgt cgcccttatt cccttttttg cggcattttg ccttcctgtt tttgctcacc 4440
   cagaaacgct ggtgaaagta aaagatgctg aagatcagtt gggtgcacga gtgggttaca 4500
   tcgaactgga tctcaacagc ggtaagatcc ttgagagttt tcgccccgaa gaacgttttc 4560
   caatgatgag cacttttaaa gttctgctat gtggcgcggt attatcccgt attgacgccg 4620
   ggcaagagca actcggtcgc cgcatacact attctcagaa tgacttggtt gagtactcac 4680
   cagtcacaga aaagcatctt acggatggca tgacagtaag agaattatgc agtgctgcca 4740
   taaccatgag tgataacact gcggccaact tacttctgac aacgatcgga ggaccgaagg 4800
   agctaaccgc ttttttgcac aacatggggg atcatgtaac tcgccttgat cgttgggaac 4860
   cggagctgaa tgaagccata ccaaacgacg agcgtgacac cacgatgcct gtagcaatgg 4920
   caacaacgtt gcgcaaacta ttaactggcg aactacttac tctagcttcc cggcaacaat 4980
   taatagactg gatggaggcg gataaagttg caggaccact tctgcgctcg gcccttccgg 5040
   ctggctggtt tattgctgat aaatctggag ccggtgagcg tgggtctcgc ggtatcattg 5100
   cagcactggg gccagatggt aagccctccc gtatcgtagt tatctacacg acggggagtc 5160
   aggcaactat ggatgaacga aatagacaga tcgctgagat aggtgcctca ctgattaagc 5220
   attggtaact gtcagaccaa gtttactcat atatacttta gattgattta aaacttcatt 5280
   tttaatttaa aaggatctag gtgaagatcc tttttgataa tctcatgacc aaaatccctt 5340
   aacgtgagtt ttcgttccac tgagcgtcag accccgtaga aaagatcaaa ggatcttctt 5400
   gagatccttt ttttctgcgc gtaatctgct gcttgcaaac aaaaaaacca ccgctaccag 5460
   cggtggtttg tttgccggat caagagctac caactctttt tccgaaggta actggcttca 5520
   gcagagcgca gataccaaat actgttcttc tagtgtagcc gtagttaggc caccacttca 5580
   agaactctgt agcaccgcct acatacctcg ctctgctaat cctgttacca gtggctgctg 5640
   ccagtggcga taagtcgtgt cttaccgggt tggactcaag acgatagtta ccggataagg 5700
   cgcagcggtc gggctgaacg gggggttcgt gcacacagcc cagcttggag cgaacgacct 5760
   acaccgaact gagataccta cagcgtgagc tatgagaaag cgccacgctt cccgaaggga 5820
   gaaaggcgga caggtatccg gtaagcggca gggtcggaac aggagagcgc acgagggagc 5880
   ttccaggggg aaacgcctgg tatctttata gtcctgtcgg gtttcgccac ctctgacttg 5940
   agcgtcgatt tttgtgatgc tcgtcagggg ggcggagcct atggaaaaac gccagcaacg 6000
   cggccttttt acggttcctg gccttttgct ggccttttgc tcacatagtc atgccccgcg 6060
   cccaccggaa ggagctgact gggttgaagg ctctcaaggg catcggtcga gatcccggtg 6120
   cctaatgagt gagctaactt acattaattg cgttgcgctc actgcccgct ttccagtcgg 6180
   gaaacctgtc gtgccagctg cattaatgaa tcggccaacg cgcggggaga ggcggtttgc 6240
   gtattgggcg ccagggtggt ttttcttttc accagtgaga cgggcaacag ctgattgccc 6300
   ttcaccgcct ggccctgaga gagttgcagc aagcggtcca cgctggtttg ccccagcagg 6360
   cgaaaatcct gtttgatggt ggttaacggc gggatataac atgagctgtc ttcggtatcg 6420
   tcgtatccca ctaccgagat atccgcacca acgcgcagcc cggactcggt aatggcgcgc 6480
   attgcgccca gcgccatctg atcgttggca accagcatcg cagtgggaac gatgccctca 6540
   ttcagcattt gcatggtttg ttgaaaaccg gacatggcac tccagtcgcc ttcccgttcc 6600
   gctatcggct gaatttgatt gcgagtgaga tatttatgcc agccagccag acgcagacgc 6660
   gccgagacag aacttaatgg gcccgctaac agcgcgattt gctggtgacc caatgcgacc 6720
   agatgctcca cgcccagtcg cgtaccgtct tcatgggaga aaataatact gttgatgggt 6780
   gtctggtcag agacatcaag aaataacgcc ggaacattag tgcaggcagc ttccacagca 6840
   atggcatcct ggtcatccag cggatagtta atgatcagcc cactgacgcg ttgcgcgaga 6900
   agattgtgca ccgccgcttt acaggcttcg acgccgcttc gttctaccat cgacaccacc 6960
   acgctggcac ccagttgatc ggcgcgagat ttaatcgccg cgacaatttg cgacggcgcg 7020
   tgcagggcca gactggaggt ggcaacgcca atcagcaacg actgtttgcc cgccagttgt 7080
   tgtgccacgc ggttgggaat gtaattcagc tccgccatcg ccgcttccac tttttcccgc 7140
   gttttcgcag aaacgtggct ggcctggttc accacgcggg aaacggtctg ataagagaca 7200
   ccggcatact ctgcgacatc gtataacgtt actggtttca cattcaccac cctgaattga 7260
   ctctcttccg ggcgctatca tgccataccg cgaaaggttt tgcgccattc gatggtgtcc 7320
   gggatctcga cgctctccct tatgcgactc ctgcattagg aagcagccca gtagtaggtt 7380
   gaggccgttg agcaccgccg ccgc                                    7404

## Description

### TECHNICAL FIELD

The present invention relates to an isoprene synthase and a gene encoding the same, and the like.

### BACKGROUND ART

Natural rubber is a very important raw material in tire and rubber industries. While its demand will be expanded in future due to motorization mainly in emerging countries, it is not easy to increase agricultural farms in view of regulation for deforestation and competition with palm, and increase in yield of natural rubber is hardly expected. Thus, balance of demand and supply is predicted to become tight. Synthesized polyisoprene is available as a material in place of the natural rubber. Its raw material monomer (isoprene (2-methyl-1,3-butadiene)) is mainly obtained by extracting from a C5 fraction obtained by cracking of naphtha. However in recent years, with the use of light feed crackers, an amount of produced isoprene tends to decrease and its supply is concerned. Also in recent years, since variation of oil price greatly influences, it is requested to establish a system in which isoprene derived from non-oil sources is produced inexpensively in order to stably ensure the supply of an isoprene monomer.

Concerning such a request, a method in which the isoprene monomer is produced using a transformant obtained by introducing an isoprene synthase gene and a mutant thereof derived from isolated Kudzu or Poplar into a microorganism for fermental production has been disclosed (see, Patent literatures 1 and 2).

### PRIOR ART REFERENCES

### PATENT LITERATURES

Patent literature 1: JP Publication No. 2011-505841
Patent literature 2: JP Publication No. 2011-518564

### NON-PATENT LITERATURES

Non-Patent Literature 1: Kesselmeier J. et al., Journal of Atmospheric Chemistry, vol.33, pp.23-88, 1999.
Non-Patent Literature 2: Monson R. K. et al., Plant Physiol., vol.98, pp.1175-1180, 1992.
Non-Patent Literature 3: Kuzma J. et al., Plant Physiol., vo.101, pp.435-440, 1993.

### DISCLOSURE OF INVENTION

### PROBLEM TO BE SOLVED BY THE INVENTION

However, an enzymatic activity of the isoprene synthase described in Patent literatures 1 and 2 was low, and there was still room to improve it in terms of producing isoprene with high productivity when these isoprene synthase genes were used.

The present invention has been made in the light of the above circumstance, and it is an object of the present invention to provide means useful for establishing an excellent isoprene monomer production system.

### MEANS FOR SOLVING PROBLEM

As a result of an extensive study for solving the above problem, the present inventors have found that an isoprene synthase derived from Mucuna (*Mucuna pruriens*) is excellent in ability to produce the isoprene monomer, and have completed the present invention.

Accordingly, the present invention is as follows.
[1] A polynucleotide of the following (a) or (b):
   (a) a polynucleotide comprising (i) the nucleotide sequence represented by SEQ ID NO:1, or (ii) the nucleotide sequence consisting of the nucleotide residues at positions 133 to 1785 in the nucleotide sequence represented by SEQ ID NO:1; or
   (b) a polynucleotide that comprises a nucleotide sequence having 90% or more identity to the nucleotide sequence of (i) or (ii), and encodes a protein having an isoprene synthase activity.
[2] The polynucleotide according to [1], wherein the polynucleotide is derived from Mucuna.
[3] A protein of the following (A), (B), or (C):
   (A) a protein comprising (i') the full length amino acid sequence represented by SEQ ID NO:2, or (ii') the amino acid sequence consisting of the amino acid residues at positions 45 to 594 in the amino acid sequence represented by SEQ OD NO:2;
   (B) a protein that comprises an amino acid sequence having 90% or more identity to the amino acid sequence of (i') or (ii'), and has an isoprene synthase activity; or
   (C) a protein that comprises an amino acid sequence having a deletion, substitution, addition or insertion of one to thirty amino acids in the amino acid sequence of (i') or (ii'), and has an isoprene synthase activity.
[4] An expression vector comprising the polynucleotide according to [1] or [2], or a polynucleotide encoding the protein according to [3].
[5] A transformant prepared by introducing the expression vector according to [4] into a host.
[6] The transformant according to [5], wherein the host has an ability to synthesize dimethylallyl diphosphate via a methylerythritol phosphate pathway.
[7] The transformant according to [6], wherein the host is *Escherichia coli*.
[8] The transformant according to any of [5] to [7], wherein the transformant has an ability to synthesize dimethylallyl diphosphate via both a mevalonate pathway and a methylerythritol phosphate pathway.
[9] The transformant according to [5], wherein the host is a microorganism belonging to genus *Corynebacterium,* genus *Pantoea,* genus *Enterobacter,* or genus *Saccharomyces*.
[10] A method of producing the protein according to [3], comprising forming the protein according to [3] using the transformant according to any of [5] to [9].
[11] A method of producing an isoprene monomer, comprising forming the isoprene monomer from dimethylallyl diphosphate in the presence of the protein according to [3].
[12] The method according to [11], wherein the isoprene monomer is formed by culturing the transformant according to any of [5] to [9].
[13] The method according to [12], wherein the dimethylallyl diphosphate is supplied from a carbon source in a medium by culturing the transformant.
[14] A method of producing an isoprene polymer, comprising (I) and (II):
   (I) forming an isoprene monomer by the method according to any of [11] to [13]; and
   (II) polymerizing the isoprene monomer to form the isoprene polymer.

### EFFECT OF THE INVENTION

According to the present invention, it is possible to establish an excellent isoprene monomer production system.

### BRIEF DESCRIPTION OF DRAWINGS

FIG. 1 is a graph showing an amount of isoprene produced per unit weight of dry leaves from various plants;
FIG. 2 is a graph showing an amount of isoprene produced per amount of total protein extracted from leaves of various plants;
FIG. 3 is a view showing an outline of a downstream and its proximal region of a mevalonate pathway on a fixed chromosome; and
FIG. 4 is a view showing the downstream and its proximal region of a mevalonate pathway controlled by a tac promoter on a chromosome.

### EMBODIMENTS FOR CARRYING OUT THE INVENTION

### <Polynucleotide encoding isoprene synthase>

The present invention provides a polynucleotide encoding an isoprene synthase.

The isoprene synthase is an enzyme that converts dimethylallyl diphosphate into isoprene. The present inventors firstly evaluated an ability to produce isoprene in various plants under the same condition at high temperature. As a result, it was found that the ability of Mucuna (*Mucuna bracteata*) to produce isoprene was several times to several ten times higher than the ability of Poplar (*Populus nigra var. italic*) and Kudzu (*Pueraria lobata*) to produce isoprene. However, it is not clarified whether this high ability to produce isoprene is attributed to a high ability of the plant to produce dimethylallyl diphosphate, high production of the isoprene synthase by the plant, or a high specific enzymatic activity of the isoprene synthase that the plant has. In particular, pH dependency and the like of an isoprene synthase activity in Mucuna,was examined using the enzyme partially purified by ammonium sulfate fractionation by Monson and Kuzma et al. in Non-Patent Literatures 2 and 3, but it was not clear whether the specific activity in Mucuna was higher than that in Kudzu and Poplar or not. Thus, the present inventors partially purified the isoprene synthase from leaves of Mucuna and Kudzu by the ammonium sulfate fractionation, and examined the specific activity of the crude extract solution. As a result, it was found that the specific activity of the isoprene synthase derived from Mucuna was several times to several ten times higher than that derived from Kudzu. It was also demonstrated that the measured specific activity of the isoprene synthase from Mucuna was several times to several ten times higher than the specific activity of the isoprene synthase in the crude enzyme from Poplar reported in Patent Literatures 1 and 2. From the above, it was suggested that Mucuna potentially had highly functional isoprene synthase.

Subsequently, a nucleotide sequence of an isoprene synthase gene from Mucuna was analyzed. One example of analysis methods will be described below.

It is known that the isoprene synthase is expressed only in the leaves in the plant and its expression level is increased under strong light and high temperature. Thus, total RNA was firstly extracted from the leaves of Mucuna where transcription of isoprene synthase mRNA had been induced under light illumination at temperature of 40°C. It was confirmed that the extracted total RNA was not decomposed and not contaminated with genomic DNA. Then, the total RNA was converted into a double strand and fragmented, and only nucleotide sequences having a poly A sequence at a 3' end were analyzed using a high performance sequencer. Overlapped sequences were assembled to obtain a plurality of contig sequences. BLAST search was performed for these contig sequences, and a contig sequence having homology (identity of nucleotide sequences) to registered sequences of the known isoprene synthase genes from Kudzu and Poplar was detected, and a partial sequence of the isoprene synthase gene from Mucuna was obtained. Based on this partial sequence, 5' RACE (Rapid Amplification of cDNA Ends) was performed using standard methods to analyze a full length nucleotide sequence of the isoprene synthase gene from Mucuna, which is represented by SEQ ID NO:1.

cDNA of the isoprene synthase from Mucuna can be obtained, for example, by RT-PCR with the total RNA obtained above as a template using primers designed based on the analyzed nucleotide sequence information of the isoprene synthase gene from Mucuna.

In one embodiment, the polynucleotide of the present invention is a polynucleotide comprising (i) the nucleotide sequence represented by SEQ ID NO:1, or (ii) the nucleotide sequence consisting of the nucleotide residues at positions 133 to 1785 in the nucleotide sequence represented by SEQ ID NO:1. The nucleotide sequence represented by SEQ ID NO:1 can encode the amino acid sequence represented by SEQ ID NO:2, a nucleotide sequence consisting of the nucleotide residues at positions 1 to 132 can encode a putative chloroplast localization signal, and the nucleotide sequence consisting of the nucleotide residues at positions 133 to 1785 can encode an amino acid sequence of mature isoprene synthase.

In another embodiment, the polynucleotide of the present invention is a polynucleotide that comprises a nucleotide sequence having 90% or more identity to the nucleotide sequence of (i) or (ii) above, and encodes a protein having an isoprene synthase activity. The percent identity to the nucleotide sequence may be 91%, 92%, 93%, 94%, 95%, 96%, 97%, 98%, or 99% or more. The isoprene synthase activity refers to an activity to form isoprene from dimethylallyl diphosphate (DMAPP) (the same meaning shall apply hereinafter).

The percent identity of the nucleotide sequences, and the percent identity of the amino acid sequences as described later can be determined using algorithm BLAST (Pro. Natl. Acad. Sci. USA, 90, 5873 (1993)) by Karlin and Altschul, and FASTA (Methods Enzymol., 183, 63 (1990)) by Pearson. The programs referred to as BLASTP and BLASTN were developed based on this algorithm BLAST (see http://www.ncbi.nlm.nih.gov). Thus, the percent identity of the nucleotide sequences and the amino acid sequences may be calculated using these programs with default setting. Also, for example, a numerical value obtained by calculating similarity as a percentage at a setting of "unit size to compare=2" using the full length of a polypeptide portion encoded in ORF with the software GENETYX Ver. 7.0.9 from Genetyx Corporation employing Lipman-Pearson method may be used as the homology of the amino acid sequences. The lowest value among the values derived from these calculations may be employed as the percent identity of the nucleotide sequences and the amino acid sequences.

In one disclosure, the polynucleotide is a polynucleotide that hybridizes under a stringent condition with a polynucleotide consisting of the nucleotide sequence complementary to the nucleotide sequence of (i) or (ii) above, and encodes a protein having an isoprene synthase activity.

The "stringent condition" refers to a condition where a so-called specific hybrid is formed whereas a nonspecific hybrid is not formed. It is difficult to clearly quantify such a condition. However, to cite a case, such a condition is a condition where substantially the same polynucleotides having the high identity, for example, the polynucleotides having the percent identity described above hybridize each other whereas polynucleotides having the lower identity than above do not hybridize each other. Specifically, such a condition may include hybridization in 6XSCC (sodium chloride/sodium citrate) at about 45°C followed by one or two or more washings in 0.2×SCC and 0.1% SDS at 50 to 65°C.

The polynucleotide of the present invention may be DNA or RNA, and is preferably DNA. The polynucleotide of the present invention can be derived from Mucuna. The polynucleotide of the present invention may also be one encoding the protein of the present invention described later.

### <Isoprene synthase>

The present invention also provides a protein having an isoprene synthase activity. The isoprene synthase activity is as described above.

In one embodiment, the protein of the present invention is a protein comprising (i') the full length amino acid sequence represented by SEQ ID NO:2, or (ii') the amino acid sequence consisting of the amino acid residues at positions 45 to 594 in the amino acid sequence represented by SEQ ID NO:2. The amino acid sequence consisting of the amino acid residues at positions 1 to 44 in the amino acid sequence represented by SEQ ID:2 can encode a putative chloroplast localization signal, and the amino acid sequence consisting of the amino acid residues at positions 45 to 594 in the amino acid sequence represented by SEQ ID:2 can encode the mature isoprene synthase.

In another embodiment, the protein of the present invention is a protein that comprises an amino acid sequence having 90% or more identity to the amino acid sequence of (i') or (ii') above, and has an isoprene synthase activity. The percent identity to the amino acid sequence may be 91%, 92%, 93%, 94%, 95%, 96%, 97%, 98% or 99% or more.

In still another embodiment, the protein of the present invention is a protein that comprises an amino acid sequence having a mutation of one to thirty amino acids in the amino acid sequence of (i') or (ii') above, and has an isoprene synthase activity. Examples of the mutation of the amino acid residues may include deletion, substitution, addition and insertion of amino acid residues. The mutation of one to thirty amino acids may be introduced into one region or multiple different regions in the amino acid sequence. The term "one to thirty" indicates a range in which a three-dimensional structure and an activity of the protein are not impaired greatly. In the case of the protein, the number represented by "one to thirty" is, for example, 1 to 30, 1 to 20, 1 to 10, or 1 to 5. The protein of the present invention may have a tag for purification, such as a histidine tag.

The protein of the present invention preferably has an isoprene synthase activity that is 50% or more, 60% or more, 70% or more, 80% or more, 90% or more, or 95% or more of the isoprene synthase activity of the protein comprising the amino acid sequence of either one of (i') or (ii') above when measured under the same condition. In terms of stability, it is also preferable that the protein of the present invention has a remaining activity that is 30% or more, 40% or more, 50% or more, 60% or more or 65% or more of the original activity when the protein is stored in a certain buffer [e.g., a solution of 50 mM Tris-HCl (pH 8.0), and 0,15 mM MgCl₂] at 4°C for 48 hours.

In the protein of the present invention, the mutation may be introduced into sites in a catalytic domain and sites other than the catalytic domain as long as an objective activity is retained. The positions of amino acid residues to be mutated which is capable of retaining the objective activity are understood by a person skilled in the art. Specifically, a person skilled in the art can recognize a correlation between structure and function, since a person skilled in the art can 1) compare the amino acid sequences of multiple proteins having the same type of activity (e.g., the amino acid sequence represented by SEQ ID NO:2 and amino acid sequences of other isoprene synthases), 2) clarify regions that are relatively conserved and regions that are not relatively conserved, and then 3) predict regions capable of playing a functionally important role and regions incapable of playing a functionally important role from the regions that are relatively conserved and the regions that are not relatively conserved, respectively. Therefore, a person skilled in the art can identify the positions of the amino acid residues to be mutated in the amino acid sequence of the isoprene synthase.

When the amino acid residue is mutated by substitution, the substitution of the amino acid residue may be conservative substitution. As used herein, the term "conservative substitution" refers to substitution of a certain amino acid residue with an amino acid residue having a similar side chain. Families of the amino acid residues having the similar side chain are well-known in the art. Examples of such families may include amino acids having a basic side chain (e.g., lysine, arginine, histidine), amino acids having an acidic side chain (e.g., aspartic acid, glutamic acid), amino acids having a non-charged polar side chain (e.g., asparagine, glutamine, serine, threonine, tyrosine, cysteine), amino acids having a non-polar side chain (e.g., glycine, alanine, valine, leucine, isoleucine, proline, phenylalanine, methionine, tryptophan), amino acids having a branched side chain at position β (e.g., threonine, valine, isoleucine), amino acids having an aromatic side chain (e.g., tyrosine, phenylalanine, tryptophan, histidine), amino acids having a hydroxyl group (e.g., alcoholic, phenolic)-containing side chain (e.g., serine, threonine, tyrosine), and amino acids having a sulfur-containing side chain (e.g., cysteine, methionine). Preferably, the conservative substitution of the amino acids may be the substitution between aspartic acid and glutamic acid, the substitution among arginine, lysine and histidine, the substitution between tryptophan and phenylalanine, the substitution between phenylalanine and valine, the substitution among leucine, isoleucine and alanine, and the substitution between glycine and alanine.

### <Expression vector>

The present invention provides an expression vector. The expression vector of the present invention comprises the polynucleotide of the present invention or the polynucleotide encoding the protein of the present invention.

Examples of the expression vector of the present invention may include a cell system vector that expresses a protein in a host cell and a cell-free system vector that utilizes a protein translation system. The expression vector may also be a plasmid or an integrative vector. The expression vector may further be a DNA vector or an RNA vector.

A known vector suitable for the host cell is used as the cell system vector. Examples of the expression vector may include ColE-based plasmids typified by pBR322 derivatives, pACYC-based plasmids having a p15A origin, pSC-based plasmids, and mini F plasmids derived from an F factor of Bac and the like in *Escherichia coli*. In addition, expression vectors having a tryptophan promoter such as trc and tac, a lac promoter, a T7 promoter, a T5 promoter, a T3 promoter, an SP6 promoter, an arabinose induction promoter, a cold shock promoter, a tetracycline induction promoter, and the like may also be included.

Examples of the cell-free system vector may include expression vectors having the T7 promoter or the T3 promoter exemplified in the cell system vector; and vectors for synthesizing proteins in wheat cell-free system, such as pEU-based plasmids having the SP6 promoter or the T7 promoter.

In protein synthesis using the cell-free system vector, cDNA for an objective protein is firstly transcribed to synthesize mRNA using a transcription system. Such a transcription system may include those known conventionally and publicly in which the cDNA is transcribed by RNA polymerase. Examples of the RNA polymerase may include T7 RNA polymerase.

Subsequently, mRNA is translated to synthesize a protein using a cell-free protein synthesis system that is a translation system. In this system, factors such as ribosome, a translation initiation factor, a translation elongation factor, a dissociation factor, and aminoacyl tRNA synthetase that are required for the translation are included. Examples of such a protein translation system may include an *E*. *coli* extract, a rabbit reticulocyte extract, and a wheat germ extract.

Further, a reconstituted cell-free protein synthesis system composed of factors required for the above translation, which are independently purified, may also be included.

The protein synthesis using the cell system vector will be described later in <Transformants>.

A protein synthesized using the cell system vector or the cell-free system vector may be purified. Examples of the methods for purification may include methods using a salting-out method and various chromatographic methods. When the expression vector is designed to express a tag sequence such as a histidine tag at an N terminus or a C terminus of the objective protein, a method for purification using affinity chromatography using a substance such as nickel or cobalt having an affinity to this tag can be employed. In addition, a purity of the protein of the present invention can be increased by an appropriate purification method in combination with ion exchange chromatography, gel filtration chromatography, or the like.

### <Transformants>

The present invention provides a transformant comprising the expression vector of the present invention. The transformant of the present invention is one obtained by introducing the expression vector of the present invention into a host. The host used for the present invention is preferably a bacterium or a fungus. The bacterium may be a gram-positive bacterium or a gram-negative bacterium.

Examples of the gram-positive bacteria may include bacteria belonging to genera *Bacillus, Listeria, Staphylococcus, Streptococcus, Enterococcus, Clostridium, Corynebacterium,* and *Streptomyces.* Bacteria belonging to genera *Bacillus* and *Corynebacterium* are preferable.

Examples of the bacteria belonging to genus *Bacillus* may include *Bacillus subtilis, Bacillus anthracis,* and *Bacillus cereus. Bacillus subtilis* is more preferable.

Examples of the bacteria belonging to genus *Corynebacterium* may include *Corynebacterium glutamicum, Corynebacterium efficiens,* and *Corynebacterium callunae. Corynebacterium glutamicum* is more preferable.

Examples of the gram-negative bacteria may include bacteria belonging to genera *Escherichia, Pantoea, Salmonella, Vivrio, Serratia,* and *Enterobacter.* The bacteria belonging to genera *Escherichia, Pantoea* and *Enterobacter* are preferable.

*Escherichia coli* is preferable as the bacteria belonging to genus *Escherichia.*

Examples of the bacteria belonging to genus Pantoea may include *Pantoea ananatis, Pantoea stewartii, Pantoea agglomerans,* and *Pantoea citrea. Pantoea ananatis* and *Pantoea citrea* are preferable. Strains exemplified in European Patent Application Publication 0952221 may be used as the bacteria belonging to genus *Pantoea.* Examples of representative strains of the bacteria belonging to genus Pantoea may include *Pantoea ananatis* AJ13355 strain (FERM BP-6614) and *Pantoea ananatis* AJ13356 strain (FERM BP-6615) disclosed in European Patent Application Publication 0952221.

Examples of the bacteria belonging to genus *Enterobacter* may include *Enterobacter agglomerans* and *Enterobacter aerogenes. Enterobacter aerogenes* is preferable. The bacterial strains exemplified in European Patent Application Publication 0952221 may be used as the bacteria belonging to genus *Enterobacter.* Examples of representative strains of the bacteria belonging to genus *Enterobacter* may include *Enterobacter agglomerans* ATCC12287 strain, *Enterobacter aerogenes* TACC13048 strain, *Enterobacter aerogenes* NBRC12010 strain (Biotechnol. Bioeng., 2007, Mar. 27; 98 (2): 340-348), and *Enterobacter aerogenes* AJ110637 (FERM BP-10955). The *Enterobacter aerogenes* AJ110637 strain was deposited to International Patent Organism Depositary (IPOD), National Institute of Advanced Industrial Science and Technology (AIST) (Chuo No. 6, Higashi 1-1-1, Tsukuba City, Ibaraki Pref., JP, Postal code 305-8566) as of August 22, 2007, and was transferred to the international deposition based on Budapest Treaty on March 13, 2008, and an accession number FERM BP-10955 was given thereto.

Examples of the fungus may include microorganisms belonging to genera *Saccharomyces, Schizosaccharomyces, Yarrowia, Trichoderma, Aspergillus, Fusarium,* and *Mucor.* The microorganisms belonging to genera *Saccharomyces, Schizosaccharomyces, Yarrowia,* or *Trichoderma* are preferable.

Examples of the microorganisms belonging to genus *Saccharomyces* may include *Saccharomyces carlsbergensis, Saccharomyces cerevisiae, Saccharomyces diastaticus, Saccharomyces douglasii, Saccharomyces kluyveri, Saccharomyces norbensis,* and *Saccharomyces oviformis. Saccharomyces cerevisiae* is preferable.

*Schizosaccharomyces pombe* is preferable as the microorganisms belonging to genus *Schizosaccharomyces.*

*Yarrowia lypolytica* is preferable as the microorganisms belonging to genus *Yarrowia.*

Examples of the microorganisms belonging to genus *Trichoderma* may include *Trichoderma harzianum, Trichoderma koningii, Trichoderma longibrachiatum, Trichoderma reesei,* and *Trichoderma viride. Trichoderma reesei* is preferable.

In addition, the host used for the present invention is not particularly limited as long as the host has an ability to synthesize dimethylallyl diphosphate (DMAPP) via a mevalonic acid (MVA) pathway and/or a methylerythritol phosphate (MEP) pathway that are involved in synthesis of dimethylallyl diphosphate that is a substrate of the isoprene synthase, and may be an insect cell, an animal cell, a plant cell, and the like.

In the transformant of the present invention, the pathway to synthesize dimethylallyl diphosphate (DMAPP) that is the substrate of the isoprene synthase may be enhanced. For such an enhancement, an expression vector that expresses an isopentenyl-diphosphate delta isomerase having an ability to convert isopentenyl diphosphate (IPP) into dimethylallyl diphosphate (DMAPP) may be introduced into the transformant of the present invention. An expression vector that expresses one or more enzymes involved in the mevalonate pathway and/or methylerythritol phosphate pathway associated with formation of IPP and/or DMAPP may also be introduced into the transformant of the present invention. The expression vector for such an enzyme may be a plasmid or an integrative vector. The expression vector for such an enzyme may also be a DNA vector or an RNA vector. The expression vector for such an enzyme may further express a plurality of enzymes (e.g., one, two, three or four or more) involved in the mevalonate pathway and/or the methylerythritol phosphate pathway, and may be, for example, an expression vector for polycistronic mRNA. Origin of one or more enzymes involved in the mevalonate pathway and/or the methylerythritol phosphate pathway may be homologous or heterologous to the host. When the origin of the enzyme involved in the mevalonate pathway and/or the methylerythritol phosphate pathway is homologous to the host, for example, the host may be a bacterium as described above (e.g., *Escherichia coli*) and the enzyme involved in the mevalonate pathway may be derived from a fungus (e.g., *Saccharomyces cerevisiae*). In addition, when the host inherently produces the enzyme involved in the methylerythritol phosphate pathway, an expression vector to be introduced into the host may express the enzyme involved in the mevalonate pathway.

Examples of isopentenyl-diphosphate delta isomerase (EC: 5.3.3.2) may include Idi1p (ACCESSION ID NP_015208), AT3G02780 (ACCESSION ID NP_186927), AT5G16440 (ACCESSION ID NP_197148) and Idi (ACCESSION ID NP_417365).

Examples of the enzymes involved in the mevalonate (MVA) pathway may include mevalonate kinase (EC: 2.7.1.36; example 1, Erg12p, ACCESSION ID NP_013935; example 2, AT5G27450, ACCESSION ID NP_001190411), phosphomevalonate kinase (EC: 2.7.4.2; example 1, Erg8p, ACCESSION ID NP_013947; example 2, AT1G31910, ACCESSION ID NP_001185124), diphosphomevalonate decarboxylase (EC: 4.1.1.33; example 1, Mvd1p, ACCESSION ID NP_014441; example 2, AT2G38700, ACCESSION ID NP_181404; example 3, AT3G54250, ACCESSION ID NP_566995), acetyl-CoA-C-acetyltransferase (EC: 2.3.1.9; example 1, Erg10p, ACCESSION ID NP_015297; example 2, AT5G47720, ACCESSION ID NP_001032028; example 3, AT5G48230, ACCESSION ID NP_568694), hydroxymethylglutaryl-CoA synthase (EC: 2.3.3.10; example 1, Erg13p, ACCESSION ID NP_013580; example 2, AT4G11820, ACCESSION ID NP_192919; example 3, MvaS, ACCESSION ID AAG02438), hydroxymethylglutaryl-CoA reductase (EC: 1.1.1.34; example 1, Hmg2p, ACCESSION ID NP_013555; example 2, Hmg1p, ACCESSION ID NP_013636; example 3, AT1G76490, ACCESSION ID NP_177775; example 4, AT2G17370, ACCESSION ID NP_179329, EC: 1.1.1.88, example, MvaA, ACCESSION ID P13702), and acetyl-CoA-C-acetyltransferase/hydroxymethylglutaryl-CoA reductase (EC: 2.3.1.9/1.1.1.34, example, MvaE, ACCESSION ID AAG02439).

Examples of the enzymes involved in the methylerythritol phosphate (MEP) pathway may include 1-deoxy-D-xylulose-5-phosphate synthase (EC: 2.2.1.7, example 1, Dxs, ACCESSION ID NP_414954; example 2, AT3G21500, ACCESSION ID NP_566686; example 3, AT4G15560, ACCESSION ID NP_193291; example 4, AT5G11380, ACCESSION ID NP_001078570), 1-deoxy-D-xylulose-5-phosphate reductoisomerase (EC: 1.1.1.267; example 1, Dxr, ACCESSION ID NP_414715; example 2, AT5G62790, ACCESSION ID NP_001190600), 4-diphosphocytidyl-2-C-methyl-D-erythritol synthase (EC: 2.7.7.60; example 1, IspD, ACCESSION ID NP_417227; example 2, AT2G02500, ACCESSION ID NP_565286), 4-diphosphocytidyl-2-C-methyl-D-erythritol kinase (EC: 2.7.1.148; example 1, IspE, ACCESSION ID NP_415726; example 2, AT2G26930, ACCESSION ID NP_180261), 2-C-methyl-D-erythritol-2,4-cyclodiphosphate synthase (EC: 4.6.1.12; example 1, IspF, ACCESSION ID NP_417226; example 2, AT1G63970, ACCESSION ID NP_564819), 1-hydroxy-2-methyl-2-(E)-butenyl-4-diphosphate synthase (EC: 1.17.7.1; example 1, IspG, ACCESSION ID NP_417010; example 2, AT5G60600, ACCESSION ID NP_001119467), and 4-hydroxy-3-methyl-2-butenyl diphosphate reductase (EC: 1.17.1.2; example 1, IspH, ACCESSION ID NP_414570; example 2, AT4G34350, ACCESSION ID NP_567965).

The introduction of the expression vector incorporating a gene into a host (transformation) can be carried out using known methods. Examples of such a method may include a competent cell method using a microbial cell treated with calcium and an electroporation method. The gene may be introduced by infecting the microbial cell with a phage vector rather than the plasmid vector.

Further, a gene encoding the enzyme involved in the mevalonate pathway or the methylerythritol phosphate pathway that synthesizes dimethylallyl diphosphate that is the substrate of the isoprene synthase may also be introduced into the transformant of the present invention.

Such an enzyme may include 1-deoxy-D-xylose-5-phosphate synthase that converts a pyruvate and D-glycelaldehyde-3-phosphate into 1-deoxy-D-xylose-5-phosphate, and isopentyl diphosphate isomerase that converts isopentenyl diphosphate into dimethylallyl diphosphate.

The protein of the present invention may be extracted or purified from the transformant of the present invention, and isoprene may be produced by culturing the transformant that expresses the protein of the present invention.

### <Methods of producing isoprene monomer and isoprene polymer>

The present invention provides a method of producing an isoprene monomer. The method of producing the isoprene monomer of the present invention comprises forming the isoprene monomer from dimethylallyl diphosphate in the presence of the protein of the present invention.

The method of producing the isoprene monomer of the present invention is not particularly limited as long as the method is performed in the presence of the protein of the present invention, and can be performed by utilizing an enzymatic reaction system with the protein itself (e.g., purified protein) of the present invention or culturing the transformant of the present invention that produces the protein of the present invention. Preferably, it is performed by culturing the transformant of the present invention. When the transformant of the present invention is used in the method of producing the isoprene monomer of the present invention, dimethylallyl diphosphate that is a raw material of the isoprene monomer is efficiently supplied from a carbon source in a culture medium by the transformant of the present invention. The transformant of the present invention produces the isoprene monomer mainly as an outgas from the carbon source in the culture medium. Thus, the isoprene monomer is recovered by collecting gas produced from the transformant. Dimethylallyl diphosphate that is the substrate of the isoprene synthase is synthesized from the carbon source in the culture medium via the mevalonate pathway or the methylerythritol phosphate pathway in the host.

The culture medium for culturing the transformant of the present invention preferably contains a carbon source to be converted into isoprene. The carbon source may include carbohydrates such as monosaccharides, disaccharides, oligosaccharides and polysaccharides; invert sugars obtained by hydrolyzing sucrose; glycerol; compounds having one carbon atom (hereinafter referred to as a C1 compound) such as methanol, formaldehyde, formate, carbon monoxide and carbon dioxide; oils such as corn oil, palm oil and soybean oil; acetate; animal fats; animal oils; fatty acids such as saturated fatty acids and unsaturated fatty acids; lipids; phospholipids; glycerolipids; glycerine fatty acid esters such as monoglyceride, diglyceride and triglyceride; polypeptides such as microbial proteins and plant proteins; renewable carbon sources such as hydrolyzed biomass carbon sources; yeast extracts, or combinations thereof. For a nitrogen source, inorganic ammonium salts such as ammonium sulfate, ammonium chloride and ammonium phosphate, organic nitrogen such as hydrolyzed soybeans, ammonia gas, ammonia water, and the like can be used. It is desirable to contain required substances such as vitamin B1 and L-homoserine, or the yeast extract and the like in an appropriate amount as an organic trace nutrient source. In addition thereto, potassium phosphate, magnesium sulfate, iron ion, manganese ion, and the like are added in a small amount if necessary. The culture medium used in the present invention may be a natural medium or a synthesized medium as long as it contains the carbon source, the nitrogen source, inorganic ions, and optionally the other organic trace ingredients.

Examples of the monosaccharides may include triose such as ketotriose (dihydroxyacetone) and aldotriose (glyceraldehyde); tetrose such as ketotetrose (erythrulose) and aldotetrose (erythrose, threose); pentose such as ketopentose (ribulose, xylulose), aldopentose (ribose, arabinose, xylose, lyxose) and deoxysaccharide (deoxyribose); hexose such as ketohexose (psychose, fructose, sorbose, tagatose), aldohexose (allose, altrose, glucose, mannose, gulose, idose, galactose, tallose), and deoxysaccharide (fucose, fucrose, rhamnose); and heptose such as sedoheptulose. C6 sugars such as fructose, mannose, galactose and glucose; and C5 sugars such as xylose and arabinose are preferable.

Examples of the disaccharides may include sucrose, lactose, maltose, trehalose, turanose, and cellobiose. Sucrose and lactose are preferable.

Examples of the oligosaccharides may include trisaccharides such as raffinose, melezitose and maltotriose; tetrasaccharides such as acarbose and stachyose; and other oligosaccharides such as fructooligosaccharide (FOS), galactooligosaccharide (GOS) and mannan-oligosaccharide (MOS).

Examples of the polysaccharides may include glycogen, starch (amylose, amylopectin), cellulose, dextrin, and glucan (β1,3-glucan), and starch and cellulose are preferable.

Examples of the microbial protein may include polypeptides derived from a yeast or bacterium.

Examples of the plant protein may include polypeptides derived from soybean, corn, canola, Jatropha, palm, peanut, sunflower, coconut, mustard, cotton seed, Palm kernel oil, olive, safflower, sesame and linseed.

Examples of the lipid may include substances containing one or more saturated or unsaturated fatty acids of C4 or more.

The oil is preferably the lipid that contains one or more saturated or unsaturated fatty acids of C4 or more and is liquid at room temperature, and examples of the oil may include lipids derived from soybean, corn, canola, Jatropha, palm, peanut, sunflower, coconut, mustard, cotton seed, Palm kernel oil, olive, safflower, sesame, linseed, oily microbial cells, Chinese tallow tree, and a combination of two or more thereof.

Examples of the fatty acid may include compounds represented by a formula RCOOH ("R" represents a hydrocarbon group).

The unsaturated fatty acid is a compound having at least one double bond between two carbon atoms in "R", and examples of the unsaturated fatty acid may include oleic acid, vaccenic acid, linoleic acid, palmitelaidic acid and arachidonic acid.

The saturated fatty acid is a compound where the "R" is a saturated aliphatic group, and examples of the saturated fatty acid may include docosanoic acid, eicosanoic acid, octadecanoic acid, hexadecanoic acid, tetradecanoic acid, and dodecanoic acid.

Among them, those containing one or more C2 to C22 fatty acids are preferable as the fatty acid, and those containing C12 fatty acid, C14 fatty acid, C16 fatty acid, C18 fatty acid, C20 fatty acid and C22 fatty acid are more preferable.

The carbon source may include salts and derivatives of these fatty acids and salts of these derivatives. Examples of the salt may include lithium salts, potassium salts and sodium salts.

Examples of the carbon source may also include combinations of carbohydrate such as glucose with the lipid, the oil, the fats, the fatty acid and glycerine fatty acid ester.

Examples of the renewable carbon source may include hydrolyzed biomass carbon sources.

Examples of the biomass carbon source may include cellulose-based substrates such as waste materials of woods, papers and pulps, leafy plants, and fruit pulps; and partial plants such as stalks, grain particles, roots and tubers.

Examples of the plants to be used as the biomass carbon source may include corn, wheat, rye, sorghum, triticate, rice, millet, barley, cassava, legume such as pea, potato, sweet potato, banana, sugar cane and tapioca.

When the renewable carbon source such as biomass is added to the culture medium, the carbon source is preferably pretreated. Examples of the pretreatment may include an enzymatic pretreatment, a chemical pretreatment, and a combination of the enzymatic pretreatment and the chemical pretreatment.

It is preferred that the renewable carbon source is entirely or partially hydrolyzed before being added to the culture medium.

Examples of the carbon source may also include the yeast extract and a combination of the yeast extract with the other carbon source such as glucose. The combination of the yeast extract with the C1 compound such as carbon dioxide and methanol is preferable.

In the method of culturing the transformant in connection with the present invention, it is preferable to culture the cell in a standard medium containing saline and nutrients.

The culture medium is not particularly limited, and examples of the culture medium may include ready-made general media that is commercially available such as Luria Bertani (LB) broth, Sabouraud dextrose (SD) broth, and yeast medium (YM) broth. The medium suitable for the cultivation of the specific host can be selected appropriately for the use.

It is desirable to contain appropriate minerals, salts, supplemental elements, buffers, and ingredients known for those skilled in the art to be suitable for the cultivation and to facilitate the production of isoprene in addition to the appropriate carbon source in the cell medium.

It is preferable to add the sugar, a metal salt, an antimicrobial substance, and the like to the medium in order to keep the expression of the protein of the present invention in the transformant of the present invention.

A culture condition for the transformant of the present invention is not particularly limited as long as the protein of the present invention can be expressed, and a standard cell culture condition can be used.

A culture temperature is preferably 20 to 37°C, a gas composition is preferably about 6 to 84% of CO₂ concentration, and a pH value is preferably about 5 to about 9.

The transformant is preferably cultured under an aerobic, oxygen-free, or anaerobic condition depending on a nature of the host.

Examples of the method of culturing the transformant may include a method using a known fermentation method such as a batch cultivation method, a feeding cultivation method or a continuous cultivation method.

In the batch cultivation method, a medium composition is added at start of the fermentation, and the transformant is inoculated in the medium composition and cultured while pH and an oxygen concentration are controlled.

In the cultivation of the transformant by the batch cultivation method, the growth of the transformant starts from a mild induction phase, passes through a logarithmic growth phase and finally goes to a stationary phase in which a growth speed is reduced or stopped. Isoprene is produced by the transformant in the logarithmic growth phase and the stationary phase.

In the feeding cultivation method, in addition to the above batch method, the carbon source is gradually added according to the progress of a fermentation process. The feeding cultivation method is effective when an amount of the carbon source is to be restricted in the medium because metabolism of the transformant tends to be reduced due to catabolite suppression. The feed cultivation can be performed using a restricted amount or an excessive amount of the carbon source such as glucose.

In the continuous cultivation method, a certain amount of the medium is continuously supplied to a bioreactor at a constant rate while the same amount of the medium is removed. In the continuous cultivation method, the culture can be kept constantly at high concentration and the transformant in the culture medium is generally in the logarithmic growth phase.

The nutrition can be supplemented by entirely or partly exchanging the medium appropriately, and accumulation of metabolic byproducts that potentially have adverse effects on the growth of the transformant, and the accumulation of dead cells can be prevented.

A promoter possessed by the expression vector of the present invention may include constitutive promoters and inducible promoters. When the expression vector of the present invention has the inducible promoter such as a lac promoter, the expression of the protein of the present invention may be induced by adding IPTG (isopropyl-β-thiogalactopyranoside) into the culture medium.

Examples of the method of evaluating an amount of isoprene produced by culturing the transformant of the present invention may include a method in which a gas phase is collected by a headspace method and this gas phase is analyzed by gas chromatography.

In detail, the isoprene monomer in a headspace which is obtained by culturing the transformant in a sealed vial with shaking the culture medium is analyzed by standard gas chromatography. Then, an area calculated by a curve measured by gas chromatography is converted into the amount of the isoprene monomer produced with the transformant using a standard curve.

Examples of the method of collecting the isoprene monomer obtained by culturing the transformant of the present invention may include gas stripping, fractional distillation, or dissociation of the isoprene monomer adsorbed to a solid phase by heat or vacuum, or extraction with a solvent.

In the gas stripping, isoprene gas is continuously removed from the outgas. Such removal of the isoprene gas can be performed by various methods. Examples of the removal may include adsorption to the solid phase, separation into a liquid phase, and a method in which the isoprene gas is directly condensed.

The isoprene monomer can be collected by a single step or multiple steps. When the isoprene monomer is collected by the single step, the isoprene monomer is converted into the liquid phase simultaneously with separating the isoprene monomer from the outgas. The isoprene monomer can also be directly condensed from the outgas to make the liquid phase. When the isoprene monomer is collected by the multiple stages, the isoprene monomer is separated from off-gas and subsequently converted into the liquid phase. For example, the isoprene monomer is adsorbed to the solid phase, and extracted from the solid phase with the solvent.

The method of collecting the isoprene monomer may further comprise purifying the isoprene monomer. Examples of the purification may include separation from a liquid phase extract by distillation and various chromatographic methods.

The protein of the present invention is more excellent in ability to produce isoprene than conventional isoprene synthase. Thus, the isoprene monomer can be produced efficiently using the transformant that expresses the protein of the present invention.

The present invention also provides a method of producing an isoprene polymer. The method of producing the isoprene polymer according to the present invention comprises the following (I) and (II):
(I) forming an isoprene monomer by the method of the present invention; and
(II) polymerizing the isoprene monomer to form an isoprene polymer.

The step (I) can be performed in the same manner as in the method of producing the isoprene monomer of the present invention described above. The polymerization of the isoprene monomer in the step (II) can be performed by any method known in the art.

### EXAMPLES

Hereinafter, the present invention will be described in detail with reference to Examples, but the present invention is not limited to the following Examples.

### Example 1: Evaluation of ability to produce isoprene in plants

### 1-1) Measurement of amount of isoprene formed per unit weight of dry leaves

First, an amount of isoprene formed per 1 g of dry leaves in the plant was measured for evaluating an ability to produce isoprene in plants. Mucuna (*Mucuna bracteata*), Weeping willow (*Salix babylonica*), American sweetgum (*Liquidambar styraciflua*), Myrtle (*Myrtus communis*), and Kudzu (*Pueraria lobata*) were used as the plants.

In the measurement of an amount of formed isoprene, a gas replaceable desiccator (trade name: Vacuum Desiccator, manufactured by AS ONE Corporation) was housed in an incubator (trade name: Growth Chamber MLR-351H, manufactured by SANYO), and the incubator was set to a high temperature induction condition (an illuminance of 100 µmol E/m²/s at 40°C) while a fan for stirring the gas, which was provided in the gas replaceable desiccator, was driven to stir an atmosphere in space in the gas replaceable desiccator. After the temperature of the atmosphere in the gas replaceable desiccator reached 40°C, a plant body of Mucuna planted in a planter was housed therein and kept for 3 hours in a state where the gas replaceable desiccator was sealed. Then, a gas component released from Mucuna was aspirated from the space in the gas replaceable desiccator by an aspiration pump through a silicon tube, an adsorption tube and a gas collection tube. Thereby, water vapor (water content) contained in the gas component released from Mucuna was adsorbed and separated in the adsorption tube, the gas component from which the water vapor had been separated was led to the gas collection tube, and the gas component was collected in the gas collection tube. Subsequently, isoprene contained in the gas component collected in the gas collection tube was quantitatively analyzed using gas chromatograph (trade name: GC-FID6890, manufactured by Agilent).

For the weight of dry leaves, a leaf area of a fresh individual leaf, and a dry weight when the fresh individual leaf is dried by a dryer at 80°C for 8 hours establish a very good positive correlation. Thus, a formula for converting from the leaf area to the dry weight was derived, and the dry weight was estimated from the entire leaf area from the plant body of Mucuna used for the measurement of an amount of formed isoprene.

The amount of formed isoprene per 1 g of the dry leaf was obtained by dividing the amount of formed isoprene from the entire plant body of Mucuna by the estimated weight of the entire plant body.

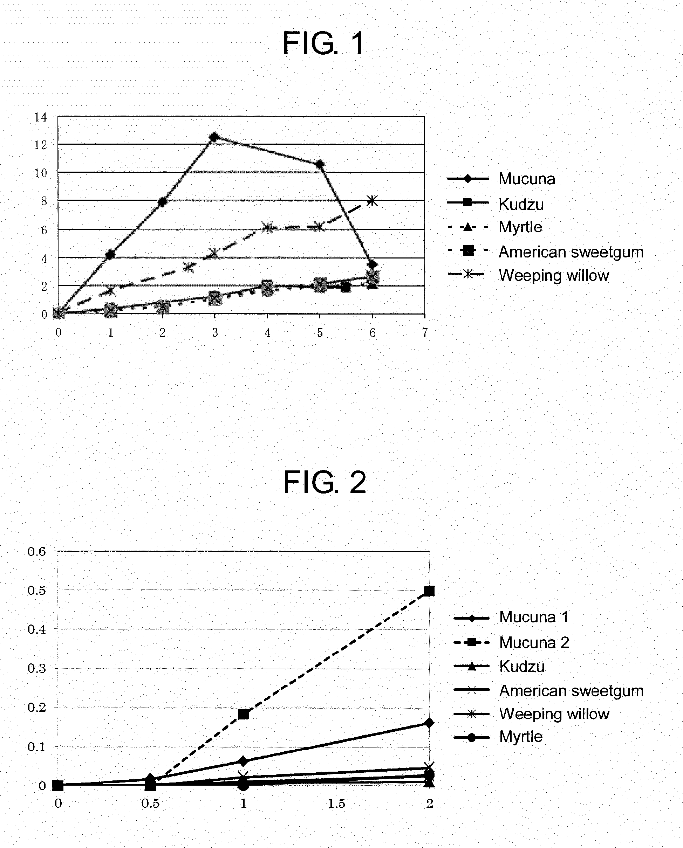

As a result, it was demonstrated that Mucuna was excellent in amount of formed isoprene per unit weight of the dry leaf (FIG. 1).

### 1-2) Measurement of amount of formed isoprene per amount of total protein

Then, the amount of formed isoprene per amount of total protein extracted from leaves of various plants was measured. Mucuna (samples 1 and 2), Weeping willow, American sweetgum, Myrtle, and Kudzu were used as the plants.

For extraction of the protein, a buffer solution (50 mM Tris-HCl, 20 mM MgCl, 5% glycerol. 0.02% Triton-X100, pH 8.0) was made, and 10% Polyclar AT, 20 mM DTT, protease complete tablet (one tablet/50 mL), and 1 mM benzamidine HCl (final concentrations, each) were added just before the use, and was used as a protein extraction buffer. 50 mL of the protein extraction buffer was added to 5 g of the sample, then the mixture was ground well in a cold mortar on ice and filtrated though doubly overlapped Miracloth. A filtrate was centrifuged at 12,000 G for 20 minutes and 40,000 G for 40 minutes to obtain a supernatant, and the supernatant was used as a crude extract.

Subsequently, this crude extract was fractionated with ammonium sulfate. Proteins precipitated in a range of 40% to 55% of final concentrations of ammonium sulfate were centrifuged at 40,000 G for 40 minutes, and an obtained pellet was re-dissolved in the protein extraction buffer to obtain an ammonium sulfate fraction.

A total (ammonium sulfate fraction) protein mass was calculated by measuring the ammonium sulfate fraction using Bradford assay. A Bradford reagent was reacted with the standard protein, bovine serum albumin, and absorbance at a wavelength of 595 nm was measured using a spectrophotometer. A standard curve for the protein was made using the obtained absorbance values. The absorbance at a wavelength of 595 nm was also measured in the ammonium sulfate fraction diluted to 50 times, and the amount of the total (ammonium sulfate fraction) protein was estimated from the standard curve for the standard protein.

In the measurement of the amount of formed isoprene, 100 µL of the crude extract or 100 µL of a crude enzyme solution boiled at 100°C was placed in a 4 mL glass vial, and then 2 µL of a 0.5 M MgCl₂ solution and 5 µL of a 0.2 M DMAPP solution were added thereto. The vial was tightly closed with a screw cap with a septum, and then the vial was gently vortexed and set in an incubator at 40°C. After 0.5, 1 and 2 hours, 0.5 to 2 mL of a gas layer in a headspace was sampled by a gas-tight syringe, and the amount of formed isoprene was measured using gas chromatograph (trade name: GC-FID6890, manufactured by Agilent). The amount of formed isoprene using the crude enzyme after 0.5, 1 and 2 hours was calculated by subtracting a measured value in the case of using the crude enzyme solution boiled at 100°C from a measured value in the case of using the crude enzyme. An enzymatic activity per 1 mg of the total protein (specific activity) was calculated from the amount of the formed isoprene per one hour. The amount of formed isoprene was measured with keeping the amount of DMAPP that was the substrate of the isoprene synthase constant.

As a result, it was demonstrated that Mucuna was excellent in amount of formed isoprene per amount of total protein (Fig. 2, Table 1). As described above, it was shown that Mucuna was excellent in ability to produce isoprene.

**Table 1. Amount of formed isoprene per amount of total protein (index numbers relative to case of Kudzu)**

| | 0 hour* | 0.5 hour* | 1 hour* | 2 hours* | Specific activity index (Value from Kudzu was set to 1) |
|---|---|---|---|---|---|
| Mucuna 1 | 0 | 16.947 | 61.895 | 160.632 | 16.87842808 |
| Mucuna 2 | 0 | 0 | 183.58 7 | 449.514 | 47.23274141 |
| American sweetgum | 0 | 0 | 22.063 | 46.132 | 4.847325838 |
| Weeping willow | 0 | 0 | 9.756 | 24.39 | 2.562782389 |
| Myrtle | 0 | 0 | 0 | 27.451 | 2.884417358 |
| Kudzu | 0 | 0 | 6.662 | 9.517 | 1 |

| | | | | | |
|---|---|---|---|---|---|
| *Unit is µg isoprene/mg protein | | | | | |

### Example 2: Cloning of isoprene synthase gene derived from Mucuna

### 2-1) Evaluation of sampling time

Isoprene gas released from leaves of Mucuna illuminated with light for 1, 2, 3 and 5 hours at temperature of 40°C was sampled and the amount of produced isoprene was quantified by gas chromatography described later, and production of 4, 8, 12 and 10 µg of isoprene/g DW leaf was confirmed. Thus, it was confirmed that an optimal light illumination time was 3 hours.

### 2-2) Extraction of total RNA lysis solution

A total RNA was extracted from leaves of Mucuna with total RNA lysis solution according to the following procedures.
(1) The leaves of Mucuna illuminated with light for 3 hours at temperature of 40°C were sampled.
(2) 100 mg of leaf tissue was pulverized in a mortar with rapidly freezing the leaf tissue with liquid nitrogen, then the leaf tissue together with the liquid nitrogen was dispensed in an RNA-free 2 mL Eppendorf tube, and the liquid nitrogen was gasified.
(3) To this Eppendorf tube, 450 µL of a dissolution buffer RLT (containing 2-mercaptoethanol) attached to RNeasy Plant Kit (manufactured by Qiagen), and mixed vigorously with Vortex to obtain a leaf tissue lysate.
(4) This leaf tissue lysate was applied to QIAshredder spin column attached to RNeasy Plant Kit, and centrifuged at 15,000 rpm for 2 minutes.
(5) A supernatant alone of a column eluate was transferred to a new RNA-free 2 mL Eppendorf tube, then special grade ethanol in a half volume of the supernatant was added to the supernatant, and the obtained solution was mixed by pipetting to obtain about 650 µL of a solution.
(6) This solution was applied to RNeasy spin column attached to RNeasy Plant Kit, centrifuged at 10,000 rpm for 15 seconds, and a filtrate was discarded.
(7) 700 µL of RW1 buffer attached to RNeasy Plant Kit was added to this RNeasy spin column, centrifuged at 10,000 rpm for 15 seconds, and a filtrate was discarded.
(8) 500 µL of BPE buffer attached to RNeasy Plant Kit was added to this RNeasy spin column, centrifuged at 10,000 rpm for 15 seconds, and a filtrate was discarded.
(9) 500 µL of BPE buffer was again added to this RNeasy spin column, centrifuged at 10,000 rpm for 2 minutes, and a filtrate was discarded.
(10) This RNeasy spin column was set to a 2 mL collective tube attached to RNeasy Plant Kit, centrifuged at 15,000 rpm for one minute, and a filtrate was discarded.
(11) This RNeasy spin column was set to a 1.5 mL collective tube attached to RNeasy Plant Kit.
(12) RNA-free distilled water attached to RNeasy Plant Kit was directly added to a membrane of this RNeasy spin column using a Pipetman, centrifuged at 10,000 rpm for one minute, and total RNA was collected. This step was repeated twice to obtain about 100 µg of total RNA.

### 2-3) Analysis of nucleotide sequence of isoprene synthase gene derived from Mucuna

Quality of RNA in the extracted total RNA solution was checked using nano-chips for RNA provided by BioAnalyzer (Agilent Technologies, Inc.), and it was confirmed that the solution was not contaminated with genomic DNA and RNA was not decomposed in the solution.

This total RNA was converted into a double strand using reverse transcriptase, and then fragmented using a nebulizer. Nucleotide sequences of 198,179 fragments having a poly A sequence at a 3' end were analyzed using 454 titanium FLX high performance sequencer (manufactured by Roche Applied Science). Overlapped sequences in the obtained fragment sequences were aligned to obtain 13,485 contig sequences. BLAST search was performed for these contig sequences, and 6 contig sequences having the homology (identity of nucleotide sequences) to registered and known isoprene synthase gene sequences from Kudzu and Poplar were extracted. These sequences were further analyzed in detail, and 3 sequences in these 6 contig sequences were found to be derived from the same gene. Thus, a partial sequence of the isoprene synthase gene derived from Mucuna was obtained. 5' RACE was performed based on this partial sequence to obtain a full length nucleotide sequence of the isoprene synthase gene derived from Mucuna, which was represented by SEQ ID NO:1.

### Example 3: Preparation of expression plasmid for isoprene synthase derived from various plants

### 3-1) Chemical synthesis of isoprene synthase derived from Pueraria montana var. lobata (Kudzu)

The nucleotide sequence and the amino acid sequence of the isoprene synthase derived from *Pueraria montana var*. *lobata* were already known (ACCESSION: AAQ84170: P. *montana var. lobata* isoprene synthase (IspS)). The amino acid sequence of the IspS protein derived from *P. montana* and the nucleotide sequence of its gene are represented by SEQ ID NO:3 and SEQ ID NO:4, respectively. The IspS gene was optimized for codon usage frequency in *E*. *coli* in order to efficiently express the IspS gene in *E*. *coli,* and further designed to cut off the chloroplast localization signal. The designed gene was designated as IspSK. A nucleotide sequence of IspSK is represented by SEQ ID NO:5. The IspSK gene was chemically synthesized, then cloned into pUC57 (manufactured by GenScript), and the resulting plasmid was designated as pUC5-IspSK.

### 3-2) Chemical synthesis of isoprene synthase derived from Populus alba x Populus tremula (Poplar)

The nucleotide sequence and the amino acid sequence of the isoprene synthase derived from *P. alba x P. tremula* were already known (ACCESSION: CAC35696: *P. alba x P. tremula* (Poplar) isoprene synthase). The amino acid sequence of the IspS protein derived from *P. alba x P. tremula* and the nucleotide sequence of its gene are represented by SEQ ID NO:6 and SEQ ID NO:7, respectively. An IspS gene that was optimized for the codon usage frequency in *E*. *coli* in the same manner as above and in which the chloroplast localization signal was cut off was designed and designated as IspSP. A nucleotide sequence of IspSP is represented by SEQ ID NO:8. The IspSP gene was chemically synthesized, then cloned into pUC57 (manufactured by GenScript), and the resulting plasmid was designated as pUC57-IspSP.

### 3-3) Chemical synthesis of isoprene synthase derived from Mucuna pruriens (Mucuna)

Based on the nucleotide sequence derived from *M*. *pruriens,* an IspS gene that was optimized for the codon usage frequency in *E*. *coli* was designed in the same manner as above. One in which the chloroplast localization signal had been conferred was designated as IspSM (L), and one in which the chloroplast localization signal had been cut off was designated as IspSM. Nucleotide sequences for IspSM (L) and IspSM are represented by SEQ ID NO:9 and SEQ ID NO:10, respectively. The IspSM gene and the IspSM (L) gene were chemically synthesized, then cloned into pUC57 (manufactured by GenScript), and the resulting plasmids were designated as pUC57-IspSM and pUC57-IspSM (L).

### 3-4) Construction of expression plasmid, pSTV28-Ptac-Ttrp

An expression plasmid pSTV28-Ptac-Ttrp for expressing IspS derived from various plants in *E*. *coli* was constructed. First, a DNA fragment comprising a tac promoter (synonym: Ptac) region (deBoer, et al.,(1983) Proc. Natl. Acad. Sci. U.S.A., 80, 21-25) and a terminator region of tryptophan operon (synonym: Ttrp) derived from *E*. *coli* (Wu et al., (1978) Proc. Natl. Acad. Sci. U.S.A., 75, 442-5446) and having a *Kpn*I site at a 5' terminus and a *BamH*I site at a 3' end was synthesized chemically (the nucleotide sequence of Ptac-Ttrp is represented by SEQ ID NO:11). The resulting Ptac-Ttrp DNA fragment was digested with *Kpn*I and *BamH*I, and ligated to pSTV28 (manufactured by Takara Bio Inc.) similarly digested with *Kpn*I and *BamH*I by a ligation reaction with DNA ligase. The resulting plasmid was designated as pSTV28-Ptac-Ttrp (its nucleotide sequence is represented by SEQ ID NO:12). This plasmid can amplify the expression of the IspS gene by cloning the IspS gene downstream of Ptac.

### 3-5) Construction of plasmid for expressing IspS gene derived from various plants

Plasmids for expressing the IspSK gene, the IspSP gene, the IspSM gene and the IspSM (L) gene in *E*. *coli* were constructed by the following procedure. PCR was performed with Prime Star polymerase (manufactured by Takara Bio Inc.) using synthesized oligonucleotides consisting of the nucleotide sequences represented by SEQ ID NOS:13 and 14 as primers with pUC57-IspSK as a template, synthesized oligonucleotides consisting of the nucleotide sequences represented by SEQ ID NOS:15 and 16 as primers with pUC57-IspSP as a template, synthesized oligonucleotides consisting of the nucleotide sequences represented by SEQ ID NOS:17 and 18 as primers with pUC57-IspSM as a template, or further synthesized oligonucleotides consisting of the nucleotide sequences represented by SEQ ID NOS:19 and 20 as primers with pUC57-IspSM (L) as a template. A reaction solution was prepared according to a composition attached to the kit, and a reaction at 98°C for 10 seconds, 54°C for 20 seconds and 68°C for 120 seconds was performed in 40 cycles. As a result, a PCR product containing the IspSK gene, the IspSP gene, the IspSM gene or the IspSM (L) gene was obtained. Likewise, PCR was performed with Prime Star polymerase (manufactured by Takara Bio Inc.) using synthesized oligonucleotides consisting of the nucleotide sequences represented by SEQ ID NOS:21 and 22 as primers with pSTV28-Ptac-Ttrp as a template. A reaction solution was prepared according to a composition attached to the kit, and a reaction at 98°C for 10 seconds, 54°C for 20 seconds and 68°C for 210 seconds was performed in 40 cycles. As a result, a PCR product containing pSTV28-Ptac-Ttrp was obtained. Subsequently, the purified IspSK gene, IspSP gene, IspSM gene, and IspSM (L) gene fragments were ligated to the PCR product for pSTV28-Ptac-Ttrp using In-Fusion HD Cloning Kit (manufactured by Clontech). The resulting plasmids for expressing the IspSK gene, the IspSP gene, IspSM gene and IspSM (L) gene were designated as pSTV28-Ptac-IspSK, pSTV28-Ptac-IspSP, pSTV28-Ptac-IspSM, and pSTV28-Ptac-IspSM (L), respectively.

**Table 2. Primer sequences used for construction of plasmids for expressing IspS genes derived from various plants**

| Subject for amplification | Sequence name | Sequence (5'-) |
|---|---|---|
| IspSK | Ptac-IspS(K)F | |
| IspSK | IspS(K)R-MCSR | |
| IspSP | Ptac-IspS(P)F | |
| IspSP | IspS(P)R-MCSR | |
| IspSM | Ptac-IspS(M)F | |
| IspSM | IspS(M)R-MCSR | ACGGCCAGTGAATTCTTAGTTAATCGGGAACGGGT (SEQ ID NO:18) |
| IspSM (L) | Ptac-IspS(M(L))F | |
| IspSM(L) | IspS(M(L))R-MCSR | ACGGCCAGTGAATTCTCAGTTAATCGGGAACGGGT (SEQ ID NO:20) |
| pSTV28-Ptac-Ttrp | pSTV28-F | GTGTGAAATTGTTATCCGCTCACAATTCC (SEQ ID NO:21) |
| pSTV28-Ptac-Ttrp | pSTV28-R | GAATTCACTGGCCGTCGTTTTACAACG (SEQ ID NO:22) |

### Example 4: Measurement of enzymatic activity of isoprene synthase derived from various plants using crude enzyme extract derived from E. coli

### 4-1) Construction of E. coli MG1655 strain having ability to produce isoprene

Competent cells of *E. coli* MG1655 strain (ATCC 700926) were prepared, and then pSTV28-Ptac-Ttrp, pSTV28-Ptac-IspSK, pSTV28-Ptac-IspSP, pSTV28-Ptac-IspSM, or further pSTV28-Ptac-IspSM (L) was introduced therein. A suspension of the cells was evenly applied onto an LB plate containing 60 mg/L of chloramphenicol, and cultured at 37°C for 18 hours. Subsequently, transformants that were resistant to chloramphenicol were obtained from the resulting plate. A strain in which pSTV28-Ptac-Ttrp, pSTV28-Ptac-IspSK, pSTV28-Ptac-IspSP, pSTV28-Ptac-IspSM, or further pSTV28-Ptac-IspSM (L) was introduced into *E. coli* MG1655 strain were designated as MG1655/pSTV28-Ptac-Ttrp, MG1655/pSTV28-Ptac-IspSK, MG1655/pSTV28-Ptac-IspSP, MG1655/pSTV28-Ptac-IspSM, or further MG1655/pSTV28-Ptac-IspSM (L) strain, respectively.

### 4-2) Method of preparing crude enzyme extract

Microbial cells of MG1655/pSTV28-Ptac-Ttrp, MG1655/pSTV28-Ptac-IspSK, MG1655/pSTV28-Ptac-IspSP, MG1655/pSTV28-Ptac-IspSM, or MG1655/pSTV28-Ptac-IspSM (L) strain were evenly applied to the LB plate containing 60 mg/L of chloramphenicol, and cultured at 37°C for 18 hours. The microbial cells corresponding to 1/6 of the resulting plate were inoculated to a Sakaguchi flask in which 20 mL of LB containing 60 mg/L of chloramphenicol had been added, and cultured at 37°C for 6 hours. The microbial cells from the culture medium were centrifuged at 5000 rpm at 4°C for 5 minutes, and washed twice with ice-cold isoprene synthase buffer (50 mM Tris-HCl, pH 8.0, 20 mM MgCl₂, 5% glycerol). The washed microbial cells were suspended in 1.8 mL of the same buffer. About 0.9 mL of beads for disruption (YBG01, diameter 0.1 mm) and 0.9 mL of the microbial cell suspension were placed in a 2 mL tube specific for a multibead shocker, and the microbial cells were disrupted using the multibead shocker manufactured by Yasui Kikai Corporation at 2500 rpm at 4°C for 3 cycles of ON for 30 seconds/OFF for 30 seconds. After the disruption, the tube was centrifuged at 20,000 g at 4°C for 20 minutes, and a supernatant was used as a crude enzyme extract.

### 4-3) Measurement of isoprene synthase activity

The crude enzyme extract from MG1655/pSTV28-Ptac-Ttrp, MG1655/pSTV28-Ptac-IspSK, MG1655/pSTV28-Ptac-IspSP, MG1655/pSTV28-Ptac-IspSM, or MG1655/pSTV28-Ptac-IspSM (L) strain (containing 2 mg as amount of total protein) together with the isoprene buffer in a total volume of 0.5 mL was placed in a headspace vial (22 mL CLEAR CRIMP TOP VIAL (Cat #B0104236) manufactured by Perkin Elmer), then 0.025 mL of a 0.5 M MgCl₂ solution and 0.01 mL of a 0.2 M DMAPP (manufactured by Cayman, Catalog No. 63180) solution were added thereto, and the mixture was lightly vortexed. Then immediately, the vial was tightly sealed with a cap with a butyl rubber septum for the headspace vial (CRIMPS (Cat #B0104240) manufactured by Perkin Elmer), and kept at 37°C for 2 hours.

After completion of the reaction, a concentration of isoprene in the headspace of the vial was measured by gas chromatography. An analysis condition for the gas chromatography will be described below.

### Headspace sampler (manufactured by Perkin Elmer, Turbo Matrix 40)

Temperature for keeping vial warm: 40°C
Time period for keeping vial warm: 30 minutes
Pressurization time: 3.0 minutes
Injection time: 0.02 minute
Needle temperature: 70°C
Transfer temperature: 80°C
Carrier gas pressure (high purity helium): 124 kPa

### Gas chromatography (manufactured by Shimadzu Corporation, GC-2010 Plus AF)

Column (Rxi (registered trademark)-1 ms: length 30 m, internal diameter 0.53 mm, liquid phase film thickness 1.5 µm, Cat #13370)
Column temperature: 37°C
Pressure: 24.8 kPa
Column flow: 5 mL/minute
Influx method: Split 1:0 (actually measured 1:18)
Transfer flow: 90 mL
GC injection volume: 1.8 mL (transfer flow × injection time)
Injection volume of sample into column: 0.1 mL
Inlet temperature: 250°C
Detector: FID (hydrogen 40 mL/minute, air 400 mL/minute, makeup gas helium 30 mL/minute)
Detector temperature: 250°C

### Preparation of isoprene standard sample

A reagent isoprene (specific gravity 0.681) was diluted to 10, 100, 1000, 10000 and 100000 times with cold methanol to prepare standard solutions for addition. Subsequently, 1 µL of each standard solution for addition was added to a headspace vial in which 1 mL of water had been added, and used as a standard sample.

The amount of formed isoprene after the reaction of each microbial strain for 2 hours is described in Table 3.

**Table 3. Amount of formed isoprene after reaction for 2 hours**

| Name of microbial strain | Amount of formed isoprene (mg/L) |
|---|---|
| MG1655/pSTV28-Ptac-Ttrp | 0.10 ± 0.01 |
| MG1655/pSTV28-Ptac-IspSK | 0.45 ± 0.02 |
| MG1655/pSTV28-Ptac-IspSM | 28.93 ± 6.04 |
| MG1655/pSTV28-Ptac-IspSM(L) | 5.06 ± 0.13 |
| MG1655/pSTV28-Ptac-IspSP | 0.10 ± 0.01 |

From the result in Table 3, the amount of formed isoprene was larger in order of MG1655/pSTV28-Ptac-IspSM, MG1655/pSTV28-Ptac-IspSM (L) and MG1655/pSTV28-Ptac-IspSK strains, and was almost equal in MG1655/pSTV28-Ptac-IspSP and MG1655/pSTV28-Ptac-Ttrp strains. From the above result, the crude enzyme extract from the strain introduced with the isoprene synthase derived from Mucuna exhibited the highest activity to form isoprene.

### Example 5: Effects of introduction of isoprene synthase derived from various plants on E. coli MG1655 strain

From the result of the crude enzymatic activity in Example 4, the highest activity was confirmed in the isoprene synthase derived from Mucuna that deleted the chloroplast localization signal. Thus, an ability to produce isoprene from glucose was compared in all isoprene synthase-introduced strains in which the chloroplast localization signal had been deleted. Microbial cells of MG1655/pSTV28-Ptac-Ttrp, MG1655/pSTV28-Ptac-IspSK, MG1655/pSTV28-Ptac-IspSP, or MG1655/pSTV28-Ptac-IspSM strain were evenly applied onto the LB plate containing 60 mg/L of chloramphenicol, and cultured at 37°C for 18 hours. One loopful of the microbial cells from the resulting plate was inoculated to 1 mL of M9 glucose medium in a headspace vial. The vial was tightly sealed with the cap with the butyl rubber septum for the headspace vial (CRIMPS (Cat #B0104240) manufactured by Perkin Elmer), and the microbial cells were cultured at 30°C for 24 hours using a reciprocal shaking cultivation apparatus (120 rpm). A composition of the M9 glucose medium is as described in Table 4.

**Table 4. Composition of M9 glucose medium**

| | |
|---|---|
| Glucose | 1.0 g/L |
| Na₂HPO₄ | 6.0 g/L |
| KH₂PO₄ | 3.0 g/L |
| NaCl | 0.5 g/L |
| NH₄Cl | 1.0 g/L |
| 1 M MgSO₄ (autoclaved) | 1.0 mL |
| 1 M CaCl₂ (autoclaved) | 0.1 mL |

Further, chloramphenicol was added at a final concentration of 60 mg/L. The volume was adjusted to 1 L and the medium was then sterilized by filtration.

After completion of the cultivation, the concentration of isoprene in the headspace in the vial was measured by the gas chromatography. An OD value was also measured at 600 nm using a spectrophotometer (HITACHI U-2900). The concentration of isoprene and the OD value in each microbial strain at the time of completing the cultivation are described in Table 5.

**Table 5. OD value, and amount (µg/L) of isoprene produced by MG1655/pSTV28-Ptac-Ttrp, MG1655/pSTV28-Ptac-IspSK, MG1655/pSTV28-Ptac-IspSP and MG1655/pSTV28-Ptac-IspSM strains at the time of completing cultivation**

| Name of microbial strain | OD value | Amount (µg/L) of formed isoprene |
|---|---|---|
| MG1655/pSTV28-Ptac-Ttrp | 1.68±0.04 | ND |
| MG1655/pSTV28-Ptac-IspSK | 1.60±0.09 | 43±6 |
| MG1655/pSTV28-Ptac-IspSM | 1.45±0.03 | 56±7 |
| MG1655/pSTV28-Ptac-IspSP | 1.59±0.07 | 26±3 |

From the results in Table 5, it was found that the amount of produced isoprene was larger in order of MG1655/pSTV28-Ptac-IspSM, MG1655/pSTV28-Ptac-IspSK, MG1655/pSTV28-Ptac-IspSP and MG1655/pSTV28-Ptac-Ttrp strains. From the above results, the strain introduced with the isoprene synthase derived from Mucuna exhibited the highest activity to produce isoprene in the wild strains.

### Example 6: Effects of introduction of isoprene synthase derived from various plants on E. coli MG1655 strain in which MEP (methylerythritol) pathway is enhanced.

### 6-1) Construction of plasmid for expressing dxs gene (pMW219-dxs)

It was already reported that the amount of formed isoprene was enhanced (Appl. Microbiol. Biotechnol., (2011) 90, 1915-1922), when the expression of a dxs (1-deoxy-D-xylulose-5-phosphate synthase) gene that constitutes the MEP pathway was enhanced in *E. coli* strain in which the isoprene synthase was introduced. Thus, it was confirmed whether an ability to produce isoprene was also different due to an origin of the isoprene synthase in the strain in which the expression of the dxs gene was enhanced. The entire genomic nucleotide sequence of *E. coli* K-12 strain was already shown (GenBank Accession No. U00096) (Science, (1997) 277, 1453-1474). pMW219 (manufactured by Nippon Gene Co., Ltd.) was used for amplifying the gene. This plasmid can increase an expression level of an objective gene when isopropyl-β-thiogalactopyranoside (IPTG) is added by introducing the objective gene into a multicloning site. Synthesized oligonucleotides were synthesized from the nucleotide sequences represented by SEQ ID NOS:23 and 24 based on the nucleotide sequence of the dxs gene in the genomic nucleotide sequence of *E. coli.* Subsequently, PCR was performed with Prime Star polymerase (manufactured by Takara Bio Inc.) using the synthesized oligonucleotides consisting of the nucleotide sequences represented by SEQ ID NOS:23 and 24 as the primers with MR1655 strain genomic DNA as the template. A reaction solution was prepared according to the composition attached to the kit, and a reaction at 98°C for 10 seconds, 54°C for 20 seconds and 68°C for 120 seconds was performed in 40 cycles. As a result, a PCR product containing the dxs gene was obtained. Likewise, PCR was performed with Prime Star polymerase (manufactured by Takara Bio Inc.) using the synthesized oligonucleotides consisting of the nucleotide sequences represented by SEQ ID NOS:25 and 26 as the primers with pMW219 as the template. A reaction solution was prepared according to the composition attached to the kit, and a reaction at 98°C for 10 seconds, 54°C for 20 seconds and 68°C for 240 seconds was performed in 40 cycles. As a result, a PCR product containing pMW219 was obtained. Subsequently, the purified dxs gene fragment was ligated to the PCR product of pMW219 using In-Fusion HD Cloning Kit (manufactured by Clontech). The resulting plasmid for expressing the dxs gene was designated as pMW219-dxs.

**Table 6. Primer sequences used for construction of plasmid for expressing dxs gene**

| Sequence name | Sequence (5'-) |
|---|---|
| *dxs*-F | CAGGAAACAGCTATGAGTTTTGATATTGCCAAATACCCGAC (SEQ ID NO:23) |
| *dxs*-R | GCTGCCACTCCTGCTATACTCGTCATAC (SEQ ID NO:24) |
| pMW219-F | CATAGCTGTTTCCTGTGTGAAATTGTTATC (SEQ ID NO:25) |
| pMW219-R | AGCAGGAGTGGCAGCGAATTCGAGCTCGGTACCCGGGGAT (SEQ ID NO:26) |

### 6-2) Introduction of pMW219-dxs into E. coli MG1655 strain having ability to produce isoprene

Competent cells of MG1655/pSTV28-Ptac-Ttrp, MG1655/pSTV28-Ptac-IspSK, MG1655/pSTV28-Ptac-IspSM, or further MG1655/pSTV28-Ptac-IspSP strain were prepared, and pMW219-dxs was introduced therein by an electroporation method. The cells were evenly applied onto the LB medium containing 60 mg/L of chloramphenicol and 50 mg/L of kanamycin hydrochloride, and the cells were cultured at 37°C for 18 hours. Transformants that were resistant to chloramphenicol and kanamycin were obtained from the resulting LB plates. Strains in which pMW219-dxs had been introduced into MG1655/pSTV28-Ptac-Ttrp, MG1655/pSTV28-Ptac-IspSK, MG1655/pSTV28-Ptac-IspSM, or further MG1655/pSTV28-Ptac-IspSP strain were designated as MG1655/pSTV28-Ptac-Ttrp/pMW219-dxs, MG1655/pSTV28-Ptac-IspSK/pMW219-dxs, MG1655/pSTV28-Ptac-IspSM/pMW219-dxs, or further MG1655/pSTV28-Ptac-IspSP/pMW219-dxs strain, respectively.

### 6-3) Effects of introduction of isoprene synthase derived from various plants on E. coli MG1655 strain in which expression of DXS is enhanced

MG1655/pSTV28-Ptac-Ttrp/pMW219-dxs, MG1655/pSTV28-Ptac-IspSK/pMW219-dxs, MG1655/pSTV28-Ptac-IspSM/pMW219-dxs, or further MG1655/pSTV28-Ptac-IspSP/pMW219-dxs strain were evenly applied onto the LB medium containing 60 mg/L of chloramphenicol and 50 mg/L of kanamycin hydrochloride, and were cultured at 37°C for 18 hours. Subsequently, the cultivation in the headspace vial was evaluated as described in Example 5. The amount (µg/L) of produced isoprene and the OD value upon completion of the cultivation are described in Table 7.

**Table 7. Amount (µg/L) of produced isoprene and OD value when the cultivation was completed in various strains having enhanced isoprene synthase which are prepared from E. coli MG1655 strain having enhanced DXS as host.**

| Name of microbial strain | OD value | Amount (µg/L) of produced isoprene |
|---|---|---|
| MG1655/pSTV28-Ptac-Ttrp/pMW219-dxs | 1.46±0.04 | ND |
| MG1655/pSTV28-Ptac-IspSK/pMW219-dxs | 1.13±0.02 | 101±28 |
| MG1655/pSTV28-Ptac-IspSM/pMW219-dxs | 1.76±0.06 | 126±23 |
| MG1655/pSTV28-Ptac-IspSP/pMW219-dxs | 2.21±0.12 | 42±17 |

From the results in Table 7, the amount of produced isoprene was larger in order of MG1655/pSTV28-Ptac-IspSM/pMW219-dxs, MG1655/pSTV28-Ptac-IspSK/pMW219-dxs, MG1655/pSTV28-Ptac-IspSP/pMW219-dxs and MG1655/pSTV28-Ptac-Ttrp/pMW219-dxs strains. From the above results, the strain introduced with the isoprene synthase derived from Mucuna also exhibited the highest ability to produce isoprene in the MEP pathway-enhanced strains.

### Example 7: Effects of introduction of isoprene synthase derived from various plants on E. coli MG1655 strain in which MVA (mevalonic acid) pathway is introduced

### 7-1) Cloning gene downstream of mevalonate pathway which is derived from yeast

A downstream region of the mevalonate pathway was obtained from *Saccharomyces cerevisiae* (WO2009076676, *Saccharomyces* Genome database http://www.yeastgenome.org/# Nucleic Acids Res., Jan 2012; 40: D700-D705). An ERG12 gene encoding mevalonate kinase, an ERG8 gene encoding phosphomevalonate kinase, an ERG19 gene encoding diphosphomevalonate decarboxylase, and an IDI1 gene encoding isopentenyl diphosphate delta isomerase were amplified by PCR with genomic DNA of *S. cerevisiae* as the template using the primer shown below (Table 8). Prime Star Max Premix sold by Takara Bio Inc. was used for a PCR enzyme, and the reaction was performed at 98°C for 2 minutes and for 30 cycles of 98°C for 10 seconds, 55°C for 5 seconds and 72°C for 5 seconds/kb. Cloning and construction of an expression vector were performed by introducing the PCR fragment into the pSTV28-Ptac-Ttrp vector (SEQ ID NO:12) treated with the restriction enzyme *Sma*I by an in-fusion cloning method. *E. coli* DH5α was transformed with the expression vector, clones having assumed sequence length from each gene were selected, a plasmid was extracted according to standard methods, and its sequence was confirmed. The nucleotide sequences of these amplified genes and the amino acid sequences of the enzymes encoded by these genes are available on *Saccharomyces* Genome database http://www.yeastgenome.org/#.

**Table 8. Primer sequences used for cloning of genes downstream of mevalonate pathway**

| Amplified gene | Sequence name | Sequence (5'-) |
|---|---|---|
| ERG12 | MVK-IFS_5742-33-1 | |
| ERG12 | MVK-IFA_5742-33-2 | |
| ERG8 | PMK-IFS_5742-33-3 | |
| ERG8 | PMK-IFA_5742-33-4 | |
| ERG19 | MVD-IFS_5742-33-5 | |
| ERG19 | MVD-IFA_5742-33-6 | |
| IDI1 | yIDI-IFS_5742-33-7 | |
| IDI1 | yIDI-IFA_5742-33-8 | |

### 7-2) Construction of artificial operon downstream of mevalonate pathway

A sequence in which the gene encoding the mevalonate kinase and the gene encoding the phosphomevalonate kinase were arranged in straight was constructed by the in-fusion cloning method. The ERG12 gene encoding the mevalonate kinase and the ERG8 gene encoding the phosphomevalonate kinase were amplified by PCR with genomic DNA from *Saccharomyces cerevisiae* as the template using the primers shown in Table 9. KOD plus sold by Toyobo was used for the PCR enzyme, and the reaction was performed at 94°C for 2 minutes and for 30 cycles of 94°C for 15 seconds, 45°C for 30 seconds and 68°C for 1 minute/kb. The cloning and the construction of an expression vector were performed by inserting the PCR fragment into pUC118 vector treated with the restriction enzyme *Sma*I by the in-fusion cloning method. *E. coli* JM109 was transformed with the expression vector, clones having assumed sequence length of each gene were selected, a plasmid was extracted according to standard methods, and its sequence was confirmed. The produced plasmid was designated as pUC-mvk-pmk. The nucleotide sequence of pUC-mvk-pmk is represented by SEQ ID NO:35.

**Table 9. Primer sequences used for ligating mevalonate kinase and phosphomevalonate kinase**

| Amplified gene | Sequence name | Sequence (5'-) |
|---|---|---|
| ERG12 | KKS1-6038-2-1 | TCGAGCTCGGTACCCATGTCATTACCGTTCTTAACTTCT (SEQ ID NO:36) |
| ERG12 | KKA1-6038-2-2 | |
| ERG8 | KKS2-6083-2-3 | |
| ERG8 | KKA2-6083-2-4 | CTCTAGAGGATCCCCTTATTTATCAAGATAAGTTTCCGG (SEQ ID NO:39) |

A sequence in which a gene encoding diphosphomevalonate decarboxylase and a gene encoding isopentenyl diphosphate delta isomerase were arranged in straight was constructed by the in-fusion cloning method. The ERG19 gene encoding the diphosphomevalonate decarboxylase and the IDI1 gene encoding the isopentenyl diphosphate delta isomerase were amplified by PCR with genomic DNA of *Saccharomyces cerevisiae* as the template using the primers shown in Table 10. KOD plus sold by Toyobo was used for the PCR enzyme, and the reaction was performed at 94°C for 2 minutes and for 30 cycles of 94°C for 15 seconds, 45°C for 30 seconds and 68°C for 1 minute/kb, and then at 68°C for 10 minutes. The cloning and the construction of an expression vector were performed by inserting the PCR fragment into TWV228 vector treated with the restriction enzyme *Sma*I by the in-fusion cloning method. *E. coli* DH5α was transformed with the expression vector, clones having assumed sequence length of each gene were selected, a plasmid was extracted according to standard methods, and its sequence was confirmed. The produced plasmid was designated as pTWV-dmd-yidi. The nucleotide sequence of pTWV-dmd-yidi is represented by SEQ ID NO:40.

**Table 10. Primer sequences used for ligating diphosphomevalonate decarboxylase and isopentenyl diphosphate delta isomerase.**

| Amplified gene | Sequence name | Sequence (5'-) |
|---|---|---|
| ERG19 | DyIS1-6083-2-5 | TCGAGCTCGGTACCCATGACCGTTTACACAGCATCC (SEQ ID NO:41) |
| ERG19 | DyIA1-6083-2-6 | |
| IDI1 | DyIS2-6083-2-7 | |
| IDI1 | DyIA2-6083-2-8 | |

A sequence in which the gene encoding the mevalonate kinase, the gene encoding the diphosphomevalonate kinase, the gene encoding the diphosphomevalonate decarboxylase and the gene encoding the isopentenyl diphosphate delta isomerase were arranged in straight was constructed by the in-fusion cloning method. An expression vector in which these four enzyme genes were arranged in straight was constructed by amplifying the the gene encoding the mevalonate kinase and the gene encoding the diphosphomevalonate kinase by PCR with pUC-mvk-pmk as the template using the primers shown in Table 11 and amplifying the gene encoding the diphosphomevalonate decarboxylase and the gene encoding the isopentenyl diphosphate delta isomerase by PCR with pTWV-dmd-yidi as the template using the primers shown in Table 11, followed by cloning the amplified products into pTrcHis2B vector by the in-fusion cloning method. Prime Star HS DNA polymerase sold by Takara Bio Inc. was used for the PCR enzyme, and the reaction was carried out at 98°C for 2 minutes followed by in 30 cycles of 98°C for 10 seconds, 52°C for 5 seconds and 72°C for 1 minute/kb, and then at 72°C for 10 minutes. The PCR fragment was inserted into pTrcHis2B vector treated with the restriction enzymes *Nco*I and *Pst*I to construct the expression vector. *E. coli* JM109 was transformed with the expression vector, clones having an objective sequence length were selected, a plasmid was extracted according to standard methods, and its sequence was confirmed. The constructed expression vector was designated as pTrc-KKDyI (β) . The nucleotide sequence of pTrc-KKDyI (β) is represented by SEQ ID NO:45.

**Table 11. Primer sequences used for amplifying genes for constructing pTrc-KKDyI (β)**

| Template plasmid | Sequence name | Sequence (5'-) |
|---|---|---|
| pUC-mvk-pmk | KKDS2_6038-3-2 | GAGGAATAAACCATGTCATTACCGTTCTTAACTTCT (SEQ ID NO:46) |
| pUC-mvk-pmk | KKMyIA_6038-2-9 | |
| pTWV-dmd-yidi | KMS_6038-6-1 | |
| pTWV-dmd-yidi | KDyIA_6038-3-3 | |

### 7-3) Fixation of downstream region of mevalonate pathway on chromosome

The sequence in which the gene encoding the mevalonate kinase, the gene encoding the diphosphomevalonate kinase, the gene encoding the diphosphomevalonate decarboxylase and the gene encoding the isopentenyl diphosphate delta isomerase were arranged in straight was expressed on a chromosome. A glucose isomerase promoter was used for the expression of the gene, and a transcription termination region of aspA gene in *E*. *coli* was used for the termination of the transcription (WO2010031062). A translocation site of Tn7 was used as a chromosomal site to be fixed (Mol Gen Genet., 1981;183 (2): 380-7). A cat gene was used as a drug marker after the fixation of the chromosome. A T7 downstream region in the chromosome region to be fixed was amplified by PCR with genomic DNA of *E*. *coli* as the template using the primers shown in Table 12. Prime Star HS DNA polymerase sold by Takara Bio Inc. was used for the PCR enzyme, and the reaction was carried out at 98°C for 2 minutes followed by in 30 cycles of 98°C for 10 seconds, 52°C for 5 seconds and 72°C for 1 minute/kb, and then at 72°C for 10 minutes. A cat gene region containing a λ phage attachment site was amplified by PCR with pMW118-attL-Cm-attR plasmid as the template using the primers shown in Table 12 (WO2010-027022). Prime Star HS DNA polymerase sold by Takara Bio Inc. was used for the PCR enzyme, and the reaction was carried out at 95°C for 3 minutes followed by in 2 cycles of 95°C for 1 minute, 34°C for 30 seconds and 72°C for 40 seconds, 2 cycles of 95°C for 30 seconds, 50°C for 30 seconds and 72°C for 40 seconds, and then at 72°C for 5 minutes. A sequence downstream of the mevalonate pathway to which a promoter and a transcription termination region had been added (hereinafter abbreviated as KKDyI) was amplified with pTrc-KKDyI (β) as the template using the primers shown in Table 12. Prime Star HS DNA polymerase sold by Takara Bio Inc. was used for the PCR enzyme, and the reaction was carried out at 98°C for 2 minutes followed by in 30 cycles of 98°C for 10 seconds, 52°C for 5 seconds and 72°C for 1 minute/kb, and then at 72°C for 10 minutes. A vector was constructed using these PCR products and pMW219 treated with the restriction enzyme *Sma*I by the in-fusion cloning method. *E*. *coli* JM109 was transformed with the expression vector, clones having an objective sequence length were selected, a plasmid was extracted according to standard methods, and its sequence was confirmed. The resulting plasmid was designated as pMW219-KKDyI-TaspA. The nucleotide sequence of pMW219-KKDyI-TaspA is represented by SEQ ID NO:50.

Subsequently, a Tn7 upstream region in the chromosome region to be fixed was amplified by PCR with the genomic DNA of *E*. *coli* as the template using the primers shown in Table 13. Prime Star HS DNA polymerase sold by Takara Bio Inc. was used for the PCR enzyme, and the reaction was carried out at 98°C for 2 minutes followed by in 30 cycles of 98°C for 10 seconds, 52°C for 5 seconds and 72°C for 1 minute/kb, and then at 72°C for 10 minutes. A vector was constructed using the PCR product and pMW219-KKDyI-TaspA treated with the restriction enzyme *Sal*I by the in-fusion cloning method. *E. coli* JM109 was transformed with the expression vector, clones having an objective sequence length were selected, a plasmid was extracted according to standard methods, and its sequence was confirmed. The resulting plasmid was designated as pMW-Tn7-Pgi-KKDyI-TaspA-Tn7. The sequence of the constructed plasmid is represented by SEQ ID NO:51.

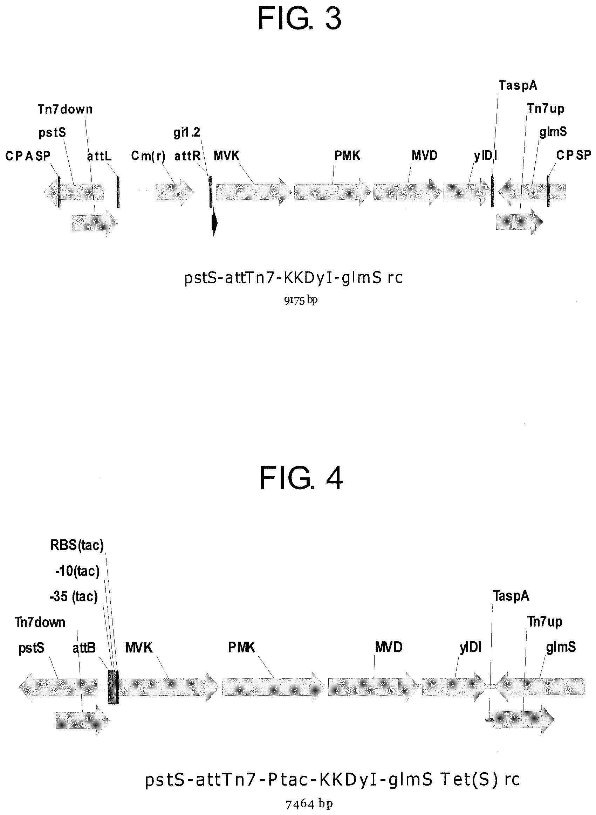

Subsequently, a chromosome having a region including the chloramphenicol resistance gene, the glucose isomerase promoter, the operon downstream of the mevalonate pathway, and the aspA gene transcription termination region was fixed using λ-Red method. A fragment for chromosome fixation was prepared by extracting the plasmid pMW-Tn7-Pgi-KKDyI-TaspA-Tn7 and then treating it with the restriction enzymes *Pvu*I and *Sal*I followed by purifying it. *E. coli* MG1655 containing a plasmid pKD46 having a temperature-sensitive replication capacity (hereinafter referred to as MG1655/pKD46) was used for the electroporation. The plasmid pKD46 [Proc. Natl. Acad. Sci. USA, 2000, vol. 97, No.12, p6640-6645] contains a DNA fragment of total 2154 nucleotides (GenBank/EMBL Accession No. J02459, 31088th to 33241st) of λ phage containing λ Red system genes (λ, β, exo genes) controlled by an arabinose-inducible ParaB Promoter. After the electroporation, a colony that had acquired the resistance to chloramphenicol was obtained, subsequently genomic DNA was extracted, and it was confirmed by PCR using the primers shown in Table 14 that the objective region was fixed on the chromosome. Further, the sequence of the objective region was confirmed by confirming the sequence of the PCR fragment. The nucleotide sequence of the mevalonate pathway downstream and its proximal region fixed on the chromosome is represented by SEQ ID NO:52, and its construction outline is shown in FIG. 3. The resulting mutant was designated as MG1655 cat-Pgi-KKDyI.

The drug marker in MG1655 cat-Pgi-KKDyI was removed by the following procedure. Competent cells of MG1655 cat-Pgi-KKDyI was made, and then pMW-int-xis was introduced therein. pMW-int-xis is a plasmid containing a gene encoding integrase (Int) of the λ phage and a gene encoding excisionase (Xis) of the λ phage and having the temperature-sensitive replication capacity (WO2007/037460, JP 2005-058827).

The chloramphenicol-resistant gene located in a region sandwiched with attL and attR that are the attachment site of the λ phage is dropped off from the chromosome by introducing pMW-int-xis. As a result, it is known that the host loses the resistance to chloramphenicol. And, a chloramphenicol-sensitive strain was obtained from the resulting colony, and subsequently cultured on the LB medium at 42°C for 6 hours. The cultured microbial cells were applied onto the LB plate medium to allow colonies to appear. A colony that had lost the resistance to ampicillin was selected from these colonies to remove the drug resistance. The mutant obtained as above was designated as MG1655 Pgi-KKDyI.

**Table 12. Primers for making PCR fragments used for construction of pMW219-KKDyI-TaspA**

| Template DNA | Amplified region | Sequence name | Sequence (5'-) |
|---|---|---|---|
| E. coli genome | Tn7 downstream | Tn7dS_6038-7-1 | |
| E. coli genome | Tn7 downstream | Tn7dA_6038-7-2 | AGGCTTCATTTTAATCAAACATCCTGCCAACTC (SEQ ID NO:54) |
| pMW-attL -Cm-attR | attL-cat-attR | Tn7dattLcmS_6 038-7-4 | ATTAAAATGAAGCCTGCTTTTTTAT (SEQ ID NO:55) |
| pMW-attL -Cm-attR | attL-cat-attR | PgiattRcmA_60 38-7-5 | GGCATCGTCAAGGGCCGCTCAAGTTAGTATAA (SEQ ID NO:56) |
| pTrc-KKDyI(β) | KKDyI | gi1.2-MVK-S_6038-7-6 | |
| pTrc-KKDyI(β) | KKDyI | pMW-TaspA-yIDIA_6038-7-7 | |

**Table 13. Primers for making PCR fragments used for construction of pMW-Tn7-Pgi-KKDyI-TaspA-Tn7**

| Template DNA | Amplified region | Sequence name | Sequence (5'-) |
|---|---|---|---|
| E. coli genome | Tn7 upstream | Tn7upSv02_6038 -24-1 | |
| E. coli genome | Tn7 upstream | Tn7upAv02_6038 -24-2 | |

**Table 14. PCR primers for confirming chromosome fixation of mevalonate pathway downstream**

| Sequence name | Sequence (5'-) |
|---|---|
| Tn7v02-F_6038-22-5 | ACGAACTGCTGTCGAAGGTT (SEQ ID NO:61) |
| Tn7v02-R_6038-22-6 | GGTGTACGCCAGGTTGTTCT (SEQ ID NO:62) |

### 7-4) Substitution of promoter downstream of mevalonate pathway on chromosome

The promoter of the operon downstream of the mevalonate pathway on the chromosome was substituted by the λ-red method. A genomic fragment having attL-Tet-attR-Ptac was used as the template for PCR. This is one in which the tac promoter, and attL and attR that are the attachment sites for a tetracycline resistant drug marker and the λ phage are aligned. This sequence is represented by SEQ ID NO:63. A PCR fragment was prepared using the promoter shown in Table 15. LA-Taq polymerase sold by Takara Bio Inc. was used for the PCR enzyme, and the reaction was carried out at 92°C for 1 minute, then for 40 cycles of 92°C for 10 seconds, 50°C for 20 seconds and 72°C for 1 minute/kb, and further at 72°C for 7 minutes. The PCR product was purified. MG1655 Pgi-KKDyI containing the plasmid pKD46 (hereinafter referred to as MG1655 Pgi-KKDyI/pKD46) having the temperature-sensitive replication capacity was used for the electroporation. The plasmid pKD46 [Proc. Natl. Acad. Sci. USA, 2000, vol. 97, No.12, p6640-6645] contains a DNA fragment of total 2154 nucleotides (GenBank/EMBL Accession No. J02459, 31088th to 33241st) of λ phage containing λ Red system genes (X, β, exo genes) controlled by an arabinose-inducible ParaB Promoter. The plasmid pKD46 is required for incorporating the PCR product into MG1655 Pgi-KKDyI.

Competent cells for the electroporation were prepared as follows. MG1655 Pgi-KKDyI/pKD46 cultured in the LB medium containing 100 mg/L of ampicillin at 30°C overnight were diluted to 100 times with 5 mL of LB medium containing ampicillin and L-arabinose (1 mM). The resulting cells in diluted suspension were grown until OD600 reached about 0.6 with ventilating at 30°C, and subsequently washed three times with ice-cold 10% glycerol solution to use for the electroporation. The electroporation was performed using 50 µL of the competent cells and about 100 ng of the PCR product. The cells after the electroporation in 1 mL of SOC medium [Molecular Cloning: Laboratory Manuals, 2nd Edition, Sambrook, J. et al., Cold Spring Harbor Laboratory Press (1989)] were cultured at 37°C for one hour, and subjected to a plate culture on LB agar medium at 37°C to select a chloramphenicol-resistant transformant. Subsequently, in order to remove the pKD46 plasmid, the transformant was subcultured on the LB agar medium containing tetracycline at 37°C. The ampicillin resistance was examined in the obtained colonies, and an ampicillin-resistant strain having no pKD46 was obtained. A mutant containing the tac promoter substitution that could be distinguished by the tetracycline-resistant gene was obtained. The obtained mutant was designated as MG1655 tet-Ptac-KKDyI.

The antibiotic marker was removed by the following procedure. Competent cells of MG1655 tet-Ptac-KKDyI were made, and then pMW-int-xis was introduced therein. pMW-int-xis is a plasmid containing the genes encoding integrase (Int) and excisionase (Xis) of the λ phage and having the temperature-sensitive replication capacity (WO2007/037460, JP Publication No. 2005-058827). The tetracycline-resistant gene located in a region sandwiched with attL and attR that are the attachment site of the λ phage is dropped off from the chromosome by introducing pMW-int-xis. As a result, it is known that the host loses the resistance to tetracycline. Thus, a tetracycline-sensitive strain was obtained from the resulting colonies. Cells of this strain were cultured on the LB medium at 42°C for 6 hours, and the cultured cells were applied onto the LB plate medium to allow colonies to appear. A clone that had lost the resistance to ampicillin was selected to remove the drug resistance. The resulting mutant was designated as MG1655 Ptac-KKDyI. The nucleotide sequence of the mevalonate pathway downstream and its proximal region controlled by the tac promoter on the chromosome is represented by SEQ ID NO:64, and its outline is shown in FIG. 4.

**Table 15. Primers for making PCR fragments for promoter substitution**

| Sequence name | Sequence (5'-) |
|---|---|
| APtacKKDyIv03_6038 -36-5 | |
| SPtacKKDyIv02_6038 -36-3 | |

### 7-5) Introduction of isoprene synthase derived from various plants into MG1655 Ptac-KKDyI strain

Competent cells of MG1655 Ptac-KKDyI strain were prepared, and then pSTV28-Ptac-Ttrp, pSTV28-Ptac-IspSK, pSTV28-Ptac-IspSM, or further pSTV28-Ptac-SP was introduced therein. The cells were evenly applied onto the LB plate containing 60 mg/L of chloramphenicol, and the cells were cultured at 37°C for 18 hours. Transformants that exhibited the chloramphenicol resistance were obtained from the resulting plate. A strain in which pSTV28-Ptac-Ttrp, pSTV28-Ptac-IspSK, pSTV28-Ptac-IspSM, or pSTV28-Ptac-IspSP had been introduced into MG1655 Ptac-KKDyI strain was designated as MG1655 Ptac-KKDyI/pSTV28-Ptac-Ttrp, MG1655 Ptac-KKDyI/pSTV28-Ptac-IspSK, MG1655 Ptac-KKDyI/pSTV28-Ptac-IspSM, or MG1655 Ptac-KKDyI/pSTV28-Ptac-IspSP, respectively.

### 7-6) Effects of introduction of isoprene synthase derived from various plants on MG1655 strain in which MVA pathway is enhanced

Microbial cells of MG1655 Ptac-KKDyI/pSTV28-Ptac-Ttrp, MG1655 Ptac-KKDyI/pSTV28-Ptac-IspSK, MG1655 Ptac-KKDyI/pSTV28-Ptac-IspSM, or further MG1655 Ptac-KKDyI/pSTV28-Ptac-IspSP were evenly applied onto the LB plate containing 60 mg/L of chloramphenicol, and the cells were cultured at 37°C for 18 hours. One loopful of the microbial cells from the resulting LB plate was inoculated to 1 mL of M9 glucose (containing mevalonic acid) medium in a headspace vial (22 mL CLEAR CRIMP TOP VIAL (Cat #B0104236) manufactured by Perkin Elmer), and subsequently the cultivation was evaluated according to the method described in Example 2. A composition of the M9 glucose (containing mevalonic acid) medium is described in Table 16. The amount of produced isoprene and the OD value upon completion of the cultivation are described in Table 17.

**Table 16. Composition of M9 glucose (containing mevalonic acid) medium**

| | |
|---|---|
| Glucose | 2.0 g/L |
| Na₂HPO₄ | 6.0 g/L |
| KH₂PO₄ | 3.0 g/L |
| NaCl | 0.5 g/L |
| NH₄Cl | 1.0 g/L |
| Mevalonic acid (manufactured by ADEKA) | 1.0 g/L |
| 1 M MgSO₄ (autoclaved) | 1.0 mL |
| 1 M CaCl₂ (autoclaved) | 0.1 mL |

Chloramphenicol was added at a final concentration of 60 mg/L.

A total volume was adjusted to 1 L, and the medium was sterilized by filtration.

**Table 17. Amount (mg/L) of produced isoprene and OD value when cultivation of MG1655 Ptac-KKDyI/pSTV28-Ptac-Ttrp, MG1655 Ptac-KKDyI/pSTV28-Ptac-IspSK, MG1655 Ptac-KKDyI/pSTV28-Ptac-IspSM, or further MG1655 Ptac-KKDyI/pSTV28-Ptac-IspSP was completed**

| Name of microbial strain | OD value | Amount (mg/L) of produced isoprene |
|---|---|---|
| MG1655 Ptac-KKDyI/pSTV28-Ptac-Ttrp | 2.08 ± 0.07 | 0.07 ± 0.01 |
| MG1655 Ptac-KKDyI/pSTV28-Ptac-IspSK | 2.48 ± 0.13 | 30.96 ± 3.04 |
| MG1655 Ptac-KKDyI/pSTV28-Ptac-IspSM | 2.48 ± 0.09 | 57.13 ± 15.00 |
| MG1655 Ptac-KKDyI/pSTV28-Ptac-IspSP | 1.95 ± 0.09 | 0.52 ± 0.01 |

From the results in Table 17, the amount of produced isoprene was larger in order of MG1655 Ptac-KKDyI/pSTV28-Ptac-IspSM, MG1655 Ptac-KKDyI/pSTV28-Ptac-IspSK, MG1655 Ptac-KKDyI/pSTV28-Ptac-IspSP, and MG1655 Ptac-KKDyI/pSTV28-Ptac-Ttrp strains. From the above results, the strain introduced with the isoprene synthase derived from Mucuna also exhibited the highest ability to produce isoprene in the strains introduced with the MVA pathway.

### Example 8: Measurement of enzymatic activity of isoprene synthase derived from various plants using crude enzyme extract derived from Corynebacterium glutamicum

### 1) Construction of plasmid for expressing IspS gene derived from various plants

*C*. *glutamicum* 2256 strain (ATCC13869) was used as a coryneform bacterium (Okumura et al., 1962, Santamaria et al., 1984, Tsuchida et al., 1986). Plasmids for expressing the IspSK gene, the IspSP gene and the IspSM gene in *C*. *glutamicum* were constructed by the following procedure. PCR was performed with Prime Star polymerase (manufactured by Takara Bio Inc.) using the synthesized oligonucleotides represented by SEQ ID NOS:67 and 68 as the primers with pUC57-IspSK as the template, the synthesized oligonucleotides represented by SEQ ID NOS:69 and 70 as the primers with pUC57-IspSP as the template, or synthesized oligonucleotides represented by SEQ ID NOS:71 and 72 as the primers with pUC57-IspSM as the template. A reaction solution was prepared according to the composition attached to the kit, and the reaction was carried out for 30 cycles of 98°C for 10 seconds, 55°C for 5 seconds and 72°C for 120 seconds. As a result, the PCR product containing the IspSK gene, the IspSP gene, or the IspSM gene was obtained. Subsequently, for the purpose of obtaining a promoter sequence of the elongation factor Tu (hereinafter P₀₄₈₀), PCR was performed using chromosomal DNA of *C*. *glutamicum* 2256 strain as the template. For the purpose of combining the IspSK gene with P₀₄₈₀, the synthesized nucleotides represented by SEQ ID NOS:73 and 74 were used as the primers for PCR. Also for the purpose of combining the IspSP gene with P₀₄₈₀, the synthesized nucleotides represented by SEQ ID NOS:73 and 75 were used as the primers for PCR. Also for the purpose of combining the IspSM gene with P₀₄₈₀, the synthesized nucleotides represented by SEQ ID NOS:73 and 76 were used as the primers for PCR. Prime Star polymerase (manufactured by Takara Bio Inc.) was used for PCR. A reaction solution was prepared according to the composition attached to the kit, and the reaction was carried out for 30 cycles of 98°C for 10 seconds, 55°C for 5 seconds and 72°C for 30 seconds. As a result, the PCR products containing P₀₄₈₀ were obtained. Information for the sequence of P₀₄₈₀ is represented by SEQ ID NO:77. A shuttle vector pVK9 for *C*. *glutamicum* and *E*. *coli* was digested with the restriction enzyme *Xba*I (manufactured by Takara Bio Inc.) (Miwa et al., 1985). Information for the sequence of pVK9 is represented by SEQ ID NO:78. The purified fragment of the IspSK gene, IspSP gene or IspSM gene, the PCR product of P₀₄₈₀ and purified pVK9 treated with X*ba*I were ligated using In-fusion HD Cloning Kit (manufactured by Clontech). The resulting plasmids for expressing the IspSK gene, the IspSP gene and IspSM gene were designated as pVK9-P₀₄₈₀-IspSK, pVK9-P₀₄₈₀-IspSP and pVK9-P₀₄₈₀-IspSM, respectively, and their sequence information were represented by SEQ ID NOS:79, 80 and 81, respectively.

### 2) Construction of C. glutamicum 2256 strain having ability to produce isoprene

Microbial cells of *C*. *glutamicum* 2256 strain were inoculated to a test tube containing 4 mL of CM-Dex medium, and cultured to a logarithmic growth phase at 30°C at 120 rpm. These microbial cells were collected, washed two to three times with ice-cold 10% glycerol, concentrated to about 100 to 200 times, and used as competent cells. The competent cells and each plasmid of pVK9, pVK9-P₀₄₈₀-IspSK, pVK9-P₀₄₈₀-IspSP or pVK9-P₀₄₈₀-IspSM were mixed well, and pulse was applied thereto. The pulse application was performed under the condition of 1.8 kV, 25 µF and 200 Ω using GenePulserXCell manufactured by BIORAD. After the pulse application, the cells were recovered by culturing in a CM-Dex medium for about 1 to 2 hours, subsequently applied onto a CM-Dex plate medium containing 50 µg/mL of Km, and cultured at 30°C for 18 to 24 hours. Transformants exhibiting the resistance to kanamycin were obtained from the plate after the cultivation. The composition of the CM-Dex medium is shown in Table 18. A strain in which pVK9, pVK9-P₀₄₈₀-IspSK, pVK9-P₀₄₈₀-IspSP or pVK9-P₀₄₈₀-IspSM had been introduced into *C*. *glutamicum* 2256 strain was designated as 2256/pVK9, 2256/pVK9-P₀₄₈₀-IspSK, 2256/pVK9-P₀₄₈₀-IspSP or 2256/pVK9-P₀₄₈₀-IspSM, respectively.

**Table 18. Composition of CM-Dex medium**

| | |
|---|---|
| Glucose | 5 g/L |
| Polypeptone | 10 g/L |
| Yeast extract | 10 g/L |
| KH₂PO₄ | 1 g/L |
| MgSO₄·7H₂O | 0.4 g/L |
| FeSO₄·H₂O | 0.01 g/L |
| MnSO₄·H₂O | 0.01 g/L |
| Urea | 3 g/L |
| Hydrolyzed soybeans | 1.2 g/L (as total nitrogen concentration) |
| pH 7.5 (NaOH) | |

The medium was sterilized at 120°C for 20 minutes. If necessary, kanamycin was added at a final concentration of 50 mg/L.
In the case of the plate, 20 g/L of agar was added.

### 3) Method of preparing crude enzyme extract

Microbial cells of 2256/pVK9, 2256/pVK9-P₀₄₈₀-IspSK, 2256/pVK9-P₀₄₈₀-IspSP or 2256/pVK9-P₀₄₈₀-IspSM were evenly applied onto the CM-Dex plate medium containing 50 mg/L of kanamycin, and cultured at 30°C for 18 hours. The microbial cells corresponding to 1/6 of the cultured plate were inoculated to a Sakaguchi flask in which 20 mL of the CM-Dex medium containing 50 mg/L of kanamycin, and cultured at 30°C for 6 hours. The microbial cells were separated from the culture medium by centrifugation at 5,000 rpm for 5 minutes, and washed twice with the isoprene synthase buffer (50 mM Tris-HCl (pH 8.0), 20 mM MgCl₂, 5% glycerol). The microbial cells after the washing were resuspended in 1.8 mL of the same buffer. Subsequently, about 0.9 mL of beads for disruption (YBG01, diameter 0.1 mm) and 0.9 mL of the microbial cell suspension were placed in a 2 mL tube specific for the multibead shocker. The microbial cells were disrupted using the multibead shocker (MB701 (S) model) manufactured by Yasui Kikai Corporation at 2500 rpm for 6 cycles of ON for 60 seconds/OFF for 60 seconds. After the disruption, the tube was centrifuged at 20,000 g for 20 minutes, and the obtained supernatant was used as a crude enzyme extract.

### 4) Measurement of isoprene synthase activity

A protein concentration of the crude enzyme extracts from 2256/pVK9, 2256/pVK9-P₀₄₈₀-IspSK, 2256/pVK9-P₀₄₈₀-IspSP and 2256/pVK9-P₀₄₈₀-IspSM strains was measured by a bicinchonic acid method (BCA method). BCA protein assay reagent kit (Thermo Scientific Japan, Cat #23227) was used as reagents for the measurement. 2 mg of the crude enzyme extract as the amount of total protein and the isoprene buffer were combined and prepared to make total 0.5 mL. This was placed in a headspace vial, subsequently 0.025 mL of a 0.5 M MgCl₂ solution and 0.01 mL of a 0.2 M DMAPP (manufactured by Cayman, Cat No. 63180) solution were added thereto, and the mixture was lightly vortexed. Then immediately, the vial was tightly sealed, and the reaction was carried out at 37°C for 2 hours. After the reaction, the concentration of isoprene in the headspace in the vial was measured by gas chromatography. 22 mL CLEAR CRIMP TOP VIAL (Cat #B0104236) manufactured by Perkin Elmer was used as the headspace vial and a cap with a butyl rubber septum for the headspace vial (Cat #B0104240) manufactured by Perkin Elmer was used as a cap for tight sealing.

The amounts of isoprene formed after the reaction for 2 hours in various microbial strains are described in Table 19.

**Table 19. Amounts of isoprene formed after the reaction for 2 hours in 2256/pVK9, 2256/pVK9-P₀₄₈₀-IspSK, 2256/pVK9-P₀₄₈₀-IspSP and 2256/pVK9-P₀₄₈₀-IspSM strains**

| Name of microbial strain | Amount (mg/L) of formed isoprene |
|---|---|
| C.glutamicum 2256/pVK9 | 0.05 ± 0.02 |
| C.glutamicum 2256/pVK9-P-₀₄₈₀-ispSP | 0.05 ± 0.02 |
| C.glutamicum 2256/pVK9-P-₀₄₈₀-ispSK | 0.68 ± 0.14 |
| C.glutamicum 2256/pVK9-P-₀₄₈₀-ispSM | 3.48 ± 0.55 |

From the results in Table 19, the amount of formed isoprene was larger in 2256/pVK9-P-₀₄₈₀-IspSM strain than in 2256/pVK9-P-₀₄₈₀-IspSK strain. The amount of formed isoprene in 2256/pVK9-P-₀₄₈₀-IspSP strain was as low as in 2256/pVK9 strain. From the above results, the crude enzyme extract derived from the strain introduced with the isoprene synthase derived from Mucuna exhibited the highest activity to form isoprene.

### Example 9: Effects of introduction of isoprene synthase derived from various plants on Corynebacterium glutamicum 2256 strain

### 1) Effects of introduction of isoprene synthase derived from various plants on C. glutamicum 2256 strain

Microbial cells of 2256/pVK9, 2256/pVK9-P₀₄₈₀-IspSK, 2256/pVK9-P-₀₄₈₀-IspSP or 2256/pVK9-P-₀₄₈₀-IspSP strain were evenly applied onto the CM-Dex plate containing 50 mg/L of kanamycin, and cultured at 30°C for 18 to 24 hours. One loopful of the microbial cells from the resulting plate were inoculated to 1 mL of MM-glucose medium in a headspace vial. Next, the headspace vial was tightly sealed, and then the cells were cultured in a reciprocal shaking apparatus at 30°C at 120 rpm for 24 hours. 22 mL CLEAR CRIMP TOP VIAL (Cat #B0104236) manufactured by Perkin Elmer was used as the headspace vial and the cap with the butyl rubber septum for the headspace vial (Cat #B0104240) manufactured by Perkin Elmer was used as the cap for tight sealing.

The composition of the MM-glucose medium is as described in Table 20.

**Table 20. Composition of MM-glucose medium**

| | |
|---|---|
| Glucose | 10 g/L |
| (NH₄)₂SO₄ | 2.5 g/L |
| Urea | 2 g/L |
| MOPS | 40 g/L |
| KH₂PO₄ | 0.5 g/L |
| MgSO₄.7H₂O | 0.25 g/L |
| CaCl₂ | 10 mg/L |
| FeSO₄ | 10 mg/L |
| MnSO₄ | 10 mg/L |
| CuSO₄ | 0.02 mg/L |
| Biotin | 50 µg/L |
| Vitamin B1 | 100 µg/L |
| Protocatechuic acid | 15 mg/L |
| pH 7.0 (KOH) | |

If necessary, kanamycin was added at a final concentration of 50 mg/L.
A total volume was adjusted to 1 mL and the medium was sterilized by filtration.

After the completion of the culture, the concentration of isoprene in the headspace in the vial was measured by gas chromatography. The OD value was measured at 660 nm using the spectrophotometer (HITACHI U-2900).

The concentrations of isoprene formed from various microbial strains upon completion of the culture are described in Table 21.

**Table 21. Amounts of isoprene produced in 2256/pVK9, 2256/pVK9-P₀₄₈₀-IspSK, 2256/pVK9-P-₀₄₈₀-IspSP and 2256/pVK9-P₀₄₈₀-IspSM strains upon completion of the culture**

| Name of microbial strain | OD value | Amount (µg/L) of formed isoprene |
|---|---|---|
| C. glutamicum 2256/pVK9 | 2.22 ± 0.06 | ND |
| C. glutamicum 2256/pVK9-P-₀₄₈₀-ispSP | 2.48 ± 0.02 | ND |
| C. glutamicum 2256/pVK9-P-₀₄₈₀-ispSP | 2.38 ± 0.09 | 21.9 ± 0.2 |
| C. glutamicum 2256/pVK9-P-₀₄₈₀-ispSP | 2.33 ± 0.13 | 24.2 ± 0.1 |

From the results in Table 21, the amount of produced isoprene was larger in order of 2256/pVK9-P-₀₄₈₀-IspSM and 2256/pVK9-P-₀₄₈₀-ispSP No isoprene was detected in 2256/pVK9 and 2256/pVK9-P-₀₄₈₀-IspSP From the above results, the strain introduced with the isoprene synthase derived from Mucuna exhibited the highest ability to produce isoprene in wild strains of *C*. *glutamicum* 2256.

### Example 10: Measurement of enzymatic activity of isoprene synthase derived from various plants using crude enzyme extract derived from P. ananatis

### 1) Construction of P. ananatis AJ13355 strain having ability to produce isoprene

Competent cells of *P*. *ananatis* AJ13355 were prepared, subsequently pSTV28-Ptac-Ttrp, pSTV28-Ptac-IspSK, pSTV28-Ptac-IspSP, or further pSTV28-Ptac-IspSM was introduced therein by the electroporation method, and the cells were evenly applied onto the LB plate containing 60 mg/L chloramphenicol and cultured at 30°C for 18 hours. Subsequently, transformants exhibiting the resistance to chloramphenicol were obtained from the resulting plates. A strain in which pSTV28-Ptac-Ttrp, pSTV28-Ptac-IspSK, pSTV28-Ptac-IspSP, or pSTV28-Ptac-IspSM had been introduced into *P*. *ananatis* AJ13355 strain was designated as AJ13355/pSTV28-Ptac-Ttrp, AJ13355/pSTV28-Ptac-IspSK, AJ13355/pSTV28-Ptac-IspSP, or AJ13355/pSTV28-Ptac-IspSM strain, respectively.

### 2) Method of preparing crude enzyme extract

The activity of the crude enzyme was measured according to the method described in Example 4. Microbial cells of AJ13355/pSTV28-Ptac-Ttrp, AJ13355/pSTV28-Ptac-IspSK, AJ13355/pSTV28-Ptac-IspSP, or AJ13355/pSTV28-Ptac-IspSM strain were evenly applied onto the LB plate containing 60 mg/L of chloramphenicol and cultured at 30°C for 18 hours. The microbial cells corresponding to 1/6 of the resulting plate were inoculated to a Sakaguchi flask in which 20 mL of LB containing 60 mg/L of chloramphenicol had been added, and cultured at 34°C for 6 hours. The microbial cells from the culture medium were centrifuged at 6000 rpm at 4°C for 5 minutes, and washed twice with ice-cold isoprene synthase buffer (50 mM Tris-HCl, pH 8.0, 20 mM MgCl₂, 5% glycerol). The washed microbial cells were suspended in 1.8 mL of the same buffer. About 0.9 mL of beads for disruption (YBG01, diameter 0.1 mm) and 0.9 mL of the microbial cell suspension were placed in a 2 mL tube specific for the multibead shocker, and the microbial cells were disrupted using the multibead shocker manufactured by Yasui Kikai Corporation at 2500 rpm at 4°C for 3 cycles of ON for 30 seconds/OFF for 30 seconds. After the disruption, the tube was centrifuged at 20,000 g at 4°C for 20 minutes, and a supernatant was used as a crude enzyme extract.

### 3) Measurement of isoprene synthase activity

A total volume of 0.5 mL of the crude enzyme extract from AJ13355/pSTV28-Ptac-Ttrp, AJ13355/pSTV28-Ptac-IspSK, AJ13355/pSTV28-Ptac-IspSP, or AJ13355/pSTV28-Ptac-IspSM strain (quantified to be 2 mg as the amount of total protein by Bradford method) and the isoprene buffer was placed in a headspace vial (22 mL CLEAR CRIMP TOP VIAL (Cat #B0104236) manufactured by Perkin Elmer), then 0.025 mL of the 0.5 M MgCl₂ solution and 0.01 mL of the 0.2 M DMAPP (manufactured by Cayman, Catalog No. 63180) solution were added thereto, and the mixture was lightly vortexed. Then immediately, the vial was tightly sealed with the cap with the butyl rubber septum for the headspace vial (CRIMPS (Cat #B0104240) manufactured by Perkin Elmer), and kept at 37°C for 2 hours.

After the completion of the reaction, the concentration of isoprene in the headspace of the vial was measured by gas chromatography.

**Table 22. Results of measuring isoprene synthase activity in P. ananatis AJ13355 strains**

| Microbial strain | Amount (mg/L) of formed isoprene |
|---|---|
| AJ13355/pSTV28-Ptac-Ttrp | 0.13 ± 0.02 |
| AJ13355/pSTV28-Ptac-IspSK | 0.58 ± 0.10 |
| AJ13355/pSTV28-Ptac-IspSM | 13.42 ± 1.67 |
| AJ13355/pSTV28-Ptac-IspSP | 0.23 + 0.04 |

From the results in Table 22, the amount of formed isoprene was larger in order of AJ13355/pSTV28-Ptac-IspSM, AJ13355/pSTV28-Ptac-IspSK, AJ13355/pSTV28-Ptac-IspSP, and AJ13355/pSTV28-Ptac-Ttrp. From the above results, the crude enzyme extract from the strain introduced with the isoprene synthase derived from Mucuna exhibited the highest activity to form isoprene.

### Example 11: Effects of introduction of isoprene synthase derived from various plants on P. ananatis AJ13355 strain

The ability to produce isoprene from glucose was compared in various *P. ananatis* AJ13355 strains in which the chloroplast localization signal had been deleted and the isoprene synthase had been introduced. Microbial cells of AJ13355/pSTV28-Ptac-Ttrp, AJ13355/pSTV28-Ptac-IspSK, AJ13355/pSTV28-Ptac-IspSP, or AJ13355/pSTV28-Ptac-IspSM strain were evenly applied onto the LB plate containing 60 mg/L of chloramphenicol and cultured at 30°C for 18 hours. One loopful of the microbial cells from the resulting plate was inoculated to a 1 mL of M9 glucose medium in a headspace vial. Next, the vial was tightly sealed with the cap with the butyl rubber septum for the headspace vial (CRIMPS (Cat #B0104240) manufactured by Perkin Elmer), and cultured in the reciprocal shaking cultivation apparatus (120 rpm) at 30°C for 24 hours. After completion of the cultivation, the concentration of isoprene in the headspace in the vial was measured by gas chromatography. The OD value was measured at 600 nm using the spectrophotometer (HITACHI U-2900). The concentration of isoprene and the OD value in various microbial strains upon completion of the cultivation are described in Table 23.

**Table 23. Amount (µg/L) of produced isoprene and OD value when the cultivation was completed in AJ13355/pSTV28-Ptac-Ttrp, AJ13355/pSTV28-Ptac-IspSK, AJ13355/pSTV28-Ptac-IspSP, and AJ13355/pSTV28-Ptac-IspSM strains**

| Name of microbial strain | OD value | Amount (µg/L) of produced isoprene |
|---|---|---|
| AJ13355/pSTV28-Ptac-Ttrp | 2.36 ± 0.11 | N.D. |
| AJ13355/pSTV28-Ptac-IspSK | 2.18 ± 0.09 | 50.0 ± 13.0 |
| AJ13355/pSTV28-Ptac-IspSM | 2.44 ± 0.11 | 63.0 ± 6.0 |
| AJ13355/pSTV28-Ptac-IspSP | 2.25 ± 0.18 | 12.0 ± 1.0 |

| | | |
|---|---|---|
| N.D. not detected | | |

From the results in Table 23, the amount of produced isoprene was larger in order of AJ13355/pSTV28-Ptac-IspSM, AJ13355/pSTV28-Ptac-IspSK, AJ13355/pSTV28-Ptac-IspSP and AJ13355/pSTV28-Ptac-Ttrp strains. From the above results, the strain introduced with the isoprene synthase derived from Mucuna also exhibited the highest ability to produce isoprene in *P. ananatis* AJ13355 strains.

### Example 12: Measurement of enzymatic activity of isoprene synthase derived from various plants using crude enzyme extract derived from E. aerogenes AJ110637

### 1) Construction of E. aerogenes AJ110637 strain having ability to produce isoprene

Competent cells of *E. aerogenes* AJ110637 were prepared, subsequently pSTV28-Ptac-Ttrp, pSTV28-Ptac-IspSK, pSTV28-Ptac-IspSP, or further pSTV28-Ptac-IspSM was introduced therein by the electroporation method, and the cells were evenly applied onto the LB plate containing 60 mg/L chloramphenicol and cultured at 37°C for 18 hours. Subsequently, transformants exhibiting the resistance to chloramphenicol were obtained from the resulting plates. A strain in which pSTV28-Ptac-Ttrp, pSTV28-Ptac-IspSK, pSTV28-Ptac-IspSP, or pSTV28-Ptac-IspSM had been introduced into *E. aerogenes* AJ110637 strain was designated as AJ110637/pSTV28-Ptac-Ttrp, AJ110637/pSTV28-Ptac-IspSK, AJ110637/pSTV28-Ptac-IspSP, or AJ110637/pSTV28-Ptac-IspSM strain, respectively.

### 2) Method of preparing crude enzyme extract

The activity of the crude enzyme was measured according to the method described in Example 4. Microbial cells of AJ110637/pSTV28-Ptac-Ttrp, AJ110637/pSTV28-Ptac-IspSK, AJ110637/pSTV28-Ptac-IspSP, or AJ110637/pSTV28-Ptac-IspSM strain were evenly applied onto the LB plate containing 60 mg/L of chloramphenicol and cultured at 37°C for 18 hours. The microbial cells corresponding to 1/6 of the resulting plate were inoculated to a Sakaguchi flask in which 20 mL of LB containing 60 mg/L of chloramphenicol had been added, and cultured at 37°C for 4 hours. The microbial cells from the culture medium were centrifuged at 8,000 rpm at 4°C for 10 minutes, and washed twice with ice-cold isoprene synthase buffer (50 mM Tris-HCl, pH 8.0, 20 mM MgCl₂, 5% glycerol). The washed microbial cells were suspended in 1.8 mL of the same buffer. About 0.9 mL of beads for disruption (YBG01, diameter 0.1 mm) and 0.9 mL of the microbial cell suspension were placed in a 2 mL tube specific for the multibead shocker, and the microbial cells were disrupted using the multibead shocker (MB701 (S) model) manufactured by Yasui Kikai Corporation at 2500 rpm at 4°C for 3 cycles of ON for 30 seconds/OFF for 30 seconds. After the disruption, the tube was centrifuged at 20,000 g at 4°C for 20 minutes, and a supernatant was used as a crude enzyme extract.

### 3) Measurement of isoprene synthase activity

A total volume of 0.5 mL of the crude enzyme extract from AJ110637/pSTV28-Ptac-Ttrp, AJ110637/pSTV28-Ptac-IspSK, AJ110637/pSTV28-Ptac-IspSP, or AJ110637/pSTV28-Ptac-IspSM strain (quantified to be 2 mg as the amount of total protein by Bradford method) and the isoprene buffer was placed in a headspace vial (22 mL CLEAR CRIMP TOP VIAL (Cat #B0104236) manufactured by Perkin Elmer), then 0.025 mL of the 0.5 M MgCl₂ solution and 0.01 mL of the 0.2 M DMAPP (manufactured by Cayman, Catalog No. 63180) solution were added thereto, and the mixture was lightly vortexed. Then immediately, the vial was tightly sealed with the cap with the butyl rubber septum for the headspace vial (CRIMPS (Cat #B0104240) manufactured by Perkin Elmer), and kept at 37°C for 2 hours.

After the completion of the reaction, the concentration of isoprene in the headspace of the vial was measured by gas chromatography.

The amount of formed isoprene in each microbial strain after reaction for 2 hours is described in Table 24.

**Table 24. Results of measuring isoprene synthase activity in E. aerogenes AJ110637 strains**

| Name of microbial strain | Amount (mg/L) of formed isoprene |
|---|---|
| AJ110637/pSTV28-Ptac-Ttrp | 0.13 ± 0.02 |
| AJ110637/pSTV28-Ptac-IspSK | 0.30 ± 0.04 |
| AJ110637/pSTV28-Ptac-IspSM | 1.46 ± 0.18 |
| AJ110637/pSTV28-Ptac-IspSP | 0.17 ± 0.03 |

From the results in Table 24, the amount of formed isoprene was larger in order of AJ110637/pSTV28-Ptac-IspSM, AJ110637/pSTV28-Ptac-IspSK, AJ110637/pSTV28-Ptac-IspSP and AJ110637/pSTV28-Ptac-Ttrp strains. From the above results, the crude enzyme extract from the strain introduced with the isoprene synthase derived from Mucuna exhibited the highest activity to form isoprene.

### Example 13: Effects of introduction of isoprene synthase derived from various plants on E. aerogenes AJ110637 strain

The ability to produce isoprene from glucose was compared in various *E. aerogenes* AJ110637 strains in which the chloroplast localization signal had been deleted and the isoprene synthase had been introduced. Microbial cells of AJ110637/pSTV28-Ptac-Ttrp, AJ110637/pSTV28-Ptac-IspSK, AJ110637/pSTV28-Ptac-IspSP, or AJ110637/pSTV28-Ptac-IspSM strain were evenly applied onto the LB plate containing 60 mg/L of chloramphenicol and cultured at 30°C for 18 hours. One loopful of the microbial cells from the resulting plate was inoculated to a 1 mL of M9 glucose medium in a headspace vial. Next, the vial was tightly sealed with the cap with the butyl rubber septum for the headspace vial (CRIMPS (Cat #B0104240) manufactured by Perkin Elmer), and cultured in the reciprocal shaking cultivation apparatus (120 rpm) at 30°C for 24 hours. After completion of the cultivation, the concentration of isoprene in the headspace in the vial was measured by gas chromatography. The OD value was measured at 600 nm using the spectrophotometer (HITACHI U-2900). The concentration of isoprene and the OD value in various microbial strains upon completion of the cultivation are described in Table 25.

**Table 25. Amount (µg/L) of produced isoprene and OD value when cultivation was completed in AJ110637/pSTV28-Ptac-Ttrp, AJ110637/pSTV28-Ptac-IspSK, AJ110637/pSTV28-Ptac-IspSP, and AJ110637/pSTV28-Ptac-IspSM strains**

| Name of microbial strain | OD value | Amount (µg/L) of produced isoprene |
|---|---|---|
| AJ110637/pSTV28-Ptac-Ttrp | 3.12 ± 0.21 | N.D. |
| AJ110637/pSTV28-Ptac-IspSK | 3.08 ± 0.14 | 130.2 ± 14.3 |
| AJ110637/pSTV28-Ptac-IspSM | 3.20 ± 0.18 | 316.1 ± 26.2 |
| AJ110637/pSTV28-Ptac-IspSP | 3.09 ± 0.12 | 12.5 ± 0.8 |

| | | |
|---|---|---|
| N.D. not detected | | |

From the results in Table 25, the amount of produced isoprene was larger in order of AJ110637/pSTV28-Ptac-IspSM, AJ110637/pSTV28-Ptac-IspSK, AJ110637/pSTV28-Ptac-IspSP and AJ110637/pSTV28-Ptac-Ttrp. From the above results, the strain introduced with the isoprene synthase derived from Mucuna also exhibited the highest ability to produce isoprene in *E. aerogenes* AJ110637 strains.

### Example 14: Measurement of enzymatic activity of isoprene synthase derived from various plants using crude enzyme extract derived from Saccharomyces cerevisiae

### 1) Construction of plasmid for expressing IspS gene derived from various plants

Plasmids for expressing the IspSK gene, the IspSP gene and the IspSM gene in *S. cerevisiae* were constructed by the following procedure. PCR was performed with Prime Star polymerase (manufactured by Takara Bio Inc.) using the synthesized oligonucleotides represented by SEQ ID NOS:82 and 83 as the primers with pUC57-IspSK as the template, the synthesized oligonucleotides represented by SEQ ID NOS:84 and 85 as the primers with pUC57-IspSP as the template, or the synthesized oligonucleotides represented by SEQ ID NOS:86 and 87 as the primers with pUC57-IspSM as the template. A reaction solution was prepared according to the composition attached to the kit, and the reaction was carried out for 30 cycles of 98°C for 10 seconds, 55°C for 5 seconds and 72°C for 120 seconds. As a result, PCR products containing the IspSK gene, the IspSP gene and the IspSM gene were obtained. Meanwhile, the shuttle vector pYES2 for *S. cerevisiae* and *E. coli* (Invitrogen, Cat No. V825-20) was digested with the restriction enzyme *Kpn*I (manufactured by Takara Bio Inc.). Subsequently, the purified PCR fragment containing the IspSK gene was ligated to purified pYES2 treated with *Kpn*I using In-fusion HD Cloning Kit (manufactured by Clontech). The resulting plasmid for expressing the IspSK gene was designated as pYES2-IspSK. Likewise, the purified PCR fragment containing the IspSP gene was ligated to purified pYES2 treated with *Kpn*I using In-fusion HD Cloning Kit (manufactured by Clontech). The resulting plasmid for expressing the IspSP gene was designated as pYES2-IspSP. Likewise, the purified PCR fragment containing the IspSM gene was ligated to purified pYES2 treated with *Kpn*I using In-fusion HD Cloning Kit (manufactured by Clontech). The resulting plasmid for expressing the IspSM gene was designated as pYES2-IspSM.

### 2) Construction of S. cerevisiae S288C strain having ability to produce isoprene

Competent cells of *S. cerevisiae* S288C were prepared using Frozen-EZ Yeast Transformation Kit II (ZYMO RESEARCH Cat. No. T2001). pYES2, pYES2-IspSK, pYES2-IspSP, or pYES2-IspSM was introduced into the competent cells of *S. cerevisiae* S288C strain according to the protocol of the same kit. The cells were evenly applied onto an SD-Ura plate, and cultured at 30°C for 2 days. Subsequently, transformants that had lost Ura requirement were obtained from the resulting plates. The composition of the SD-Ura medium is as described in Table 26. A strain in which pYES2, pYES2-IspSK, pYES2-IspSP, or pYES2-IspSM had been introduced into *S. cerevisiae* S288C strain was designated as S288C/pYES2, S288C/pYES2-IspSK, S288C/pYES2-IspSP, or S288C/pYES2-IspSM strain, respectively.

**Table 26. SD-Ura medium**

| | |
|---|---|
| Yeast Nitrogen Base w/o AA (Difco, Cat No. 291940) | 6.7 g/L |
| -Ura DO supplement (Clontech, Cat No. 630416) | 0.77 g/L |
| Glucose | 20 g/L (sterilized separately) |
| Agar | 20 g/L (only whenrequired) |
| pH 5.6 to 6.0 (KOH) | |
| Sterilized at 120°C for 15 minutes | |

### 3) Method of preparing crude enzyme extract

Microbial cells of S288C/pYES2, S288C/pYES2-IspSK, S288C/pYES2-IspSP, or S288C/pYES2-IspSM strain were evenly applied to the SD-Ura plate and cultured at 30°C for 24 hours. The microbial cells corresponding to 1/4 of the resulting plate were inoculated to a Sakaguchi flask in which 50 mL of SD-Ura galactose (SD-Ura medium in which glucose was replaced with galactose), and cultured at 30°C for 16 hours. The composition of the SD-Ura galactose medium is shown in Table 27. The microbial cells were separated from the culture medium by centrifugation at 3,000 rpm for 5 minutes, and washed twice with the isoprene synthase buffer (50 mM Tris-HCl, pH 8.0, 20 mM MgCl₂, 5% glycerol). The washed microbial cells were suspended in 1.8 mL of the same buffer. About 0.9 mL of beads for disruption (YBG05, diameter 0.5 mm) and 0.9 mL of the microbial cell suspension were placed in a 2 mL tube specific for the multibead shocker, and the microbial cells were disrupted using the multibead shocker (MB701 (S) model) manufactured by Yasui Kikai Corporation at 2500 rpm for 12 cycles of ON for 60 seconds/OFF for 60 seconds. After the disruption, the tube was centrifuged at 20,000 g for 20 minutes, and a supernatant was used as a crude enzyme extract.

**Table 27. SD-Ura Galactose medium**

| | |
|---|---|
| Yeast Nitrogen Base w/o AA (Difco, Cat No. 291940) | 6.7 g/L |
| -Ura DO supplement (Clontech, Cat No. 630416) | 0.77 g/L |
| Galactose | 20 g/L (sterilized separately) |
| Agar | 20 g/L (only when required) |
| pH | 5.6 to 6.0 (KOH) |
| Sterilized | at 120°C for15 minutes |

### 4) Measurement of isoprene synthase activity

A protein concentration of the crude enzyme extracts from S288C/pYES2, S288C/pYES2-IspSK, S288C/pYES2-IspSP and S288C/pYES2-IspSM strains was measured by the bicinchonic acid method (BCA method). BCA protein assay reagent kit (Thermo Scientific Japan, Cat #23227) was used as reagents for the measurement. 0.4 mg of the crude enzyme extract as the amount of total protein and the isoprene buffer were combined and prepared to make total 0.25 mL. This was placed in a headspace vial, subsequently 0.0125 mL of the 0.5 M MgCl₂ solution and 0.005 mL of the 0.2 M DMAPP (manufactured by Cayman, Cat No. 63180) solution were added thereto, and the mixture was lightly vortexed. Then immediately, the vial was tightly sealed with the cap with the butyl rubber septum for the headspace vial (Cat #B0104240) manufactured by Perkin Elmer, and the reaction was carried out at 37°C for 2 hours. After the reaction, the concentration of isoprene in the headspace in the vial was measured by gas chromatography. 22 mL CLEAR CRIMP TOP VIAL (Cat #B0104236) manufactured by Perkin Elmer was used as the headspace vial and the cap with the butyl rubber septum for the headspace vial (Cat #B0104240) manufactured by Perkin Elmer was used as the cap for tight sealing.

The amounts of isoprene formed in various microbial strains after the reaction for 2 hours are described in Table 28.

**Table 28. Amounts of isoprene formed in S288C/pYES2, S288C/pYES2-IspSK, S288C/pYES2-IspSP and S288C/pYES2-IspSM strains after reaction for 2 hours**

| Name of microbial strain | Amount (µg/L) of formed isoprene |
|---|---|
| S.cerevisiae S288C/pYES2 | 70.33 ± 5.64 |
| S.cerevisiae S288C/pYES2-IspSP | 66.74 ± 0.09 |
| S.cerevisiae S288C/pYES2-IspSK | 86.02 ± 3.05 |
| S.cerevisiae S288C/pYES2-IspSM | 119.72 ± 6.57 |

From the results in Table 28, the amount of formed isoprene was larger in S288C/pYES2-IspSM strain than in S288C/pYES2-IspSK strain, and lower in S288C/pYES2-IspSP strain and S288C/pYES2 strain. From the above results, the crude enzyme extract from the strain introduced with the isoprene synthase derived from Mucuna exhibited the highest activity to form isoprene.

### Example 15: Effects of introduction of isoprene synthase derived from various plants on Saccharomyces cerevisiae S288C strain

### 1) Effects of introduction of isoprene synthase derived from various plants on S. cerevisiae S288C strain

Microbial cells of S288C/pYES2, S288C/pYES2-IspSK, S288C/pYES2-IspSP or S288C/pYES2-IspSM were evenly applied onto a YDP plate, and cultured at 30°C for 18 to 24 hours. One loopful of the microbial cells from the resulting plate were inoculated to 1 mL of SD-Ura2 medium (containing each 1 g/L of glucose and galactose) in a headspace vial (22 mL CLEAR CRIMP TOP VIAL (Cat #B0104236) manufactured by Perkin Elmer), the vial was tightly sealed with a cap with butyl rubber septum for the head space vial (CRIMPS (Cat #B0104240) manufactured by Perkin Elmer), and the microbial cells were cultured at 30°C at 120 rpm for 24 hours using the reciprocal shaking cultivation apparatus. The compositions of the YPD medium and SD-Ura2 medium are described in Tables 29 and 30, respectively.

**Table 29. Composition of YPD medium**

| | |
|---|---|
| Yeast extract | 10 g/L |
| Peptone | 10 g/L |
| Glucose | 20 g/L (sterilized separately) |
| Agar | 20 g/L (only when required) |
| pH 5.6 to 6.0 (KOH) | |
| Sterilized at 120°C for 15 minutes | |

**Table 30. SD-Ura2 medium**

| | |
|---|---|
| Yeast nitrogen base w/o AA (Difco, CatNo. 291940 | 6.7 g/L |
| -Ura DO supplement (Clontech, Cat No. 630416) | 0.77 g/L |
| Glucose | 1 g/L (sterilized separately) |
| Galactose | 1 g/L (sterilized separately) |
| Agar | 20 g/L (only when required) |
| pH 5.6 to 6.0 (KOH) | |
| Sterilized at 120°C for 15 minutes | |

After completion of the cultivation, the concentration of isoprene in the headspace in the vial was measured by gas chromatography.

The OD value was measured at 600 nm using the spectrophotometer (HITACHI U-2900). The concentration in each microbial strain upon completion of the cultivation is described in Table 31.

**Table 31. Amounts (µg/L) of produced isoprene when cultivation was completed in S288C/pYES2, S288C/pYES2-IspSK, S288C/pYES2-IspSP and S288C/pYES2-IspSM strains**

| Strains | OD600 | Isoprene (µg/L) |
|---|---|---|
| S.cerevisiae S288C/pYES2_2 | 2.76 ± 0.48 | ND |
| S.cerevisiae S288C/pYES2-ispSK-72 | 2.59 ± 0.09 | 13.05 ± 0.09 |
| S.cerevisiae S288C/pYES2-ispSP-101 | 2.48 ± 0.20 | ND |
| S.cerevisiae S288C/pYES2-ispSM-201 | 2.76 ± 0.18 | 16.15 ± 0.09 |

From the results in Table 31, the amount of produced isoprene was larger in S288C/pYES2-IspSM than S288C/pYES2-IspSK. No isoprene was detected in S288C/pYES2 and S288C/pYES2-IspSP. From the above results, the strain introduced with the isoprene synthase derived from Mucuna exhibited the highest ability to produce isoprene in the wild strains.

### Example 16: Comparison of stability of isoprene synthase

### 1) Construction of plasmid for expression

A plasmid for abundantly expressing the isoprene synthase derived from Mucuna was constructed by the following procedure. A vector was made by PCR using the synthesized oligonucleotides represented by SEQ ID NO:88 and 89 with pCold-TF (manufactured by TaKaRa, #3365, its sequence information is obtained from GenBank/EMBL/DDBJ accession ID AB213654) as the template. An insert was made by PCR using the synthesized oligonucleotides represented by SEQ ID NOS:90 and 91 with pUC57-IspSM as the template. PrimeStar HS (manufactured by Takara Bio Inc.) was used as the polymerase for PCR. The reaction solution was prepared according to the composition attached to the kit. The reaction was carried out by repeating a cycle of 95°C for 10 seconds, 55°C for 5 seconds and 72°C for 6 minutes 30 times. The resulting these DNA fragments were ligated using In-Fusion HD Cloning Kit (manufactured by Clontech). The constructed plasmid was designated as pCold-TF-IspSM. The nucleotide sequence of pCold-TF-IspSM is represented by SEQ ID NO:92.

A plasmid for abundantly expressing the isoprene synthase derived from Poplar was constructed by the following procedure. A vector was made by PCR using the synthesized oligonucleotides represented by SEQ ID NO:88 and 89 with pCold-TF as the template. An insert was made by PCR using the synthesized oligonucleotides represented by SEQ ID NOS:93 and 94 with pUC57-ispSP as the template. PrimeStar HS (manufactured by Takara Bio Inc.) was used as the polymerase for PCR. The reaction solution was prepared according to the composition attached to the kit. The reaction was carried out by repeating a cycle of 95°C for 10 seconds, 55°C for 5 seconds and 72°C for 6 minutes 30 times. The resulting these DNA fragments were ligated using In-Fusion HD Cloning Kit (manufactured by Clontech). The constructed plasmid was designated as pCold-TF-IspSP. The nucleotide sequence of pCold-TF-IspSP is represented by SEQ ID NO:95.

A plasmid for abundantly expressing the isoprene synthase derived from Kudzu was constructed by the following procedure. A vector was made by PCR using the synthesized oligonucleotides represented by SEQ ID NO:88 and 89 with pCold-TF as the template. An insert was made by PCR using the synthesized oligonucleotides represented by SEQ ID NOS:96 and 97 with pUC57-ispSK as the template. PrimeStar HS (manufactured by Takara Bio Inc.) was used as the polymerase for PCR. The reaction solution was prepared according to the composition attached to the kit. The reaction was carried out by repeating a cycle of 95°C for 10 seconds, 55°C for 5 seconds and 72°C for 6 minutes 30 times. The resulting these DNA fragments were ligated using In-Fusion HD Cloning Kit (manufactured by Clontech). The constructed plasmid was designated as pCold-TF-IspSK. The nucleotide sequence of pCold-TF-IspSK is represented by SEQ ID NO:98.

In the construction of these expression vectors, the isoprene synthase was designed to be expressed as a protein fused with a trigger factor (TF) at its N terminus. The fusion protein of the isoprene synthase and TF was designated as TF-IspS. The fusion proteins derived from Mucuna, Poplar and Kudzu were designated as TF-IspSM, TF-IspSP and TF-IspSK, respectively.

Competent cells of *E. coli* BL21 (DE3) (one shot BL21 (DE3) manufactured by Life Technologies) were transformed with pCold-TF-IspSM, pCold-TF-IspSP or pCold-TF-IspSK by a heat shock method. After heat shock at 42°C for 30 seconds, the cells were recovered by culturing in SOC medium at 37°C at 120 rpm for one hour. Subsequently, all of the cells were seeded on the LB plate containing 100 mg/L of ampicillin and cultured statically at 37°C for 14 hours.

### 2) Cultivation for preparation of isoprene synthase in large amount

A formed colony was picked up, inoculated to 5 mL of the LB medium containing 100 mg/L of ampicillin, and cultured at 37°C at 200 rpm until OD600 value reached 1.0. After confirming that the OD value reached 1.0, the total cells were inoculated to a 500 mL Sakaguchi flask in which 100 mL of LB medium containing 100 mg/L of ampicillin, and cultured at 37°C at 200 rpm until OD600 value reached 1.0. Subsequently, 1 M isopropyl-β-thiogalactopyranoside (IPTG) was added at a final concentration of 1 mM, and the cells were cultured at 15°C at 100 rpm overnight. After completion of the cultivation, the microbial cells were collected by centrifugation at 8,000×g for 10 minutes, and stored at -20°C until the purification was performed.

### 3) Purification of isoprene synthase

The isoprene synthase was purified using a His-tag column. The microbial cells obtained from 100 mL of the broth after completion of the cultivation were suspended in 240 mL of a disruption buffer composed of 50 mM phosphate buffer (pH 8.0) and 500 mM NaCl, and disrupted using a sonication machine (Sonifier 250 manufactured by Baransan) under a condition of duty cycle 50% and output control 6 on ice for 8 minutes. After the sonication, a disruption supernatant was obtained by centrifugation at 14,000×g for 20 minutes. The following purification steps were all carried out at 4°C. His-select nickel affinity gel (manufactured by Sigma) corresponding to 2 mL of a bed volume was applied to a polyprep column (manufactured by BioRad) and filled by a gravity method. Subsequently, 10 mL of the disruption buffer was applied to the column, and equilibrated by the gravity method. A total volume of the disruption supernatant after the sonication was applied to this column and TF-IspS was adsorbed to the column.

It was confirmed that the total volume of the applied disruption supernatant passed through the column, and then the column was washed with 10 mL of the disruption buffer. The column was further additionally washed with 10 mL of buffer for measuring the activity, composed of 50 mM Tris-HCl (pH 8.0) and 15 mM MgCl₂. Subsequently, 4 mL of a washing solution composed of 50 mM Tris-HCl (pH 8.0), 15 mM MgCl₂ and 10 mM imidazole was applied to the column, and a passed solution was discarded. Finally, 4 mL of an eluant composed of 50 mM Tris-HCl (pH 8.0), 15 mM MgCl₂ and 200 mM imidazole was applied to the column to elute TF-IspS. TF-IspS after the elution was concentrated by a gel filtration column (Amicon Ultra MWCO100k manufactured by Millipore).

Subsequently, the fused portion of the TF-IspS fusion protein was cleaved. Factor Xa (manufactured by Novagen) was used for the cleavage. A reaction buffer was composed of 50 mM Tris-HCl (pH 8.0), 100 mM NaCl and 5 mM CaCl₂, 26 U of Factor Xa was added, and the reaction was carried out at 4°C for 14 hours. TF was removed from the reaction solution by utilizing the fact that TF alone is adsorbed to the His-select nickel affinity gel by passing the reaction solution after the reaction through the His-select nickel affinity gel. Subsequently, IspS was concentrated by a gel filtration column (Amicon Ultra MWCO50k manufactured by Millipore). The resulting concentrated solution was developed on NuPAGE 4 to 12% (manufactured by Life Technologies) to test a purity. As a result of staining the developed gel with CBB, no band derived from impurities other than IspS was observed. Thus, the purity was estimated to be about 99%.

The IspSM solution and the IspSK solution thus obtained were stored in the buffer for measuring the activity, to which glycerol was added at a final concentration of 5%, in a freezer at -80°C until being subjected to the measurement of the activity. The IspSP solution was stored in the same buffer to which PEG600 was added at a final concentration of 5% in the freezer at - 80°C until being subjected to the measurement of the activity.

### 4) Measurement of isoprene synthase activity

IspSM, IspSK and IspSP were thawed on ice, and glycerol and PEG600 were removed by buffer exchange. Protein concentrations were unified by colorimetric quantification on SDS-PAGE. 50 µL of an isoprene reaction solution was composed of 50 mM Tris-HCl, pH 8.0, 15 mM MgCl₂, 4 mM DMAPP and 1 µg of IspS. This 50 µL of the isoprene reaction solution was placed in a 0.2 mL PCR tube (manufactured by Nippon Genetics Co., Ltd.), and a hole was made in its cap. Subsequently, this tube was placed in a 22 mL vial (manufactured by Perkin Elmer) in which 450 µL of purified water had been added, and then immediately the vial was tightly sealed with the cap with the butyl rubber septum for the headspace vial (manufactured by Perkin Elmer). The reaction for forming isoprene was carried out at 40°C for 238 hours. Formed isoprene was quantified according to the method described in 4-2) and 4-3). The results are shown in Table 32. As is shown in the results, it was demonstrated that the isoprene synthase derived from Mucuna had about 10 times higher ability to produce isoprene than the isoprene synthase derived from Kudzu.

**Table 32. Comparison of accumulated isoprene amounts by IspS (238 hours, reaction at 40°C)**

| Type of IspS | isp (µmol/L/mg IspS) | SEM (n=2) | Cone.* | Purity** |
|---|---|---|---|---|
| IspSM | 254.7 | 4.2 | 20 mg/L | >99% |
| IspSK | 25.7 | 4.0 | 20 mg/L | >99% |

| | | | | |
|---|---|---|---|---|
| *Concentration of IspS **Purity of IspS | | | | |

### 5) Comparison of stability of isoprene synthase

Each IspS purified by the above method was stored in a solution of 50 mM Tris-HCl (pH 8.0) and 15 mM MgCl₂ at 4°C for 48 hours. The amount of isoprene produced by the enzymatic reaction of each isoprene synthase before and after the storage was analyzed by gas chromatography according to the aforementioned method. The residual activities were compared by dividing the amount of isoprene produced after the storage by the amount of isoprene produced before the storage. As a result, it was demonstrated that the isoprene synthase derived from Mucuna had the more excellent stability than the isoprene synthase derived from Kudzu and the isoprene synthase derived from Poplar.

**Table 33. Comparison of residual activity of each IspS (after stored at 4°C for 48 hours)**

| Type of IspS | Residual activity |
|---|---|
| IspSM | 66% |
| IspSK | 9% |
| IspSP | 23% |

## Claims

1. A polynucleotide of the following (a) or (b):
(a) a polynucleotide comprising (i) the nucleotide sequence represented by SEQ ID NO:1, or (ii) the nucleotide sequence consisting of the nucleotide residues at positions 133 to 1785 in the nucleotide sequence represented by SEQ ID NO:1; or
(b) a polynucleotide that comprises a nucleotide sequence having 90% or more identity to the nucleotide sequence of (i) or (ii), and encodes a protein having an isoprene synthase activity.

2. The polynucleotide according to claim 1, wherein the polynucleotide is derived from Mucuna.

3. A protein of the following (A), (B), or (C):
(A) a protein comprising (i') the full length amino acid sequence represented by SEQ ID NO:2, or (ii') the amino acid sequence consisting of the amino acid residues at positions 45 to 594 in the amino acid sequence represented by SEQ OD NO:2;
(B) a protein that comprises an amino acid sequence having 90% or more identity to the amino acid sequence of (i') or (ii'), and has an isoprene synthase activity; or
(C) a protein that comprises an amino acid sequence having a deletion, substitution, addition or insertion of one to thirty amino acids in the amino acid sequence of (i') or (ii'), and has an isoprene synthase activity.

4. An expression vector comprising the polynucleotide according to claim 1 or 2, or a polynucleotide encoding the protein according to claim 3.

5. A transformant prepared by introducing the expression vector according to claim 4 into a host.

6. The transformant according to claim 5, wherein the host has an ability to synthesize dimethylallyl diphosphate via a methylerythritol phosphate pathway.

7. The transformant according to claim 6, wherein the host is Escherichia coli.

8. The transformant according to any one of claims 5 to 7, wherein the transformant has an ability to synthesize dimethylallyl diphosphate via both a mevalonate pathway and a methylerythritol phosphate pathway.

9. The transformant according to claim 5, wherein the host is a microorganism belonging to genus Corynebacterium, genus Pantoea, genus Enterobacter, or genus Saccharomyces.

10. A method of producing a protein, comprising forming the protein according to claim 3 using the transformant according to any one of claims 5 to 9.

11. A method of producing an isoprene monomer, comprising forming the isoprene monomer from dimethylallyl diphosphate in the presence of the protein according to claim 3.

12. The method according to claim 11, wherein the isoprene monomer is formed by culturing the transformant according to any one of claims 5 to 9.

13. The method according to claim 12, wherein the dimethylallyl diphosphate is supplied from a carbon source in a medium by culturing the transformant.

14. A method of producing an isoprene polymer, comprising (I) and (II):
(I) forming an isoprene monomer by the method according to any one of claims 11 to 13; and
(II) polymerizing the isoprene monomer to form the isoprene polymer.

## Patentansprüche

1. Polynucleotid nach den folgenden Punkten (a) oder (b):
(a) Polynucleotid, umfassend (i) die durch SEQ ID NO: 1 dargestellte Nucleotidsequenz oder (ii) die aus den Nucleotidresten an den Positionen 133 bis 1785 in der durch SEQ ID NO: 1 dargestellten Nucleotidsequenz bestehende Nucleotidsequenz, oder
(b) Polynucleotid, das eine Nucleotidsequenz umfasst, die 90 % oder mehr Identität mit der Nucleotidsequenz von (i) oder (ii) aufweist und ein Protein mit einer Isoprensynthase-Aktivität aufweist.

2. Polynucleotid gemäß Anspruch 1, wobei das Polynucleotid von Mucuna abgeleitet ist.

3. Protein nach den folgenden Punkten (A), (B) oder (C):
(A) Protein, umfassend (i') die durch SEQ ID NO: 2 dargestellte Aminosäuresequenz voller Länge oder (ii') die aus den Aminosäureresten an den Positionen 45 bis 594 in der durch SEQ ID NO: 2 dargestellten Aminosäuresequenz bestehende Aminosäuresequenz, oder
(B) Protein, das eine Aminosäuresequenz umfasst, die 90 % oder mehr Identität mit der Aminosäuresequenz von (i') oder (ii') aufweist und eine Isoprensynthase-Aktivität aufweist, oder
(C) Protein, das eine Aminosäuresequenz umfasst, die eine Deletion, Substitution, Addition oder Insertion von einer bis dreißig Aminosäuren in der Aminosäuresequenz von (i') oder (ii') aufweist und eine Isoprensynthase-Aktivität aufweist.

4. Expressionsvektor, umfassend das Polynucleotid gemäß Anspruch 1 oder 2 oder Polynucleotid, welches das Protein gemäß Anspruch 3 kodiert.

5. Transformant, hergestellt durch Einführen des Expressionsvektors gemäß Anspruch 4 in einen Wirt.

6. Transformant gemäß Anspruch 5, wobei der Wirt eine Fähigkeit aufweist, Dimethylallyldiphosphat über einen Methylerythritolphosphat-Weg zu synthetisieren.

7. Transformant gemäß Anspruch 6, wobei es sich bei dem Wirt um Escherichia coli handelt.

8. Transformant gemäß einem der Ansprüche 5 bis 7, wobei der Transformant eine Fähigkeit aufweist, Dimethylallyldiphosphat über sowohl einen Mevalonat-Weg als auch einen Methylerythritolphosphat-Weg zu synthetisieren.

9. Transformant gemäß Anspruch 5, wobei es sich bei dem Wirt um einen zur Gattung Corynebacterium, Gattung Pantoea, Gattung Enterobacter oder Gattung Saccharomyces gehörenden Mikroorganismus handelt.

10. Verfahren zum Herstellen eines Proteins, umfassend Bilden des Proteins gemäß Anspruch 3 unter Verwendung des Transformanten gemäß einem der Ansprüche 5 bis 9.

11. Verfahren zum Herstellen eines Isoprenmonomers, umfassend Bilden des Isoprenmonomers aus Dimethylallyldiphosphat in Anwesenheit des Proteins gemäß Anspruch 3.

12. Verfahren gemäß Anspruch 11, wobei das Isoprenmonomer durch Kultivieren des Transformanten gemäß einem der Ansprüche 5 bis 9 gebildet wird.

13. Verfahren gemäß Anspruch 12, wobei das Dimethylallyldiphosphat aus einer Kohlenstoffquelle in einem Medium durch Kultivieren des Transformanten zur Verfügung gestellt wird.

14. Verfahren zum Herstellen eines Isoprenpolymers, umfassend (I) und (II):
(I) Bilden eines Isoprenmonomers durch das Verfahren gemäß einem der Ansprüche 11 bis 13 und
(II) Polymerisieren des Isoprenmonomers, um das Isoprenpolymer zu bilden.

## Revendications

1. Polynucléotide selon (a) ou (b) ci-après :
(a) polynucléotide comprenant (i) la séquence de nucléotides représentée par la SEQ ID NO : 1, ou (ii) la séquence de nucléotides constituée des résidus de nucléotide au niveau des positions 133 à 1785 dans la séquence de nucléotides représentée par la SEQ ID NO : 1 ; ou
(b) polynucléotide qui comprend une séquence de nucléotides ayant une identité de 90 % ou plus vis-à-vis de la séquence de nucléotides selon (i) ou (ii), et code pour une protéine ayant une activité d'isoprène synthase.

2. Polynucléotide selon la revendication 1, le polynucléotide étant dérivé de *Mucuna.*

3. Protéine selon (A), (B) ou (C) ci-après :
(A) protéine comprenant (i') la totalité de la séquence d'acides aminés de longueur complète représentée par la SEQ ID NO : 2, ou (ii') la séquence d'acides aminés constituée des résidus d'acides aminés au niveau des positions 45 à 594 dans la séquence d'acides aminés représentée par la SEQ ID NO : 2 ;
(B) protéine qui comprend une séquence d'acides aminés ayant une identité de 90 % ou plus vis-à-vis de la séquence d'acides aminés de (i') ou (ii'), et présente une activité d'isoprène synthase ; ou
(C) protéine qui comprend une séquence d'acides aminés ayant une délétion, une substitution, une addition ou une insertion de un à trente acides aminés dans la séquence d'acides aminés de (i') ou (ii'), et présente une activité d'isoprène synthase.

4. Vecteur d'expression comprenant le polynucléotide selon la revendication 1 ou 2, ou un polynucléotide codant pour la protéine selon la revendication 3.

5. Transformant préparé par introduction du vecteur d'expression selon la revendication 4 dans un hôte.

6. Transformant selon la revendication 5, l'hôte ayant une aptitude à synthétiser le diméthylallyle diphosphate par une voie au méthylérythritol phosphate.

7. Transformant selon la revendication 6, l'hôte étant *Escherichia coli.*

8. Transformant selon l'une quelconque des revendications 5 à 7, le transformant ayant une aptitude à synthétiser le diméthylallyle diphosphate à la fois par une voie au mévalonate et une voie au méthylérythritol phosphate.

9. Transformant selon la revendication 5, l'hôte étant un microorganisme appartenant au genre *Corynebacterium,* au genre *Pantoea,* au genre *Enterobacter* ou au genre *Saccharomyces.*

10. Procédé de production d'une protéine, comprenant la formation de la protéine selon la revendication 3 en utilisant le transformant selon l'une quelconque des revendications 5 à 9.

11. Procédé de production d'un monomère isoprène, comprenant la formation du monomère d'isoprène à partir de diphosphate de diméthylallyle en présence de la protéine selon la revendication 3.

12. Procédé selon la revendication 11, dans lequel le monomère isoprène est formé par culture du transformant selon l'une quelconque des revendications 5 à 9.

13. Procédé selon la revendication 12, dans lequel le diphosphate de diméthylallyle est fourni par une source de carbone dans un milieu par culture du transformant.

14. Procédé de production d'un polymère d'isoprène, comprenant (I) et (II) :
(I) formation d'un monomère isoprène par le procédé selon l'une quelconque des revendications 11 à 13 ; et
(II) polymérisation du monomère isoprène pour former le polymère d'isoprène.
